# EUROPEAN PATENT SPECIFICATION

(11) **EP 2 608 671 B1**
(45) Date of publication and mention of the grant of the patent: **12.12.2018**
(21) Application number: 11820603.6
(22) Date of filing: 24.08.2011
(51) Int. Cl.: A61K 31/415, C07D 233/60, C07D 403/04, C07D 417/12, C07D 417/14, C07D 263/32, C07D 277/24, C07D 277/42, A61P 35/00, C07D 403/12, C07D 417/04

(54) **COMPOUNDS FOR TREATMENT OF CANCER**
VERBINDUNGEN ZUR KREBSBEHANDLUNG
COMPOSÉS UTILISÉS DANS LE TRAITEMENT DU CANCER

(30) Priority: 24.08.2010 US 376675 P; 29.12.2010 WO PCT/US2010/062418
(43) Date of publication of application: 03.07.2013
(73) Proprietor: Gtx, Inc., Memphis, TN 38103 (US); University of Tennessee Research Foundation, Knoxville, TN 37996-4122 (US)
(72) Inventor: DALTON, James, T., Lakeland, TN 38002 (US); MILLER, Duane, D., Germantown, TN 38139-6437 (US); LI, Chien-Ming, Memphis, TN 38103 (US); AHN, Sunjoo, Memphis, TN 38103 (US); LU, Yan, Bartlett, TN 38135 (US); WANG, Zhao, Downingtown, PA 19335 (US); CHEN, Jianjun, Memphis, TN 38104 (US); LI, Wei, Germantown, TN 38139 (US); DUKE, Charles, Memphis, TN 38117 (US)
(74) Representative: Pearl Cohen Zedek Latzer Baratz UK LLP
(86) International application number: PCT/US2011/048980
(87) International publication number: WO 2012/027481

(56) References cited:
- WO-A1-03/016338
- WO-A1-2011/109059
- US-A1- 2009 326 020
- US-B2- 6 706 717

## Description

### FIELD OF THE INVENTION

The present invention relates to novel compounds having anti-cancer activity, methods of making these compounds, and their use for treating cancer, treating drug-resistant tumors, drug-resistant cancer, metastatic cancer, metastatic melanoma, drug resistant melanoma, prostate cancer and drug resistant prostate cancer.

### BACKGROUND OF THE INVENTION

Cancer is the second most common cause of death in the United States, exceeded only by heart disease. In the United States, cancer accounts for 1 of every 4 deaths. The 5-year relative survival rate for all cancer patients diagnosed in 1996-2003 is 66%, up from 50% in 1975-1977 (Cancer Facts & Figures American Cancer Society: Atlanta, GA (2008)). This improvement in survival reflects progress in diagnosing at an earlier stage and improvements in treatment. Discovering highly effective anticancer agents with low toxicity is a primary goal of cancer research.

Microtubules are cytoskeletal filaments consisting of αβ-tubulin heterodimers and are involved in a wide range of cellular functions, including shape maintenance, vesicle transport, cell motility, and division. Tubulin is the major structural component of the microtubules and a well verified target for a variety of highly successful anti-cancer drugs. Compounds that are able to interfere with microtubule-tubulin equilibrium in cells are effective in the treatment of cancers. Anticancer drugs like taxol and vinblastine that are able to interfere with microtubule-tubulin equilibrium in cells are extensively used in cancer chemotherapy. There are three major classes of antimitotic agents. Microtubule-stabilizing agents, which bind to fully formed microtubules and prevent the depolymerization of tubulin subunits, are represented by taxanes and epothilones. The other two classes of agents are microtubule-destabilizing agents, which bind to tubulin dimers and inhibit their polymerization into microtubules. Vina alkaloids such as vinblastine bind to the vinca site and represent one of these classes. Colchicine and colchicine-site binders interact at a distinct site on tubulin and define the third class of antimitotic agents.

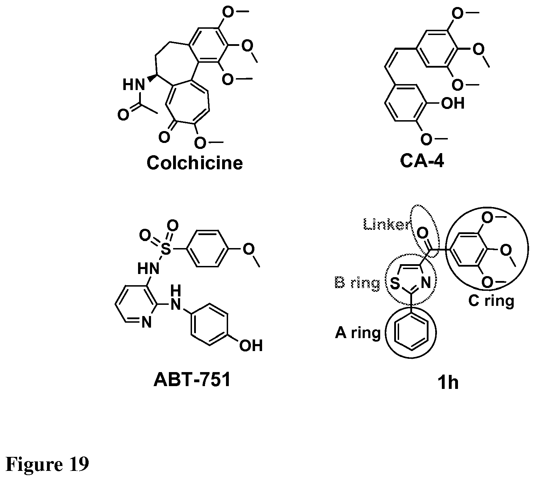

Both the taxanes and vinca alkaloids are widely used to treat human cancers, while no colchicine-site binders are currently approved for cancer chemotherapy yet. However, colchicine binding agents like combretastatin A-4 (CA-4) and ABT-751 (**Figure 19**), are now under clinical investigation as potential new chemotherapeutic agents (Luo, Y.; Hradil, V. P.; Frost, D. J.; Rosenberg, S. H.; Gordon, G. B.; Morgan, S. J.; Gagne, G. D.; Cox, B. F.; Tahir, S. K.; Fox, G. B., ABT-751, "A novel tubulin-binding agent, decreases tumor perfusion and disrupts tumor vasculature". Anticancer Drugs 2009, 20(6), 483-92.; Mauer, A. M.; Cohen, E. E.; Ma, P. C.; Kozloff, M. F.; Schwartzberg, L.; Coates, A. I.; Qian, J.; Hagey, A. E.; Gordon, G. B., "A phase II study of ABT-751 in patients with advanced non-small cell lung cancer". J Thorac Oncol 2008, 3(6), 631-6.; Rustin, G. J.; Shreeves, G.; Nathan, P. D.; Gaya, A.; Ganesan, T. S.; Wang, D.; Boxall, J.; Poupard, L.; Chaplin, D. J.; Stratford, M. R.; Balkissoon, J.; Zweifel, M., "A Phase Ib trial of CA4P (combretastatin A-4 phosphate), carboplatin, and paclitaxel in patients with advanced cancer". Br J Cancer 2010, 102(9), 1355-60.).

Unfortunately, microtubule-interacting anticancer drugs in clinical use share two major problems, resistance and neurotoxicity. A common mechanism of multidrug resistance (MDR), namely ATP binding cassette (ABC) transporter protein-mediated drug efflux, limits their efficacy (Green, H.; Rosenberg, P.; Soderkvist, P.; Horvath, G.; Peterson, C., "beta-Tubulin mutations in ovarian cancer using single strand conformation analysis-risk of false positive results from paraffin embedded tissues". Cancer Letters 2006, 236(1), 148-54.; Wang, Y.; Cabral, F., "Paclitaxel resistance in cells with reduced beta -tubulin". Biochimica et Biophysica Acta, Molecular Cell Research 2005, 1744(2), 245-255.; Leslie, E. M.; Deeley, R. G.; Cole, S. P. C., "Multidrug resistance proteins: role of P-glycoprotein, MRP1, MRP2, and BCRP (ABCG2) in tissue defense". Toxicology and Applied Pharmacology 2005, 204(3), 216-237.).

P-glycoproteins (P-gp, encoded by the MDR1 gene) are important members of the ABC superfamily. P-gp prevents the intracellular accumulation of many cancer drugs by increasing their efflux out of cancer cells, as well as contributing to hepatic, renal, or intestinal clearance pathways. Attempts to co-administer P-gp modulators or inhibitors to increase cellular availability by blocking the actions of P-gp have met with limited success (Gottesman, M. M.; Pastan, I., "The multidrug transporter, a double-edged sword". J Biol Chem 1988, 263(25), 12163-6.; Fisher, G. A.; Sikic, B. I., "Clinical studies with modulators of multidrug resistance". Hematology/Oncology Clinics of North America 1995, 9(2), 363-82).

The other major problem with taxanes, as with many biologically active natural products, is its lipophilicity and lack of solubility in aqueous systems. This leads to the use of emulsifiers like Cremophor EL and Tween 80 in clinical preparations. A number of biologic effects related to these drug formulation vehicles have been described, including acute hypersensitivity reactions and peripheral neuropathies (Hennenfent, K. L.; Govindan, R., "Novel formulations of taxanes: a review. Old wine in a new bottle?" Ann Oncol 2006, 17(5), 735-49.; ten Tije, A. J.; Verweij, J.; Loos, W. J.; Sparreboom, A., "Pharmacological effects of formulation vehicles : implications for cancer chemotherapy". Clin Pharmacokinet 2003, 42(7), 665-85.).

Compared to compounds binding the paclitaxel- or vinca alkaloid binding site, colchicine-binding agents usually exhibit relatively simple structures. Thus providing a better opportunity for oral bioavailability via structural optimization to improve solubility and pharmacokinetic (PK) parameters. In addition, many of these drugs appear to circumvent P-gp-mediated MDR. Therefore, these novel colchicine binding site targeted compounds hold great promise as therapeutic agents, particularly since they have improved aqueous solubility and overcome P-gp mediated MDR.

Prostate cancer is one of the most frequently diagnosed noncutaneous cancers among men in the US and is the second most common cause of cancer deaths with over 180,000 new cases and almost 29,000 deaths expected this year. Patients with advanced prostate cancer undergo androgen deprivation therapy (ADT), typically either by luteinizing hormone releasing hormone (LHRH) agonists or by bilateral orchiectomy. Androgen deprivation therapy not only reduces testosterone, but estrogen levels are also lower since estrogen is derived from the aromatization of testosterone, which levels are depleted by ADT. Androgen deprivation therapy-induced estrogen deficiency causes significant side effects which include hot flushes, gynecomastia and mastalgia, bone loss, decreases in bone quality and strength, osteoporosis and life-threatening fractures, adverse lipid changes and higher cardiovascular disease and myocardial infarction, and depression and other mood changes.

Leuprolide acetate (Lupron®) is a synthetic nonapeptide analog of naturally occurring gonadotropin-releasing hormone (GnRH or LHRH). Leuprolide acetate is an LHRH superagonist that eventually suppresses LH secretion by the pituitary. Leuprolide acetate acts as a potent inhibitor of gonadotropin secretion, resulting in suppression of ovarian and testicular steroidogenesis. In humans, administration of leuprolide acetate results in an initial increase in circulating levels of luteinizing hormone (LH) and follicle stimulating hormone (FSH), leading to a transient increase in levels of the gonadal steroids (testosterone and dihydrotestosterone in males, and estrone and estradiol in premenopausal females). However, continuous administration of leuprolide acetate results in decreased levels of LH and FSH. In males, testosterone is reduced to castrate levels (below 50 ng/dL). In premenopausal females, estrogens are reduced to postmenopausal levels. Testosterone is a known stimulus for cancerous cells of the prostate. Suppressing testosterone secretion or inhibiting the actions of testosterone is thus a necessary component of prostate cancer therapy. Leuprolide acetate can be used for LH suppression, which is the reduction and lowering of serum testosterone to castrate levels to treat prostate cancer.

Malignant melanoma is the most dangerous form of skin cancer, accounting for about 75% of skin cancer deaths. The incidence of melanoma is rising steadily in Western populations. The number of cases has doubled in the past 20 years. Around 160,000 new cases of melanoma are diagnosed worldwide each year, and it is more frequent in males and Caucasians. According to a WHO Report, about 48,000 melanoma-related deaths occur worldwide per year.

Currently there is no effective way to treat metastatic melanoma. It is highly resistant to current chemotherapy, radiotherapy, and immunotherapy. Metastatic melanoma has a very poor prognosis, with a median survival rate of 6 months and a 5-year survival rate of less than 5%. In the past 30 years, dacarbazine (DTIC) is the only FDA-approved drug for metastatic melanoma. However, it provides only less than 5% of complete remission in patients. In recent years, great efforts have been attempted in fighting metastatic melanoma. Neither combinations of DTIC with other chemotherapy drugs (e.g., cisplatin, vinblastine, and carmustine) nor adding interferon- α2b to DTIC have shown a survival advantage over DTIC treatment alone. Most recently, clinical trials with antibodies and vaccines to treat metastatic melanoma also failed to demonstrate satisfactory efficacy.

Melanoma cells have low levels of spontaneous apoptosis *in vivo* compared with other tumor cell types, and they are relatively resistant to drug-induced apoptosis *in vitro*. The natural role of melanocytes is to protect inner organs from UV light, a potent DNA damaging agent. Therefore, it is not surprising that melanoma cells may have special DNA damage repair systems and enhanced survival properties. Moreover, recent studies showed that, during melanoma progression, it acquired complex genetic alterations that led to hyperactivation of efflux pumps, detoxification enzymes, and a multifactorial alteration of survival and apoptotic pathways. All these have been proposed to mediate the multidrug-resistant (MDR) phenotype of melanoma. With the rapidly rising incidence of this disease and the high resistance to current therapeutic agents, developing more effective drugs for advanced melanoma and other cancer types that can effectively circumvent MDR will provide significant benefits to cancer patients.

US Patent Publication No. 2009/0326020 A1 discloses compounds of formula VIII 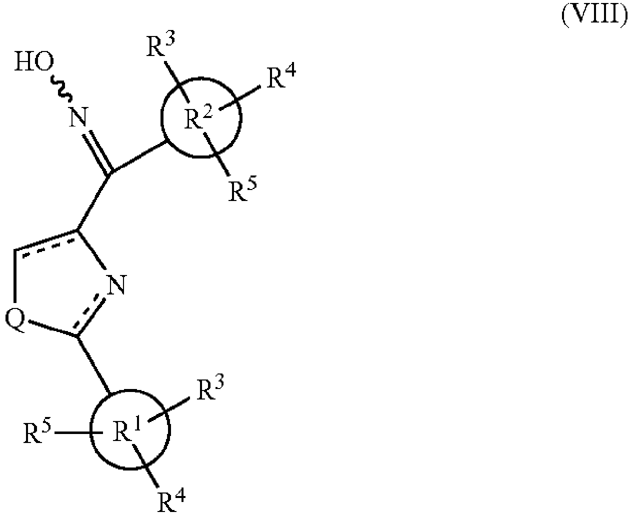 wherein Q is S, N or O, and their use in treating cancer.

### SUMMARY OF THE INVENTION

In one embodiment, this invention is directed to a compound represented by the structure of formula **XI**: 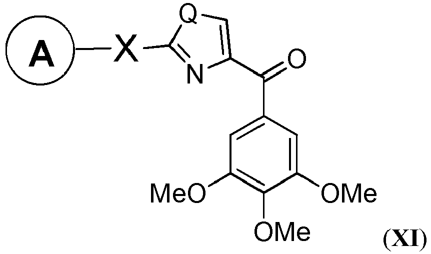 wherein
**X** is NH;
**Q** is S; and
**A** is a substituted or unsubstituted phenyl or indolyl ring;
wherein said A ring is optionally substituted by 1-5 substituents which are independently O-alkyl, O-haloalkyl, F, Cl, Br, I,,haloalkyl, CF₃, CN, -CH₂CN, NH₂, hydroxyl, -(CH₂)ᵢNHCH₃, -(CH₂)ᵢNH₂, -(CH₂)ᵢN(CH₃)₂, -OC(O)CF₃, C₁-C₅ linear or branched alkyl, haloalkyl, alkylamino, aminoalkyl, -OCH₂Ph, -NHCO-alkyl, COOH, -C(O)Ph, C(O)O-alkyl, C(O)H, -C(O)NH₂ or NO₂; and
**i** is an integer between 0-5.

In one embodiment, this invention is directed to a compound represented by the structure of formula **VIII**: 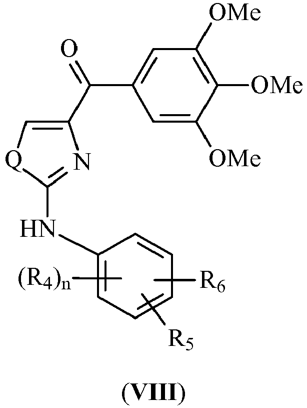
**R₄**, **R₅** and **R₆** are independently hydrogen, O-alkyl, O-haloalkyl, F, Cl, Br, I, haloalkyl, CF₃, CN, -CH₂CN, NH₂, hydroxyl, -(CH₂)ᵢNHCH₃, -(CH₂)ᵢNH₂, - (CH₂)ᵢN(CH₃)₂, -OC(O)CF₃, C₁-C₅ linear or branched alkyl, haloalkyl, alkylamino, aminoalkyl, -OCH₂Ph, -NHCO-alkyl, COOH, -C(O)Ph, C(O)O-alkyl, C(O)H, - C(O)NH₂ or NO₂;
Q is S;
**i** is an integer between 0-5; and
**n** is an integer between 1-3.

In one embodiment, this invention is directed to a compound represented by the structure of formula **XI**(c): 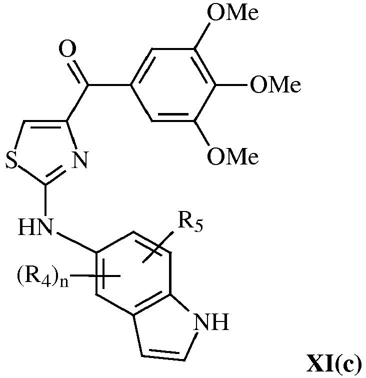
wherein R₄ and R₅ are independently hydrogen, O-alkyl, O-haloalkyl, F, Cl, Br, I, haloalkyl, CF₃, CN, -CH₂CN, NH₂, hydroxyl, -(CH₂)ᵢNHCH₃, -(CH₂)ᵢNH₂, - (CH₂)ᵢN(CH₃)₂, -OC(O)CF₃, C₁-C₅ linear or branched alkyl, haloalkyl, alkylamino, aminoalkyl, -OCH₂Ph, -NHCO-alkyl, COOH, -C(O)Ph, C(O)O-alkyl, C(O)H, - C(O)NH₂ or NO₂;
**i** is an integer from 0-5; and
**n** is an integer between 1-4.

In another embodiment, this invention is directed to the following compounds: (2-(phenylamino)thiazol-4-yl)(3,4,5-trimethoxyphenyl)methanone (**5a**), (2*-*(*p-*tolylamino)thiazol-4-yl)(3,4,5-trimethoxyphenyl)methanone (**5b**), (2-(*p-*fluorophenylamino)thiazol-4-yl)(3,4,5-trimethoxyphenyl)methanone (**5c**), and (2-(4-chlorophenylamino)thiazol-4-yl)(3,4,5-trimethoxyphenyl)methanone (**5d**).

In another embodiment, the compound of this invention is its pharmaceutically acceptable salt, pharmaceutical product, tautomer, hydrate, *N*-oxide, or combinations thereof.

In one embodiment, this invention is directed to a pharmaceutical composition comprising a compound of this invention and a pharmaceutically acceptable carrier.

In one embodiment this invention is directed to a compound of this invention for use in (a) treating, suppressing, reducing the severity, reducing the risk, or inhibiting cancer; (b) treating a drug resistant tumor or tumors; and (c) destroying a cancerous cell. In another embodiment the cancer is selected from the group consisting of prostate cancer, breast cancer, ovarian cancer, skin cancer, melanoma, lung cancer, colon cancer, leukemia, renal cancer, CNS cancer, and combinations thereof.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

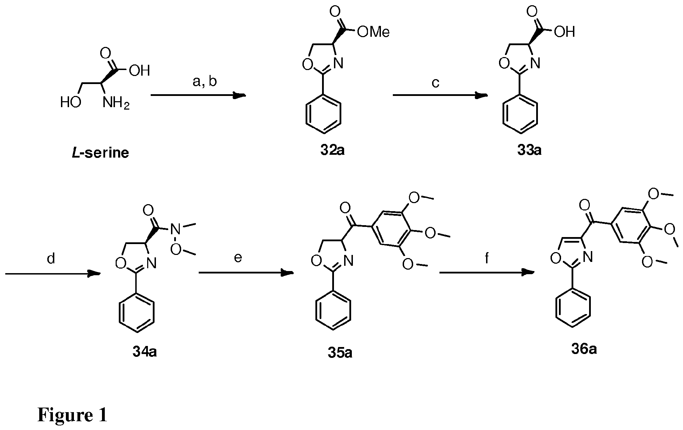

**Figure 1** depicts the synthesis of the diverse B-ring template: oxazole. Reagents and conditions: (a) MeOH, CH₃COCl, 83%; (b) Benzimidic acid ethyl ester, CH₂Cl₂, Et₃N, 96%; (c) LiOH, MeOH, H₂O, 65%; (d) EDCI, HOBt, NMM, CH₃OCH₃NH•HCl, 61%; (e) 3,4,5-trimethoxyphenylmagnesium bromide, THF, 48%-71%; (f) CBrCl₃, DBU, CH₂Cl₂, 56%.

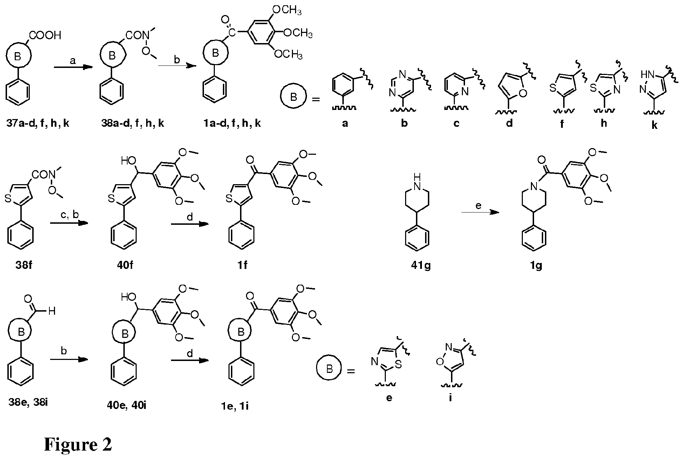

**Figure 2** depicts the synthesis of the diverse B-ring templates. Reagents and conditions: (a) EDCI, HOBt, NMM, CH₃OCH₃NH•HCl, CH₂Cl₂, 51-95%; (b) 3,4,5-trimethoxyphenyl-magnesium bromide, THF, 48-78%; (c) LAH, -78 °C, THF, 85%; (d) Dess-Martin reagent, CH₂Cl₂, 81%; (e) EDCI, HOBt, NMM, 3,4,5-trimethoxybenzoic acid, CH₂Cl₂, 58%.

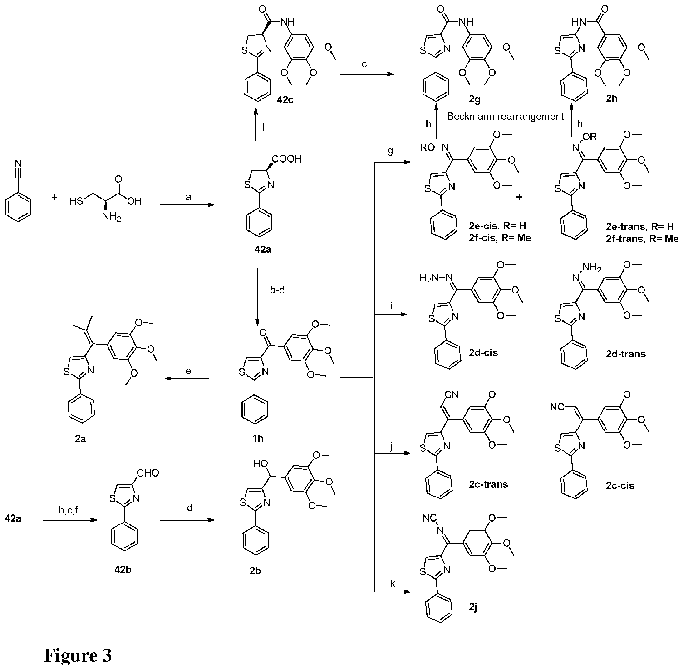

**Figure 3** depicts the synthetic scheme of reference compounds. Reagents and conditions: (a) MeOH/pH=6.4 phosphate buffer, RT; (b) EDCI, HOBt, NMM, HNCH₃OCH₃; (c) CBrCl₃, DBU, CH₂Cl₂; (d) 3,4,5-trimethoxyphenylmagnesium bromide, THF; (e) isopropyl triphenylphosphonium iodide, *n*-BuLi, THF; (f) LAH, THF; (g) For **2e-cis** and **2e-trans**, NH₂OH•HCl, C₂H₅OH, H₂O, NaOH; For **2g** and **2h**, NH₂OMe•HCl, pyridine; (h) TsCl, NaH, basic Al₂O₃; (i) NH₂NH₂•xH₂O, CH₂Cl₂, *t*-BuOH; (j) diethyl cyanomethylphosphonate, *n*-BuLi, THF; (k) bis-trimethylsilylcarbodiimide, TiCl₄, CH₂Cl₂; (1) EDCI, HOBt, Et₃N, 3,4,5-trimethoxyaniline, CH₂Cl₂.

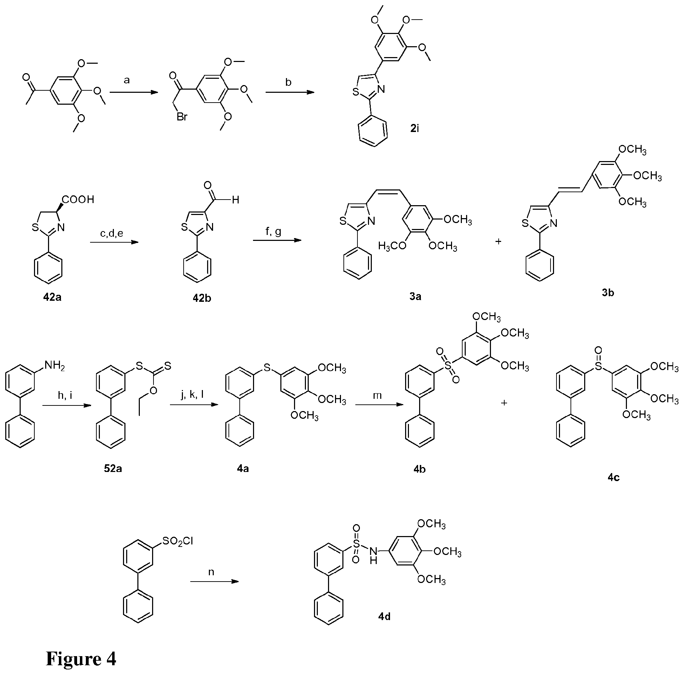

**Figure 4** depicts the synthetic scheme of reference compounds. Reagents and conditions: (a) bromine, EtOH; (b) benzothioamide, EtOH, reflux; (c) EDCI, HOBt, NMM, HNCH₃OCH₃, CH₂Cl₂; (d) CBrCl₃, DBU, CH₂Cl₂; (e) LAH, THF; (f) 5-(bromomethyl)-1,2,3-trimethoxybenzene, Ph₃P, THF; (g) *n*-BuLi, THF; (h) (1) HCl, H₂O; (2) NaNO₂, H₂O, 0 °C; (i) ethyl potassium xanthate; (j) KOH/EtOH; (k) H₂O, HCl; (1) 5-iodo-1,2,3-trimethoxybenzene, CuI, *t*-BuONa; (m) 2 equiv or 1 equiv *m*-CPBA, CH₂Cl₂; (n) 3,4,5-trimethoxyaniline, NEt₃, DMF.

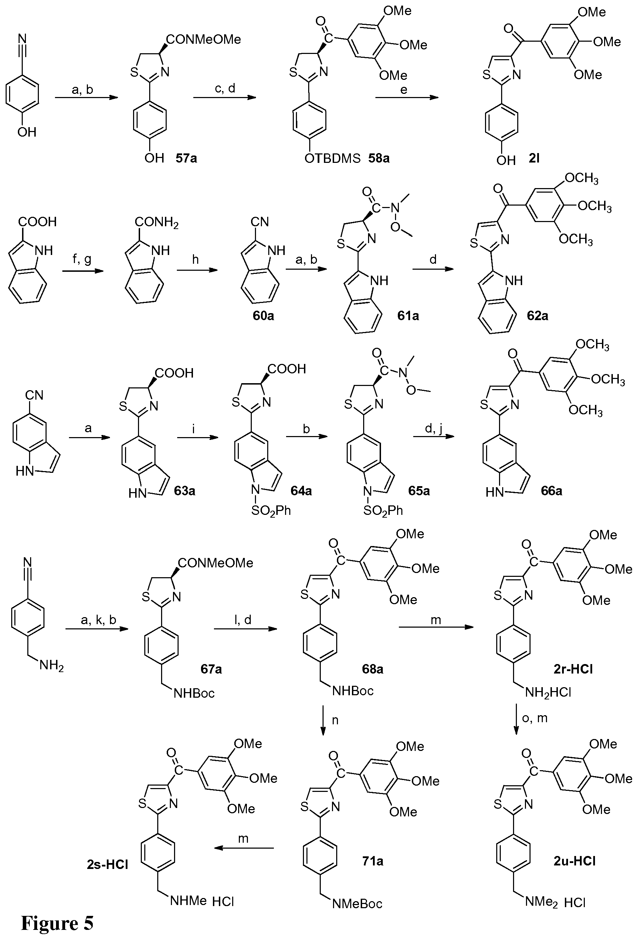

**Figure 5** depicts the synthetic scheme of reference compounds. Reagents and conditions: (a) *L*-cysteine, EtOH, 65 °C; (b) EDCI, HOBt, NMM, HNCH₃OCH₃, CH₂Cl₂; (c) TBDMSCl, imidazole, THF; (d) 3,4,5-trimethoxyphenylbromide, BuLi, THF; (e) TBAF, THF; (f) SOCl₂, Et₂O; (g) NH₃, MeOH; (h) POCl₃; (i) PhSO₂Cl, Bu₄NHSO₄, toluene, 50% NaOH; (j) 1 N NaOH, EtOH, reflux; (k) Boc₂O, 1 N NaOH, 1,4-dioxane; (1) CBrCl₃, DBU, CH₂Cl₂; (m) 4 N HCl in 1,4-dioxane; (n) NaH, DMF, MeI; (o) HCHO, NaBH₃CN, Et₃N.

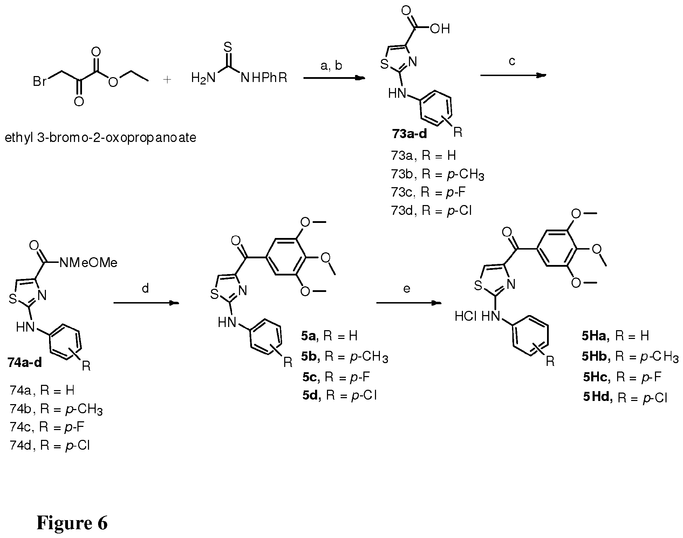

**Figure 6** depicts the synthetic scheme of compounds of this invention. Reagents and conditions: (a) EtOH, 65 °C; (b) NaOH, C₂H₅OH, refluxing; (c) EDCI, HOBt, NMM, HNCH₃OCH₃, CH₂Cl₂; (d) 3,4,5-trimethoxyphenylbromide, BuLi, THF; (e) 2 N HCl in 1,4-dioxane.

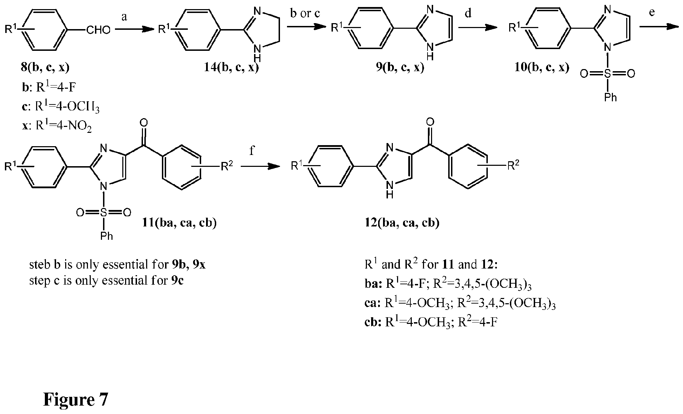

**Figure 7** depicts a synthetic scheme for the preparation of Aryl-Benzoyl-Imidazole (ABI) compounds as reference compounds. Reagents and conditions: (a) *t*-BuOH, I₂, ethylenediamine, K₂CO₃, reflux; (b) PhI (OAc)₂, K₂CO₃, DMSO; (c) DBU, CBrCl₃, DMF; (d) NaH, PhSO₂Cl, THF, 0 °C - RT; (e) *t*-BuLi, substituted benzoyl chloride, THF, -78 °C; (f) Bu₄NF, THF, RT.

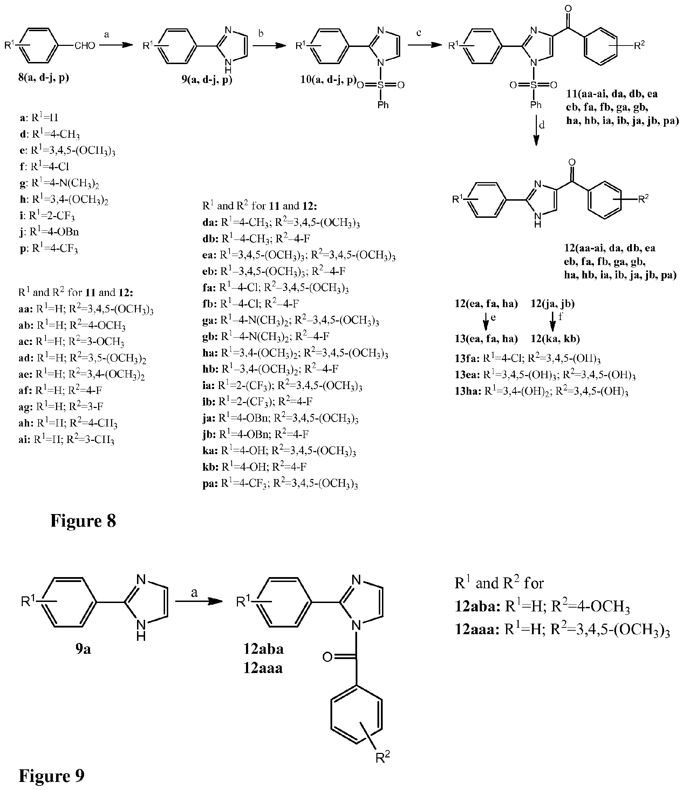

**Figure 8** depicts a synthetic scheme for the preparation of Aryl-Benzoyl-Imidazole (ABI) compounds as reference compounds. Reagents and conditions: (a) NH₄OH, oxalaldehyde, ethanol, RT; (b) NaH, PhSO₂Cl, THF, 0 °C - RT; (c) *t*-BuLi, substituted benzoyl chloride, THF, -78 °C; (d) Bu₄NF, THF, RT; (e) BBr₃, CH₂Cl₂; (f) c-HCl, AcOH, reflux.
**Figure 9** depicts a synthetic scheme for the preparation of Aryl-Benzoyl-Imidazole (ABI) compounds as reference compounds. Reagents and conditions: (a) NaH, substituted benzoyl chloride, THF.

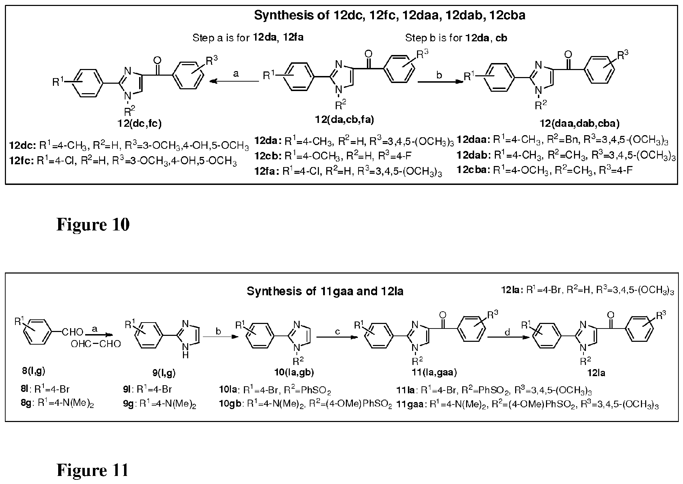

**Figure 10** depicts the synthetic scheme of compounds **12dc**, **12fc**, **12daa**, **12dab**, **12cba**. (a) AlCl₃, THF, reflux; (b) NaH, CH₃I for **12dab** and **12cba** and BnBr for **12daa**, THF, reflux.
**Figure 11** depicts the synthetic scheme of compounds **11gaa, 12la**. (a) NH₄OH, ethanol, glyoxal, RT; (b) NaH, substituted PhSO₂Cl, THF, 0 °C - RT; (c) *t*-BuLi (1.7 M in pentane), substituted benzoyl chloride, THF, -78 °C; (d) Bu₄NF, RT.

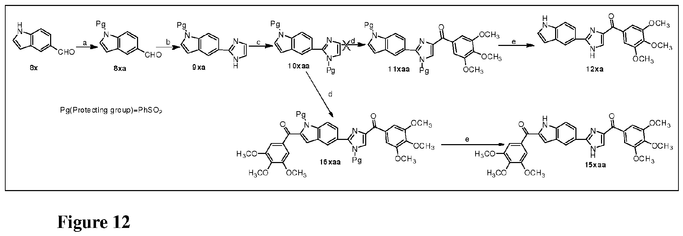

**Figure 12** depicts the synthetic scheme of compound **15xaa** and **12xa**. (a) 1. KOH, ethanol; 2. PhSO₂Cl, acetone; (b) NH₄OH, glyoxal, ethanol, RT; (c) NaH, PhSO₂Cl, THF, 0 °C - RT; (d) *t*-BuLi (1.7 M in pentane), benzoyl chloride, THF, -78 °C; (e) NaOH, ethanol, H₂O, reflux.

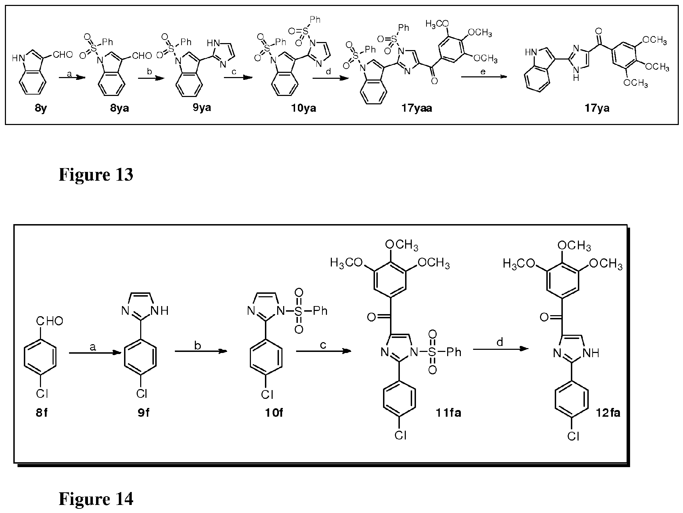

**Figure 13** depicts synthetic scheme of **17ya**. (a) 1. KOH, ethanol, 2. PhSO₂Cl, acetone, RT; (b) NH₄OH, glyoxal, ethanol, RT; (c) NaH, PhSO₂Cl, THF, 0 °C - RT; (d) *t-*BuLi (1.7 M in pentane), benzoyl chloride, THF, -78 °C; (e) NaOH, ethanol, H₂O, reflux.
**Figure 14** depicts synthetic scheme of **12fa**. (a) NH₄OH, oxalaldehyde, ethanol, RT; (b) NaH, PhSO₂Cl, THF, 0 °C - RT; (c) *t*-BuLi, 3,4,5-trimethoxybenzoyl chloride, THF, -78 °C; (d) Bu₄NF, THF, RT.

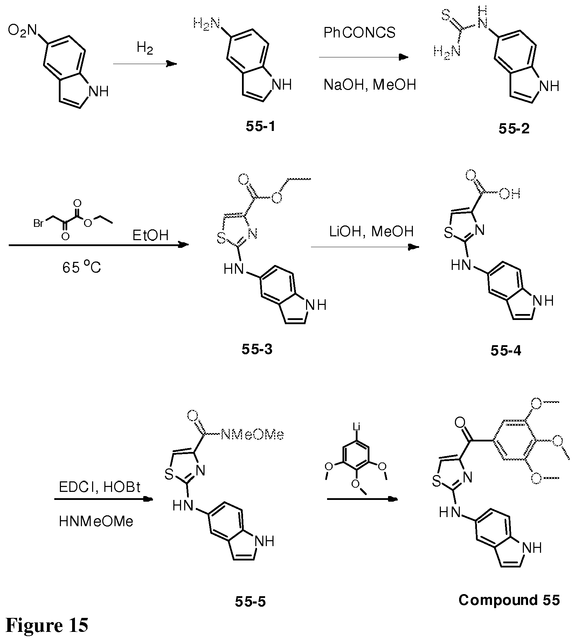

**Figure 15** depicts a synthetic scheme of compound **55**.

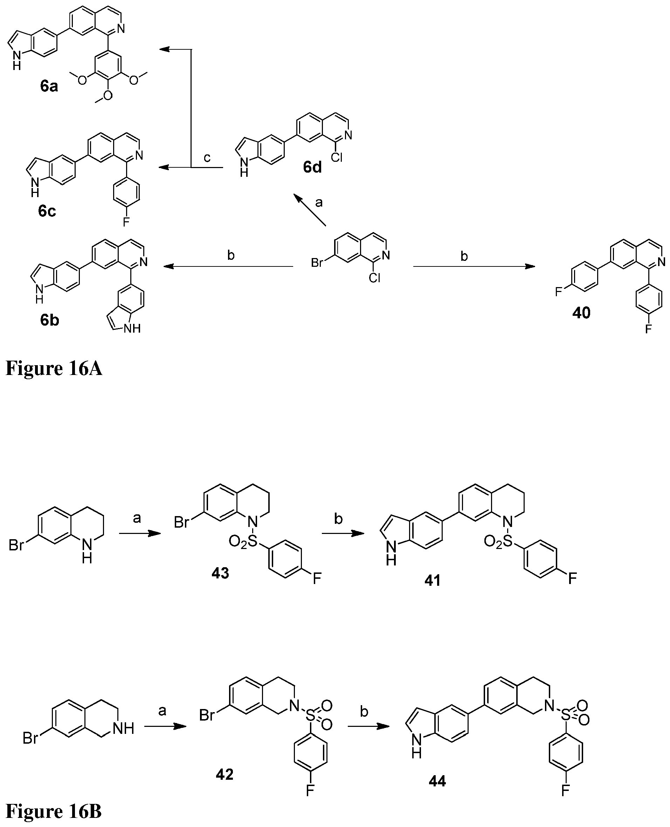

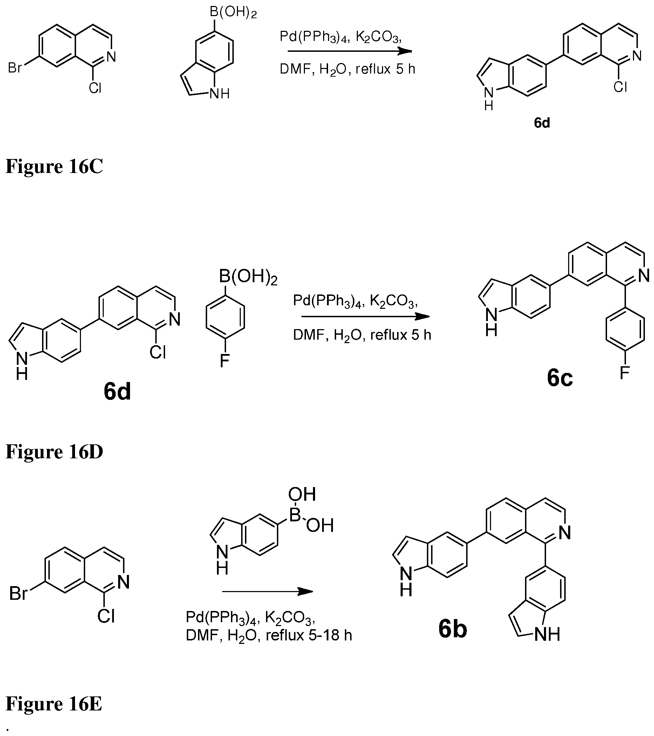

**Figure 16** depicts a synthetic scheme of isoquinoline and quinoline based compounds. **Figure 16A** depicts the synthetic scheme of isoquinoline derivatives. Reagents and conditions: a) arylboronic acid (1 equiv.), Pd(PPh₃)₄ (0.01 equiv.), K₂CO₃, H₂O, DMF, 5 h; b) arylboronic acid (2.4 equiv.), Pd(PPh₃)₄ (0.04 equiv.), K₂CO₃, H₂O, DMF, 16 h; c) arylboronic acid (1.2 equiv.), Pd(PPh₃)₄ (0.04 equiv.), K₂CO₃, H₂O, DMF, 16 h. **Figure 16B** depicts the synthetic scheme of compounds **41** and **44**. Reagents and conditions: a) *p-*fluorobenzenesulfonyl chloride, pyridine, pyridine, 80 °C, 3 h; b) 5-indoleboronic acid (1.2 equiv.), Pd(PPh₃)₄ (0.02 equiv.), K₂CO₃, H₂O, DMF, 16 h. **Figure 16C** depicts the synthetic scheme of isoquinoline derivative **6d**. **Figure 16D** depicts the synthetic scheme of isoquinoline derivative **6c**. **Figure 16E** depicts the synthetic scheme of isoquinoline derivative **6b**.

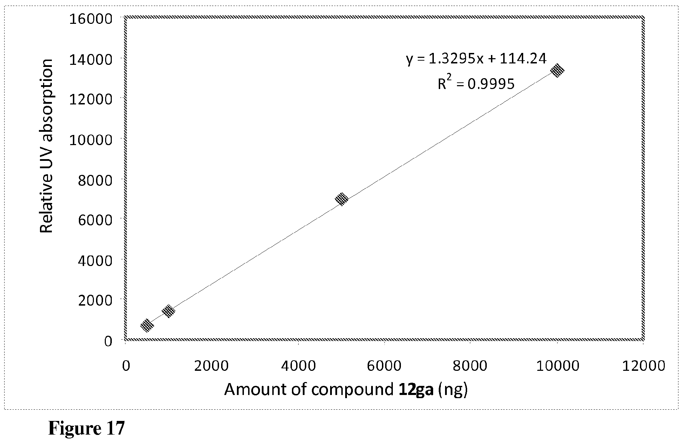

**Figure 17** depicts a standard solubility curve for ABI compound **12ga** (dissolved in acetonitrile). X-axis is the amount of compound and y-axis is the m/z peak area.

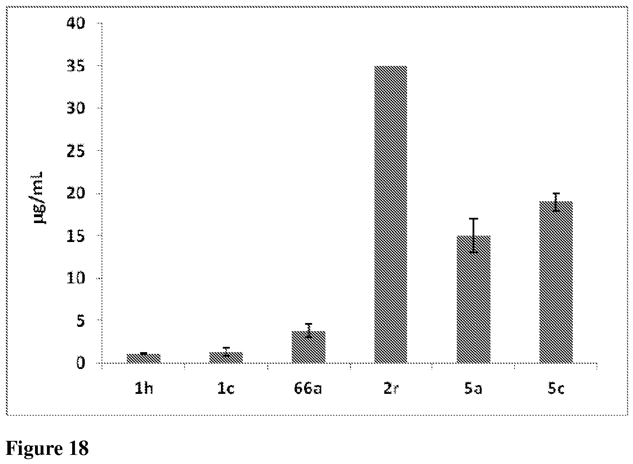

**Figure 18** depicts the measured aqueous solubility for anti-tubulin compounds **1h**, **1c**, **66a, 2r-HCl, 5a**, and **5c**.
**Figure 19** depicts the structures of colchicine-binding site tubulin inhibitors.

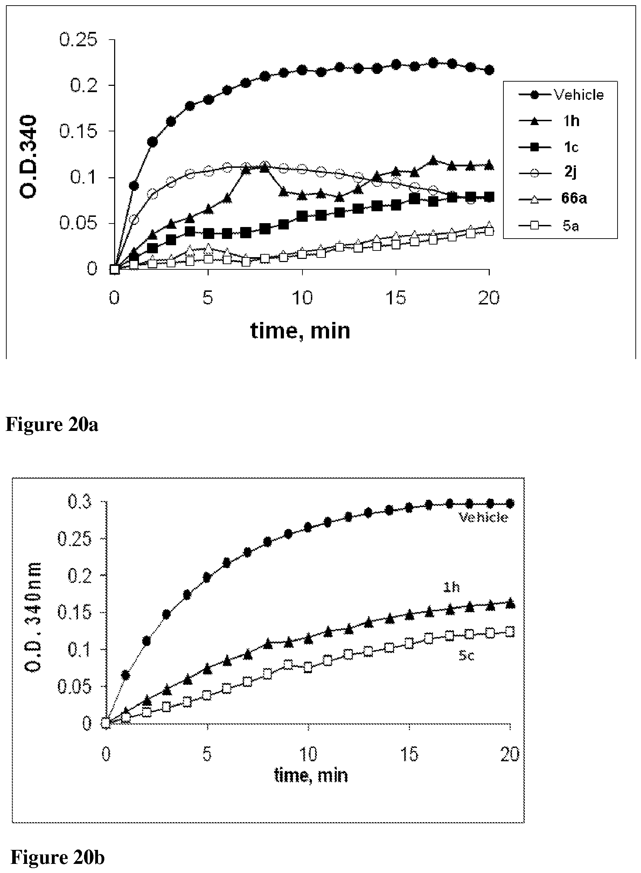

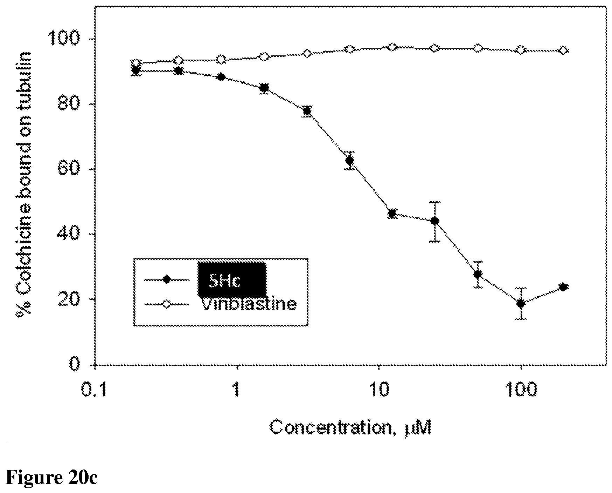

**Figure 20** depicts the ability of anti-tubulin compounds **1h**, **1c**, **2j**, **66a** and **5a** to inhibit tubulin polymerization *in vitro* (**Figure 20a**) and **5c** (**Figure 20b**), and the **5Hc** binding to colchicine site (**Figure 20c**).

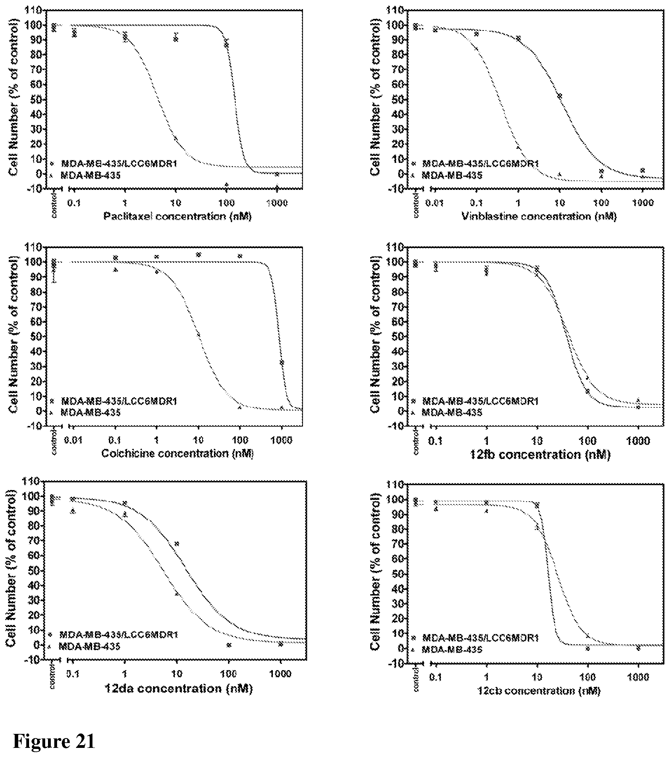

**Figure 21** depicts dose-response curves of 2-aryl-4-benzoyl-imidazole compounds (ABIs) compared with other anticancer drugs and compounds on multidrug resistant melanoma cell line (MDR cell) and the matched sensitive parent cell line (Normal Melanoma cell). The large distance between the two curves for paclitaxel, vinblastine, and colchicine indicates that they were substrates for P-glycoprotein (P-gp). The overlapping two curves of each ABI compound indicate that the ABI compounds were not substrates for P-gp and overcame multidrug resistance.

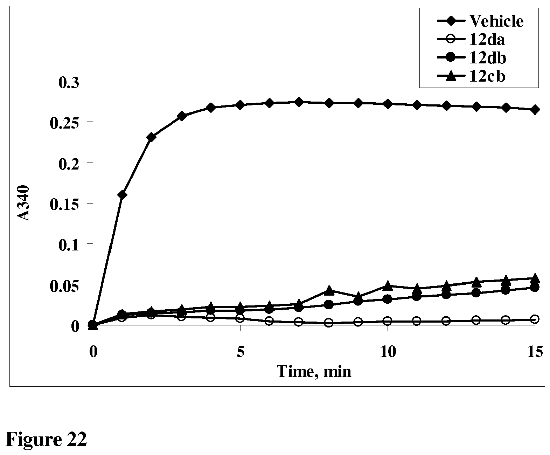

**Figure 22** presents the effect of ABI compounds on tubulin polymerization *in vitro*. Tubulin (0.4 mg/assay) was exposed to 10 µM ABI compounds (vehicle control, 5% DMSO). Absorbance at 340 nm was monitored at 37 °C every minute for 15 min and demonstrated that ABI compounds **12da, 12db**, and **12cb** inhibited tubulin polymerization *in vitro*.

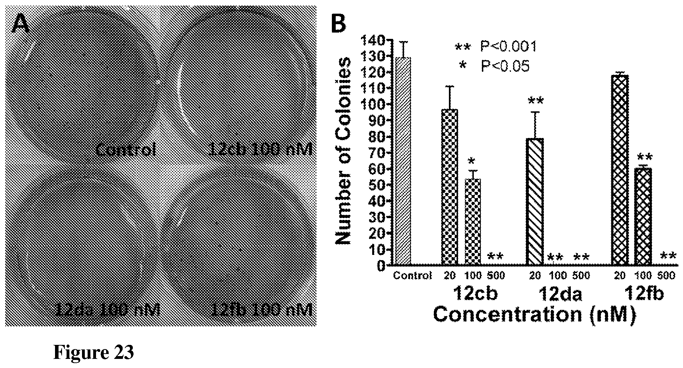

**Figure 23** depicts B16-F1 melanoma colony formation assay in soft agar which showed that ABI compounds inhibited colony formation in a concentration-dependent manner. **Figure 23A** depicts representative pictures of control and each tested compound (**12cb**, **12da**, and **12fb**) at 100 nM. The diameter of each well was 35 mm. **Figure 23B** depicts a quantified representation of assay results for each tested compound (**12cb**, **12da**, and **12fb**). P value was calculated comparing with control using Student's t test by GraphPad Prism software. Columns, means of three replicates; bars, SD.

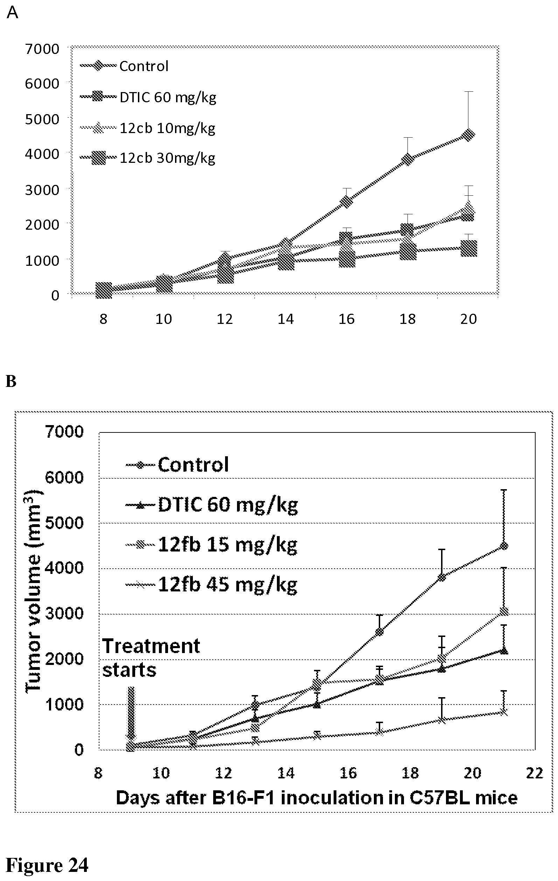

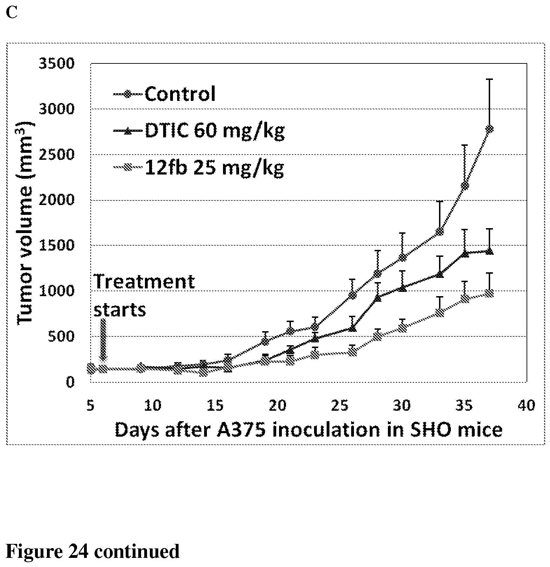

**Figure 24** depicts *in vivo* study of ABI compounds. **Figure 24A** depicts the *in vivo* activity of **12cb** against B16-F1 melanoma tumors in C57/BL mice. **Figure 24B** depicts the *in vivo* activity of **12fb** against B16-F1 melanoma in C57BL/6 mice and SHO nude mice. Results showed that **12fb** inhibited melanoma tumor growth in a dose-dependent manner. C57BL/6 mice bearing B16-F1 melanoma allograft (n=5 per group). Each mouse received 0.5x10⁶ cells by s.c. injection into the flank. 30 µL i.p. daily treatments were started when tumor size reached -100 mm³. **Figure 24C** depicts the *in vivo* activity of **12fb** against an A375 human melanoma xenograft. SHO nude mice bearing an A375 human melanoma xenograft (n=5 per group). Each mouse received 2.5x10⁶ cells by s.c. injection into the flank. 30 µL i.p. daily treatments were started when the tumor size reached -150 mm³. Control, vehicle solution only; points, means; bars, SD. DTIC, (5-(3,3,-dimethyl-1-triazenyl)-imidazole-4-carboxamide, dacarbazine.

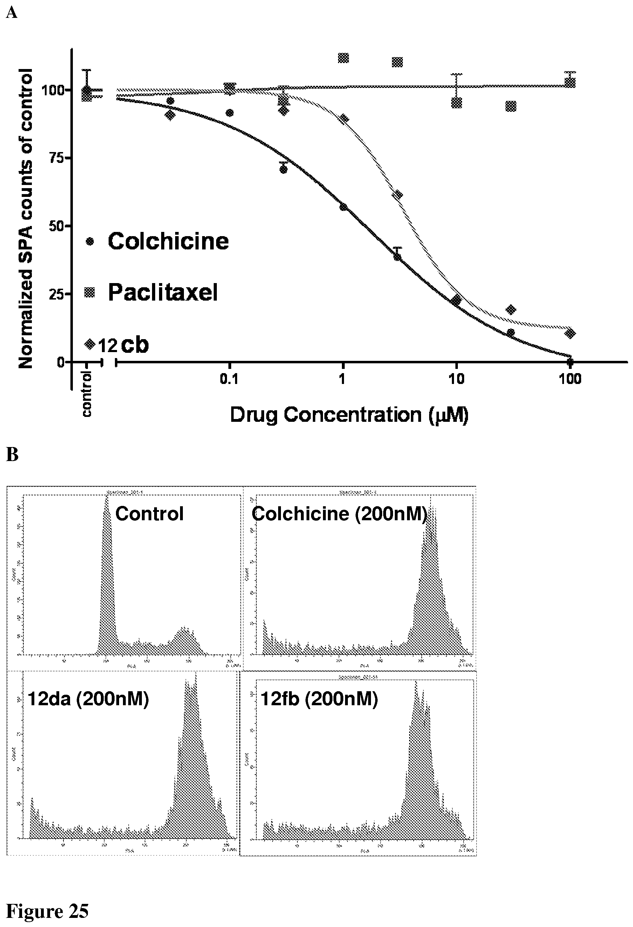

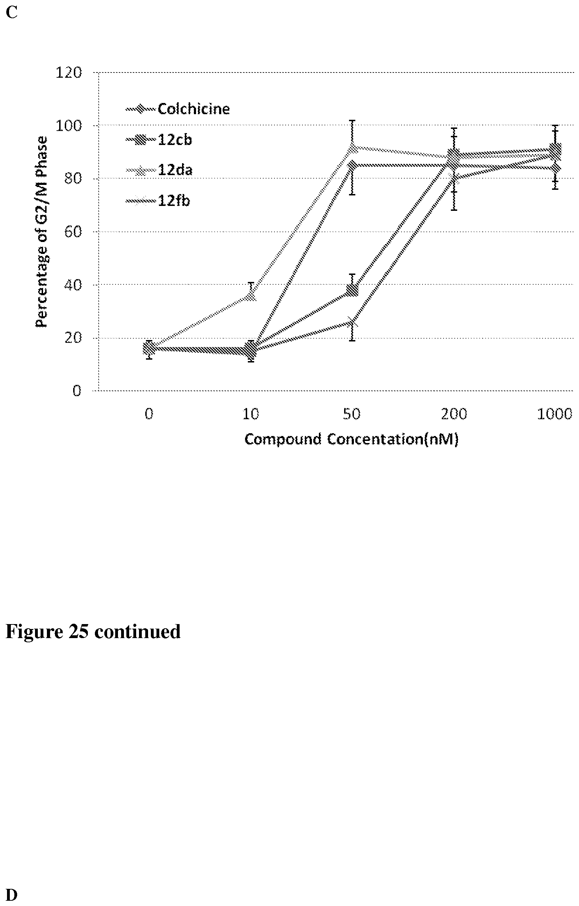

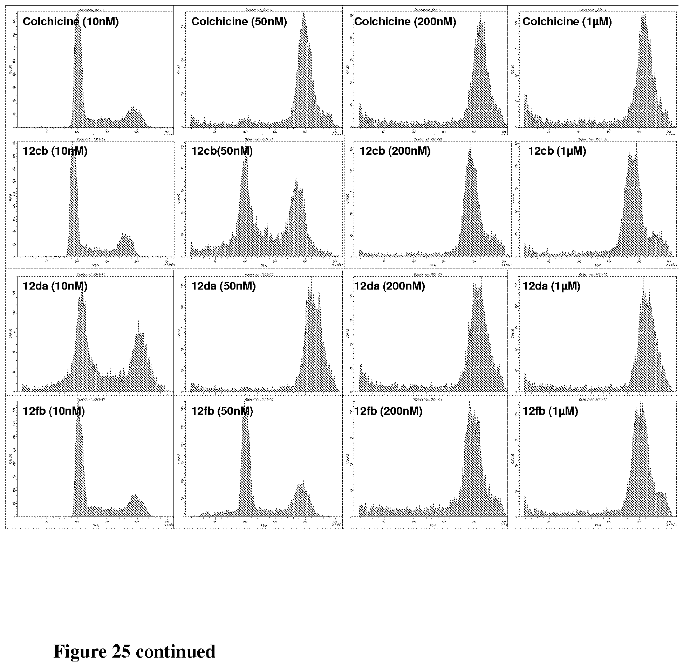

**Figure 25** depicts a competitive colchicine binding assay. **Figure 25A** depicts a [³H]-colchicine competition-binding scintillation proximity assay which showed that **12cb** competitively bound to tubulin colchicine binding site. **Figure 25B** depicts representative graphs of cell cycle analysis using flow cytometry which showed that ABI compounds (examples shown for **12da** and **12fb**) arrested A375 cells in the G2/M phase after 24 h incubation. The effect and potency were similar to those of colchicine. **Figure 25C** shows quantified graphic depictions of cell cycle analysis. All tested compounds (examples shown for **12cb**, **12da**, and **12fb**) arrested A375 cells in the G2/M phase in a dose-dependent manner. ABI **12da** showed greater potency than did colchicine. **Figure 25D** depicts a cell cycle analysis using flow cytometry of A375 cells after being incubated with **12cb**, **12da**, **and 12fb** at different concentrations for 24 h. Colchicine arrested most cells in the G2/M phase starting from 50 nM. **12cb**, **12da**, **and 12fb** also arrested most cells in the G2/M phase starting from 200, 50, and 200 nM respectively.

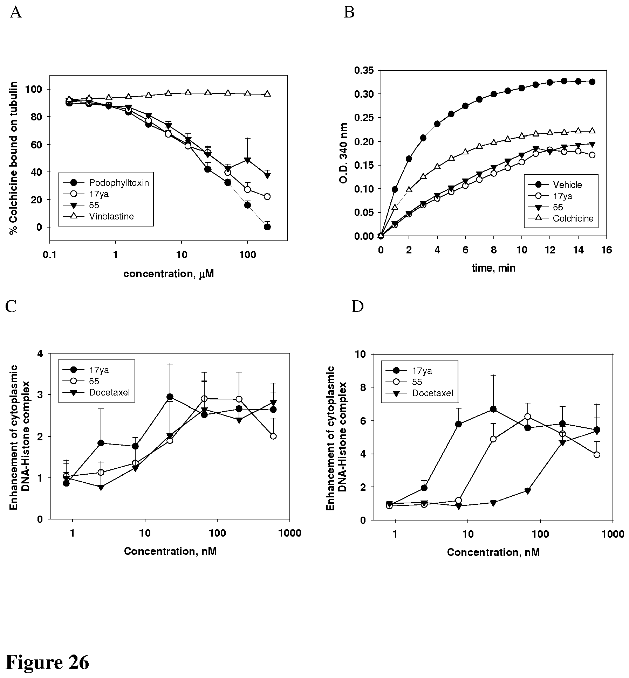

**Figure 26** depicts the effect of **17ya** and **55** on tubulin polymerization. Compounds **17ya** and **55** bind to colchicine-binding site on tubulin, and inhibit tubulin polymerization. **Figure 26A**, competitive mass binding. Tubulin (1 mg/mL) and colchicine (1.2 µM) were incubated with various concentrations of podophylltoxin, vinblastine, compounds **17ya**, and 55. N = 3; mean ± SD. Podophylltoxin and vinblastine were used as positive and negative controls, respectively. **Figure 26B**, effect on tubulin polymerization. Tubulin (0.4 mg) was exposed to test compounds (5 µM). Colchicine was used as positive control. **Figure 26C** and **26D**, ability of **17ya** and **55** to enhance cytoplasmic DNA-Histone complex formation (apoptosis) at 24 h in PC-3 (**C**) and PC-3/TxR (**D**) cells (N =3); mean ± SD. Docetaxel was used as positive control.

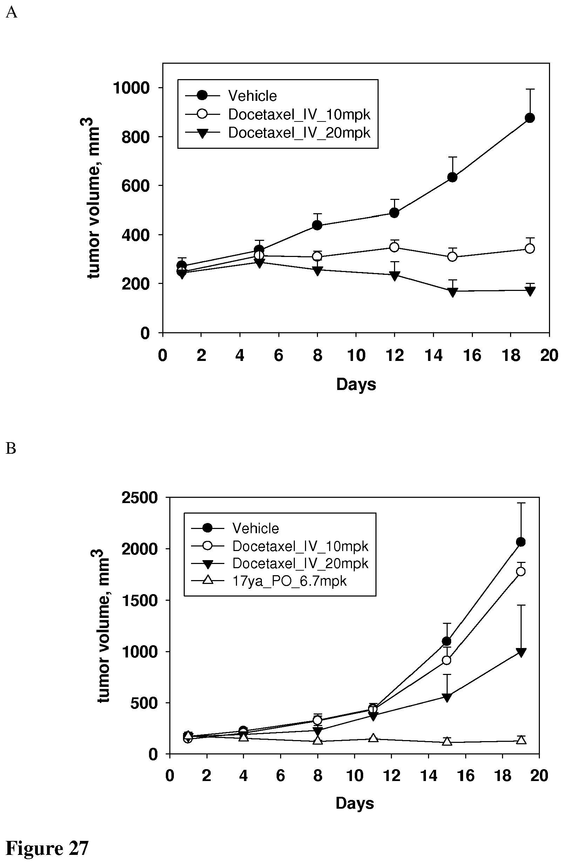

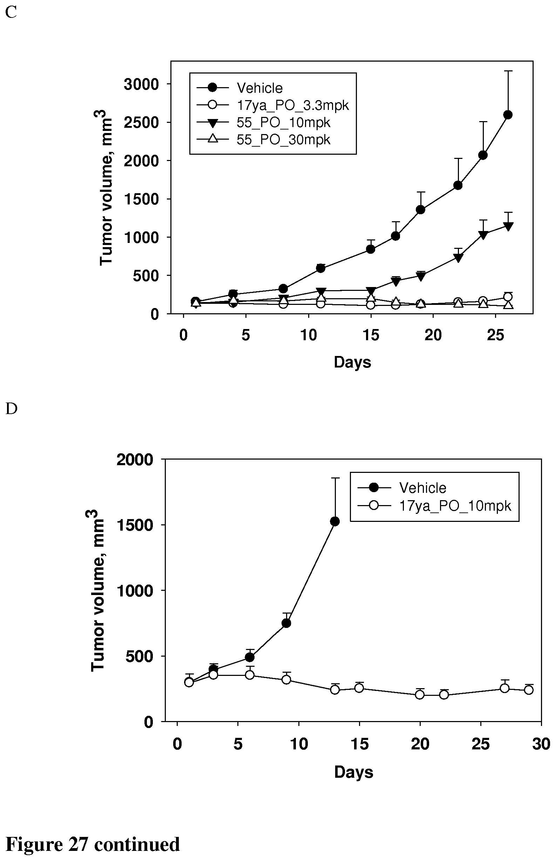

**Figure 27** depicts *in vivo* anticancer efficacy. **Figure 27A****,** Nude mice bearing PC-3 tumors were treated with docetaxel (i.v., 10 or 20 mg/kg) on day 1 and 9. (N = 5-6). Bars, SE. **Figure 27B**, Nude mice bearing PC-3/TxR tumors were treated with docetaxel (i.v., 10 or 20 mg/kg) on day 1 and 9, compound **17ya** treatments (p.o., 6.7 mg/kg) once daily, five days a week. (N = 4-5). Bars, SE. **Figure 27C**, Nude mice bearing PC-3/TxR tumors were treated with compound **17ya** (PO, 3.3 mg/kg) twice a day for four days in the first week, and then dosed once a day, five days a week for weeks 2-4 (N = 7), with compound **55** treatments (p.o., 10 or 30 mg/kg) twice a day, five days a week for four weeks (N = 7). Bars, SE. **Figure 27D**, Nude mice bearing PC-3/TxR tumors were treated with compound **17ya** (PO, 10 mg/kg) three times a week for four weeks (N = 5). Bars, SE.

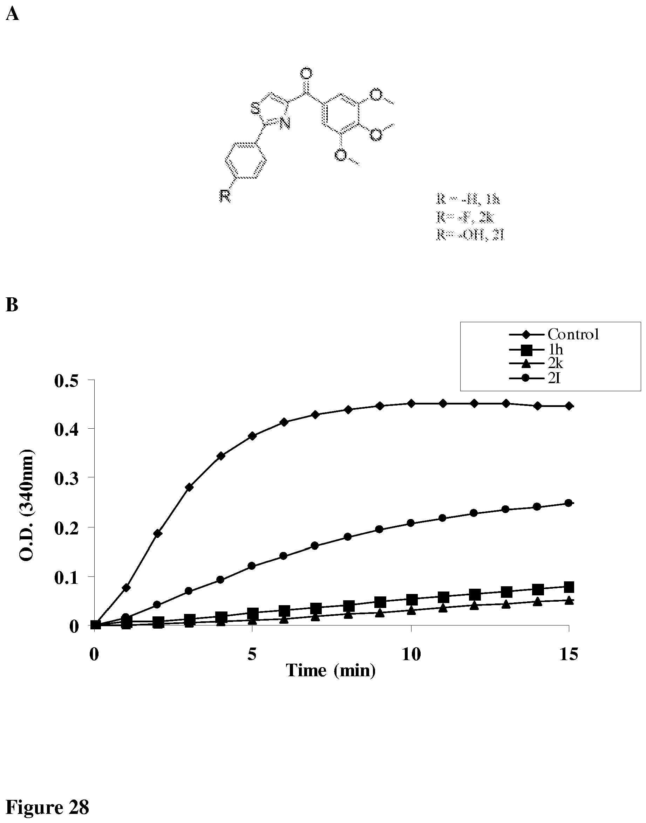

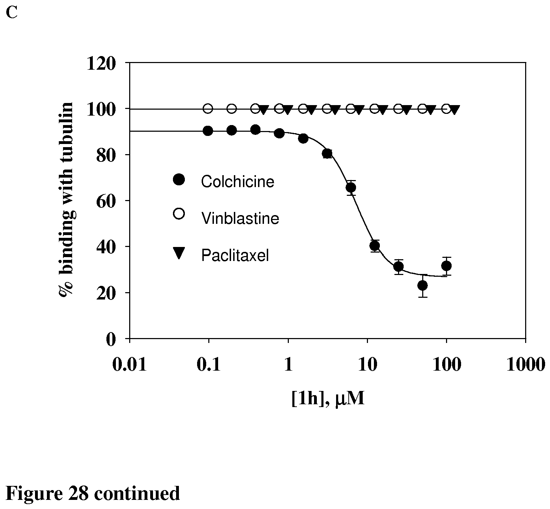

**Figure 28** depicts that compounds **1h**, **2k**, and **2l** inhibit tubulin polymerization via binding to the colchicine binding site on tubulin. (**Figure 28A**) Structures of **1h** (-H), **2k** (-F), and **2l** (-OH). (**Figure 28B**) Effect of the compounds on tubulin polymerization. Tubulin (0.4 mg) was exposed to compounds **1h**, **2k**, and **2l** (10 µM). Absorbance at 340 nm was monitored every min for 15 min. (**Figure 28C**) Ability of **1h** to compete for colchicine, vinblastine and paclitaxel binding sites on tubulin using mass spectrometry competitive binding assay (n = 3); bars, SD.

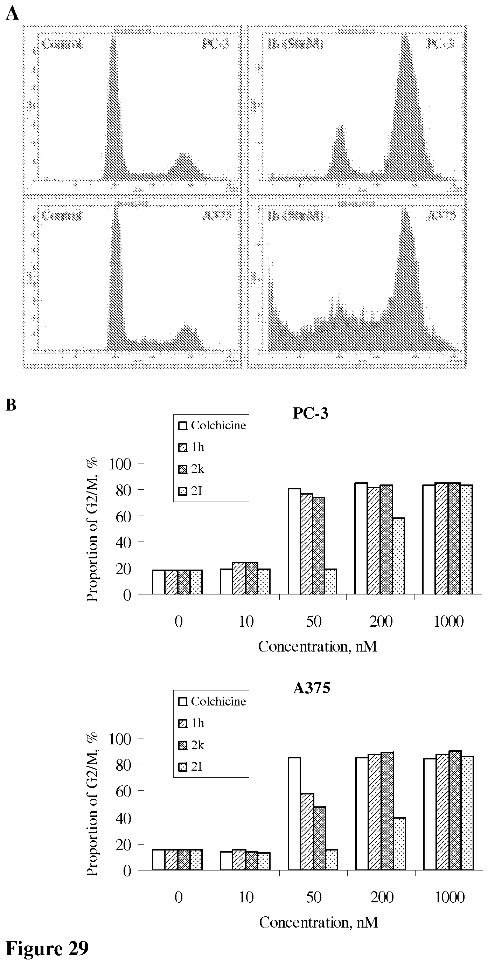

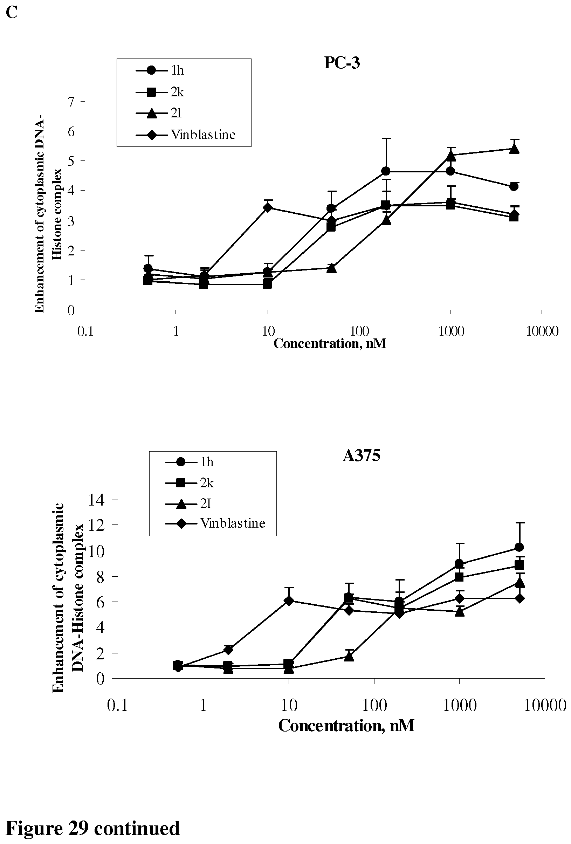

**Figure 29** depicts that compounds **1h**, **2k** and **2l** arrested cells into G2/M phase and induced apoptosis. (**Figure 29A**) Representative graphs of cell cycle analysis after compounds treatment for 24 h on PC-3 and A375 cells. (**Figure 29B**) The changes in G2/M proportion induced by **1h**, **2k**, and **2l** in PC-3 and A375 cells after 24 h treatment. (**Figure 29C**) Ability of **1h**, **2k**, and **2l** to enhance cytoplasmic DNA-Histone complex formation in 24 h (n = 3); bars, SD. Colchicine and vinblastine were used as positive controls.

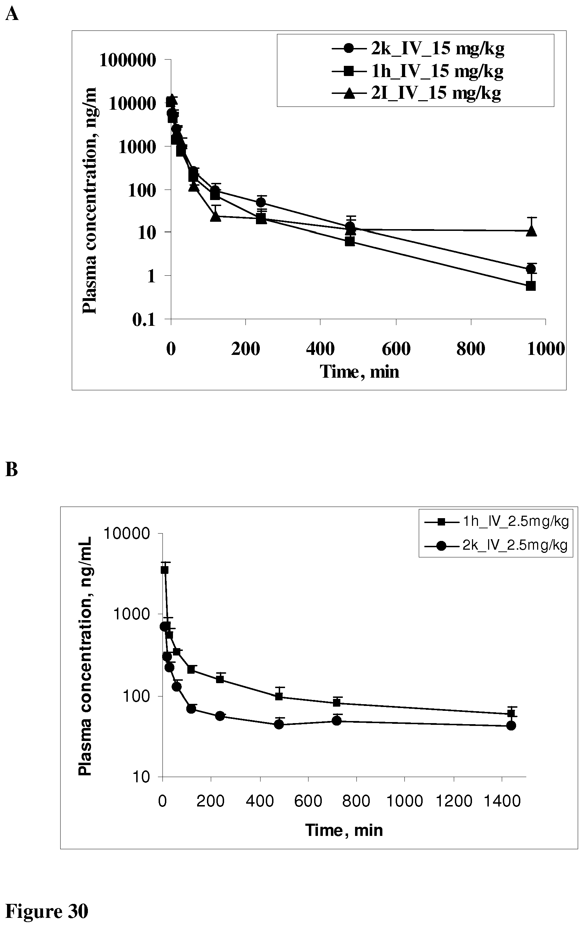

**Figure 30** depicts pharmacokinetic studies of **1h**, **2k** and **2l** administered i.p. in mice and rats. (**Figure 30A**) Concentration-time curve of SMART compounds in ICR mice (n = 3); bars, SD. SMART compounds were administrated 15 mg/kg i.v. by tail vein injection. (**Figure 30B**) Concentration-time curve of **1h** and **2k** in SD rats (n = 4); bars, SD. Spague-Dawley rats were dosed 2.5 mg/kg i.v. with the formulation DMSO/PEG300 (1/4).

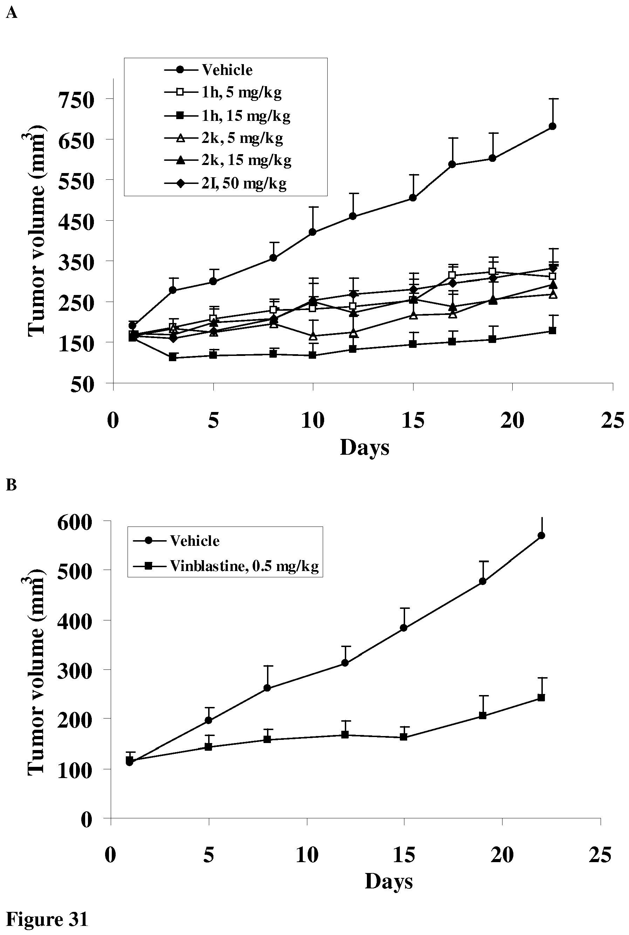

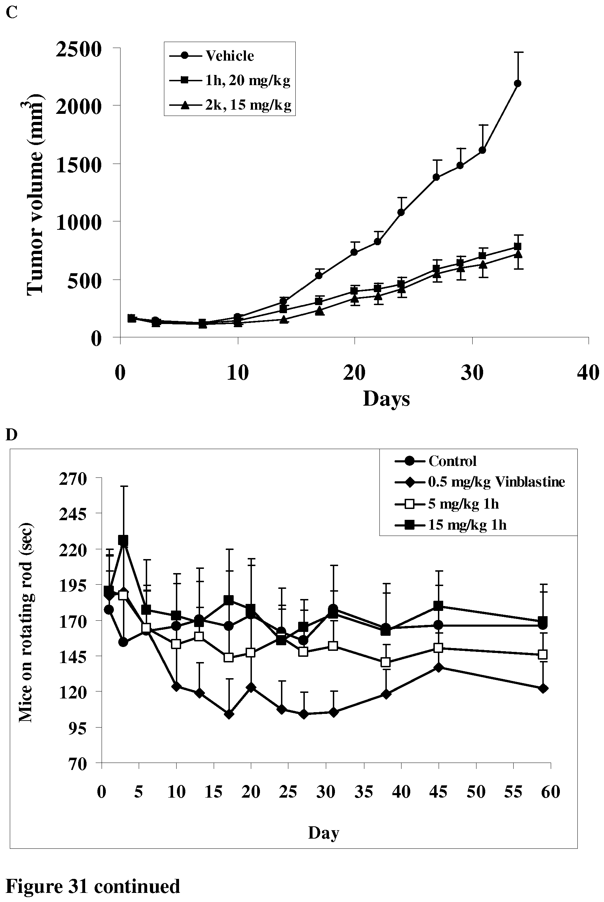

**Figure 31** presents *in vivo* anti-cancer efficacy (administered i.p.) and neurotoxicity of SMART compounds in mice. (**Figure 31A**) SMART compounds efficacy for PC-3 prostate tumor xenografted on nude mice (n = 6-8). (**Figure 31B**) Vinblastine efficacy for PC-3 prostate tumor xenografted on nude mice (n = 8). This served as the positive control. (**Figure 31C**) *In vivo* efficacy of **1h** and **2k** in nude mice bearing A375 melanoma xenografts (n = 10). Nude mice were inoculated with 2.5 x 10⁶ PC-3 or A375 cells and dosed i.p. daily (SMART compounds) and q2d (vinblastine) after tumor formation (150-200 mm³). Each point represents mean tumor volume for animals in each group. (**Figure 31D**) *In vivo* neurotoxicity (rotarod test) of **1h** in ICR mice (n = 7 or 8). **1h** (5 and 15 mg/kg), vinblastine (0.5 mg/kg) and vehicle were given i.p. daily, and vinblastine was used as the positive control. The dosing was stopped on day 31. *, *p* < 0.05. Bars, SE.

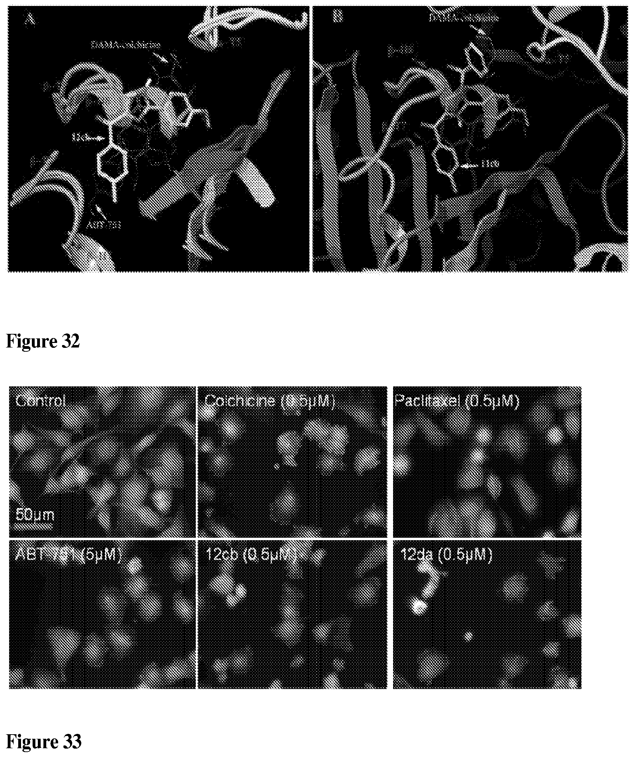

**Figure 32** depicts molecular modeling of ABI compounds that target tubulin in the colchicine binding site. **Figures 32A and 32B** depict molecular modeling of compound **12cb** and **11cb**, respectively.
**Figure 33** depicts microscopic images of immunofluorescence-labeled microtubules in WM-164 melanoma cells, which showed microtubule modality was dramatically changed after compound treatment for 18 h. This provides visual proof that ABI compounds target tubulin and disrupt functional microtubule formation.

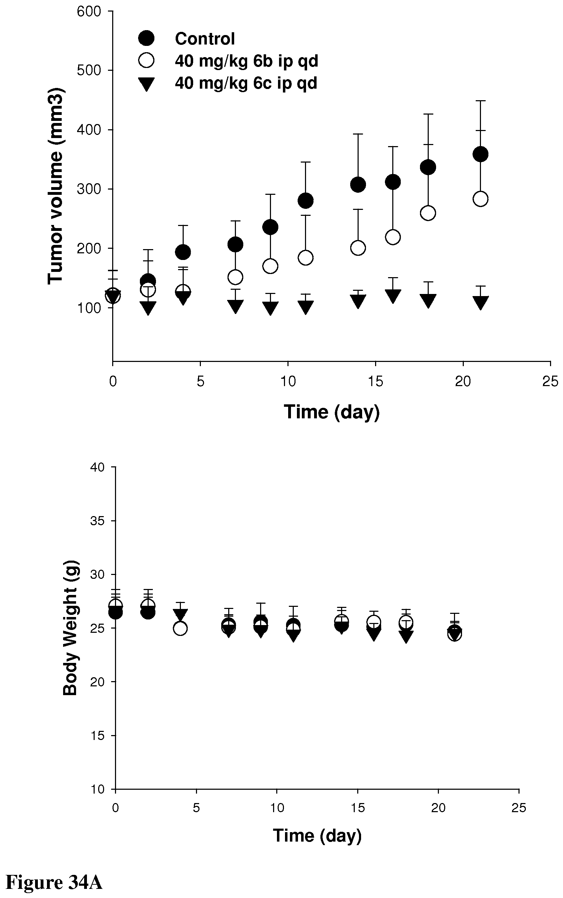

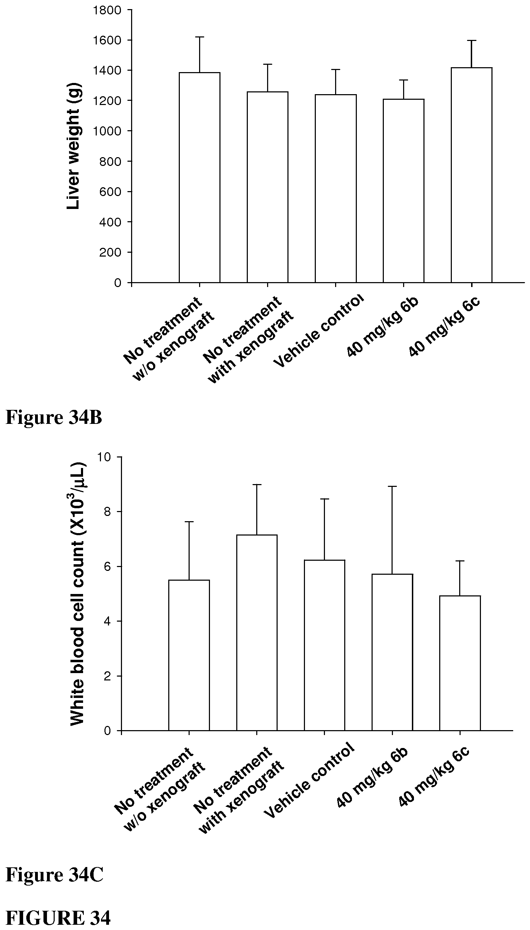

**Figure 34** depicts the efficacy and tolerability of **6b** and **6c** in xenograft models after i.p. injection. **Figure 34A****.** PC-3 xenografts were treated with vehicle (qd), **6b** (40 mg/kg, qd), or **6c** (40 mg/kg, qd) for 3 weeks. Dosing vehicles were composed of 20% Captex200 in Tween80. The tumor volumes (mm³) were plotted against time and are the means ± SD from eight animals. The tumor volumes were shown in left panel and body weights were shown in right panel. **Figure 34B****.** The liver size (g) of each nude mouse was measured after 3 weeks treatment. **Figure 34C****.** The number of white blood cells was counted in whole blood collected from animal after 3 weeks treatment.

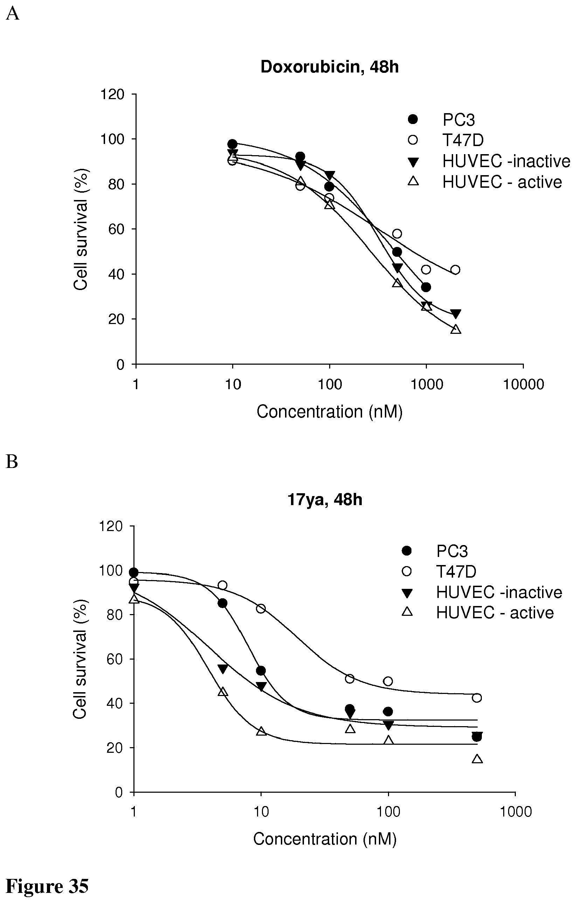

**Figure 35****- Compound 17ya showed potent endothelial cell growth inhibition.** Cell growth inhibition of doxorubicin (**Figure 35A**) and compound 17ya (**Figure 35B**) was investigated in several cell lines by SRB study. The definitions HUVEC-active and HUVEC-inactive represent growth factor-supplemented and growth factor-deprived endothelial cell cultures, respectively.

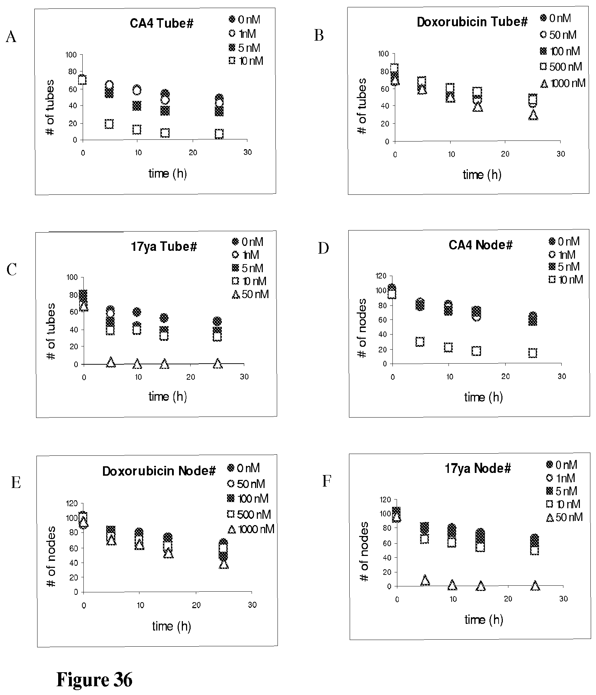

**Figure 36****- Disruption of preformed capillary by 17ya.** HUVEC cells loaded on Matrigel were allowed to make tube for 16 h and the test compound was treated to the preformed tubes. The number of tubes (A, B, and C) and nodes (D, E, and F) were counted up to 25 h after drug treatment. Panels A and D are conditions in the presence of CA4, panels B and E are conditions in the presence of doxorubicin and panels C and F are conditions in the presence of 17ya.

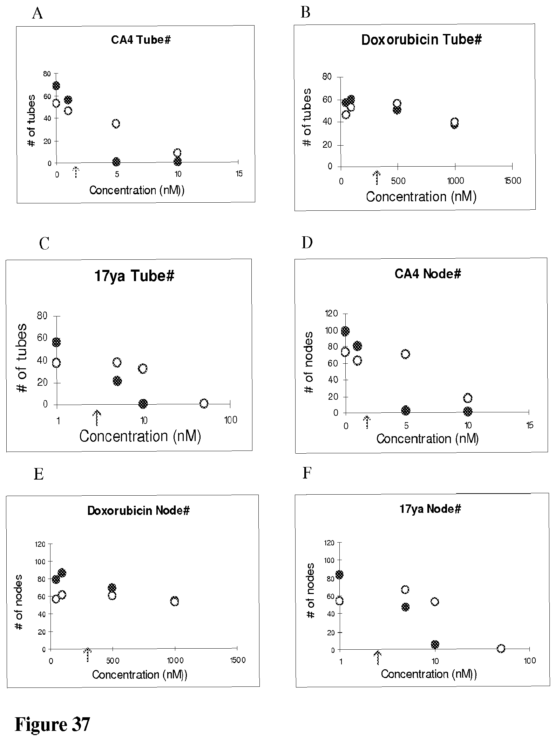

**Figure 37****- Inhibition of the endothelial capillary formation and disruption of preformed capillaries.** Inhibition of capillary formation (●) and disruption of preformed capillary (○) were compared *in vitro* study using HUVEC cells after 15 h CA4 (A and D), DOX (B and E), and 17ya (C and F) treatment. Arrow shows the IC₅₀ value of each compound in HUVEC cell growth inhibition.

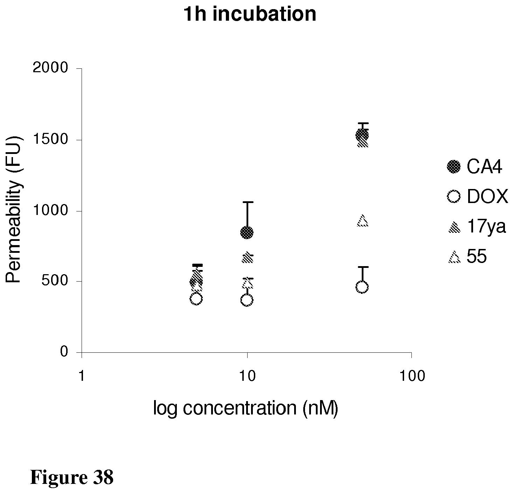

**Figure 38****- 17ya and 55 increased the permeability of endothelial cell monolayers.** Confluent HUVEC monolayers were exposed to test compound. The leakage of FITC-conjugated dextran through the monolayer was assessed by relative fluorescence measurements at λ = 485 nm excitation and λ = 530 nm emission in a receiver to determine changes in monolayer permeability following exposure.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE INVENTION

This disclosure is directed to a compound represented by the structure of formula **XI**: 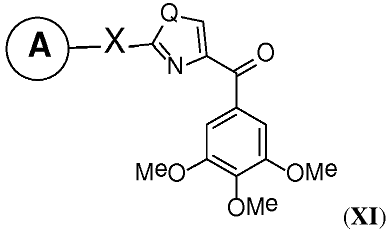 wherein
**X** is NH;
**Q** is S; and
**A** is a substituted or unsubstituted phenyl or indolyl ring; and
**i** is an integer from 0-5;
or its pharmaceutically acceptable salt, hydrate, or tautomer.

In one embodiment, **A** of compound of Formula **XI** is Ph. In another embodiment, **A** of compound of Formula **XI** is substituted Ph. In another embodiment, the substitution is 4-F. In another embodiment, the substitution is 4-Me. Non limiting examples of compounds of Formula **XI** are selected from: (2-(phenylamino)thiazol-4-yl)(3,4,5-trimethoxyphenyl)methanone **(5a),** (2-(*p*-tolylamino)thiazol-4-yl)(3,4,5-trimethoxyphenyl)methanone **(5b),** (2-(p-fluorophenylamino)thiazol-4-yl)(3,4,5-trimethoxyphenyl)methanone **(5c),** (2-(4-chlorophenylamino)thiazol-4-yl)(3,4,5-trimethoxyphenyl)methanone **(5d),** (2-(phenylamino)thiazol-4-yl)(3,4,5-trimethoxyphenyl)methanone hydrochloride salt **(5Ha),** (2-(*p*-tolylamino)thiazol-4-yl)(3,4,5-trimethoxyphenyl)methanone hydrochloride salt **(5Hb),** (2-(*p-*fluorophenylamino)thiazol-4-yl)(3,4,5-trimethoxyphenyl)methanone hydrochloride salt **(5Hc),** (2-(4-chlorophenylamino)thiazol-4-yl)(3,4,5-trimethoxyphenyl)methanone htdrochloride salt **(5Hd).**

In one embodiment, this invention is directed to a compound represented by the structure of formula **XI(c):** 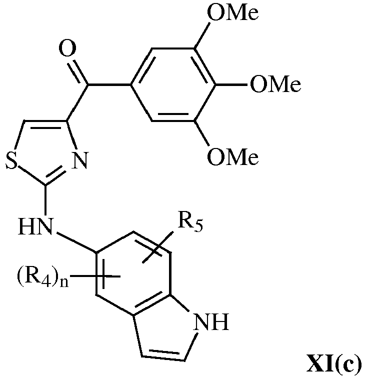
wherein **R₄** and **R₅** are independently hydrogen, O-alkyl, O-haloalkyl, F, Cl, Br, I, haloalkyl, CF₃, CN, -CH₂CN, NH₂, hydroxyl, -(CH₂)ᵢNHCH₃, -(CH₂)ᵢNH₂, - (CH₂)ᵢN(CH₃)₂, -OC(O)CF₃, C₁-C₅ linear or branched alkyl, haloalkyl, alkylamino, aminoalkyl, -OCH₂Ph, -NHCO-alkyl, COOH, -C(O)Ph, C(O)O-alkyl, C(O)H, -C(O)NH₂ or NO₂;
**i** is an integer from 0-5; and
**n** is an integer between 1-4;
or its pharmaceutically acceptable salt, hydrate, or tautomer.

In one embodiment, this invention is directed to a compound represented by the structure of formula **XI(d):** 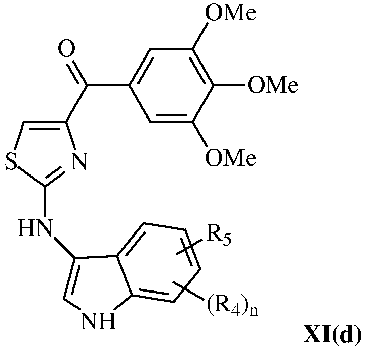
wherein **R₄** and **R₅** are independently hydrogen, O-alkyl, O-haloalkyl, F, Cl, Br, I, haloalkyl, CF₃, CN, -CH₂CN, NH₂, hydroxyl, -(CH₂)ᵢNHCH₃, -(CH₂)ᵢNH₂, - (CH₂)ᵢN(CH₃)₂, -OC(O)CF₃, C₁-C₅ linear or branched alkyl, haloalkyl, alkylamino, aminoalkyl, -OCH₂Ph, -NHCO-alkyl, COOH, -C(O)Ph, C(O)O-alkyl, C(O)H, -C(O)NH₂ or NO₂;
**i** is an integer from 0-5; and
**n** is an integer between 1-4;
or its pharmaceutically acceptable salt, hydrate, or tautomer.

In another embodiment, a compound of formula XI is represented by the structure of compound **55**:

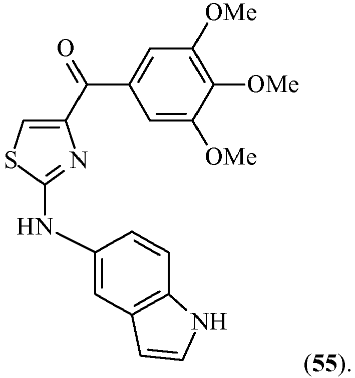

It is well understood that in structures presented in this invention wherein the nitrogen atom has less than 3 bonds, H atoms are present to complete the valence of the nitrogen.

In one embodiment, the **A** group is substituted or unsubstituted phenyl. In another embodiment, the A group is phenyl substituted by Cl, F or methyl. In one embodiment, the **A** group includes substituted or unsubstituted indolyl groups; most preferably, substituted and unsubstituted 3-indolyl and 5-indolyl.

In one embodiment, the **A** group of formula **XI** can be substituted or unsubstituted. Thus, although the exemplary groups recited in the preceding paragraph are unsubstituted, it should be appreciated by those of skill in the art that these groups can be substituted by one or more, two or more, three or more, and even up to five substituents (other than hydrogen).

In one embodiment, the most preferred **A** groups are substituted by 3,4,5-trimethoxyphenyl. In another embodiment the **A** groups are substituted by alkoxy. In another embodiment the **A** groups are substituted by methoxy. In another embodiment the **A** groups are substituted by alkyl. In another embodiment the **A** groups are substituted by methyl. In another embodiment the **A** groups are substituted by halogen. In another embodiment, the **A** groups are substituted by F. In another embodiment, the **A** groups are substituted by Cl. In another embodiment, the A rings are substituted by Br.

The substituents of these **A** groups of formula **XI** are independently selected from the group of hydrogen (e.g., no substitution at a particular position), hydroxyl, an aliphatic straight- or branched-chain C₁ to C₁₀ hydrocarbon, alkoxy, haloalkoxy, aryloxy, nitro, cyano, alkyl-CN, halo (e.g., F, Cl, Br, I), haloalkyl, dihaloalkyl, trihaloalkyl, COOH, C(O)Ph, C(O)-alkyl, C(O)O-alkyl, C(O)H, C(O)NH₂, -OC(O)CF₃, OCH₂Ph, amino, aminoalkyl, alkylamino, mesylamino, dialkylamino, arylamino, amido, NHC(O)-alkyl, urea, alkyl-urea, alkylamido (e.g., acetamide), haloalkylamido, arylamido, aryl, and C₅ to C₇ cycloalkyl, arylalkyl, and combinations thereof. Single substituents can be present at the *ortho, meta,* or *para* positions. When two or more substituents are present, one of them is preferably, though not necessarily, at the *para* position.

In one embodiment, R₄, R₅ and R₆ of formula VIII, XI(c), or XI(d) are independently hydrogen. In another embodiment, R₄, R₅ and R₆ are independently O-alkyl. In another embodiment, R₄, R₅ and R₆ are independently O-haloalkyl. In another embodiment, R₄, R₅ and R₆ are independently F. In another embodiment, R₄, R₅ and R₆ are independently Cl. In another embodiment, R₄, R₅ and R₆ are independently Br. In another embodiment, R₄, R₅ and R₆ are independently I. In another embodiment, R₄, R₅ and R₆ are independently haloalkyl. In another embodiment, R₄, R₅ and R₆ are independently CF₃. In another embodiment, R₄, R₅ and R₆ are independently CN. In another embodiment, R₄, R₅ and R₆ are independently -CH₂CN. In another embodiment, R₄, R₅ and R₆ are independently NH₂. In another embodiment, R₄, R₅ and R₆ are independently hydroxyl. In another embodiment, R₄, R₅ and R₆ are independently -(CH₂)ᵢNHCH₃. In another embodiment, R₄, R₅ and R₆ are independently -(CH₂)ᵢNH₂. In another embodiment, R₄, R₅ and R₆ are independently - (CH₂)ᵢN(CH₃)₂. In another embodiment, R₄, R₅ and R₆ are independently -OC(O)CF₃. In another embodiment, R₄, R₅ and R₆ are independently C₁-C₅ linear or branched alkyl. In another embodiment, R₄, R₅ and R₆ are independently haloalkyl. In another embodiment, R₄, R₅ and R₆ are independently alkylamino. In another embodiment, R₄, R₅ and R₆ are independently aminoalkyl. In another embodiment, R₄, R₅ and R₆ are independently - OCH₂Ph. In another embodiment, R₄, R₅ and R₆ are independently -NHCO-alkyl. In another embodiment, R₄, R₅ and R₆ are independently COOH. In another embodiment, R₄, R₅ and R₆ are independently -C(O)Ph. In another embodiment, R₄, R₅ and R₆ are independently C(O)O-alkyl. In another embodiment, R₄, R₅ and R₆ are independently C(O)H. In another embodiment, R₄, R₅ and R₆ are independently -C(O)NH₂. In another embodiment, R₄, R₅ and R₆ are independently NO₂.

As used herein, "single-, fused- or multiple-ring, aryl or (hetero)cyclic ring systems" can be any such ring, including but not limited to phenyl, biphenyl, triphenyl, naphthyl, cycloalkyl, cycloalkenyl, cyclodienyl, fluorene, adamantane, etc.

"Saturated or unsaturated *N*-heterocycles" can be any such N-containing heterocycle, including but not limited to aza- and diaza-cycloalkyls such as aziridinyl, azetidinyl, diazatidinyl, pyrrolidinyl, piperidinyl, piperazinyl, and azocanyl, pyrrolyl, pyrazolyl, imidazolyl, pyridinyl, pyrimidinyl, pyrazinyl, pyridazinyl, triazinyl, tetrazinyl, pyrrolizinyl, indolyl, quinolinyl, isoquinolinyl, benzimidazolyl, indazolyl, quinolizinyl, cinnolinyl, quinololinyl, phthalazinyl, naphthyridinyl, quinoxalinyl, etc.

"Saturated or unsaturated *O*-Heterocycles" can be any such O-containing heterocycle including but not limited to oxiranyl, oxetanyl, tetrahydrofuranyl, tetrahydropyranyl, dioxanyl, furanyl, pyrylium, benzofuranyl, benzodioxolyl, etc.

"Saturated or unsaturated *S*-heterocycles" can be any such S-containing heterocycle, including but not limited to thiranyl, thietanyl, tetrahydrothiophene-yl, dithiolanyl, tetrahydrothiopyranyl, thiophene-yl, thiepinyl, thianaphthenyl, etc.

"Saturated or unsaturated mixed heterocycles" can be any heterocycle containing two or more S-, N-, or O-heteroatoms, including but not limited to oxathiolanyl, morpholinyl, thioxanyl, thiazolyl, isothiazolyl, thiadiazolyl, oxazolyl, isoxazolyl, oxadiaziolyl, etc.

As used herein, "aliphatic straight- or branched-chain hydrocarbon" refers to both alkylene groups that contain a single carbon and up to a defined upper limit, as well as alkenyl groups and alkynyl groups that contain two carbons up to the upper limit, whether the carbons are present in a single chain or a branched chain. Unless specifically identified, a hydrocarbon can include up to about 30 carbons, or up to about 20 hydrocarbons, or up to about 10 hydrocarbons. Alkenyl and alkynyl groups can be mono-unsaturated or polyunsaturated. In another embodiment, an alkyl includes C₁-C₆ carbons. In another embodiment, an alkyl includes C₁-C₈ carbons. In another embodiment, an alkyl includes C₁-C₁₀ carbons. In another embodiment, an alkyl is a C₁-C₁₂ carbons. In another embodiment, an alkyl is a C₁-C₅ carbons.

As used herein, the term "alkyl" can be any straight- or branched-chain alkyl group containing up to about 30 carbons unless otherwise specified. In another embodiment, an alkyl includes C₁-C₆ carbons. In another embodiment, an alkyl includes C₁-C₈ carbons. In another embodiment, an alkyl includes C₁-C₁₀ carbons. In another embodiment, an alkyl is a C₁-C₁₂ carbons. In another embodiment, an alkyl is a C₁-C₂₀ carbons. In another embodiment, cyclic alkyl group has 3-8 carbons. In another embodiment, branched alkyl is an alkyl substituted by alkyl side chains of 1 to 5 carbons.

Preferred alkyl groups are methyl, ethyl, and propyl.

As used herein, the term "aryl" refers to any aromatic ring that is directly bonded to another group. Exemplary aryl groups include, without limitation, phenyl, tolyl, xylyl, furanyl, naphthyl, pyridinyl, pyrimidinyl, pyridazinyl, pyrazinyl, triazinyl, thiazolyl, oxazolyl, isooxazolyl, pyrazolyl, imidazolyl, thiophene-yl, pyrrolyl, phenylmethyl, phenylethyl, phenylamino, phenylamido, etc.

As used herein, the term "aminoalkyl" refers to an amine group substituted by an alkyl group as defined above. Aminoalkyl refers to monoalkylamine, dialkylamine or trialkylamine. Nonlimiting examples of aminoalkyl groups are -N(Me)₂, -NHMe, -NH₃.

A "haloalkyl" group refers, in another embodiment, to an alkyl group as defined above, which is substituted by one or more halogen atoms, e.g. by F, Cl, Br or I. Nonlimiting examples of haloalkyl groups are CF₃, CF₂CF₃, CH₂CF₃.

In one embodiment, this invention provides a compound of this invention or its, pharmaceutically acceptable salt, pharmaceutical product, tautomer, hydrate, *N*-oxide, or crystal or combinations thereof. In another embodiment, this invention provides a pharmaceutically acceptable salt of the compound of this invention. In another embodiment, this invention provides a pharmaceutical product of the compound of this invention. In another embodiment, this invention provides a tautomer of the compound of this invention. In another embodiment, this invention provides a hydrate of the compound of this invention. In another embodiment, this invention provides an *N*-oxide of the compound of this invention. In another embodiment, this invention provides a crystal of the compound of this invention. In another embodiment, this invention provides composition comprising a compound of this invention, as described herein, or, in another embodiment, a combination of a pharmaceutically acceptable salt, pharmaceutical product, tautomer,hydrate, *N*-oxide, or crystal of the compound of this invention.

Compounds of the present invention can also be in the form of a hydrate, which means that the compound further includes a stoichiometric or non-stoichiometric amount of water bound by non-covalent intermolecular forces.

Compounds of the present invention may exist in the form of one or more of the possible tautomers and depending on the particular conditions it may be possible to separate some or all of the tautomers into individual and distinct entities. It is to be understood that all of the possible tautomers, including all additional enol and keto tautomers. For example the following tautomers, but not limited to these, are included.

### Tautomerization of the imidazole ring

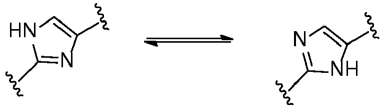

The invention includes "pharmaceutically acceptable salts" of the compounds of this invention, which may be produced, by reaction of a compound of this invention with an acid or base. Certain compounds, particularly those possessing acid or basic groups, can also be in the form of a salt, preferably a pharmaceutically acceptable salt. The term "pharmaceutically acceptable salt" refers to those salts that retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxylic acid, maleic acid, malonic acid, succinic acid, fumaric acid, tartaric acid, citric acid, benzoic acid, cinnamic acid, mandelic acid, methanesulfonic acid, ethanesulfonic acid, *p-*toluenesulfonic acid, salicylic acid, *N*-acetylcysteine and the like. Other salts are known to those of skill in the art and can readily be adapted for use in accordance with the present invention.

Suitable pharmaceutically-acceptable salts of amines of compounds of this invention may be prepared from an inorganic acid or from an organic acid. In one embodiment, examples of inorganic salts of amines are bisulfates, borates, bromides, chlorides, hemisulfates, hydrobromates, hydrochlorates, 2-hydroxyethylsulfonates (hydroxyethanesulfonates), iodates, iodides, isothionates, nitrates, persulfates, phosphate, sulfates, sulfamates, sulfanilates, sulfonic acids (alkylsulfonates, arylsulfonates, halogen substituted alkylsulfonates, halogen substituted arylsulfonates), sulfonates and thiocyanates.

In one embodiment, examples of organic salts of amines may be selected from aliphatic, cycloaliphatic, aromatic, araliphatic, heterocyclic, carboxylic and sulfonic classes of organic acids, examples of which are acetates, arginines, aspartates, ascorbates, adipates, anthranilates, algenates, alkane carboxylates, substituted alkane carboxylates, alginates, benzenesulfonates, benzoates, bisulfates, butyrates, bicarbonates, bitartrates, citrates, camphorates, camphorsulfonates, cyclohexylsulfamates, cyclopentanepropionates, calcium edetates, camsylates, carbonates, clavulanates, cinnamates, dicarboxylates, digluconates, dodecylsulfonates, dihydrochlorides, decanoates, enanthuates, ethanesulfonates, edetates, edisylates, estolates, esylates, fumarates, formates, fluorides, galacturonates gluconates, glutamates, glycolates, glucorate, glucoheptanoates, glycerophosphates, gluceptates, glycollylarsanilates, glutarates, glutamate, heptanoates, hexanoates, hydroxymaleates, hydroxycarboxlic acids, hexylresorcinates, hydroxybenzoates, hydroxynaphthoates, hydrofluorates, lactates, lactobionates, laurates, malates, maleates, methylenebis(beta-oxynaphthoate), malonates, mandelates, mesylates, methane sulfonates, methylbromides, methylnitrates, methylsulfonates, monopotassium maleates, mucates, monocarboxylates, naphthalenesulfonates, 2-naphthalenesulfonates, nicotinates, nitrates, napsylates, *N*-methylglucamines, oxalates, octanoates, oleates, pamoates, phenylacetates, picrates, phenylbenzoates, pivalates, propionates, phthalates, phenylacetate, pectinates, phenylpropionates, palmitates, pantothenates, polygalacturates, pyruvates, quinates, salicylates, succinates, stearates, sulfanilate, subacetates, tartrates, theophyllineacetates, *p*-toluenesulfonates (tosylates), trifluoroacetates, terephthalates, tannates, teoclates, trihaloacetates, triethiodide, tricarboxylates, undecanoates and valerates.

In one embodiment, examples of inorganic salts of carboxylic acids or hydroxyls may be selected from ammonium, alkali metals to include lithium, sodium, potassium, cesium; alkaline earth metals to include calcium, magnesium, aluminium; zinc, barium, cholines, quaternary ammoniums.

In another embodiment, examples of organic salts of carboxylic acids or hydroxyl may be selected from arginine, organic amines to include aliphatic organic amines, alicyclic organic amines, aromatic organic amines, benzathines, *t*-butylamines, benethamines (*N*-benzylphenethylamine), dicyclohexylamines, dimethylamines, diethanolamines, ethanolamines, ethylenediamines, hydrabamines, imidazoles, lysines, methylamines, meglamines, *N*-methyl-*D*-glucamines, *N*,*N*'-dibenzylethylenediamines, nicotinamides, organic amines, ornithines, pyridines, picolies, piperazines, procain, tris(hydroxymethyl)methylamines, triethylamines, triethanolamines, trimethylamines, tromethamines and ureas.

In one embodiment, the salts may be formed by conventional means, such as by reacting the free base or free acid form of the product with one or more equivalents of the appropriate acid or base in a solvent or medium in which the salt is insoluble or in a solvent such as water, which is removed in vacuo or by freeze drying or by exchanging the ions of a existing salt for another ion or suitable ion-exchange resin.

Also disclosed herein is a process for the preparation of the compounds of this invention. In one embodiment, the aryl-imidazole is prepared by reacting an appropriately substituted benzaldehyde with ethylenediamine to construct the imidazoline ring, followed by oxidation of the imidazoline by an oxidizing agent to the corresponding imidazole. In another embodiment the oxidizing agent is diacetoxyiodobenzene, bromotrichloromethane and 1,8-diazabicyclo[5.4.0]undec-7-ene (DBU), carbon-O₂ system or palladium-carbon system. In another embodiment, the aryl-imidazole is prepared by reacting an appropriately substituted benzaldehyde with ethylene diamine in the presence of iodine and potassium carbonate in order to construct the imidazoline ring, followed by oxidation of the imidazoline ring catalyzed by diacetoxyiodobenzene, bromotrichloromethane and 1,8-diazabicyclo[5.4.0]undec-7-ene (DBU), carbon-O₂ system or palladium-carbon system to the corresponding imidazole. In another embodiment, the aryl-imidazole is prepared by reacting an appropriately substituted benzaldehyde with ethylene diamine in the presence of iodine and potassium carbonate in order to construct the imidazoline ring, followed by oxidation of the imidazoline ring catalyzed by diacetoxyiodobenzene to the corresponding imidazole. In another embodiment, the aryl-imidazole is prepared by reacting an appropriately substituted benzaldehyde with ethylene diamine in the presence of iodine and potassium carbonate in order to construct the imidazoline ring, followed by oxidation of the imidazoline ring catalyzed by bromotrichloromethane and 1,8-diazabicyclo[5.4.0]undec-7-ene (DBU) to the corresponding imidazole. In one embodiment, the aryl-imidazole is prepared by reacting the appropriate benzaldehyde in ethanol with oxalaldehyde and ammonia hydroxide to construct the imidazole ring system.

As disclosed herein an aryl-benzoyl-imidazole compound may be prepared by protecting the aryl-imidazole followed by coupling with an appropriately substituted benzoyl chloride, followed by removing the protecting group. In another embodiment, the protecting group is a phenyl sulfonyl group, phthalimide, di-*tert*-butyl dicarbonate (Boc), fluorenylmethyloxycarbonyl (Fmoc), benzyloxycarbonyl (Cbz), or monomethoxytrityl (MMT). In another embodiment, the aryl-imidazole is protected with phenyl sulfonyl to yield the *N*-sulfonyl protected aryl-imidazole. In another embodiment, the protected aryl-imidazole compound is prepared by reacting the aryl-imidazole with phenylsulfonyl chloride and sodium hydride in THF. In another embodiment, the protected aryl-imidazole is prepared according to Figures 7 and 8.

As disclosed herein, the protected aryl-imidazole may be coupled with an appropriately substituted benzoyl chloride to obtain a protected aryl-benzoyl imidazole. In another embodiment, aryl-imidazole is coupled with an appropriately substituted benzoyl chloride in the presence of *tert-butyl* lithium to obtain aryl-phenylsulfonyl (2-aryl-1-(phenylsulfonyl)-1*H*-imidazol-4-yl)methanone. In another embodiment, the (2-aryl-1-(phenylsulfonyl)-1*H*-imidazol-4-yl)methanone is prepared according to Figures 7 and 8 steps e and c, respectively.

As disclosed herein, an aryl-benzoyl-imidazole may be prepared by removing the protecting group of the aryl-benzoyl-imidazole. In another embodiment, the removal of the protecting group depends on the protecting group used and can be removed by known conditions which are known in the art. In another embodiment, the phenyl sulfonyl protecting group is removed by tetrabutylammonium fluoride in THF. In another embodiment, phenylsulfonyl is removed according to Figures 7 and 8.

In one embodiment, reference compounds are prepared according to Figures 1, 2 and 5. In another embodiment, compounds of formula XI are prepared according to Figure 6.

In another embodiment, reference compounds are prepared according to Figure 12. In another embodiment, reference compounds are prepared according to Figure 13. In another embodiment, reference compounds are prepared according to Figure 14. In another embodiment, compounds of formula **XI and XIc** are prepared according to Figure 15.

### Pharmaceutical composition

Another aspect of the present invention relates to a pharmaceutical composition including a pharmaceutically acceptable carrier and a compound according to the aspects of the present invention. The pharmaceutical composition can contain one or more of the above-identified compounds of the present invention. Typically, the pharmaceutical composition of the present invention will include a compound of the present invention or its pharmaceutically acceptable salt, as well as a pharmaceutically acceptable carrier. The term "pharmaceutically acceptable carrier" refers to any suitable adjuvants, carriers, excipients, or stabilizers, and can be in solid or liquid form such as, tablets, capsules, powders, solutions, suspensions, or emulsions.

Typically, the composition will contain from about 0.01 to 99 percent, preferably from about 20 to 75 percent of active compound(s), together with the adjuvants, carriers and/or excipients. While individual needs may vary, determination of optimal ranges of effective amounts of each component is within the skill of the art. Typical dosages comprise about 0.01 to about 100 mg/kg body wt. The preferred dosages comprise about 0.1 to about 100 mg/kg body wt. The most preferred dosages comprise about 1 to about 100 mg/kg body wt. Treatment regimen for the administration of the compounds of the present invention can also be determined readily by those with ordinary skill in art. That is, the frequency of administration and size of the dose can be established by routine optimization, preferably while minimizing any side effects.

The solid unit dosage forms can be of the conventional type. The solid form can be a capsule and the like, such as an ordinary gelatin type containing the compounds of the present invention and a carrier, for example, lubricants and inert fillers such as, lactose, sucrose, or cornstarch. In another embodiment, these compounds are tabulated with conventional tablet bases such as lactose, sucrose, or cornstarch in combination with binders like acacia, cornstarch, or gelatin, disintegrating agents, such as cornstarch, potato starch, or alginic acid, and a lubricant, like stearic acid or magnesium stearate.

The tablets, capsules, and the like can also contain a binder such as gum tragacanth, acacia, corn starch, or gelatin; excipients such as dicalcium phosphate; a disintegrating agent such as corn starch, potato starch, alginic acid; a lubricant such as magnesium stearate; and a sweetening agent such as sucrose, lactose, or saccharin. When the dosage unit form is a capsule, it can contain, in addition to materials of the above type, a liquid carrier such as a fatty oil.

Various other materials may be present as coatings or to modify the physical form of the dosage unit. For instance, tablets can be coated with shellac, sugar, or both. A syrup can contain, in addition to active ingredient, sucrose as a sweetening agent, methyl and propylparabens as preservatives, a dye, and flavoring such as cherry or orange flavor.

For oral therapeutic administration, these active compounds can be incorporated with excipients and used in the form of tablets, capsules, elixirs, suspensions, syrups, and the like. Such compositions and preparations should contain at least 0.1% of active compound. The percentage of the compound in these compositions can, of course, be varied and can conveniently be between about 2% to about 60% of the weight of the unit. The amount of active compound in such therapeutically useful compositions is such that a suitable dosage will be obtained. Preferred compositions according to the present invention are prepared so that an oral dosage unit contains between about 1 mg and 800 mg of active compound.

The active compounds of the present invention may be orally administered, for example, with an inert diluent, or with an assimilable edible carrier, or they can be enclosed in hard or soft shell capsules, or they can be compressed into tablets, or they can be incorporated directly with the food of the diet.

The pharmaceutical forms suitable for injectable use include sterile aqueous solutions or dispersions and sterile powders for the extemporaneous preparation of sterile injectable solutions or dispersions. In all cases, the form should be sterile and should be fluid to the extent that easy syringability exists. It should be stable under the conditions of manufacture and storage and should be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol), suitable mixtures thereof, and vegetable oils.

The compounds or pharmaceutical compositions of the present invention may also be administered in injectable dosages by solution or suspension of these materials in a physiologically acceptable diluent with a pharmaceutical adjuvant, carrier or excipient. Such adjuvants, carriers and/or excipients include, but are not limited to, sterile liquids, such as water and oils, with or without the addition of a surfactant and other pharmaceutically and physiologically acceptable components. Illustrative oils are those of petroleum, animal, vegetable, or synthetic origin, for example, peanut oil, soybean oil, or mineral oil. In general, water, saline, aqueous dextrose and related sugar solution, and glycols, such as propylene glycol or polyethylene glycol, are preferred liquid carriers, particularly for injectable solutions.

These active compounds may also be administered parenterally. Solutions or suspensions of these active compounds can be prepared in water suitably mixed with a surfactant such as hydroxypropylcellulose. Dispersions can also be prepared in glycerol, liquid polyethylene glycols, and mixtures thereof in oils. Illustrative oils are those of petroleum, animal, vegetable, or synthetic origin, for example, peanut oil, soybean oil, or mineral oil. In general, water, saline, aqueous dextrose and related sugar solution, and glycols such as, propylene glycol or polyethylene glycol, are preferred liquid carriers, particularly for injectable solutions. Under ordinary conditions of storage and use, these preparations contain a preservative to prevent the growth of microorganisms.

For use as aerosols, the compounds of the present invention in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

In one embodiment, the compounds of this invention are administered in combination with an anti-cancer agent. In one embodiment, the anti-cancer agent is a monoclonal antibody. In some embodiments, the monoclonal antibodies are used for diagnosis, monitoring, or treatment of cancer. In one embodiment, monoclonal antibodies react against specific antigens on cancer cells. In one embodiment, the monoclonal antibody acts as a cancer cell receptor antagonist. In one embodiment, monoclonal antibodies enhance the patient's immune response. In one embodiment, monoclonal antibodies act against cell growth factors, thus blocking cancer cell growth. In one embodiment, anti-cancer monoclonal antibodies are conjugated or linked to anti-cancer drugs, radioisotopes, other biologic response modifiers, other toxins, or a combination thereof. In one embodiment, anti-cancer monoclonal antibodies are conjugated or linked to a compound of this invention as described hereinabove.

Yet another aspect of the present invention relates to a pharmaceutical composition comprising a compound according to the present invention and a pharmaceutically acceptable carrier for use in treating cancer in a subject in need of treatment for cancer.

When administering the compounds of the present invention, they can be administered systemically or, alternatively, they can be administered directly to a specific site where cancer cells or precancerous cells are present. Thus, administering can be accomplished in any manner effective for delivering the compounds or the pharmaceutical compositions to the cancer cells or precancerous cells. Exemplary modes of administration include, without limitation, administering the compounds or compositions orally, topically, transdermally, parenterally, subcutaneously, intravenously, intramuscularly, intraperitoneally, by intranasal instillation, by intracavitary or intravesical instillation, intraocularly, intraarterially, intralesionally, or by application to mucous membranes, such as, that of the nose, throat, and bronchial tubes.

### Biological Activity

In one embodiment, the invention provides compounds and compositions, including any embodiment described herein, for use in any of the medical uses of this invention. In one embodiment, use of a compound of this invention or a composition comprising the same, will have utility in inhibiting, suppressing, enhancing or stimulating a desired response in a subject, as will be understood by one skilled in the art. In another embodiment, the compositions may further comprise additional active ingredients, whose activity is useful for the particular application for which the compound of this invention is being administered.

In one embodiment, this invention is directed to a compound of this invention for use in treating, suppressing, reducing the severity, reducing the risk of developing or inhibiting cancer in a subject suffering from cancer.

Drug resistance is the major cause of cancer chemotherapy failure. One major contributor to multidrug resistance is overexpression of P-glycoprotein (P-gp). This protein is a clinically important transporter protein belonging to the ATP-binding cassette family of cell membrane transporters. It can pump substrates including anticancer drugs out of tumor cells through an ATP-dependent mechanism.

In one embodiment, this invention provides compounds of the invention and/or a pharmaceutically acceptable salt, pharmaceutical product, tautomer, hydrate, or *N*-oxide of said compound, or any combination thereof for use in: a) treating, suppressing, reducing the severity, reducing the risk, or inhibiting drug resistant tumors; b) treating, suppressing, reducing the severity, reducing the risk, or inhibiting metastatic cancer; c) treating, suppressing, reducing the severity, reducing the risk, or inhibiting drug resistant cancer; d) treating, suppressing, reducing the severity, reducing the risk, or inhibiting a drug resistant cancer wherein the cancer is melanoma; e) treating, suppressing, reducing the severity, reducing the risk, or inhibiting a drug resistant cancer wherein the cancer is prostate cancer; f) treating, suppressing, reducing the severity, reducing the risk, or inhibiting metastatic melanoma; g) treating, suppressing, reducing the severity, reducing the risk, or inhibiting prostate cancer; h) treating, suppressing, reducing the severity, reducing the risk, or inhibiting cancer in a subject, wherein the subject has been previously treated with chemotherapy, radiotherapy, or biological therapy.

The compounds of the present invention are useful in the treatment, reducing the severity, reducing the risk, or inhibition of cancer, metastatic cancer, drug resistant tumors, drug resistant cancer and various forms of cancer. In a preferred embodiment the cancer is prostate cancer, breast cancer, ovarian cancer, skin cancer (e.g., melanoma), lung cancer, colon cancer, leukemia, lymphoma, head and neck, pancreatic, esophageal, renal cancer or CNS cancer (e.g., glioma, glioblastoma). Treatment of these different cancers is supported by the Examples herein. Moreover, based upon their believed mode of action as tubulin inhibitors, it is believed that other forms of cancer will likewise be treatable or preventable upon administration of the compounds or compositions of the present invention to a patient. Preferred compounds of the present invention are selectively disruptive to cancer cells, causing ablation of cancer cells but preferably not normal cells. Significantly, harm to normal cells is minimized because the cancer cells are susceptible to disruption at much lower concentrations of the compounds of the present invention.

In some embodiments, this invention provides for a compound of the invention as herein described, or its pharmaceutically acceptable salt, pharmaceutical product, tautomer, crystal, *N*-oxide, hydrate or any combination thereof, for use in treating, suppressing, reducing the severity, reducing the risk, or inhibiting cancer in a subject. In another embodiment, the cancer is adrenocortical carcinoma, anal cancer, bladder cancer, brain tumor, brain stem tumor, breast cancer, glioma, cerebellar astrocytoma, cerebral astrocytoma, ependymoma, medulloblastoma, supratentorial primitive neuroectodermal, pineal tumors, hypothalamic glioma, breast cancer, carcinoid tumor, carcinoma, cervical cancer, colon cancer, central nervous system (CNS) cancer, endometrial cancer, esophageal cancer, extrahepatic bile duct cancer, Ewing's family of tumors (Pnet), extracranial germ cell tumor, eye cancer, intraocular melanoma, gallbladder cancer, gastric cancer, germ cell tumor, extragonadal, gestational trophoblastic tumor, head and neck cancer, hypopharyngeal cancer, islet cell carcinoma, laryngeal cancer, leukemia, acute lymphoblastic, leukemia, oral cavity cancer, liver cancer, lung cancer, non-small cell lung cancer, small cell, lymphoma, AIDS-related lymphoma, central nervous system (primary), lymphoma, cutaneous T-cell, lymphoma, Hodgkin's disease, non-Hodgkin's disease, malignant mesothelioma, melanoma, Merkel cell carcinoma, metasatic squamous carcinoma, multiple myeloma, plasma cell neoplasms, mycosis fungoides, myelodysplastic syndrome, myeloproliferative disorders, nasopharyngeal cancer, neuroblastoma, oropharyngeal cancer, osteosarcoma, ovarian cancer, ovarian epithelial cancer, ovarian germ cell tumor, ovarian low malignant potential tumor, pancreatic cancer, exocrine, pancreatic cancer, islet cell carcinoma, paranasal sinus and nasal cavity cancer, parathyroid cancer, penile cancer, pheochromocytoma cancer, pituitary cancer, plasma cell neoplasm, prostate cancer, rhabdomyosarcoma, rectal cancer, renal cancer, renal cell cancer, salivary gland cancer, Sezary syndrome, skin cancer, cutaneous T-cell lymphoma, skin cancer, Kaposi's sarcoma, skin cancer, melanoma, small intestine cancer, soft tissue sarcoma, soft tissue sarcoma, testicular cancer, thymoma, malignant, thyroid cancer, urethral cancer, uterine cancer, sarcoma, unusual cancer of childhood, vaginal cancer, vulvar cancer, Wilms' tumor, or any combination thereof. In another embodiment the subject has been previously treated with chemotherapy, radiotherapy or biological therapy.

In some embodiments, this invention provides for a compound of the invention as herein described, or its pharmaceutically acceptable salt, pharmaceutical product, tautomer, crystal, *N*-oxide, hydrate or any combination thereof, for use in treating, suppressing, reducing the severity, reducing the risk, or inhibiting a metastatic cancer in a subject. In another embodiment, the cancer is adrenocortical carcinoma, anal cancer, bladder cancer, brain tumor, brain stem tumor, breast cancer, glioma, cerebellar astrocytoma, cerebral astrocytoma, ependymoma, medulloblastoma, supratentorial primitive neuroectodermal, pineal tumors, hypothalamic glioma, breast cancer, carcinoid tumor, carcinoma, cervical cancer, colon cancer, central nervous system (CNS) cancer, endometrial cancer, esophageal cancer, extrahepatic bile duct cancer, Ewing's family of tumors (Pnet), extracranial germ cell tumor, eye cancer, intraocular melanoma, gallbladder cancer, gastric cancer, germ cell tumor, extragonadal, gestational trophoblastic tumor, head and neck cancer, hypopharyngeal cancer, islet cell carcinoma, laryngeal cancer, leukemia, acute lymphoblastic, leukemia, oral cavity cancer, liver cancer, lung cancer, non-small cell lung cancer, small cell, lymphoma, AIDS-related lymphoma, central nervous system (primary), lymphoma, cutaneous T-cell, lymphoma, Hodgkin's disease, non-Hodgkin's disease, malignant mesothelioma, melanoma, Merkel cell carcinoma, metasatic squamous carcinoma, multiple myeloma, plasma cell neoplasms, mycosis fungoides, myelodysplastic syndrome, myeloproliferative disorders, nasopharyngeal cancer, neuroblastoma, oropharyngeal cancer, osteosarcoma, ovarian cancer, ovarian epithelial cancer, ovarian germ cell tumor, ovarian low malignant potential tumor, pancreatic cancer, exocrine, pancreatic cancer, islet cell carcinoma, paranasal sinus and nasal cavity cancer, parathyroid cancer, penile cancer, pheochromocytoma cancer, pituitary cancer, plasma cell neoplasm, prostate cancer, rhabdomyosarcoma, rectal cancer, renal cancer, renal cell cancer, salivary gland cancer, Sezary syndrome, skin cancer, cutaneous T-cell lymphoma, skin cancer, Kaposi's sarcoma, skin cancer, melanoma, small intestine cancer, soft tissue sarcoma, soft tissue sarcoma, testicular cancer, thymoma, malignant, thyroid cancer, urethral cancer, uterine cancer, sarcoma, unusual cancer of childhood, vaginal cancer, vulvar cancer, Wilms' tumor, or any combination thereof.

In some embodiments, this invention provides for a compound of the invention as herein described, or its pharmaceutically acceptable salt, pharmaceutical product, tautomer, crystal, *N*-oxide, hydrate or any combination thereof, for use in treating, suppressing, reducing the severity, reducing the risk, or inhibiting a drug-resistant cancer or resistant cancer in a subject. In another embodiment, the cancer is adrenocortical carcinoma, anal cancer, bladder cancer, brain tumor, brain stem tumor, breast cancer, glioma, cerebellar astrocytoma, cerebral astrocytoma, ependymoma, medulloblastoma, supratentorial primitive neuroectodermal, pineal tumors, hypothalamic glioma, breast cancer, carcinoid tumor, carcinoma, cervical cancer, colon cancer, central nervous system (CNS) cancer, endometrial cancer, esophageal cancer, extrahepatic bile duct cancer, Ewing's family of tumors (Pnet), extracranial germ cell tumor, eye cancer, intraocular melanoma, gallbladder cancer, gastric cancer, germ cell tumor, extragonadal, gestational trophoblastic tumor, head and neck cancer, hypopharyngeal cancer, islet cell carcinoma, laryngeal cancer, leukemia, acute lymphoblastic, leukemia, oral cavity cancer, liver cancer, lung cancer, non-small cell lung cancer, small cell, lymphoma, AIDS-related lymphoma, central nervous system (primary), lymphoma, cutaneous T-cell, lymphoma, Hodgkin's disease, non-Hodgkin's disease, malignant mesothelioma, melanoma, Merkel cell carcinoma, metasatic squamous carcinoma, multiple myeloma, plasma cell neoplasms, mycosis fungoides, myelodysplastic syndrome, myeloproliferative disorders, nasopharyngeal cancer, neuroblastoma, oropharyngeal cancer, osteosarcoma, ovarian cancer, ovarian epithelial cancer, ovarian germ cell tumor, ovarian low malignant potential tumor, pancreatic cancer, exocrine, pancreatic cancer, islet cell carcinoma, paranasal sinus and nasal cavity cancer, parathyroid cancer, penile cancer, pheochromocytoma cancer, pituitary cancer, plasma cell neoplasm, prostate cancer, rhabdomyosarcoma, rectal cancer, renal cancer, renal cell cancer, salivary gland cancer, Sezary syndrome, skin cancer, cutaneous T-cell lymphoma, skin cancer, Kaposi's sarcoma, skin cancer, melanoma, small intestine cancer, soft tissue sarcoma, soft tissue sarcoma, testicular cancer, thymoma, malignant, thyroid cancer, urethral cancer, uterine cancer, sarcoma, unusual cancer of childhood, vaginal cancer, vulvar cancer, Wilms' tumor, or any combination thereof.

In one embodiment "metastatic cancer" refers to a cancer that spread (metastasized) from its original site to another area of the body. Virtually all cancers have the potential to spread. Whether metastases develop depends on the complex interaction of many tumor cell factors, including the type of cancer, the degree of maturity (differentiation) of the tumor cells, the location and how long the cancer has been present, as well as other incompletely understood factors. Metastases spread in three ways - by local extension from the tumor to the surrounding tissues, through the bloodstream to distant sites or through the lymphatic system to neighboring or distant lymph nodes. Each kind of cancer may have a typical route of spread. The tumor is called by the primary site (ex. breast cancer that has spread to the brain is called metastatic breast cancer to the brain).

In one embodiment "drug-resistant cancer" refers to cancer cells that acquire resistance to chemotherapy. Cancer cells can acquire resistance to chemotherapy by a range of mechanisms, including the mutation or overexpression of the drug target, inactivation of the drug, or elimination of the drug from the cell. Tumors that recur after an initial response to chemotherapy may be resistant to multiple drugs (they are multidrug resistant). In the conventional view of drug resistance, one or several cells in the tumor population acquire genetic changes that confer drug resistance. Accordingly, the reasons for drug resistance, *inter alia*, are: a) some of the cells that are not killed by the chemotherapy mutate (change) and become resistant to the drug. Once they multiply, there may be more resistant cells than cells that are sensitive to the chemotherapy; b) Gene amplification. A cancer cell may produce hundreds of copies of a particular gene. This gene triggers an overproduction of protein that renders the anticancer drug ineffective; c) cancer cells may pump the drug out of the cell as fast as it is going in using a molecule called p-glycoprotein; d) cancer cells may stop taking in the drugs because the protein that transports the drug across the cell wall stops working; e) the cancer cells may learn how to repair the DNA breaks caused by some anti-cancer drugs; f) cancer cells may develop a mechanism that inactivates the drug. One major contributor to multidrug resistance is overexpression of P-glycoprotein (P-gp). This protein is a clinically important transporter protein belonging to the ATP-binding cassette family of cell membrane transporters. It can pump substrates including anticancer drugs out of tumor cells through an ATP-dependent mechanism. Thus, the resistance to anticancer agents used in chemotherapy is the main cause of treatment failure in malignant disorders, provoking tumors to become resistant. Drug resistance is the major cause of cancer chemotherapy failure.

In one embodiment "resistant cancer" refers to drug-resistant cancer as described herein above. In another embodiment "resistant cancer" refers to cancer cells that acquire resistance to any treatment such as chemotherapy, radiotherapy or biological therapy.

In one embodiment, this disclosure is directed to treating, suppressing, reducing the severity, reducing the risk, or inhibiting cancer in a subject, wherein the subject has been previously treated with chemotherapy, radiotherapy or biological therapy.

In one embodiment "Chemotherapy" refers to chemical treatment for cancer such as drugs that kill cancer cells directly. Such drugs are referred as "anti-cancer" drugs or "antineoplastics." Today's therapy uses more than 100 drugs to treat cancer. To cure a specific cancer. Chemotherapy is used to control tumor growth when cure is not possible; to shrink tumors before surgery or radiation therapy; to relieve symptoms (such as pain); and to destroy microscopic cancer cells that may be present after the known tumor is removed by surgery (called adjuvant therapy). Adjuvant therapy is given to prevent a possible cancer reoccurrence.

In one embodiment, "Radiotherapy" refers to high energy x-rays and similar rays (such as electrons) to treat disease. Many people with cancer will have radiotherapy as part of their treatment. This can be given either as external radiotherapy from outside the body using x-rays or from within the body as internal radiotherapy. Radiotherapy works by destroying the cancer cells in the treated area. Although normal cells can also be damaged by the radiotherapy, they can usually repair themselves. Radiotherapy treatment can cure some cancers and can also reduce the chance of a cancer coming back after surgery. It may be used to reduce cancer symptoms.

In one embodiment "Biological therapy" refers to substances that occur naturally in the body to destroy cancer cells. There are several types of treatment including: monoclonal antibodies, cancer growth inhibitors, vaccines and gene therapy. Biological therapy is also known as immunotherapy.

In one embodiment, this invention provides a compound of the invention or its pharmaceutically acceptable salt, pharmaceutical product, tautomer, hydrate, *N*-oxide, or any combination thereof, or a composition comprising the same, for use in treating a subject suffering from prostate cancer, metastatic prostate cancer, resistant prostate cancer or drug-resistant prostate cancer.In another embodiment, the compound is compound **55**.

In one embodiment, this invention provides a compound of the invention or its pharmaceutically acceptable salt, pharmaceutical product, tautomer, hydrate, *N*-oxide, or any combination thereof, or a composition comprising the same, for use in suppressing, reducing the severity, reducing the risk, delaying the progression, or inhibiting prostate cancer, metastatic prostate cancer, resistant prostate cancer or drug-resistant prostate cancer in a subject. In another embodiment, the compound is compound **55**.

In one embodiment, this invention provides a compound of the invention or its pharmaceutically acceptable salt, pharmaceutical product, tautomer, hydrate, *N*-oxide, or any combination thereof, or a composition comprising the same, for use in treating a subject suffering from breast cancer, metastatic breast cancer, resistant breast cancer or drug-resistant breast cancer. In another embodiment, the subject is a male or female. In another embodiment, the compound is compound **55**.

In one embodiment, this invention provides a compound of the invention or its pharmaceutically acceptable salt, pharmaceutical product, tautomer, hydrate, *N*-oxide, or any combination thereof, or a composition comprising the same, for use in suppressing, reducing the severity, reducing the risk, delaying the progression, or inhibiting breast cancer, metastatic breast cancer, resistant breast cancer or drug-resistant breast cancer in a subject. In another embodiment, the compound is compound **55**.

In another embodiment, this invention provides a compound of the invention as herein described, or its pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting ovarian cancer, metastatic ovarian cancer, resistant ovarian cancer or drug-resistant ovarian cancer in a subject. In another embodiment, the compound is compound **55**.

In one embodiment, this invention provides a compound of the invention or its pharmaceutically acceptable salt, pharmaceutical product, tautomer, hydrate, *N*-oxide, or any combination thereof, or a composition comprising the same, for use in treating, suppressing, reducing the severity, reducing the risk or inhibiting melanoma, metastatic melanoma, resistant melanoma or drug-resistant melanoma in a subject. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting lung cancer, metastatic lung cancer, resistant lung cancer or drug-resistant lung cancer. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting non-small cell lung cancer, metastatic small cell lung cancer, resistant small cell lung cancer or drug-resistant small cell lung cancer. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting colon cancer, metastatic colon cancer, resistant colon cancer or drug-resistant colon cancer. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting of leukemia, metastatic leukemia, resistant leukemia or drug-resistant leukemia. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting lymphoma, metastatic lymphoma, lymphoma or drug-resistant lymphoma. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting head and neck cancer, metastatic head and neck cancer, resistant head and neck cancer or drug-resistant head and neck cancer. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting of pancreatic cancer, metastatic pancreatic cancer, resistant pancreatic cancer or drug-resistant pancreatic cancer.In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting esophageal cancer, metastatic esophageal cancer, resistant esophageal cancer or drug-resistant esophageal cancer. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting renal cancer, metastatic renal cancer, resistant renal cancer or drug-resistant renal cancer. In another embodiment, the compound is compound **55**.

In another embodiment, this invention provides a compound of the invention as herein described, or pharmaceutically acceptable salt, pharmaceutical product, tautomer, hydrate, *N*-oxide, crystal or any combination thereof, for use in treating, suppressing, reducing the severity, reducing the risk, delaying the progression, or inhibiting CNS cancer, metastatic CNS cancer, resistant CNS cancer or drug-resistant CNS cancer. In another embodiment, the compound is compound **55**.

In some embodiments, this invention provides a compound of the invention as herein described, or its pharmaceutically acceptable salt, pharmaceutical product, tautomer, crystal, *N*-oxide, hydrate or any combination thereof, for use in treating, suppressing, reducing the severity, reducing the risk, or inhibiting a drug resistant cancerous tumor or tumors in a subject. In another embodiment, the cancer is adrenocortical carcinoma, anal cancer, bladder cancer, brain tumor, brain stem tumor, breast cancer, glioma, cerebellar astrocytoma, cerebral astrocytoma, ependymoma, medulloblastoma, supratentorial primitive neuroectodermal, pineal tumors, hypothalamic glioma, breast cancer, carcinoid tumor, carcinoma, cervical cancer, colon cancer, central nervous system (CNS) cancer, endometrial cancer, esophageal cancer, extrahepatic bile duct cancer, Ewing's family of tumors (Pnet), extracranial germ cell tumor, eye cancer, intraocular melanoma, gallbladder cancer, gastric cancer, germ cell tumor, extragonadal, gestational trophoblastic tumor, head and neck cancer, hypopharyngeal cancer, islet cell carcinoma, laryngeal cancer, leukemia, acute lymphoblastic, leukemia, oral cavity cancer, liver cancer, lung cancer, non-small cell lung cancer, small cell, lymphoma, AIDS-related lymphoma, central nervous system (primary), lymphoma, cutaneous T-cell, lymphoma, Hodgkin's disease, non-Hodgkin's disease, malignant mesothelioma, melanoma, Merkel cell carcinoma, metasatic squamous carcinoma, multiple myeloma, plasma cell neoplasms, mycosis fungoides, myelodysplastic syndrome, myeloproliferative disorders, nasopharyngeal cancer, neuroblastoma, oropharyngeal cancer, osteosarcoma, ovarian cancer, ovarian epithelial cancer, ovarian germ cell tumor, ovarian low malignant potential tumor, pancreatic cancer, exocrine, pancreatic cancer, islet cell carcinoma, paranasal sinus and nasal cavity cancer, parathyroid cancer, penile cancer, pheochromocytoma cancer, pituitary cancer, plasma cell neoplasm, prostate cancer, rhabdomyosarcoma, rectal cancer, renal cancer, renal cell cancer, salivary gland cancer, Sezary syndrome, skin cancer, cutaneous T-cell lymphoma, skin cancer, Kaposi's sarcoma, skin cancer, melanoma, small intestine cancer, soft tissue sarcoma, soft tissue sarcoma, testicular cancer, thymoma, malignant, thyroid cancer, urethral cancer, uterine cancer, sarcoma, unusual cancer of childhood, vaginal cancer, vulvar cancer, Wilms' tumor, or any combination thereof. In another embodiment, the compound is compound **55**.

In another embodiment, the tumor is prostate cancer tumor. In another embodiment, the tumor is ovarian cancer tumor. In another embodiment, the tumor is a melanoma tumor. In another embodiment, the tumor is a multidrug resistant (MDR) melanoma tumor.

In one embodiment, this invention is directed to a method of destroying a cancerous cell *ex vivo* comprising: providing a compound of this invention and contacting the cancerous cell with the compound under conditions effective to destroy the contacted cancerous cell. According to various embodiments of destroying the cancerous cells, the cells to be destroyed are located *ex vivo* (i.e., in culture). In another embodiment, the compound is compound **55**.

In another embodiment, the cancer is selected from the group consisting of prostate cancer, breast cancer, ovarian cancer, skin cancer, melanoma, lung cancer, colon cancer, leukemia, renal cancer, CNS cancer, and combinations thereof.

A still further aspect of the present invention relates to a compound of the invention for use in treating or preventing a cancerous condition by administering an effective amount of the compound to a patient in a manner effective to treat or prevent a cancerous condition.

According to one embodiment, the patient to be treated is characterized by the presence of a precancerous condition, and the administering of the compound is effective to prevent development of the precancerous condition into the cancerous condition. This can occur by destroying the precancerous cell prior to or concurrent with its further development into a cancerous state.

According to another embodiment, the patient to be treated is characterized by the presence of a cancerous condition, and the administering of the compound is effective either to cause regression of the cancerous condition or to inhibit growth of the cancerous condition, i.e., stopping its growth altogether or reducing its rate of growth. This preferably occurs by destroying cancer cells, regardless of their location in the patient body. That is, whether the cancer cells are located at a primary tumor site or whether the cancer cells have metastasized and created secondary tumors within the patient body.

As used herein, subject or patient refers to any mammalian patient, including without limitation, humans and other primates, dogs, cats, horses, cows, sheep, pigs, rats, mice, and other rodents. In one embodiment, the subject is male. In another embodiment, the subject is female. In some embodiments, the compounds as described herein may be useful for treating either males or females.

When administering the compounds of the present invention, they can be administered systemically or, alternatively, they can be administered directly to a specific site where cancer cells or precancerous cells are present. Thus, administering can be accomplished in any manner effective for delivering the compounds or the pharmaceutical compositions to the cancer cells or precancerous cells. Exemplary modes of administration include, without limitation, administering the compounds or compositions orally, topically, transdermally, parenterally, subcutaneously, intravenously, intramuscularly, intraperitoneally, by intranasal instillation, by intracavitary or intravesical instillation, intraocularly, intraarterially, intralesionally, or by application to mucous membranes, such as, that of the nose, throat, and bronchial tubes.

The compounds of the present invention are useful in the treatment or prevention of various forms of cancer, particularly prostate cancer, breast cancer, ovarian, skin cancer (e.g., melanoma), lung cancer, colon cancer, leukemia, renal cancer, and CNS cancer (e.g., glioma, glioblastoma). Treatment of these different cancers is supported by the Examples herein. Moreover, based upon their believed mode of action as tubulin inhibitors, it is believed that other forms of cancer will likewise be treatable or preventable upon administration of the compounds or compositions of the present invention to a patient. Preferred compounds of the present invention are selectively disruptive to cancer cells, causing ablation of cancer cells but preferably not normal cells. Significantly, harm to normal cells is minimized because the cancer cells are susceptible to disruption at much lower concentrations of the compounds of the present invention.

The compounds of the present invention are useful in the treatment, reducing the severity, reducing the risk, or inhibition of cancer, metastatic cancer, resistant cancer or drug-resistant cancer. In another embodiment, the cancer is prostate cancer, breast cancer, ovarian, skin cancer (e.g., melanoma), lung cancer, colon cancer, leukemia, lymphoma, head and neck, pancreatic, esophageal, renal cancer or CNS cancer. Treatment of these different cancers is supported by the Examples herein. Moreover, based upon their believed mode of action as tubulin inhibitors, it is believed that other forms of cancer will likewise be treatable or preventable upon administration of the compounds or compositions of the present invention to a patient. Preferred compounds of the present invention are selectively disruptive to cancer cells, causing ablation of cancer cells but preferably not normal cells. Significantly, harm to normal cells is minimized because the cancer cells are susceptible to disruption at much lower concentrations of the compounds of the present invention. In another embodiment, the compound is compound **55**.

In one embodiment, the compound is administered in combination with another anti-cancer agent.

When the compounds or pharmaceutical compositions of the present invention are administered to treat, suppress, reduce the severity, reduce the risk, or inhibit a cancerous condition, the pharmaceutical composition can also contain, or can be administered in conjunction with, other therapeutic agents or treatment regimen presently known or hereafter developed for the treatment of various types of cancer. Examples of other therapeutic agents or treatment regimen include, without limitation, radiation therapy, immunotherapy, chemotherapy, surgical intervention, and combinations thereof.

The following examples are presented in order to more fully illustrate the preferred embodiments of the invention. To the extent that the following examples do not relate to a compound of formula **XI** as claimed, they are comparative examples.

### EXAMPLES

The Examples set forth below are for illustrative purposes only and are not intended to limit, in any way, the scope of the present invention.

### Materials and Methods:

**General.** All reagents were purchased from Sigma-Aldrich Chemical Co., Fisher Scientific (Pittsburgh, PA), AK Scientific (Mountain View, CA), Oakwood Products (West Columbia, SC), etc. and were used without further purification. Moisture-sensitive reactions were carried under an argon atmosphere. ABT-751 was prepared according methods reported by Yoshino *et al*.²⁶ Routine thin layer chromatography (TLC) was performed on aluminum backed Uniplates (Analtech, Newark, DE). Melting points were measured with Fisher-Johns melting point apparatus (uncorrected). NMR spectra were obtained on a Bruker AX 300 (Billerica, MA) spectrometer or Varian Inova-500 (Vernon Hills, Illinois) spectrometer. Chemical shifts are reported as parts per million (ppm) relative to TMS in CDCl₃. Mass spectral data was collected on a Bruker ESQUIRE electrospray/ion trap instrument in positive and negative ion modes. Elemental analyses were performed by Atlantic Microlab Inc.

**Cell Culture and Cytotoxicity Assay of Prostate Cancer and Melanoma.** All cell lines were obtained from ATCC (American Type Culture Collection, Manassas, VA, USA), while cell culture supplies were purchased from Cellgro Mediatech (Herndon, VA, USA). We examined the antiproliferative activity of our anti-tubulin compounds in four human prostate cancer cell lines (LNCaP, DU 145, PC-3, and PPC-1) and two human melanoma cell lines (A375 and WM-164). Human ovarian cell line OVCAR-8 and its resistant cell line that over-expresses P-gp (NCI/ADR-RES) were used as MDR models. Both ovarian cell lines were obtained from National Cancer Institutes (NCI). All cell lines were tested and authenticated by either ATCC or NCI. All prostate cancer and ovarian cancer cell lines were cultured in RPMI 1640, supplemented with 10% fetal bovine serum (FBS). Melanoma cells were cultured in DMEM, supplemented with 5% FBS, 1% antibiotic/antimycotic mixture (Sigma-Aldrich, Inc., St. Louis, MO, USA) and bovine insulin (5 µg/mL; Sigma-Aldrich). The cytotoxic potential of the anti-tubulin compounds was evaluated using the sulforhodamine B (SRB) assay after 96 h of treatment.

**Aqueous Solubility.** The solubility of drugs was determined by Multiscreen Solubility Filter Plate (Millipore Corporate, Billerica, MA) coupled with LC-MS/MS. Briefly, 198 µL of phosphate buffered saline (PBS) buffer (pH 7.4) was loaded into 96-well plate, and 2 µL of 10 mM test compounds (in DMSO) was dispensed and mixed with gentle shaking (200-300 rpm) for 1.5 h at RT (N = 3). The plate was centrifuged at 800g for 5 min, and the filtrate was used to determine its concentration and solubility of test compound by LC-MS/MS as described below.

**Pharmacokinetic Study.** Female Sprague-Dawley rats (n = 3 or 4; 254 ± 4 g) were purchased from Harlan Inc. (Indianapolis, IN). Rat thoracic jugular vein catheters were purchased from Braintree Scientific Inc. (Braintree, MA). On arrival at the animal facility, the animals were acclimated for 3 days in a temperature-controlled room (20-22 °C) with a 12 h light/dark cycle before any treatment. Compound **1h** was administered intravenously (i.v.) into the jugular vein catheters at a dose of 2.5 mg/kg (in DMSO/PEG300, 2/8), whereas **5Ha** and **5Hc** were dosed at 5 mg/kg (in DMSO/PEG300, 1/9). An equal volume of heparinized saline was injected to replace the removed blood, and blood samples (250 µL) were collected via the jugular vein catheters at 10, 20, 30 min, and 1, 2, 4, 8, 12, 24 h. Compounds **1h**, **5Ha** and **5Hc** were given (p.o.) by oral gavage at 10 mg/kg (in Tween80/DMSO/H₂O, 2/1/7). All blood samples (250 µL) after oral administration were collected via the jugular vein catheters at 30, 60, 90 min, 120 min, 150 min, 180 min, 210 min, 240 min, and 8, 12, 24 h. Heparinized syringes and vials were prepared prior to blood collection. Plasma samples were prepared by centrifuging the blood samples at 8,000 g for 5 min. All plasma samples were stored immediately at -80 °C until analyzed.

Analytes were extracted from 100 µL of plasma with 200 µL of acetonitrile containing 200 nM the internal standard ((3,5-dimethoxyphenyl)(2-phenyl-1*H*-imidazol-4-yl)methanone). The samples were thoroughly mixed, centrifuged, and the organic extract was transferred to autosampler for LC-MS/MS analysis. Multiple reaction monitoring (MRM) mode, scanning *m*/*z* 356 → 188 (compound **1h**), *m*/*z* 371 → 203 (compound **5Ha**), *m*/*z* 389 → 221 (compound **5Hc**), and m/z 309 → 171 (the internal standard), was used to obtain the most sensitive signals. The pharmacokinetic parameters were determined using non-compartmental analysis (WinNonlin, Pharsight Corporation, Mountain View, CA)

**Analytical Method.** Sample solution (10 µL) was injected into an Agilent series HPLC system (Agilent 1100 Series Agilent 1100 Chemstation, Agilent Technology Co, Ltd). All analytes were separated on a narrow-bore C18 column (Alltech Alltima HP, 2.1×100 mm, 3 µm, Fisher, Fair Lawn, NJ). Two gradient modes were used. Gradient mode was used to achieve the separation of analytes using mixtures of mobile phase A [ACN/H₂O (5%/95%, v/v) containing 0.1% formic acid] and mobile phase B [ACN/H₂O (95%/5%, v/v) containing 0.1% formic acid] at a flow rate of 300 µL/min. Mobile phase A was used at 15% from 0 to 1 min followed by a linearly programmed gradient to 100% of mobile phase B within 6 min, 100% of mobile phase B was maintained for 0.5 min before a quick ramp to 15% mobile phase A. Mobile phase A was continued for another 12 min towards the end of analysis.

***In Vitro* Tubulin Polymerization Assay.** Bovine brain tubulin (0.4 mg, >97% pure) (Cytoskeleton, Denver, CO) was mixed with 10 µM of the test compounds and incubated in 100 µLof general tubulin buffer (80 mM PIPES, 2.0 mM MgCl₂, 0.5 mM EGTA, and 1 mM GTP) at pH 6.9. The absorbance of wavelength at 340 nm was monitored every 1 min for 20 min by the SYNERGY 4 Microplate Reader (Bio-Tek Instruments, Winooski, VT). The spectrophotometer was set at 37 °C for tubulin polymerization.

A triple-quadruple mass spectrometer, API Qtrap 4000™ (Applied Biosystems/MDS SCIEX, Concord, Ontario, Canada), operating with a TurboIonSpray source was used. The spraying needle voltage was set at 5 kV for positive mode. Curtain gas was set at 10; Gas 1 and gas 2 were set 50. Collision-Assisted-Dissociation (CAD) gas at medium and the source heater probe temperature at 500°C. Data acquisition and quantitative processing were accomplished using Analyst™ software, Ver. 1.4.1 (Applied Biosystems).

The purity of the final compounds was tested via RP-HPLC on a Waters 2695 HPLC system installed with a Photodiode Array Detector. Two RP-HPLC methods were conducted using a Supelco Ascentis™ 5µM C-18 column (250 x 4.6 mm) at ambient temperature, and a flow rate of 0.7 mL/min. HPLC1: Gradient: Solvent A (water) and Solvent B (methanol): 0-20 min 40-100%B (linear gradient), 20-27 min 100%B. HPLC2: Gradient: Solvent A (water) and Solvent B (methanol): 0-15 min 40-100%B (linear gradient), 15-25 min 100%B. UV detection at 254nm.

The compounds of this invention and other reference compounds were prepared according to Figures 1-17.

### REFERENCE EXAMPLE 1

### SYNTHESIS OF B RING VARIANT COMPOUNDS

B ring variant compounds were synthesized according to Figures 1 and 2.

### Oxazole B ring:

### Synthesis of (2-Phenyl-oxazol-4-yl)-(3,4,5-trimethoxy-phenyl)-methanone (36a) (Figure 1):

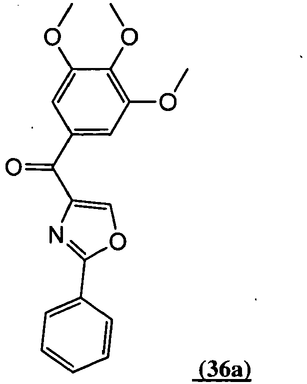

**(*2R*)-2-Phenyl-4,5-dihydro-oxazole-4-carboxylic acid methyl ester (32a).** Acetyl chloride (6.8 mL) was added dropwise to ice-cold methanol (30 mL). After the addition of *L*-serine (0.48 mmol), the reaction mixture was warmed to room temperature (RT) and stirred overnight. Evaporation of the solvent gave white solid (*2R*)-3-hydroxy-2-methyl-propionic acid methyl ester HCl salt, which was used without purification in the next step. Triethylamine (11 mL, 72.3 mmol) was added slowly to a solution of ethyl benzimidate hydrochloride (11.6 g, 62.8 mmol) in CH₂Cl₂ (150 mL). The reaction mixture was stirred at RT for 30 min and (2*R*)-3-hydroxy-2-methyl-propionic acid methyl ester HCl salt (13.5 g, 79.6 mmol) was added by portion. The resulting mixture was stirred for 48 h and concentrated under reduced pressure. The compound **32a** was separated from flash column as a yellow oil (12.3 g, 95.9%). ¹H NMR (CDCl₃) δ 7.99 -7.38 (m, 5 H), 4.97 (dd, 1 H, *J* = 7.8 Hz, *J* = 10.5 Hz), 4.70 (t, 1 H, *J* = 8.7 Hz), 4.62 (dd, 1 H, *J* = 8.7 Hz, *J* = 10.5 Hz), 3.82 (s, 3 H); MS (ESI) m/z 206.1 (M + H)⁺.

**(*2R*)-2-Phenyl-4,5-dihydro-oxazole-4-carboxylic acid (33a).** To an ice-cooled solution of **32a** in MeOH/H₂O was added LiOH (2.5 equiv) with stirring. The mixture was allowed to warm to RT in 1 h, concentrated in vacuo, and the white solid was dissolved in H₂O and acidified with 1 N HCl to pH 2.0 and extracted with MgSO₄, filtered and concentrated in vacuo to provide the acid **33a** as a white solid (95.8 %). ¹H NMR (CDCl₃) δ 7.98 (d, 2 H), 7.57-7.42 (m, 3 H), 5.04 (dd, 1 H, *J* = 7.8 Hz, *J* = 10.8 Hz), 4.80 (t, 1 H, *J* = 8.7 Hz), 4.70 (dd, 1 H, *J* = 9.0 Hz, *J*= 10.8 Hz); MS (ESI) m/z 191.9 (M + H)⁺, 189.7 (M - H)⁻, 145.8 (M - COOH)⁻.

**(*2R*)-2-Phenyl-4,5-dihydro-oxazole-4-carboxylic acid methoxy-methyl-amide (34a).** To a mixture of **33a** (5 mmol), EDCI (6 mmol), HOBt (5 mmol) and Et₃N (5 mmol) in CH₂Cl₂ (50 mL) was added HNCH₃OCH₃ (5 mmol) and stirring continued at RT for 6-8 h. The reaction mixture was diluted with CH₂Cl₂ (100 mL) and sequentially washed with water, satd. NaHCO₃, brine and dried over MgSO₄. The solvent was removed under reduced pressure to yield a crude product **34a**, which was purified by column chromatography as a white solid (61.0 %). ¹H NMR (CDCl₃) δ 7.98-7.36 (m, 5 H), 7.57-7.42 (m, 3 H), 5.35 (br, t, 1 H), 4.81 (br, t, 1 H), 4.52 (dd, 1 H, *J* = 8.7 Hz, *J* = 10.2 Hz), 3.90 (s, 3 H), 3.27 (s, 3 H); MS (ESI) m/z 257.0 (M + H)⁺.

**(*2R*)-(2-Phenyl-4,5-dihydro-oxazol-4-yl)-(3,4,5-trimethoxy-phenyl)-methanone (35a).** To a solution of *n*-BuLi (1.6 M, 0.713 mL) in 8 mL THF was added a solution of 3,4,5-trimethoxybromobenzene (1.09 mmol) in 3 mL THF under -78 °C. The mixture was allowed to stir for 2 h and a solution of Weinreb amide **34a** (1.14 mmol) in 3 mL THF was charged. The temperature was allowed to increase at RT and stirred overnight. The reaction mixture was quenched with satd. NH₄Cl, extracted with ethyl ether, dried with MgSO₄. The solvent was removed under reduced pressure to yield a crude product, which was purified by column chromatography to obtain pure compound **35a** as a white solid (47.9 %). ¹H NMR (CDCl₃) δ 7.97 -7.94 (m, 2 H), 7.62 (s, 2 H), 7.54-7.37 (m, 3 H), 5.61 (q, 1 H, *J* = 7.5 Hz, 9.9 Hz), 5.12 (t, 1 H, *J* = 7.5 Hz), 4.57 (q, 1 H, *J* = 7.8 Hz, 9.9 Hz), 3.96 (s, 6 H), 3.95 (s, 3 H); MS (ESI) m/z 364.1 (M + Na)⁺, 340.1 (M - H)⁻.

**(2-Phenyl-oxazol-4-yl)-(3,4,5-trimethoxy-phenyl)-methanone (36a).** A mixture of **35a** (1.48 mmol), CBrCl₃ (2.59 mmol) and DBU (2.97 mmol) in CH₂Cl₂ (20 mL) was stirred overnight. The reaction mixture was absorbed on silica gel and purified by column chromatography to yield pure **36a** as desired (61.6 %). ¹H NMR (CDCl₃) δ 8.37 (s, 1 H), 8.14-8.12 (m, 2 H), 7.74 (s, 2 H), 7.52-7.49 (m, 3 H), 3.97 (s, 9 H); MS (ESI) m/z 362.1(M + Na)⁺.

**Benzene, pyrimidine, pyridine, furan, thiophene, thiazole, pyrazole and piperidine B ring variants (****Figure 2****):** B ring variants **(1a-1d, 1k)** were obtained from their corresponding acids **(37a-37d, 37k).** Compound 1**f** with thiophene in B ring position can not be separated from the mixture of 1**f** and a Grignard reagent coupling by-product 3,4,5,3',4',5'-hexamethoxybiphenyl using flash column. So an alternative method was used to prepare **1f:** Weinreb amide **38f** was converted into its corresponding aldehyde which was further reacted with 3,4,5-trimethoxyphenylmagnesium bromide to afford the alcohol **40f**, which can be easily separated from 3,4,5,3',4',5'-hexamethoxybiphenyl using flash column chromatography. Oxidation with pyridinium dichromate (PDC) or DMSO did not afford 1**f** from secondary alcohol **40f** with good yields. But using Dess-Martin periodinane reagent as oxidant successfully formed the desired ketone compound **1f. 1e** and **1i** were prepared from alcohols **40e** and **40i** using a similar method. Compound **1g** was obtained via a coupling reaction from piperidine **41g** and 3,4,5-trimethoxybenzoic acid.

### Benzene B ring:

### Synthesis of Biphenyl-3-yl(3,4,5-trimethoxyphenyl)methanone (1a) (Figure 2)

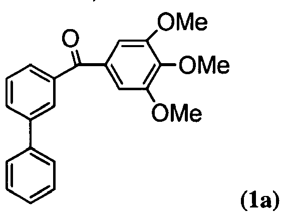

***N*-Methoxy-*N*-methylbiphenyl-3-carboxamide (38a).** To a mixture of **37a** (5 mmol), EDCI (6 mmol), HOBt (5 mmol) and NMM (11 mmol) in CH₂Cl₂ (50 mL) was added HNCH₃OCH₃HCl salt (5 mmol) and stirring continued at RT for 2 h. The reaction mixture was diluted with CH₂Cl₂ (100 mL) and sequentially washed with water, satd. NaHCO₃, brine and dried over MgSO₄. The solvent was removed under reduced pressure to yield a colorless oil, which was used for next step (58.4 %). MS (ESI) m/z 264.0 (M + Na)⁺.

**Biphenyl-3-yl(3,4,5-trimethoxyphenyl)methanone (1a).** To a solution of **38a** (Figure 2) (0.174 g, 0.72 mmoL) in 5 mL THF was added a THF solution of 3,4,5-trimethoxyphenylmagnesiumbromide (0.5 N, 1.08 mmol) at 0 °C. The mixture was allowed to stir for 30 min and quenched with satd. NH₄Cl, extracted with ethyl ether, dried with MgSO₄. The solvent was removed under reduced pressure to yield a crude product, which was purified by column chromatography to obtain pure compound **1a** as a white solid (43.8%). ¹H NMR (CDCl₃) δ 8.02 (t, 1 H), 7.84-7.74 (m, 2 H), 7.64-7.38 (m, 6 H), 7.11 (s, 2 H), 3.95 (s, 3 H), 3.88 (s, 6 H); MS (ESI) m/z 371.1 (M + Na)⁺.

### Pyrimidine B ring:

### Synthesis of (6-Phenylpyrimidin-4-yl)(3,4,5-trimethoxyphenyl)methanone (1b) (Figure 2)

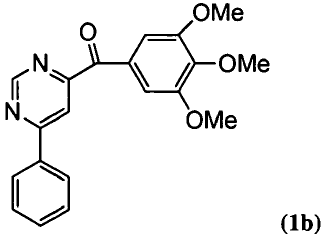

***N*-Methoxy-*N*-methyl-6-phenylpyrimidine-4-carboxamide (38b).** To a mixture of **37b** (5 mmol), EDCI (6 mmol), HOBt (5 mmol) and NMM (11 mmol) in CH₂Cl₂ (50 mL) was added HNCH₃OCH₃HCl salt (5 mmol) and stirring continued at RT for overnight. The reaction mixture was diluted with CH₂Cl₂ (100 mL) and sequentially washed with water, satd. NaHCO₃, brine and dried over MgSO₄. The solvent was removed under reduced pressure to yield a crude product, which was purified by column chromatography to obtain pure compound **38b** as a yellow solid (62.3 %). ¹H NMR (CDCl₃) δ 9.28 (s, 1 H), 8.14-8.06 (m, 2 H), 7.96 (br, s, 1 H), 7.54-7.50 (m, 3 H), 5.35 (br, t, 1 H), 4.81 (br, t, 1 H), 4.52 (dd, 1 H, *J* = 8.7 Hz, *J* = 10.2 Hz), 3.79 (s, 3 H), 3.42 (s, 3 H); MS (ESI) m/z 266.0 (M + Na)⁺.

**(6-Phenylpyrimidin-4-yl)(3,4,5-trimethoxyphenyl)methanone (1b).** To a solution of **38b** (0.243 g, 1 mmoL) in 5 mL THF was added a THF solution of 3,4,5-trimethoxyphenylmagnesiumbromide (0.5 N, 5.6 mL, 1.4 mmol) at 0 °C. The mixture was allowed to stir for 30 min and quenched with satd. NH₄Cl, extracted with ethyl ether, dried with MgSO₄. The solvent was removed under reduced pressure to yield a crude product, which was purified by column chromatography to obtain pure compound **1b** (52.3%). ¹H NMR (CDCl₃) δ 9.40 (d, 1 H, *J* = 1.5 Hz), 8.29 (d, 1 H, *J* = 1.5 Hz), 8.22-8.18, 7.57-7.54 (m, 5 H), 7.46 (s, 2 H), 3.96 (s, 3 H), 3.91 (s, 6 H); MS (ESI) m/z 351.1 (M + H)⁺.

### Pyridine B ring:

### Synthesis of (6-Phenylpyridin-2-yl)(3,4,5-trimethoxyphenyl)methanone (1c) (Figure 2)

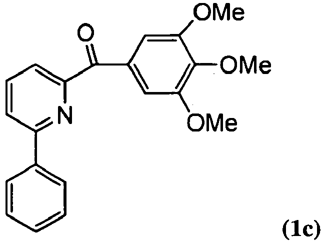

***N*-Methoxy-*N*-methyl-6-phenylpicolinamide (38c).** To a mixture of **37c** (1.77 mmol), EDCI (2.12 mmol), HOBt (1.86 mmol) and NMM (3.54 mmol) in CH₂Cl₂ (20 mL) was added HNCH₃OCH₃HCl salt (1.86 mmol) and stirring continued at RT for overnight. The reaction mixture was diluted with CH₂Cl₂ (40 mL) and sequentially washed with water, satd. NaHCO₃, brine and dried over MgSO₄. The solvent was removed under reduced pressure to yield a crude product, which was purified by column chromatography to obtain pure compound **38c** as a colorless oil (51:2 %). ¹H NMR (CDCl₃) δ 8.02 (d, 1 H, *J* = 7.0 Hz), 7.86-7.81 (m, 2 H), 7.55 (br, 1 H), 7.48 (t, 2 H), 7.44-7.41 (m, 1 H), 3.82 (s, 3 H), 3.44 (s, br, 3 H); MS (ESI) m/z 265.0 (M + Na)⁺.

**(6-Phenylpyridin-2-yl)(3,4,5-trimethoxyphenyl)methanone (1c).** To a solution of **38c** (0.210g, 0.86 mmoL) in 5 mL THF was added a THF solution of 3,4,5-trimethoxyphenylmagnesiumbromide (0.5 N, 3.5 mL, 1.73 mmol) at 0 °C. The mixture was allowed to stir for 30 min and quenched with water, extracted with ethyl acetate and dried with MgSO₄. The solvent was removed under reduced pressure to yield a crude product, which was purified by column chromatography to obtain pure **1c** as white needle crystals (78%). ¹H NMR (CDCl₃) δ 8.10 (d, br, 2 H), 8.02-8.00 (m, 1 H), 7.97-7.96 (m, 2 H), 7.66 (s, 2 H), 7.49-7.43 (m, 3 H), 3.97 (s, 3 H), 3.89 (s, 6 H); MS (ESI) m/z 372.6 (M + Na)⁺.

### Furan B ring:

### Synthesis of (5-Phenylfuran-2-yl)(3,4,5-trimethoxyphenyl)methanone (1d) (Figure 2)

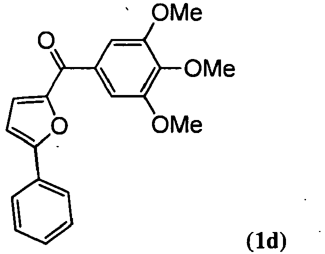

***N*-Methoxy-*N*-methyl-5-phenylfuran-2-carboxamide (38d).** To a mixture of **37d** (10 mmol), EDCI (12 mmol), HOBt (11 mmol) and NMM (21 mmol) in CH₂Cl₂ (200 mL) was added HNCH₃OCH₃HCl salt (10.5 mmol) and stirring continued at RT for overnight. The reaction mixture was diluted with CH₂Cl₂ (200 mL) and sequentially washed with water, satd. NaHCO₃, brine and dried over MgSO₄. The solvent was removed under reduced pressure to yield a crude product, which was purified by column chromatography to obtain pure compound **38d**. (95.2 %). ¹H NMR (CDCl₃) δ 7.82 (d, 1 H, *J* = 7.0 Hz), 7.46-7.43 (t, 2 H), 7.37-7.34 (m, 1 H), 7.25 (d, 1 H, *J* = 4.0 Hz), 6.78 (d, 1 H, *J* = 4.0 Hz), 3.86 (s, 3 H), 3.41 (s, 3 H); MS (ESI) m/z 254.1 (M + Na)⁺.

**(5-Phenylfuran-2-yl)(3,4,5-trimethoxyphenyl)methanone (1d).** To a solution of **38d** (0.231 g, 1 mmoL) in 5 mL THF was added a THF solution of 3,4,5-trimethoxyphenylmagnesiumbromide (0.5 N, 4.0 mL, 2 mmol) at 0 °C. The mixture was allowed to stir for 30 min and quenched with water, extracted with ethyl acetate and dried with MgSO₄. The solvent was removed under reduced pressure to yield a crude product, which was purified by column chromatography to obtain pure compound 1**d** as white crystals (35.5%). ¹H NMR (CDCl₃) δ 7.85-7.82 (m, 1 H), 7.48-7.36 (m, 4 H), 7.35 (s, 2 H), 7.25 (d, 1 H, *J* = 4:0 Hz), 6.86 (d, 1 H, *J* = 4.2 Hz), 3.96 (s, 3 H), 3.95 (s, 6 H); MS (ESI) m/z 339.1 (M + H)⁺.

### Thiazole B ring:

### Synthesis of (2-Phenylthiazol-5-yl)(3-4,5-trimethoxyphenyl)methanone (1e) (Figure 2)

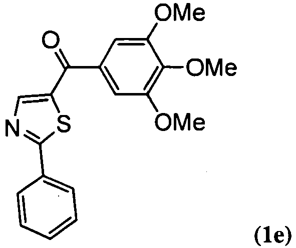

**(2-Phenylthiazol-5-yl)(3,4,5-trimethoxyphenyl)methanol (40e).** To a solution of 2-phenylthiazole-5-carbaldehyde **38e** (0.567 g, 3 mmoL) in 15 mL THF was added a THF solution of 3,4,5-trimethoxyphenylmagnesiumbromide (0.5 N, 6.5 mL, 3.25 mmol) at 0 °C. The mixture was allowed to stir for 30 min and quenched with satd. NH₄Cl, extracted with ethyl ether, dried with MgSO₄. The solvent was removed under reduced pressure to yield a crude product, which was purified by column chromatography to obtain pure compound **40e** (72.9 %). ¹H NMR (CDCl₃) δ 7.90 (m, 2 H), 7.64 (s, 1 H), 7.41 (m, 3 H), 6.69 (s, br, 2 H), 6.04 (s, 1 H), 3.86 (s, 6 H), 3.85 (s, 3 H), 1.57 (d, 1 H, *J* = 5.5 Hz); MS (ESI) m/z 358.1 (M + Na)⁺.

**(2-Phenylthiazol-5-yl)(3,4,5-trimethoxyphenyl)methanone (1e).** To a solution of **40e** (0.357 g, 1 mmoL) in 40 mL anhydrous CH₂Cl₂ was added Dess-Martin reagent (0.848 g, 2 mmol). The mixture was allowed to stir for 30 min and quenched with sat. Na₂S₂O₃ solution, extracted with ethyl acetate and dried with MgSO₄. The solvent was removed under reduced pressure to yield a crude product, which was purified by column chromatography to give pure compound **1e** (80.1%). ¹H NMR (CDCl₃) δ 8.33 (s, 1 H), 8.04 (m, 2 H), 7.51 (m, 3 H), 7.18 (s, 2 H), 3.96 (s, 3 H), 3.93 (s, 6 H); MS (ESI) m/z 378.1 (M + H)⁺.

### Thiophene B ring:

### Synthesis of (5-Phenylthiophen-3-yl)(3,4,5-trimethoxyphenyl)methanone (1f) (Figure 2)

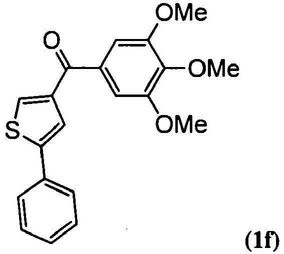

***N*-Methoxy-*N*-methyl-5-phenylthiophene-3-carboxamide (38f).** To a mixture of **37f** (2.5 mmol), EDCI (2.9 mmol), HOBt (2.6 mmol) and NMM (5.3 mmol) in CH₂Cl₂ (30 mL) was added HNCH₃OCH₃HCl salt (2.6 mmol) and stirring continued at RT for overnight. The reaction mixture was diluted with CH₂Cl₂ (20 mL) and sequentially washed with water, satd. NaHCO₃, brine and dried over MgSO₄. The solvent was removed under reduced pressure to yield a crude product, which was purified by column chromatography to obtain pure compound **38f**. (90.8 %). ¹H NMR (CDCl₃) δ 8.28 (d, 1 H, *J* = 1.5 Hz), 7.69 (d, 1 H, *J* = 1.5 Hz), 7.64 (d, 2 H, *J* = 7.0 Hz), 7.44 (t, 2 H, *J* = 7.0 Hz), 7.35-7.32 (m, 1 H), 6.78 (d, 1 H, *J* = 4.0 Hz), 3.86 (s, 3 H), 3.41 (s, 3 H); MS (ESI) m/z 270.0 (M + Na)⁺.

**(5-Phenylthiophen-3-yl)(3,4,5-trimethoxyphenyl)methanol (40f).** At -78 °C, to a solution of **38f** (2.5 mmol) in 5 mL THF under argon protection was added a solution of LiAlH₄ in THF (1 N, 1.42 mL) and stirring continued at 1 h at -20 °C. The reaction mixture was placed on an ice bath and quenched by 20% H₂SO₄ solution, extracted with ethyl acetate and dried over MgSO₄. The solvent was removed under reduced pressure and purified by column chromatography to yield 5-phenylthiophene-3-carbaldehyde (not shown) (84.8%). ¹H NMR (CDCl₃) δ 9.98 (s, 1 H), 8.04 (d, 1 H, *J* = 1.5 Hz), 7.86 (br, 1 H), 7.61-7.58 (br, 2 H), 7.47-7.33 (m, 3 H), 7.35-7.32 (m, 1 H), 6.78 (d, 1 H, *J* = 4.0 Hz); MS (ESI) m/z 210.9 (M + Na)⁺. To a solution of 5-phenylthiophene-3-carbaldehyde (0.195 g, 1.04 mmoL) in 5 mL THF was added a THF solution of 3,4,5-trimethoxyphenylmagnesiumbromide (0.5 N, 2.3 mL, 1.14 mmol) at 0 °C. The mixture was allowed to stir for 30 min and quenched with satd. NH₄Cl, extracted with ethyl ether, dried with MgSO₄. The solvent was removed under reduced pressure to yield a crude product, which was purified by column chromatography to obtain pure compound **40f**. (70.5%).¹H NMR (CDCl₃) δ 7.55-7.52 (m, 2 H), 7.40-7.35 (m, 3 H), 7.30 (br, 1 H), 7.20 (br, 1 H), 6.72 (s, 2 H), 6.01 (d, 1 H, *J* = 3.9 Hz), 3.86 (s, 6 H), 3.85 (s, 3 H), 2.42 (d, 1 H, *J* = 3.9 Hz); MS (ESI) m/z 339.1 (M - OH)⁻.

**(5-Phenylthiophen-3-yl)(3,4,5-trimethoxyphenyl)methanone (1f).** To a solution of **40f** (0.260 g, 0.73 mmoL) in 20 mL anhydrous CH₂Cl₂ was added Dess-Martin reagent (0.465 g, 1.36 mmol). The mixture was allowed to stir for 30 min and quenched with sat. Na₂S₂O₃ solution, extracted with ethyl acetate and dried with MgSO₄. The solvent was removed under reduced pressure to yield a crude product, which was purified by column chromatography to give pure compound **1f** as light yellow crystals (60.9%). ¹H NMR (CDCl₃) δ 7.97 (d, 1 H, *J* = 1.5 Hz), 7.82 (d, 1 H, *J* = 1.5 Hz), 7.59-7.57 (m, 2 H), 7.45-7.34 (m, 3 H), 7.19 (s, 2 H), 3.95 (s, 3 H), 3.93 (s, 6 H); MS (ESI) m/z 355.1 (M + H)⁺.

### Piperidine B ring:

### Synthesis of (4-Phenylpiperidin-1-yl)(3,4,5-trimethoxyphenyl)methanone (1g) (Figure 2)

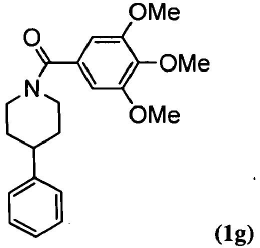

**(4-Phenylpiperidin-1-yl)(3,4,5-trimethoxyphenyl)methanone (1g).** To a mixture of 4-phenylpiperidine **41g** (5 mmol), EDCI (6 mmol), HOBt (5.5 mmol) and NMM (6 mmol) in CH₂Cl₂ (50 mL) was added 3,4,5-trimethoxybenzoic acid (5.3 mmol) and stirring continued at RT for overnight. The reaction mixture was diluted with CH₂Cl₂ (100 mL) and sequentially washed with water, satd. NaHCO₃, brine and dried over MgSO₄. The solvent was removed under reduced pressure to yield a crude product, which was purified by column chromatography to obtain pure compound **1g**. (57.9%). ¹H NMR (CDCl₃) δ 7.35-7.21 (m, 5 H), 6.66 (s, 2 H), 4.84 (br, 1 H), 3.95 (br, 1 H), 3.88 (s, 6 H), 3.86 (s, 3 H), 3.20-2.87 (br, 2 H), 2.85-2.74 (tt, 1 H, *J* = 3.6 Hz, *J* = 15.6 Hz) 1.92 (br, 2 H), 1.70 (br, 2 H); MS (ESI) m/z 378.1 (M + Na)⁺.

### Isoxazole B ring:

### Synthesis of (5-Phenylisoxazol-3-yl)(3,4,5-trimethoxyphenyl)methanone (1i) (Figure 2)

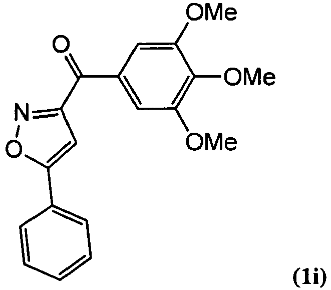

**(5-Phenylisoxazol-3-yl)(3,4,5-trimethoxyphenyl)methanol (40i).** To a solution of 5-phenylisoxazole-3-carbaldehyde 38i (0.365 g, 2.1 mmol) in 15 mL THF was added a THF solution of 3,4,5-trimethoxyphenylmagnesiumbromide (0.5 N, 5.5 mL, 2.74 mmol) at 0 °C. The mixture was allowed to stir for 30 min and quenched with satd. NH₄Cl, extracted with ethyl ether, dried with MgSO₄. The solvent was removed under reduced pressure to yield a crude product, which was purified by column chromatography to obtain pure compound **40i** as a white solid. (48.8%).¹H NMR (CDCl₃) δ 7.78-7.77 (m, 2 H), 7.48-7.46 (m, 3 H), 6.74 (s, 2 H), 6.45 (s, 1 H), 5.98 (d, 1 H, *J* = 3.5 Hz) 3.89 (s, 6 H), 3.86 (s, 3 H), 2.77 (d, 1 H, *J* = 3.5 Hz); MS (ESI) m/z 364.1 (M + Na)⁺.

**(5-Phenylisoxazol-3-yl)(3,4,5-trimethoxyphenyl)methanone (1i).** To a solution of **40i** (0.110 g, 0.73 mmoL) in 8 mL anhydrous CH₂Cl₂ was added Dess-Martin reagent (0.274 g, 0.645 mmol). The mixture was allowed to stir for 30 min and quenched with sat. Na₂S₂O₃ solution, extracted with ethyl acetate and dried with MgSO₄. The solvent was removed under reduced pressure to yield a crude product, which was purified by column chromatography to give pure compound **1i** (70.1 %). ¹H NMR (CDCl₃) δ 7.87-7.85 (m, 2 H), 7.72 (s, 2 H), 7.53-7.49 (m, 3 H), 7.05 (s, 1 H), 7.82 (d, 1 H, *J* = 1.5 Hz), 3.97 (s, 3 H), 3.96 (s, 6 H); MS (ESI) m/z 362.1 (M + H)⁺.

### Pyrazole B ring:

### Synthesis of (3-Phenyl-1H-pyrazol-5-yl)(3,4,5-trimethoxyphenyl)methanone (1k) (Figure 2)

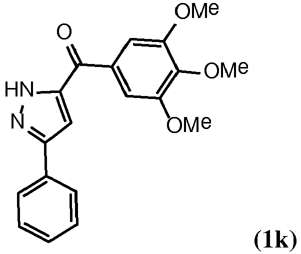

**(3-Phenyl-1*H*-pyrazol-5-yl)(3,4,5-trimethoxyphenyl)methanone (1k)** was prepared using the same method as used of compound **1c** from 3-phenyl-1*H*-pyrazole-5-carboxylic acid. ¹H NMR (500MHz, CDCl₃ δ 10.97 (br, 1 H), 7.77 (s, br, 2 H), 7.48-7.38 (m, 5 H), 7.14 (s, br, 1 H), 3.96 (s, 3 H), 3.94 (s, 6 H); MS (ESI) m/z 361.1(M + Na)⁺, 337.0 (M - H)⁻.

### REFERENCE EXAMPLE 2

### SYNTHESIS OF COMPOUNDS HAVING DIFFERENT Y LINKERS

Related compounds possess different Y linkers. Such compounds, with different Y linkers, were synthesized according to Figures 3 and 4.

Compound **1h** was synthesized from 2-phenyl-4,5-dihydro-thiazole-4-carboxylic acid **42a** through three steps described before (Lu, Y.; Wang, Z.; Li, C. M.; Chen, J.; Dalton, J. T.; Li, W.; Miller, D. D., Synthesis, in vitro structure-activity relationship, and in vivo studies of 2-arylthiazolidine-4-carboxylic acid amides as anticancer agents. Bioorg Med Chem 2010, 18, (2), 477-95). **1h** was converted to oxime isomers **2e-cis,trans** and **2f-cis,trans** upon reaction with hydroxylamines, NH₂OH or NH₂OCH₃. Assignments were made on the basis of chemical and spectral data as described *infra*. An improved Beckmann rearrangement readily produced the rearranged amides **2g** and **2h** from the two geometric stereoisomers **2e-cis** and **2e-trans** via their reaction with tosyl chloride and subsequent basic aluminum oxide column. Hydrazide derivatives **2d-cis** and **2d-trans** were prepared by mixing **1h** with hydrazine hydrate in ethanol and refluxing for 24 h. Acrylonitriles **2c-trans,cis** were obtained from Wittig reaction of **1h** with diethyl cyanomethylphosphonate. Cyanoimine **2j** was prepared using the procedure as by described by Cuccia (Cuccia, S. J.; Fleming, L. B.; France, D. J., A novel and efficient synthesis of 4-phenyl-2-chloropyrimidines from acetophenone cyanoimines. Synthetic Communications 2002, 32, (19), 3011-3018). The carbonyl group in compound **1h** was also reduced to a secondary alcohol **2b** or converted to an alkene **(2a)** as illustrated in Figure 3.

Attempts to remove the carbonyl group between B and C rings in **1h**, resulted in the formation of compound **2i** as shown in Figure 4. Introducing *cis-* and *trans-*double bonds into the carbonyl position formed compounds (**3a** and **3b**), which were synthesized from a Wittig reaction with 2-phenylthiazole-4-carbaldehyde. The sulfide compound **4a**, sulfone **4b** and sulfoxide **4c** were prepared using 3-aminobiphenyl as starting material through an initial Sandmeyer reaction to yield carbonodithioate **52a**, followed by CuI catalyzed coupling reaction and *m*-CPBA oxidation. Sulfonamide linked compound **4d** was prepared from reaction of 3-biphenylsulfonyl chloride with 3,4,5-trimethoxyaniline in the presence of NEt₃ in DMF.

### Synthesis of (2-Phenyl-thiazol-4-yl)-(3,4,5-trimethoxy-phenyl)-methanone (1h) [Figure 3]

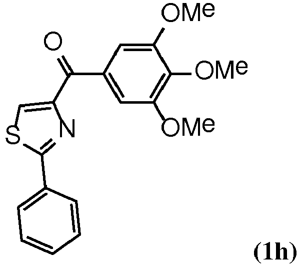

**(2-Phenyl-thiazol-4-yl)-(3,4,5-trimethoxy-phenyl)-methanone (1h).** A mixture of 2-phenyl-4,5-dihydrothiazole-4-carboxylic acid (5 mmol), EDCI (6 mmol) and HOBt (5 mmol) in CH₂Cl₂ (50 mL) was stirred for 10 min. To this solution, NMM (5 mmol) and HNCH₃OCH₃ (5 mmol) were added and stirring continued at RT for 6-8 h. The reaction mixture was diluted with CH₂Cl₂ (100 mL) and sequentially washed with water, satd. NaHCO₃, brine and dried over MgSO₄. The solvent was removed under reduced pressure to yield a crude product, which was purified by column chromatography to get 2-phenyl-4,5-dihydrothiazole-4-carboxylic acid methoxymethylamide. A solution of 2-phenyl-4,5-dihydrothiazole-4-carboxylic acid methoxymethylamide (1 equiv) in CH₂Cl₂ was cooled to 0 °C, and distilled DBU (2 equiv) was added. Bromotrichloromethane (1.7 equiv) was then introduced dropwise via syringe over 10 min. The reaction mixtures were allowed to warm to RT and stirred overnight. Upon washing with satd. aqueous NH₄Cl (2 × 50 mL), the aqueous phase was extracted with EtOAc (3x 50 mL). The combined organic layers were dried on MgSO₄, filtered and concentrated in vacuo. The residue was purified by flash chromatography as needed providing 2-phenyl-thiazole-4-carboxylic acid methoxymethylamide (73.6 %). ¹H NMR (300MHz, CDCl₃) δ 8.01 (s, 1 H), 7.99-7.96 (m, 2 H), 7.47-7.44 (m, 3 H), 3.88 (s, 3 H), 3.49 (s, 3 H). MS (ESI) *m*/*z* 271.0 (M + Na)⁺. To a solution of 3,4,5-trimethoxyphenylmagnesium bromide (0.5 N, 3 mL) in 2 mL THF was charged a solution of 2-phenyl-thiazole-4-carboxylic acid methoxymethylamide (1 mmol) in 3 mL THF at 0 °C. The mixtures were stirred for 30 min until amides disappeared on TLC plates. The reaction mixture was quenched with satd. NH₄Cl, extracted with ethyl ether, dried with MgSO₄. The solvent was removed under reduced pressure to yield a crude product, which was purified by column chromatography to obtain pure compound **1h**. Yield: 27.3 %. ¹H NMR (300 MHz, CDCl₃) δ 8.29 (s, 1 H), 8.03 (q, 2 H), 7.80 (s, 2 H), 7.49-7.47 (m, 3 H), 3.96 (s, 6 H), 3.97 (s, 3 H). MS (ESI) *m*/*z* 378.1 (M + Na)⁺.

### Synthesis of 4-(2-Methyl-1-(3,4,5-trimethoxyphenyl)prop-1-enyl)-2-phenylthiazole (2a) [Figure 3]

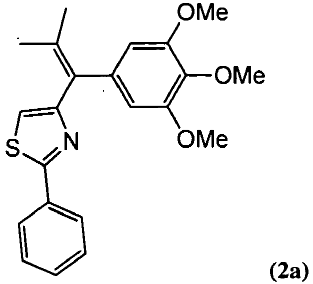

**4-(2-Methyl-1-(3,4,5-trimethoxyphenyl)prop-1-enyl)-2-phenylthiazole (2a) [****Figure 3****].** At -78 °C, to a solution of 223 mg isopropyl triphenylphosphonium iodide (0.52 mmol) in 5 mL of THF was added dropwise 0.4 mL of 1.6 N *n*-BuLi in hexane under Ar₂ protection. And the mixture was stirred at 0 °C for 40 min. A solution of 140 mg (0.39 mmol) of **1h** in 5 mL of THF was added dropwise at 0 °C, and the mixture was stirred for 1 h at RT. The reaction mixture was treated with saturated NH₄Cl solution. After a conventional workup, column chromatography (silica gel, petroleum ether/ethyl acetate) gave compound **2a** (86 mg, 57.3 %). ¹H NMR (300 MHz, CDCl₃) δ 7.98-7.97 (m, 2 H), 7.45-7.40 (m, 3 H), 6.77 (s, 1 H), 6.48 (s, 2 H), 3.86 (s, 3 H), 3.82 (s, 6 H), 2.15 (s, 3 H), 1.81 (s, 3 H). MS (ESI) *m*/*z* 404.1 (M + Na)⁺.

### Synthesis of (2-Phenylthiazol-4-yl)(3,4,5-trimethoxyphenyl)methanol (2b)[Figure 3]

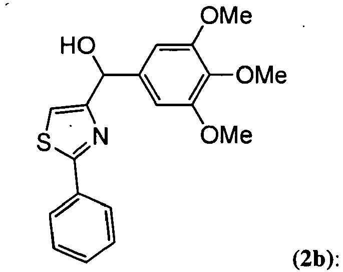

**2-Phenyl-4,5-dihydrothiazole-4-carboxylic acid (42a).** Benzonitrile (40 mmol) was combined with *L*-cysteine (45 mmol) in 100 mL of 1:1 MeOH/pH 6.4 phosphate buffer solution. The reaction was stirred at 40 °C for 3 days. The precipitate was removed by filtration, and MeOH was removed using rotary evaporation. To the remaining solution was added 1M HCl to adjust to pH = 2 under 0 °C. The resulting precipitate was filtered to yield a white solid 2-phenyl-4,5-dihydrothiazole-4-carboxylic acid **42a**, which was used directly to next step without purification.

**2-Phenylthiazole-4-carbaldehyde (42b).** At -78 °C, to a solution of 2-phenyl-thiazole-4-carboxylic acid methoxymethylamide (1equiv) in THF was added LiAlH₄ (1 equiv, 1 N in THF) and stirring for 1 h at -20 °C. The reaction mixture was placed on an ice bath and quenched by 20% H₂SO₄ solution, extracted with ethyl acetate and dried over MgSO₄. The solvent was removed under reduced pressure and purified by column chromatography to yield **42b** (45.8 %). ¹H NMR (300 MHz, CDCl₃) δ 10.1 (s, 1 H), 8.17 (s, 1 H), 8.02-8.00 (m, 2 H), 7.50-7.48 (m, 3 H). MS (ESI) *m*/*z* 244.1 (M + Na + MeOH)⁺.

**(2-Phenylthiazol-4-yl)(3,4,5-trimethoxyphenyl)methanol (2b) [****Figure 3****].** At 0 °C, to a solution of 104 mg of **42b** (0.55 mmol, 1 eq.) in 6 mL THF was added 3,4,5-trimethoxyphenylmagnesium bromide (0.5 N in THF, 2.9 mL). The mixtures were stirred for 30 min until aldehyde disappeared on TLC plates. The reaction mixture was quenched with satd. NH₄Cl, extracted with ethyl ether, dried with MgSO₄. The solvent was removed under reduced pressure to yield a crude product, which was purified by column chromatography to obtain pure compound **(2b)**. ¹H NMR (300 MHz, CDCl₃) δ 7.95-7.92 (m, 2 H), 7.44-7.43 (m, 4 H), 6.97 (s, 1 H), 6.76 (s, 2 H), 5.93 (d, 1 H, *J* = 3.6 Hz), 3.86 (s, 9 H). MS (ESI) *m*/*z* 402.1 (M + Na)⁺.

### Synthesis of (Z)-3-(2-phenylthiazol-4-yl)-3-(3,4,5-trimethoxyphenyl)acrylonitrile (2c-trans) and (E)-3-(2-phenylthiazol-4-yl)-3-(3,4,5-trimethoxyphenyl)acrylonitrile (2c-cis) [Figure 3]

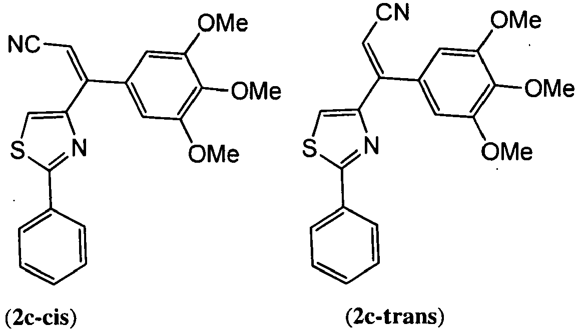

**(*Z*)-3-(2-phenylthiazol-4-yl)-3-(3,4,5-trimethoxyphenyl)acrylonitrile (2c-trans).** To a solution of 0.4 mL of 2.5 N *n*-BuLi in hexane and 10 mL of THF was added dropwise a solution of 177 mg (1 mmol) of diethyl cyanomethylphosphonate in 5 mL of THF at 0 °C under Ar₂. The ice bath was removed, and the mixture was stirred at 25 °C for 40 min. A solution of 200 mg (0.56 mmol) of **1h** in 10 mL of THF was added dropwise at 0 °C, and the mixture was stirred for 1 h at RT. The reaction mixture was treated with saturated NH₄Cl solution. After a conventional workup, column chromatography (silica gel, petroleum ether/ethyl acetate) gave compounds **2c-trans** (83 mg) and 2c-cis (76 mg). ¹H NMR (300 MHz, CDCl₃) δ 8.01-7.99 (m, 2 H), 7.44-7.40 (m, 3 H), 7.21 (s, 1 H), 6.74 (s, 2 H), 6.67 (s, 1 H), 3.93 (s, 3 H), 3.89 (s, 6 H). MS (ESI) *m*/*z* 401.1 (M + Na)⁺.

**(*E*)-3-(2-phenylthiazol-4-yl)-3-(3,4,5-trimethoxyphenyl)acrylonitrile (2c-cis).** ¹H NMR (300 MHz, CDCl₃) δ 8.07-8.05 (m, 2 H), 7.49-7.46 (m, 4 H), 6.66 (s, 2 H), 5.64 (s, 1 H), 3.91 (s, 3 H), 3.86 (s, 6 H). MS (ESI) *m*/*z* 401.1 (M + Na)⁺.

### Synthesis of (Z)-4-(hydrazono(3,4,5-trimethoxyphenyl)methyl)-2-phenylthiazole (2d-cis) and (E)-4-(hydrazono(3,4,5-trimethoxyphenyl)methyl)-2-phenylthiazole (2d-trans) [Figure 3]

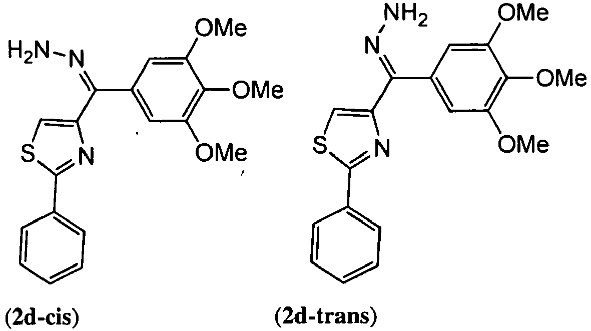

**(*Z*)-4-(hydrazono(3,4,5-trimethoxyphenyl)methyl)-2-phenylthiazole (2d-cis).** To a mixture of **1h** (230 mg, 0.65 mmol) in 3 mL CH₂Cl₂ and 3 mL ethanol was added hydrazine hydrate (2 mL). Then the mixture was refluxed for overnight. After completion of the reaction, the residue was absorbed on silica gel and purified by column chromatography to give compounds **2d-cis** (80 mg) and **2d-trans** (56 mg). ¹H NMR (300 MHz, CDCl₃) δ 8.01-7.98 (m, 2 H), 7.49-7.46 (m, 5 H), 7.33 (s, 1 H), 6.82 (s, 2 H), 3.87 (s, 3 H), 3.85 (s, 6 H). MS (ESI) *m*/*z* 370.1 (M + H)⁺.

**(*E*)-4-(hydrazono(3,4,5-trimethoxyphenyl)methyl)-2-phenylthiazole (2d-trans).** ¹H NMR (300 MHz, CDCl₃) δ 8.04-8.01 (m, 2 H), 7.44-7.40 (m, 3 H), 6.95 (s, 1 H), 6.65 (s, 2 H), 5.62 (s, 2 H), 3.93 (s, 3 H), 3.87 (s, 6 H). MS (ESI) *m*/*z* 370.1 (M + H)⁺.

### Synthesis of (Z)-(2-Phenylthiazol-4-yl)(3,4,5-trimethoxyphenyl)methanone oxime (2e-cis) and (E)-(2-Phenylthiazol-4-yl)(3,4,5-trimethoxyphenyl)methanone oxime (2e-trans) [Figure 3]

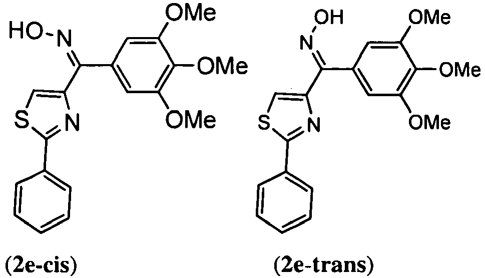

**(*Z*)-(2-Phenylthiazol-4-yl)(3,4,5-trimethoxyphenyl)methanone oxime (2e-cis)** To a suspension of **1h** (210 mg, 0.59 mmol) in 10 mL ethanol was added an aqueous solution (2 mL) of hydroxylamine hydrochloride (127 mg, 1.83 mmol). Then 2 mL 1 N NaOH was added dropwise to the reaction mixture and the mixture was stirred at 55 °C for 3 h. After completion of the reaction, the residue was absorbed on silica gel and purified by column chromatography to give compounds **2e-cis** (85 mg) and **2e-trans** (50 mg). ¹H NMR (300 MHz, DMSO-*d₆*) δ 11.95 (s, 1 H), 8.35 (s, 1 H), 7.91-7.89 (m, 2 H), 7.50-7.44 (br, 3 H), 6.85 (s, 2 H), 3.73 (s, 6 H), 3.70 (s, 3 H). MS (ESI) *m*/*z* 393.1 (M + Na)⁺; 368.9 (M - H)⁻.

**(*E*)-(2-Phenylthiazol-4-yl)(3,4,5-trimethoxyphenyl)methanone oxime 2e-trans).** ¹H NMR (300 MHz, DMSO-*d₆*) δ 11.49 (s, 1 H), 7.92-7.89 (m, 2 H), 7.64 (s, 1 H), 7.51-7.49 (m, 3 H), 7.34 (s, 1 H), 6.75 (s, 2 H), 3.75 (s, 6 H), 3.72 (s, 3 H). MS (ESI) *m*/*z* 393.1 (M + Na)⁺; 368.9 (M - H)⁻.

### Synthesis of (Z)-(2-Phenylthiazol-4-yl)(3,4,5-trimethoxyphenyl)methanone O-methyl oxime (2f-cis) and (E)-(2-Phenylthiazol-4-yl)(3,4,5-trimethoxyphenyl)methanone O-methyl oxime (2f-trans) [Figure 3]

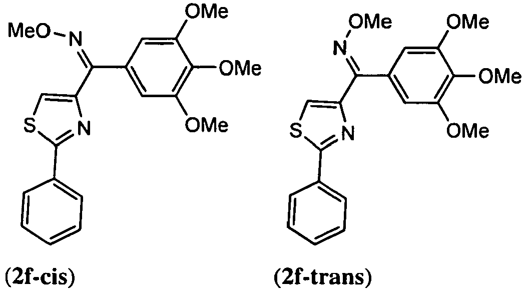

**(*Z*)-(2-Phenylthiazol-4-yl)(3,4,5-trimethoxyphenyl)methanone *O*-methyl oxime (2f-cis).** To a suspension of **1h** (110 mg, 0.59 mmol) in 10 mL pyridine was added O-methylhydroxylamine hydrochloride (52 mg, 0.63 mmol) and the mixture was stirred at 60 °C for overnight. The reaction was quenched with 1 N HCl solution, extracted with ethyl acetate and dried with MgSO₄. The solvent was removed under reduced pressure to yield a crude product, which was purified by column chromatography to give pure compounds **2f-cis** (41 mg) and **2f-trans** (33 mg). ¹H NMR (500 MHz, CDCl₃) δ 8.13 (s, 1 H), 7.96-7.94 (m, 2 H), 7.45-7.44 (m, 3 H), 6.94 (s, 2 H), 4.13 (s, 3 H), 3.91 (s, 6 H), 3.88 (s, 3 H). MS (ESI) *m*/*z* 407.2 (M + Na)⁺.

**(*E*)-(2-Phenylthiazol-4-yl)(3,4,5-trimethoxyphenyl)methanone *O*-methyl oxime (2f-trans).** ¹H NMR (500 MHz, CDCl₃) δ 8.00-7.98 (m, 2 H), 7.44-7.43 (m, 3 H), 7.28 (s, 1 H), 6.70 (s, 2 H), 4.08 (s, 3 H), 3.91 (s, 6 H), 3.85 (s, 3 H). MS (ESI) *m*/*z* 407.0 (M + Na)⁺.

### Synthesis of 2-Phenyl-N-(3,4,5-trimethoxyphenyl)thiazole-4-carboxamide (2g)

### [Figure 3]

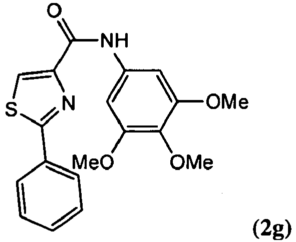

**2-Phenyl-*N*-(3,4,5-trimethoxyphenyl)thiazole-4-carboxamide (2g).** To a solution of **2e-cis** (21 mg, 0.06 mmol) in 5 mL CH₂Cl₂ was added p-toluenesulfonyl chloride (23 mg, 0.12 mmol) and NaH (5 mg, 60% in light mineral oil). Then the reaction mixture was stirred for 20 min. After completion of the reaction, the residue was absorbed on silica gel and purified by Al₂O₃ column chromatography to give compound **2g** (15 mg). ¹H NMR (300 MHz, CDCl₃) δ 9.22 (s, 1H), 8.19 (s, 1 H), 8.02-7.99 (m, 2 H), 7.52-7.50 (m, 3 H), 7.07 (s, 2 H), 3.92 (s, 6 H), 3.85 (s, 3 H). MS (ESI) *m*/*z* 371.1 (M + H)⁺.

### Synthesis of 3,4,5-Trimethoxy-N-(2-phenylthiazol-4-yl)benzamide (2h) [Figure 3]

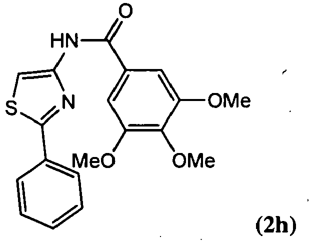

**3,4,5-Trimethoxy-*N*-(2-phenylthiazol-4-yl)benzamide (2h).** To a solution of **2e-**trans (26 mg, 0.07 mmol) in 5 mL CH₂Cl₂ was added *p*-toluenesulfonyl chloride (27 mg, 0.14 mmol) and NaH (5 mg, 60% in light mineral oil). Then the reaction mixture was stirred for 20 min. After completion of the reaction, the residue was absorbed on silica gel and purified by Al₂O₃ column chromatography to give compound **2h** (15 mg). ¹H NMR (300 MHz, CDCl₃) δ 8.88 (s, 1H), 7.94-7.91 (m, 2 H), 7.83 (s, 1 H), 7.48-7.46 (m, 3 H), 7.18 (s, 2 H), 3.97 (s, 6 H), 3.94 (s, 3 H). MS (ESI) *m*/*z* 393.1 (M + Na)⁺.

### Synthesis of N-((2-phenylthiazol-4-yl)(3,4,5-trimethoxyphenyl)methylene)cyanamide (2j) [Figure 3]

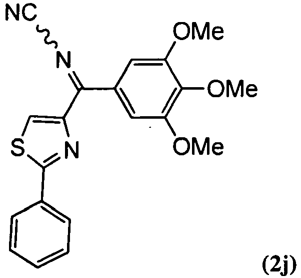

***N*-((2-phenylthiazol-4-yl)(3,4,5-trimethoxyphenyl)methylene)cyanamide (2j).** 100 mg of **1h** (0.28 mmol, 1 eq.) was dissolved in 10 mL methylene chloride. Titanium tetrachloride in methylene chloride (1.0 N, 0.7 mL, 2.5 eq.) was added dropwise at 0 °C and stirred for 30 min. Bis-trimethylsilylcarbodiimide (2.4 eq.) in 2 mL methylene chloride was added and the reaction stirred overnight protected from air and moisture. The reaction was treated with ice-water mixture followed by extraction with methylene chloride. The organic phase was dried over magnesium sulfate, filtered through celite and concentrated to give the crude acetophenone cyanoimines which were purified by flash column as isomers with a ratio of 3:7. ¹H NMR (300 MHz, CDCl₃) δ 8.72 (br, 0.3 H), 8.63 (s, 0.7 H), 8.09-8.07 (m, 1.4 H), 7.99 (br, 0.6 H), 7.58-7.56 (br, 3 H), 7.26 (s, 1.4 H), 7.18 (s, 0.6 H), 3.84, 3.83 (s, s, 6 H), 3.82 (s, 3 H). MS (ESI) *m*/*z* 402.1 (M + Na)⁺.

### Synthesis of N-((4-hydroxy-3,5-dimethoxyphenyl)(2-phenylthiazol-4-yl)methylene)cyanamide (32).

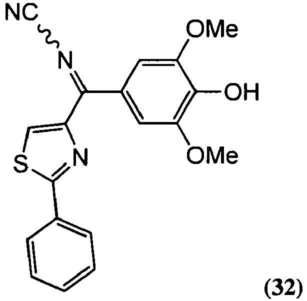

***N*-((4-hydroxy-3,5-dimethoxyphenyl)(2-phenylthiazol-4-yl)methylene)cyanamide (32)** was obtained as a by-product from synthesis of **2j.** ¹H NMR (500MHz, CDCl₃) δ 8.23 (s, 1 H), 8.02 (m, 2 H), 7.92 (s, 2 H), 7.55 (m, 3 H), 6.02 (s, 1 H), 3.99 (s, 6 H). MS (ESI) m/z 364.1(M + H)⁺.

### Synthesis of (Z)-2-Phenyl-4-(3,4,5-trimethoxystyryl)thiazole (3a) and (E)-2-Phenyl-4-(3,4,5-trimethoxystyryl)thiazole (3b) [Figure 4]

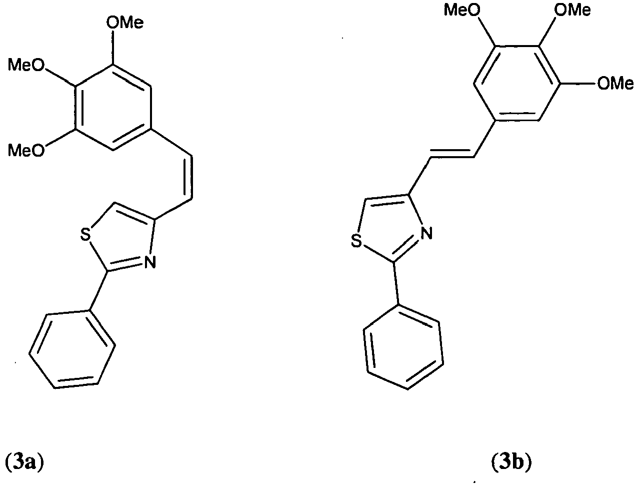

**(*Z*)-2-Phenyl-4-(3,4,5-trimethoxystyryl)thiazole (3a).** Triphenylphosphine (3.41 g, 13 mmol) was added to a solution of 5-(bromomethyl)-1,2,3-trimethoxybenzene (2.61 g, 10 mmol) in dry THF (30 mL). The mixture was refluxed with stirring for 6 h. The resulting white solid was filtered and washed with ether/hexane to afford the product 3,4,5-trimethoxybenzyltriphenylphosphonium bromide in 96.4% yield. ¹H NMR (500 MHz, CDCl₃) δ 7.77-7.73, 7.65-7.61 (m, 15 H), 6.44 (d, 2 H, *J* = 1.5 Hz), 5.37 (d, 2 H, *J* = 14 Hz), 3.76 (s, 3 H), 3.51 (d, 6 H); MS (ESI) *m*/*z* 443.1 (M - Br]⁺. At -78 °C, n-BuLi (0.42 mL, 2.5 N in hexane) was added to a solution of 3,4,5-trimethoxybenzyltriphenylphosphonium bromide (500 mg, 0.96 mmol) in 10 mL THF. After stirring at RT for 2 h, aldehyde **42b** (109 mg, 0.58 mmol) in 3 mL THF was charged and stirred for 30 min. The reaction mixture was treated with saturated NH₄Cl solution. After a conventional workup, column chromatography (silica gel, petroleum ether/ethyl acetate) gave compounds **3a** (57 mg) and **3b** (99 mg). ¹H NMR (500 MHz, CDCl₃) δ 7.90-7.89 (m, 2 H), 7.42-7.40 (m, 3 H), 7.07 (s, 1 H), 6.71 (s, 2 H), 6.66 (s, 1 H), 3.87 (s, 6 H), 3.75 (s, 3 H); MS (ESI) *m*/*z* 376.1 (M + Na)⁺.

**(*E*)-2-Phenyl-4-(3,4,5-trimethoxystyryl)thiazole (3b).** ¹H NMR (500 MHz, CDCl₃) δ 8.03-8.01 (m, 2 H), 7.52 (d, 1 H, *J* = 16 Hz), 7.47-7.44 (m, 3 H), 7.16 (s, 1 H), 7.05 (d, 1 H, *J* = 16 Hz), 6.79 (s, 2 H), 3.92 (s, 6 H), 3.88 (s, 3 H). MS (ESI) *m*/*z* 354.1 (M + H)⁺.

### Synthesis of Biphenyl-3-yl(3,4,5-trimethoxyphenyl)sulfane (4a), 3-(3,4,5-Trimethoxyphenylsulfonyl)biphenyl (4b) and 3-(3,4,5-Trimethoxyphenylsulfinyl)biphenyl (4c) [Figure 4]

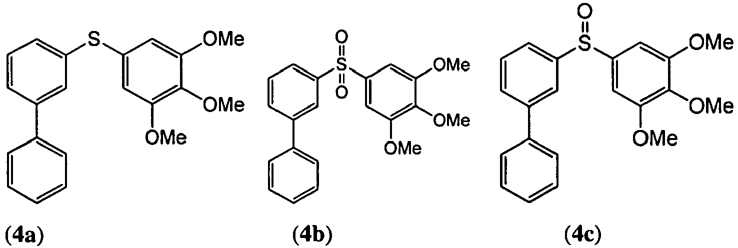

***S*-Biphenyl-3-yl *O*-ethyl carbonodithioate (52a).** To a solution of 1 equiv. of biphenyl-3-amine (1 g, 5.92 mmol) in water (7.3 mL) at 0 °C was added concentrated hydrochloric acid (1 mL). A cold solution of 1.1 equiv. of sodium nitrite (450 mg, 6.5 mmol) in water (3 mL) was added slowly and stirred for 15 min. The cold diazonium solution was added slowly to a solution of 1.3 equiv. of potassium ethyl xanthate (1.16 g, 1.3 mmol) in water (1.3 mL) at 45 °C. The reaction mixture was stirred for an additional 30 min at 45 °C and then cooled to RT. The reaction mixture was extracted with diethyl ether (3 x 50 mL). The combined organic extracts were washed with 1 N NaOH solution (100 mL), water (3 x 50 mL), brine (50 mL), dried over MgSO₄, filtered and evaporated under reduced pressure. The resulting crude xanthate **52a** was used directly in the next step without further purification. MS (ESI) *m*/*z* 275.0 (M + H)⁺.

**Biphenyl-3-yl(3,4,5-trimethoxyphenyl)sulfane (4a).** To a solution of **52a** (1.1 g, crude compound) in ethanol (8 mL) was added potassium hydroxide (2.1 g, 12 mL) and heated to reflux for overnight. The solution was cooled to RT and the ethanol was evaporated under reduced pressure. The residue was dissolved in water and washed with diethyl ether (10 mL). The aqueous layer was acidified with 2 N HCl and extracted with diethyl ether (3 x 50 mL). The organic extracts were washed with water (50 mL), brine (50 mL), dried over MgSO₄, filtered and evaporated under reduced pressure to afford 0.85 g (77.3 %) of crude biphenyl-3-thiol product (overall, 3 steps). Into a round-bottomed flask, stirred magnetically, were placed 0.1 g (1.04 mmol) of sodium *tert*-butoxide and 83 mg of copper iodide (0.43 mmol). After the reaction vessel was sealed, 0.13 g (0.71 mmol) of 4-methoxybenzenethiol and 0.19 g (0.65 mmol) of 5-iodo-1,2,3-trimethoxybenzene in 3.0 mL of toluene were injected through the septum. The reaction mixture was heated for overnight at 110 °C. Purification was performed by flash chromatography, and an amorphous solid was obtained (40% yield). ¹H NMR (500 MHz, CDCl₃) δ 7.54-7.52 (m, 3 H), 7.44-7.41 (m, 3 H), 7.37-7.33 (m, 2 H), 7.23 (s, br, 1 H), 6.69 (s, 2 H), 3.86 (s, 3 H), 3.80 (s, 6 H). MS (ESI) *m*/*z* 353.2 (M + H)⁺.

**3-(3,4,5-Trimethoxyphenylsulfonyl)biphenyl (4b).** To a solution of 60 mg (0.17 mmol) of compound 4a and 5 mL of dichloromethane was added very slowly 2 equiv. of *m-*CPBA over 3 h. Sulfoxide formation was monitored by thin-layer chromatography. Purification was performed with a flash chromatographic column, and an amorphous powder of **(4b)** was obtained (73% yield). ¹H NMR (500 MHz, CDCl₃) δ 8.14 (br, 1 H), 7.89 (d, 1 H), 7.78 (d, 1 H), 7.59-7.56 (m, 3 H), 7.49-7.39 (m, 3 H), 7.19 (s, 2 H), 3.89 (s, 6 H), 3.87 (s, 3 H). MS (ESI) *m*/*z* 385.0 (M + Na)⁺.

**3-(3,4,5-Trimethoxyphenylsulfinyl)biphenyl (4c).** At 0 °C, to a solution of 500 mg (1.42 mmol) of compound **(4a)** and 5 mL of dichloromethane was added very slowly 1 equiv. of *m*-CPBA over 3 h. Sulfoxide formation was monitored by thin-layer chromatography. Purification was performed with a flash chromatographic column, and an amorphous powder of **(4c)** was obtained (87% yield). ¹H NMR (500 MHz, CDCl₃) δ 7.92 (br, 1 H), 7.71 (d, 2 H), 7.62-7.60 (m, 3 H), 7.58-7.40 (m, 4 H), 6.94 (s, 2 H), 3.79 (s, 3 H), 3.74 (s, 6 H). MS (ESI) *m*/*z* 369.1 (M + H)⁺.

### Synthesis of N-(3,4,5-trimethoxyphenyl)biphenyl-3-sulfonamide (4d) [Figure 4]

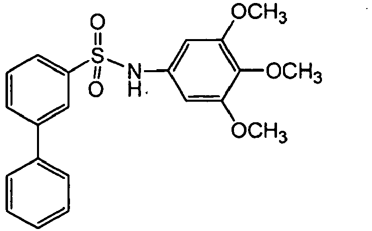

***N*-(3,4,5-Trimethoxyphenyl)biphenyl-3-sulfonamide (4d).** A mixture of 65 mg of biphenyl-3-sulfonyl chloride (0.25 mmol), 44 mg of 3,4,5-trimethoxyaniline (0.24 mmol), and 0.3 mmol of triethylamine in 5 mL DMF was stirred overnight. The reaction mixture was treated with water and extracted with ethyl acetate. After a conventional workup, column chromatography (silica gel, petroleum ether/ethyl acetate) gave 88 mg compounds **(4d)** (91.7%). ¹H NMR (500 MHz, CDCl₃) δ 7.96 (t, 1 H, *J* = 1.8 Hz), 7.81-7.74 (m, 2 H), 7.57-7.40 (m, 6 H), 6.33 (s, 2 H), 3.86 (s, 3 H), 3.80 (s, 6 H). MS (ESI) *m*/*z* 422.1 (M + Na)⁺.

### 2-Phenyl-4-(3,4,5-trimethoxyphenyl)thiazole (2i) [Figure 4]

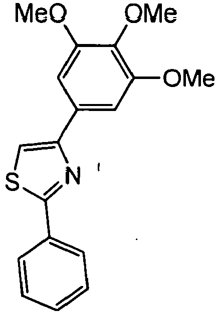

**2-Phenyl-4-(3,4,5-trimethoxyphenyl)thiazole (2i).** Bromine (160 mg, 1 mmol) was added dropwise to a stirred solution of an 1-(3,4,5-trimethoxyphenyl)ethanone (210 mg, 1 mmol) in ethanol (30 mL) and the solution was stirred at 0 °C for 1 h and then poured into water to form a precipitate. This was recrystallized from ethanol to give bromoacetophenone (70%) and used directly for next step. A mixture of bromoacetophenone (288 mg, 1 mmol) and benzothioamide (137 mg, 1 mmol) in ethanol was refluxed for 1 h. The reaction mixture was concentrated in vacuo and purified with flash column to give **2i** (167 mg, 51.1%). ¹H NMR (500 MHz, CDCl₃) δ 8.05-8.03 (m, 2 H), 7.48-7.44 (m, 3 H), 7.41 (s, 1 H), 7.22 (s, 2 H), 3.97 (s, 6 H), 3.89 (s, 3 H). MS (ESI) *m*/*z* 350.1 (M + Na)⁺.

### REFERENCE EXAMPLE 3

### SYNTHESIS OF METHOXY BENZOYL THIAZOLE COMPOUNDS HAVING DIFFERENT "A" RINGS AND/OR SUBSTITUTED "A" RING

The compounds of this disclosure possess different substituted or unsubstituted A rings such as phenyl or indolyl. Such compounds were synthesized according to Figures 5 and 6.

Hydroxyl and aminomethyl were introduced at the *para*-position of the phenyl A-ring, as well as the phenyl was replaced with 5-indolyl and 2-indolyl rings. Weinreb amides **57a, 61a, 65a,** and **67a** were prepared by the procedure presented in Figure 5 using aryl nitriles as starting materials. 2-Cyano-indole **60a** was prepared according to a standard procedure (Pletnev, A. A.; Tian, Q.; Larock, R. C., Carbopalladation of nitriles: synthesis of 2,3-diarylindenones and polycyclic aromatic ketones by the Pd-catalyzed annulation of alkynes and bicyclic alkenes by 2-iodoarenenitriles. J Org Chem 2002, 67(26), 9276-87). Protections of hydroxyl (TBDMSCl), indolyl (PhSO₂Cl) and amino (Boc₂O) groups were used in preparations. Deprotection of TBDMS and oxidation from thiazoline (**58a**) to thiazole (**21**) took place in one-step using TBAF/THF solution. This thiazoline-thiazole oxidation takes place spontaneously in the reaction of thiazoline Weinreb amide and Grignard reagent. The same phenomena is observed during preparation of the indole compounds **62a** and **66a**.

Compound **62a** was separated as a pure thiazole compound after reaction with 3,4,5-trimethoxphenyllithium without the need for further oxidation. Compound **66a** was obtained by removing the phenylsulfonyl protecting groups in hot NaOH ethanol solution. *para-OH* and NH₂ on the A ring of **2l** and **2r** were obtained by similar Grignard reactions from the Weinreb amides **58a** and **68a**. Compound **2r** was further converted to the HCl salt (**2r-HCl**) and the HCl salt of monomethyl amine **2s-HCl** using NaH/MeI conditions and dimethylamine **2u** under HCHO/NaBH₃CN conditions.

### Substituted A ring:

### Synthesis of (2-(4-Hydroxyphenyl)thiazol-4-yl)(3,4,5-trimethoxyphenyl)methanone (2l) [Figure 5]

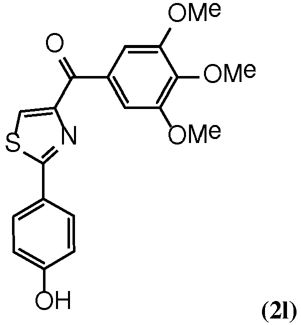

**(*R*)-2-(4-Hydroxyphenyl)-*N*-methoxy-*N*-methyl-4,5-dihydrothiazole-4-carboxamide (57a) was synthesized using the same method as used for 38d.** Quantitative yield. ¹H NMR (500 MHz, CDCl₃) δ 7.56 (d, 2 H, *J* = 8.5 Hz), 6.84 (br, 1 H), 6.73 (d, 2 H, *J* = 8.5 Hz), 5.64 (t, br, 1 H), 3.87 (s, 3 H), 3.30 (s, 3 H). MS (ESI) *m*/*z* 289.0 (M + Na)⁺, 264.9 (M - H)⁻.

**(*R*)-(2-(4-(*tert*-Butyldimethylsilyloxy)phenyl)-4,5-dihydrothiazol-4-yl)(3,4,5-trimethoxyphenyl)methanone (58a) was synthesized using the same method as used for (35a)-see Example 1.** 67.0% yield. ¹H NMR (300 MHz, CDCl₃) δ 7.73 (d, 2 H, *J* = 8.7 Hz), 7.61 (s, 2 H), 6.83 (d, 2 H, *J* = 8.7 Hz), 5.95 (dd, 1 H, *J* = 8.1 Hz, 9.0 Hz), 4.09, (dd, 1 H, *J* = 7.8 Hz, 11.1 Hz), 3.95 (s, 3 H), 3.94 (s, 6 H), 3.55 (dd, 1 H, *J* = 9.3 Hz, 11.1 Hz), 0.97 (s, 9 H), 0.19 (s, 6 H). MS (ESI) *m*/*z* 510.4 (M + Na)⁺, 486.0 (M -H)⁻.

**(2-(4-Hydroxyphenyl)thiazol-4-yl)(3,4,5-trimethoxyphenyl)methanone (2l).** At 0 °C, to a solution of **58a** (0.2 mmol) in 5 mL CH₂Cl₂ was added a solution of tetrabutylammonium fluoride in THF (1 N, 0.6 mmol) and stirred at RT for around 14 h until reaction was finished by TLC monitor. 67.0% yield. ¹H NMR (500 MHz, DMSO-*d₆*) δ 10.1 (s, 1 H), 8.51 (s, 1 H), 7.85 (d, 2 H, *J* = 8.50 Hz), 7.62 (s, 2 H), 6.91 (d, 2 H, *J* = 8.5 Hz), 3.86 (s, 6 H), 3.79 (s, 3 H). MS (ESI) *m*/*z* 394.1 (M + Na)⁺, 369.9 (M -H)⁻.

### (2-(4-(Aminomethyl)phenyl)thiazol-4-yl)(3,4,5-trimethoxyphenyl)methanone hydrochloride (2r or 2r-HCl) [Figure 5]

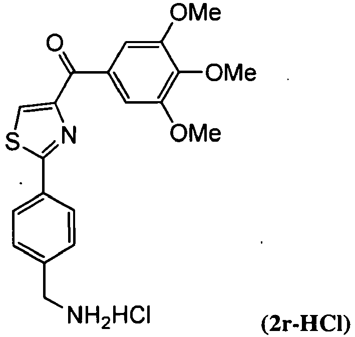

**(*R*)-*tert*-Butyl 4-(4-(methoxy(methyl)carbamoyl)-4,5-dihydrothiazol-2-yl)benzyl carbamate (67a).** 4-(Aminomethyl)benzonitrile (25.09 g, 0.149 mol) and *L*-cysteine (18.1 g, 0.149 mol) were suspended in 500 mL MeOH and pH 6.4 buffer solutions (1:1) and stirred for 3 days at RT. Triethylamine (30 mL) was added to the mixture and Boc₂O (68 g, 0.31mol) was added to this mixture and stirred for 2 h. The solvents were removed and filtered to yield white solid (*R*)-2-(4-((*tert*-butoxycarbonylamino)methyl)phenyl)-4,5-dihydrothiazole-4-carboxylic acid (38.4 g, 76.8%). Compound **67a** was obtained from this acid following the same method as used for **38d**. Yield: 84.4 %. ¹H NMR (500 MHz, CDCl₃) δ 7.75 - 7.77 (d, 2 H, *J* = 7.5 Hz), 7.27 - 7.26 (d, 2 H, *J* = 7.5 Hz), 7.23 (s, 1 H), 5.62 (br, 1 H), 4.87 (br, 1 H), 4.30 (br, 2 H), 3.86 (s, 3 H), 3.78 (t, *J* = 10.0 Hz, 1 H), 3.48 - 3.4 (m, 1 H), 3.25 (s, 3 H), 1.42 (s, 9 H). MS (ESI) m/z 402.1(M + Na)⁺, 378.0 (M - H)⁻.

***tert*-Butyl 4-(4-(3,4,5-trimethoxybenzoyl)thiazol-2-yl)benzylcarbamate (68a).** A mixture of **67a** (2.5 mmol), CBrCl₃ (3.2 mmol) and DBU (5.0 mmol) in CH₂Cl₂ (20 mL) was stirred overnight. The reaction mixture was absorbed on silica gel and purified by column chromatography to yield an intermediate thiazole Weinreb amide. To a solution of (3,4,5-trimethoxyphenyl)magnesium bromide (0.5 M, 5.5 mL) in THF was added a solution of the intermediate thiazole Weinreb amide (1.83 mmol) in 10 mL THF under 0 °C and stirred for 30 min. The reaction mixture was quenched with satd. NH₄Cl, extracted with ethyl ether, dried with MgSO₄. The solvent was removed under reduced pressure to yield a crude product, which was purified by column chromatography to obtain pure compound as a light yellow solid (32.3 %). ¹H NMR (300M, CDCl₃) δ 8.27 (s, 1 H), 7.98 (d, 2 H, *J* = 8.1 Hz), 7.78 (s, 2 H), 7.39 (d, 2 H, *J* = 8.1 Hz), 7.27 - 7.26 (d, 2 H, *J* = 7.5 Hz), 7.23 (s, 1 H), 4.93 (br, 1 H), 4.37 (br, d, 1 H), 3.96 (s, 3 H), 3.95 (s, 6 H), 1.47 (s, 9 H); MS (ESI) m/z 507.1 (M + Na)⁺.

**(2-(4-(Aminomethyl)phenyl)thiazol-4-yl)(3,4,5-trimethoxyphenyl)methanone hydrochloride (2r** or **2r-HCl).** At 0 °C, to a solution of 68a (200 mg) in 10 mL CH₂Cl₂ was added a solution of HCl in 1,4-dioxane (4 N, 2 mL) and stirred at RT for 4 h. The precipitate **(2r)** was filtered and washed with diethyl ether. Yield: 81.3%. ¹H NMR (500 MHz, DMSO-*d₆*) δ 8.68 (s, 1 H), 8.38 (br, 3 H), 8.10 (d, 2 H, *J* = 8.4 Hz), 7.66 (d, 2 H, *J* = 8.4 Hz), 7.62 (s, 2 H), 4.11 (s, 2 H), 3.87 (s, 6 H), 3.80 (s, 3 H). MS (ESI) *m*/*z* 385.1 (M + H)⁺.

### (2-(4-((Dimethylamino)methyl)phenyl)thiazol-4-yl)(3,4,5-trimethoxyphenyl)methanone hydrochloride (2u or 2u-HCl) [Figure 5]

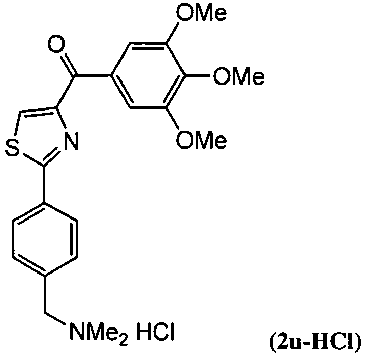

***tert*-Butyl methyl(4-(4-(3,4,5-trimethoxybenzoyl)thiazol-2-yl)benzyl)carbamate (71a).** At 0 °C, to a solution of compound **68a** (100 mg, 0.2 mmol) in 5 mL DMF was added sodium hydride (10 mg, 0.2 mmol), then iodomethane (77 mg, 0.4 mmol) was added to the reaction mixture and stirred at RT overnight. The mixture was quenched with a sat. NaHCO₃ solution, extracted with ethyl acetate and dried with MgSO₄. The solvent was removed under reduced pressure to yield a crude product, which was purified by column chromatography to obtain pure compound **71a**. Yield: 61.3%. ¹H NMR (500 MHz, DMSO-*d₆*) δ 8.30 (s, 1 H), 8.02 (d, 2 H, *J* = 8.0 Hz), 7.82 (s, 2 H), 7.36 (br, 2 H), 4.50 (s, 2 H), 4.00 (s, 3 H), 3.98 (s, 6 H), 2.90 (d, br, 3 H), 1.50 (s, 9 H). MS (ESI) *m*/*z* 521.2 (M + Na)⁺, 496.9 (M - H)⁻.

**(2-(4-((Methylamino)methyl)phenyl)thiazol-4-yl)(3,4,5-trimethoxyphenyl)methanone hydrochloride (2s** or **2s-HCl).** At 0 °C, to a solution of **71a** (60 mg) in 5 mL CH₂Cl₂ was added a solution of HCl in 1,4-dioxane (4 N, 2 mL) and stirred at RT for overnight. The precipitate (**2s-HCl**) was filtered and washed with diethyl ether. Yield: 81.3%. ¹H NMR (500 MHz, CDCl₃) δ 10.0 (s, 1 H), 8.29 (s, 1 H), 8.05 (d, 2 H, *J* = 6.0 Hz), 7.74 (s, 2 H), 7.72 (d, 2 H, *J* = 6.0 Hz), 4.15 (s, 2 H), 3.99 (s, 3 H), 3.96 (s, 6 H), 2.61 (s, 3 H). MS (ESI) *m*/*z* 399.1 (M + H)⁺.

**(2-(4-((Dimethylamino)methyl)phenyl)thiazol-4-yl)(3,4,5-trimethoxyphenyl)methanone hydrochloride (2u or 2u-HCl).** To a solution of **2r** (53 mg, 0.14 mmol) in 5 mL CH₂Cl₂ was added formaldehyde solution (37% in H₂O, 340 mg, 4.2 mmol), and sodium cyanoborohydride (34 mg, 0.55 mmol), the reaction mixture was absorbed on silica gel and free base was purified after flash column (41 mg, 70.9%). At 0 °C, to a solution of free base (41 mg) in 5 mL CH₂Cl₂ was added a solution of HCl in 1, 4-dioxane (4 N, 2 mL) and stirred at RT for overnight. The precipitate **(2u)** was filtered and washed with diethyl ether. Yield: 71.3%. ¹H NMR (500 MHz, CDCl₃) δ 13.0 (s, 1 H), 8.34 (s, 1 H), 8.13 (d, 2 H, *J* = 7.0 Hz), 7.82 (d, 2 H, *J* = 7.5 Hz), 7.75 (s, 2 H), 4.24 (s, 2 H), 3.99 (s, 3 H), 3.97 (s, 6 H), 2.83 (s, 6 H). MS (ESI) *m*/*z* 413.1 (M + H)⁺.

### 2-(4-(4-(3,4,5-Trimethoxybenzoyl)thiazol-2-yl)phenyl)acetonitrile (2n)

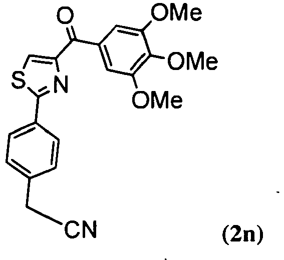

**2-(4-(4-(3,4,5-Trimethoxybenzoyl)thiazol-2-yl)phenyl)acetonitrile (2n)** was prepared using the same method as used of compound **1h** from terephthalonitrile and cysteine. ¹H NMR (500MHz, CDCl₃) δ 8.30 (s, 1 H), 8.04 (d, 2 H), 7.76 (s, 2 H), 7.46 (d, 2 H), 3.97 (s, 3 H), 3.95 (s, 6 H), 3.83 (s, 2 H).

### Synthesis of (2-(4-(Dimethylamino)phenyl)thiazol-4-yl)(3,4,5-trimethoxyphenyl)methanone (2o)

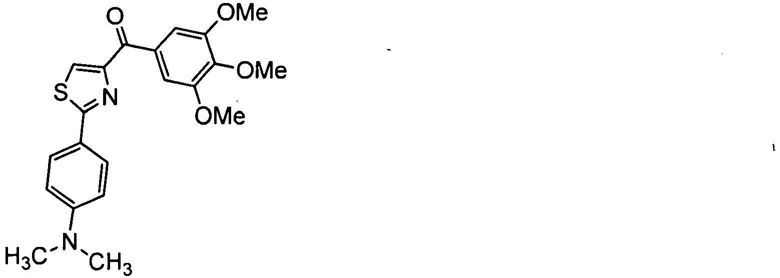

**(2-(4-(Dimethylamino)phenyl)thiazol-4-yl)(3,4,5-trimethoxyphenyl)methanone (2o)** was prepared using the same method as used of compound **1h** from 4-(dimethylamino)benzonitrile and cysteine. ¹H NMR (300MHz, CDCl₃) δ 8.12 (s, 1 H), 7.88 (d, 2 H), 7.80 (s, 2 H), 6.73 (d, 2 H), 3.96 (s, 3 H), 3.95 (s, 6 H), 3.05 (s, 6 H); MS (ESI) m/z 421.1 (M + Na)⁺.

### Indolyl A ring:

### Synthesis of (2-(1H-indol-2-yl)thiazol-4-yl)(3,4,5-trimethoxyphenyl)methanone (62a) [Figure 5]

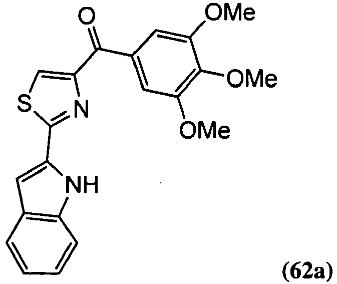

**1*H*-Indole-2-carbonitrile (60a).** To a cooled solution of indole-2-carboxylic acid (2.0 g, 12.4 mmol) in 60 mL of anhydrous Et₂O was added 1.9 mL of SOCl₂ (26 mmol). After stirring for 40 min at RT, the ether was removed under reduced pressure at a temperature not exceeding 35 °C. The obtained acyl chloride was dissolved in 40 mL of anhydrous Et₂O and the resulting solution was added immediately to a stirred solution of liquid ammonia in 80 ml of Et₂O. The reaction mixture was stirred at RT for 24 h. The solvent was then evaporated under reduced pressure, and the white indole-2-carboxamide was crystallized from 50% aq EtOH and dried in air, after which it was dissolved in POCl₃ and heated under reflux for 5 min. The cooled solution was poured onto crushed ice and aq NH₄OH was added to maintain a basic pH. The aqueous mixture was extracted with Et₂O, the extracts were dried over Na₂SO₄ and evaporated. The brown indole-2-carbonitrile **60a** (63.3% overall yield from indole-2-carboxylic acid) was obtained. ¹H NMR (500 MHz, CDCl₃) δ 8.56 (br, s, 1 H), 7.68 (d, 1 H, *J* = 8.0 Hz), 7.43-7.34 (m, 2 H), 7.24-7.21 (m, 2 H). MS (ESI) *m*/*z* 144.0 (M + H)⁺, 140.8 (M -H)⁻.

**(*R*)-2-(1*H*-indol-2-yl)-*N*-methoxy-*N*-methyl-4,5-dihydrothiazole-4-carboxamide (61a) was synthesized using the same method** as **used of 38d.** 67.1% yield. ¹H NMR (300 MHz, CDCl₃) δ 9.06 (s, br, 1 H), 7.64 (d, 2 H, *J* = 8.1 Hz), 7.36-7.24 (m, 2 H), 7.12 (dt, 1 H, *J* = 8.1 Hz, 1.2 Hz), 6.95 (d, 1 H, *J* = 1.8 Hz), 5.60 (t, br, 1 H, *J* = 8.7 Hz), 3.86 (s, 3 H), 3.78 (t, 1 H, *J* = 10.2 Hz), 3.58 (dd, 1 H, *J* = 9.0 Hz, 10.2 Hz), 3.30 (s, 3 H). MS (ESI) *m*/*z* 312.1 (M + Na)⁺, 287.9 (M - H)⁻.

**(2-(1*H*-indol-2-yl)thiazol-4-yl)(3,4,5-trimethoxyphenyl)methanone** (62a) was synthesized from 61a using the same method as used for 35a. 45.8% yield. ¹H NMR (500 MHz, DMSO-*d₆*) δ 9.26 (s, 1 H), 8.11 (s, 1 H), 7.66 (d, 1 H, *J* = 8.0 Hz), 7.46 (s, 2 H), 7.42 (d, 1 H, *J* = 8.0 Hz), 7.29 (t, 1 H, *J* = 7.5 Hz), 7.16 (t, 1 H, *J* = 7.5 Hz), 7.10 (s, 1 H), 3.97 (s, 3 H), 3.93 (s, 6 H). MS (ESI) *m*/*z* 417.1 (M + Na)⁺, 392.9 (M -H)⁻.

### Synthesis of (2-(1H-indol-5-yl)thiazol-4-yl)(3,4,5-trimethoxyphenyl)methanone (66a) [Figure 5]

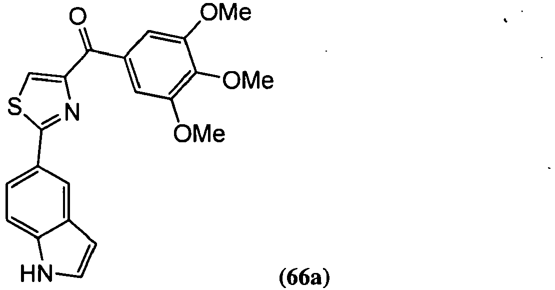

**(*R*)-2-(1-(Phenylsulfonyl)-1*H*-indol-5-yl)-4,5-dihydrothiazole-4-carboxylic acid (64a).** (*R*)-2-(1*H*-indol-5-yl)-4,5-dihydrothiazole-4-carboxylic acid **63a** was synthesized using the same method as used for **42a** from 1*H*-indole-5-carbonitrile and used without further purification. To a vigorously stirring solution of **63a** (1 mmol) and tetrabutylammonium hydrogen sulfate (0.15 mmol) in toluene (10 mL) at 0 °C was added 50% aqueous sodium hydroxide (10 mL) and sulfonyl chloride (2 mmol). The resultant solution was stirred at RT for 6 h. Then 1 N HCl was added to acidify the mixture to pH=2 and extracted with CH₂Cl₂, the organic layer was separated and dried (MgSO₄); then evaporated to dryness to yield **64a,** which were used in subsequent steps without further purification.

**(*R*)-*N*-methoxy-*N*-methyl-2-(1-(phenylsulfonyl)-1*H*-indol-5-yl)-4,5-dihydrothiazole-4-carboxamide (65a) was prepared from 64a with the same method as used for 38d.** 57.1% yield. ¹H NMR (500 MHz, CDCl₃) δ 7.92 (m, 2 H), 7.77 (m, 3 H), 7.51 (d, 1 H, *J* = 3.0 Hz), 7.46 (t, 1 H), 7.35 (t, 1H), 6.61 (d, 1 H), 5.58 (br, t, 1 H) 3.82 (s, 3 H), 3.73 (t, 1 H), 3.43 (m, 1 H), 3.21 (s, 3 H). MS (ESI) *m*/*z* 452.1 (M + Na)⁺.

**(2-(1*H*-indol-5-yl)thiazol-4-yl)(3,4,5-trimethoxyphenyl)methanone (66a).** To a solution of *n*-BuLi (1.6 M, 1.7 mL) in 8 mL THF was added a solution of 3,4,5-trimethoxybromobenzene (2.47 mmol) in 3 mL THF under -78 °C. The mixture was allowed to stir for 2h and a solution of Weinreb amide **65a** (1.24 mmol) in 3 mL THF was charged. The temperature was allowed to increase at RT and stirred overnight. The reaction mixture was quenched with satd. NH₄Cl, extracted with ethyl ether, dried with MgSO₄. The solvent was removed under reduced pressure to yield a crude product, which was refluxed in 1 N NaOH in 5 mL ethanol solution to obtain the deprotected compound 66a and purified by column chromatography to obtain pure compound as a light yellow solid (36.3 %). ¹H NMR (300M, CDCl₃) δ 8.36 (br, s, 1 H), 8.31 (s, 1 H), 8.21 (s, 1 H), 7.92, 7.89 (dd, 1 H, *J* = 1.8, 2.7 Hz), 7.46 (d, 1 H,) 7.62 (s, 2 H, *J* = 8.7 Hz), 7.29 (t, 1 H, *J* = 2.7 Hz), 6.64 (br, 1 H), 3.97 (s, 6 H), 3.97 (s, 3 H); MS (ESI) m/z 417.1 (M + Na)⁺, 392.9 (M - H)⁻.

### Synthesis of (2-(1H-Indol-2-yl)thiazol-4-yl)(1H-indol-2-yl)methanone (8).

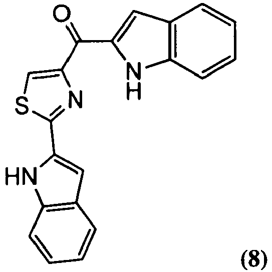

**(2-(1*H*-Indol-2-yl)thiazol-4-yl)(1*H*-indol-2-yl)methanone (8)** was prepared using the similar method as used of compound **1h** from 2-(1*H*-indol-2-yl)-4,5-dihydrothiazole-4-carboxylic acid and cysteine. ¹H NMR (500MHz, CDCl₃) δ 9.39 (s, 1 H), 8.54 (s, 1 H), 8.46 (s, 1 H), 8.06 (s, 1 H), 8.03 (dd, 1 H), 7.66 (d, 1 H), 7.51 (d, 1 H), 7.41 (d, 1 H), 7.33 (t, 1 H), 7.29 (d, 1 H), 7.15 (t, 1 H), 7.09 (d, 1 H), 6.72 (s, 1 H). MS (ESI) m/z 366.1 (M + Na)⁺, 341.9 (M - H)⁻.

### Synthesis of (2-(1H-indol-2-yl)thiazol-4-yl)(1H-indol-5-yl)methanone (21).

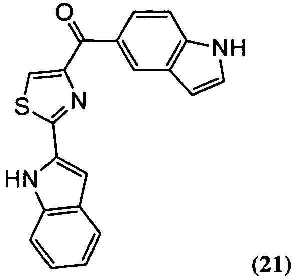

**(2-(1*H*-indol-2-yl)thiazol-4-yl)(1*H*-indol-5-yl)methanone (21)** was prepared using the similar method as used of compound **1h** from 2-(1*H*-indol-2-yl)-4,5-dihydrothiazole-4-carboxylic acid and cysteine. ¹H NMR (500MHz, CDCl₃) δ 9.60 (s, 1 H), 9.26 (s, 1 H), 8.31 (s, 1 H), 8.03 (s, 1 H), 7.83 (dd, 1 H), 7.69 (d, 1 H), 7.53-7.49 (m, 2 H), 7.41 (t, 1 H), 7.33 (t, 1 H), 7.21-7.18 (m, 2 H), 7.13 (s, 1 H). MS (ESI) m/z 366.1(M + Na)⁺, 341.9 (M - H)⁻.

### EXAMPLE 4

### SYNTHESIS OF COMPOUNDS OF THIS INVENTION HAVING A NITROGEN LINKER (X=NH)

To improve bioavailability, an NH linker was introduced between A phenyl and B thiazole rings. This new series of compounds was synthesized as shown in **Figure 6**. Reaction of 3-bromo-2-oxopropanoic acid ethyl ester and arylthiourea in ethanol under 65 °C produced 2-(arylamino)-thiazole-4-carboxylic acids **73a-d** with high yields. These acids were converted to Weinreb amides **74a-d**, followed by reactions with 3,4,5-trimethoxphenyllithium that yielded aniline linked free bases **5a-d**, which can be converted into HCl salts **5Ha-d**.

### Synthesis of (2-(Phenylamino)thiazol-4-yl)(3,4,5-trimethoxyphenyl)methanone derivatives (5a-d) and their HCl salt [Figure 6]

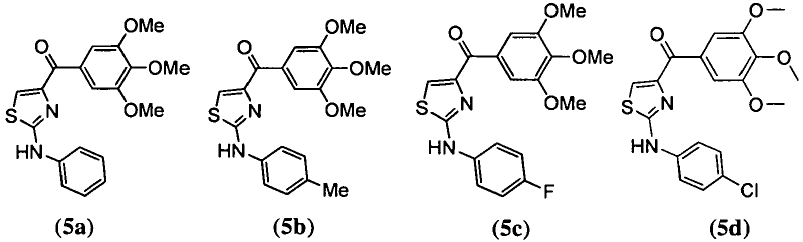

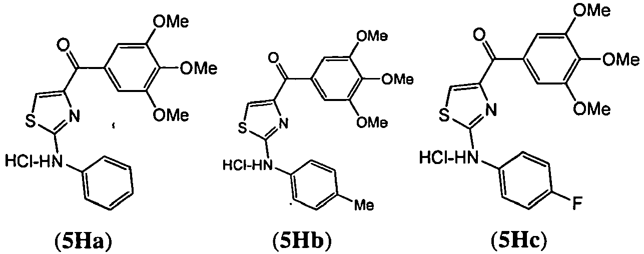

**General procedure for the synthesis of 2-(arylamino) thiazole-4-carboxylic acids (37a-d).** *N*-Aryl thiourea (0.01 mol) and ethyl bromopyruvate (0.011 mol) were dissolved in 3 mL ethanol and held at reflux for 2 h. The reaction was cooled, the crystalline ethyl 2-(substituted phenylamino) thiazole-4-carboxylate were collected by filtration and washed with ethanol. Refluxing the mixture of ethyl esters with the NaOH-ethanol solution gave final compounds **73a-d** which were used directly in the next steps.

***N*-Methoxy-*N*-methyl-2-(arylamino)thiazole-4-carboxamides (74a-d)** were synthesized using the same method as used for **38d** (see Example 1, Figure 2).

***N*-Methoxy-*N*-methyl-2-(phenylamino)thiazole-4-carboxamide (74a).** 90.2% yield. ¹H NMR (500 MHz, CDCl₃) δ 7.39 (s, 2 H), 7.38 (br, 1 H), 7.36-7.33 (m, br, 4 H), 7.09 (t, br, 1 H), 3.77 (s, 3 H), 3.43 (s, 3 H), 2.33 (s, 3 H). MS (ESI) *m*/*z* 286.0 (M + Na)⁺.

***N*-Methoxy-*N*-methyl-2-(*p*-tolylamino)thiazole-4-carboxamide (74b).** 93.3% yield. ¹H NMR (500 MHz, CDCl₃) δ 7.35 (s, 1 H), 7.31 (br, 1 H), 7.22 (d, 2 H), 7.16 (d, 2 H), 3.76 (s, 3 H), 3.42 (s, 3 H), 2.33 (s, 3 H). MS (ESI) *m*/*z* 278.0 (M + H)⁺.

**2-(4-Fluorophenylamino)-*N*-methoxy-*N*-methylthiazole-4-carboxamide (74c).** 89.7% yield. ¹H NMR (500 MHz, CDCl₃) δ 7.36 (s, 1 H), 7.36-7.31 (m, 2 H), 7.07-7.04 (m, 6 H), 3.76 (s, 3 H), 3.42 (s, 3 H). MS (ESI) *m*/*z* 282.0 (M + Na)⁺, 280.8 (M - H)⁻.

**2-(4-Chlorophenylantino)-*N*-methoxy-*N*-methylthiazole-4-carboxamide (74d).** ¹H NMR (500 MHz, CDCl₃) δ 7.66 (s, br, 1 H), 7.41 (s, 1 H), 7.34 (d, 2 H), 7.29 (d, 2 H), 3.76 (s, 3 H), 3.42 (s, 3 H). MS: 295.8 (M-1)⁻; 320.0 (M+Na)⁺.

**General procedure for the synthesis of (2-(arylamino)thiazol-4-yl)(3,4,5-trimethoxyphenyl)methanones (5a-d).** At -78 °C, to a solution of 5-bromo-1,2,3-trimethoxybenzene (1.235 g, 5.0 mmol) in 30 mL THF was charged *n*-BuLi in hexane (2.5 N, 2.4 mL, 6 mmol) under Ar₂ protection and stirred for 10 min. Weinreb amide **74a-d** (1 mmol) in 10 mL THF was added to the lithium reagent and allowed to stir at RT for 2 hs. The reaction mixture was quenched with satd. NH₄Cl, extracted with ethyl ether, dried with MgSO₄. The solvent was removed under reduced pressure to yield a crude product, which was purified by column chromatography to obtain pure compound **(5a-d)**.

**(2-(Phenylamino)thiazol-4-yl)(3,4,5-trimethoxyphenyl)methanone (5a).** 33.3% yield. ¹H NMR (500 MHz, DMSO-*d₆*) δ 10.4 (s, 1 H), 7.85 (s, 1 H), 7.68 (d, 2 H, *J* = 8.0 Hz), 7.31 (t, 2 H, *J* = 8.0 Hz), 6.98 (t, 1 H, *J* = 8.0 Hz), 3.83 (s, 6 H), 3.78 (s, 3 H). MS (ESI) *m*/*z* 393.1 (M + H)⁺, 368.9 (M -H)⁻.

**(2-(*p*-Tolylamino)thiazol-4-yl)(3,4,5-trimethoxyphenyl)methanone (5b).** 40.6% yield. ¹H NMR (500 MHz, CDCl₃) δ 7.48 (s, 1 H), 7.47 (s, 2 H), 7.30 (br, 1 H), 7.27 (d, 2 H, *J* = 8.5 Hz), 7.17 (d, 2 H, *J* = 8.5 Hz), 3.93 (s, 3 H). 3.90 (s, 6 H), 2.34 (s, 3 H). MS (ESI) *m*/*z* 385.1 (M + H)⁺, 382.9 (M -H)⁻.

**(2-(*p*-Fluorophenylamino)thiazol-4-yl)(3,4,5-trimethoxyphenyl)methanone (5c).** 39.6% yield. ¹H NMR (500 MHz, CDCl₃) δ 7.52 (br, 1 H), 7.49 (s, 1 H), 7.45 (s, 2 H), 7.40-7.37 (q, 2 H, *J* = 4.5 Hz), 7.08-7.04 (t, 2 H, *J* = 8.0 Hz), 3.93 (s, 3 H), 3.89 (s, 6H). MS (ESI) *m*/*z* 389.3 (M + H)⁺, 386.9 (M -H)⁻.

**(2-((4-Chlorophenyl)amino)thiazol-4-yl)(3,4,5-trimethoxyphenyl)methanone (5d)** was prepared using the same method as used for **5a** from 1-(4-chlorophenyl)thiourea and ethyl bromopyruvate. Melting point: 165-166°C. ¹H NMR (500 MHz, CDCl₃) δ 7.60 (s, br, 1 H), 7.56 (s, 1 H), 7.47 (s, 2 H), 7.38 (d, 2 H), 7.31 (d, 2 H), 3.94 (s, 3 H), 3.89 (s, 6 H). MS: 402.9 (M-1)⁻; 427.0 (M+Na)⁺.

**General procedure for the synthesis of hydrochloride salts (5Ha-c).** At 0 °C, to a solution of compound **5a-c** (0.1 mmol) in 5 mL CH₂Cl₂ was added a solution of HCl in 1,4-dioxane (4 N, 2 mL) and stirred at RT for overnight. The precipitates **5Ha-c** were collected and washed with diethyl ether.

**(2-(Phenylamino)thiazol-4-yl)(3,4,5-trimethoxyphenyl)methanone hydrochloride salt (5Ha).** 91.6% yield. ¹H NMR (500 MHz, DMSO-*d₆*) δ 12.9 (br, 1 H), 7.49-7.46 (m, 2 H), 7.42-7.40 (m, 2 H),7.37-7.34 (m, br, 2 H), 7.11 (s, 2 H), 3.94 (s, 3 H), 3.92 (s, 6 H). MS (ESI) *m*/*z* 389.1 (M + H)⁺.

**(2-(*p*-Tolylamino)thiazol-4-yl)(3,4,5-trimethoxyphenyl)methanone hydrochloride salt (5Hb).** 39.6% yield. ¹H NMR (500 MHz, CDCl₃) δ 7.30-7.25 (m, br, 5 H), 7.12 (s, 2 H), 3.94 (s, 3 H), 3.92 (s, 6 H), 2.38 (s, 3 H). MS (ESI) *m*/*z* 389.1 (M + H)⁺.

**(2-(*p*-Fluorophenylamino)thiazol-4-yl)(3,4,5-trimethoxyphenyl)methanone hydrochloride salt (5Hc).** 89.3% yield. ¹H NMR (500 MHz, CDCl₃) δ 10.55 (s, 1 H), 7.85 (s, 1 H), 7.72-7.69 (q, 2 H, *J* = 4.5 Hz), 7.50 (s, 2 H), 7.18-7.15 (t, 2 H, *J* = 8.5 Hz), 4.30 (br, 1 H), 3.82 (s, 6H), 3.78 (s, 3 H). MS (ESI) *m*/*z* 389.3 (M + H)⁺.

The following compound (5e) is a reference example.

### Synthesis of (2-(Phenylamino)-1H-imidazol-4-yl)(3,4,5-trimethoxyphenyl)methanone (5e)

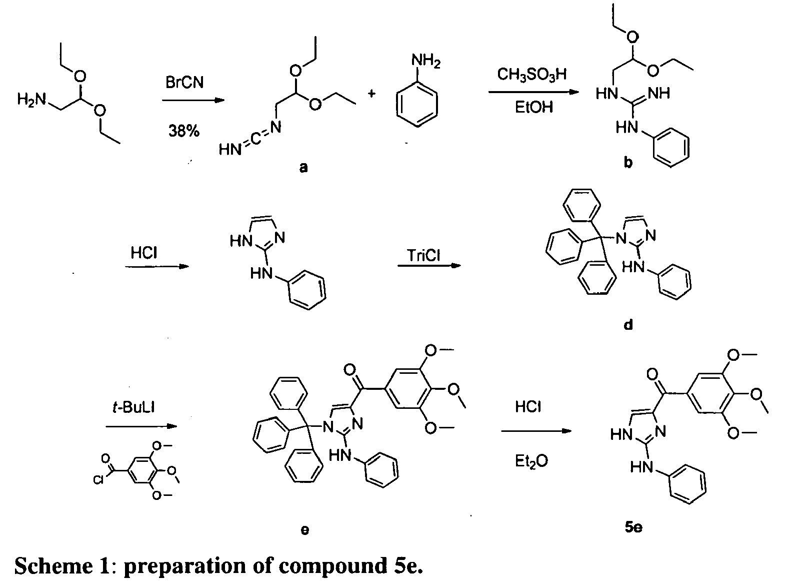

**2,2-Diethoxy-*N*-(iminomethylene)ethanamine (a).** A solution of the aminoacetaldehyde diethyl acetal (5.32 g, 40 mmol) in ether (20 mL) was added to a suspension of CNBr (4.22 g, 40 mmol) in hexane (20 mL) at RT. The reaction mixture was stirred at RT overnight. The solid was removed by filtration and washed with ether. The combined filtrate was concentrated. Flash chromatography of the concentrated residue afforded 2.82 g (45%) of the *N-*(2,2-diethoxyethyl)carbodiimide **(a)**. ¹H NMR (500 MHz, CDCl₃): 4.58 (t, *J* = 5.5 Hz, 1 H), 3.85 (br s, 1 H), 3.73 (m, 2 H), 3.56 (m, 2 H), 3.16 (*J* = 5.5 Hz, 2 H), 1.23 (t, *J* = 7.0 Hz, 3 H), MS: 156.8 (M-H)⁻; 180.9 (M+Na)⁺.

**1-(2,2-Diethoxyethyl)-3-phenylguanidine (b).** Aniline (1.66 g, 17.8 mmol) was dissolved in ethanol (25 mL), and *N*-(2,2-diethoxyethyl)carbodiimide **(a)**, (2.82 g, 17.8 mmol), was added dropwise. Then methanesulfonic acid (1.71 g, 17.8 mmol) was added, and the mixture was warmed at reflux for 24 h. The reaction mixture was poured into NaOH (0.5 M) and extracted with CH₂Cl₂. Drying and concentration afforded a product that was subjected to flash chromatography to give the intermediate guanidine **(b)** (3.3 g, 73.8%). ¹H NMR (500 MHz, DMSO-*d₆*) δ 7.27-6.90 (m, 5 H), 4.55 (t, 1 H), 3.76-3.70 (m, 2 H), 3.60-3.54 (m, 2 H), 3.35-3.34 (d, 2 H), 1.22 (pent, 6 H). MS: 249.8 (M-H)⁻; 252.1(M+H)⁺.

***N*-Phenyl-1*H*-imidazol-2-amine (c).** The guanidine **(b)** was dissolved in HCl (5 mL, 6 M) at 0 °C and then stirred for 2 h. After the starting material was consumed, NaOH (25%) was added until a precipitate formed. This mixture was stirred for 30 min. The reaction was then poured into NaOH (0.5 M), extracted with CH₂Cl₂, dried and concentrated. Flash chromatography afforded (c) (0.95 g, 50 %). ¹H NMR (500 MHz, DMSO-*d₆*) δ 8.58 (s, br, 1 H), 7.34-6.74 (m, 5 H), 6.68 (s, 2 H), 6.62 (br, 2 H), 3.82 (s, 6 H), 3.73 (s, 3 H). MS: 157.6 (M-H)⁻; 160.0 (M+H)⁺.

***N*-Phenyl-1-trityl-1*H*-imidazol-2-amine (d).** Trityl chloride (2.79 g, 10 mmol) was added to an ice-cooled solution of phenyl amino imidazole (c) (1.59 g, 10 mmol) and triethylamine (1.01 g, 10 mmol) in methylene dichloride (50 mL). The reaction mixture was allowed to warm to RT and stirred overnight. The mixture was diluted with methylene dichloride, washed successively with H₂O, saturated NaHCO₃, brine and dried with MgSO₄. Filtration and evaporation of the solvent followed by chromatography separation gave the product **(d).** ¹H NMR (500 MHz, CDCl₃) δ 7.52-7.35 (m, 5 H), 7.28-7.43 (m, 15 H), 6.85 (s, 2 H), 6.41 (s, 1 H), 6.08 (s, 1 H). MS: 1399.8 (M-H)⁻; 402.8 (M+H)⁺.

**(2-(Phenylamino)-1-trityl-1*H*-imidazol-4-yl)(3,4,5 trimethoxyphenyl)methanone (e).** At -78 °C, *t*-BuLi in THF (1.7 M, 0.34 mL, 0.58 mmol) was added to a solution of trityl protected compound **(d)** (116 mg, 0.289 mmol) in THF. Then 3,4,5-trimethoxybenzoyl chloride (66.5 mg, 0.289 mmol) was added and stirred overnight. The reaction mixture was quenched with saturated NH₄Cl, and dried with MgSO₄. Filtration and evaporation of the solvent followed by chromatography afforded compound **(e)** (75 mg, 43.7%). ¹H NMR (500 MHz, CDCl₃) δ 7.55-7.41 (m, 5 H), 7.32 (s, 1 H), 7.28-7.18 (m, 15 H), 6.94 (s, 2 H), 3.78 (s, 6 H), 3.70 (s, 3 H). MS: 594.2 (M-H)⁻; 596.3 (M+H)⁺.

**(2-(Phenylamino)-1*H*-imidazol-4-yl)(3,4,5-trimethoxyphenyl)methanone (5e).** To a solution of trityl protected compound **(e)** (50 mg, 0.084 mmol) in ethyl ether was added 2 M HCl in ether (1 mL, 1 mmol). The reaction mixture was stirred overnight and washed with saturated NaHCO₃ and dried with MgSO₄. Filtration and evaporation of the solvent followed by flash chromatography to yield de-protection compound **5e** (18 mg, 63%). ¹H NMR (500 MHz, DMSO-*d₆*) δ 7.54 (s, br, 1 H), 7.51-7.43 (m, 3 H), 7.33 (d, 2 H), 7.04 (s, 2 H), 6.62 (br, 2 H) 3.82 (s, 6 H), 3.73 (s, 3 H). MS: 352.1 (M-H)⁻; 354.3 (M+H)⁺.

### REFERENCE EXAMPLE 5

### SYNTHESIS OF SELECTED ARYL-BENZOYL-IMIDAZOLE COMPOUNDS

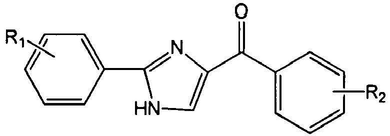

### Preparation of 2-aryl-4,5-dihydro-1H-imidazoles 14b, 14c, 14x (Figure 7).

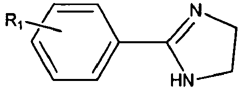

To a solution of appropriate benzaldehyde **8(b,** c, x) (60 mmol) in *t*-BuOH (300 mL) was added ethylenediamine (66 mmol) and stirred for 30 min at RT. Potassium carbonate (75 mmol) and iodine (180 mmol) were added to the reaction mixture sequentially followed by stirring at 70 °C for 3 h. Sodium sulfite (Na₂SO₃) was added and the mixture was extracted by chloroform. The organic layer was dried over magnesium sulfate and concentrated. The residue was purified by flash column chromatography (chloroform: methanol 20:1) to give a white solid. Yield: 50-60%.

### Preparation of 2-aryl-1H-imidazoles (9a-j, p, x; Figures 7 and 8).

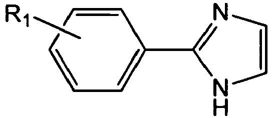

Method A (essential for only **9b, 9x** **Figure 7**): To a solution of 2-aryl-4,5-dihydro-1*H*-imidazole **14b, x** (35 mmol) in DMSO (100 mL) was added potassium carbonate (38.5 mmol) and diacetoxyiodobenzene (38.5 mmol). The reaction mixture was stirred overnight in darkness. Water was added followed by extraction with dichloromethane. The organic layer was dried over magnesium sulfate and concentrated. The residue was subjected to flash column chromatography (hexane: ethyl acetate 3:2) to give a white solid. Yield: 30%-50%.

Method B (essential for only **9c;** **Figure 7**): To a solution of 2-aryl-4,5-dihydro-1*H*-imidazole **14c** (50 mmol) in DMF (70 mL) was added DBU (55 mmol) and CBrCl₃ (55 mmol). The reaction mixture was stirred overnight and a saturated NaHCO₃ (aqueous) solution was added followed by extraction with dichloromethane. The organic layer was dried over magnesium sulfate and concentrated. The residue was subjected to flash column chromatography (chloroform: methanol 50:1) to yield a white solid. Yield: 7%.

Method C (essential for **9a, 9d-j, 9p;** **Figure 8**): To a solution of appropriate benzaldehyde (**8a, 8d-j, 8p**) (100 mmol) in ethanol (350 mL) at 0 °C was added a solution of 40% oxalaldehyde in water (12.8 mL, 110 mmol) and a solution of 29% ammonium hydroxide in water (1000 mmol, 140 mL). After stirring for 2-3 days at RT, the reaction mixture was concentrated and the residue was subjected to flash column chromatography with dichloromethane as eluent to yield the titled compound as a yellow powder. Yield: 20%-40%.

### Preparation of 2-aryl-1-(phenylsulfonyl)-1H-imidazoles (10a-j, p, x; Figures 7 and 8).

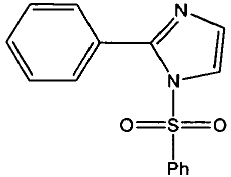

To a solution of 2-aryl-1*H*-imidazole **9a-j, p, x** (20 mmol) in anhydrous THF (200 mL) at 0 °C was added sodium hydride (60% dispersion in mineral oil, 1.2 g, 30 mmol) and stirred for 30 min. Benzenesulfonyl chloride (2.82 mL, 22 mmol) was added and the reaction mixture was stirred overnight. After dilution by 100 mL of saturated NaHCO₃ solution (aqueous), the reaction mixture was extracted by ethyl acetate (500 mL). The organic layer was dried over magnesium sulfate and concentrated. The residue was purified by flash column chromatography (hexane: ethyl acetate 2:1) to give a pale solid. Yield: 50%-70%.

### Preparation of aryl (2-aryl-1-(phenylsulfonyl)-1H-imidazol-4-yl)methanones (11aa-ai, ba, ca, cb, da, db, ea, eb, fa, fb, ga, gb, ha, hb, ia, ib, ja, jb, pa; Figures 7 and 8).

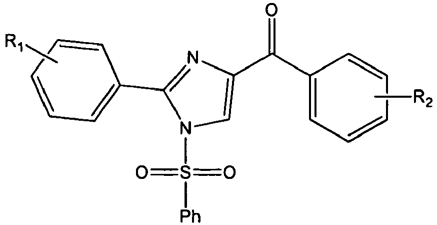

To a solution of 2-aryl-1-(phenylsulfonyl)-1*H*-imidazole (6.0 mmol) **10a-j, p, x** in anhydrous THF (30 mL) at -78 °C was added 1.7M *tert*-butyllithium in pentane (5.3 mL, 9.0 mmol) and stirred for 10 min. Appropriate substituted benzoyl chloride (7.2 mmol) was added at -78 °C and stirred for overnight. The reaction mixture was diluted with 100 mL of saturated NaHCO₃ solution (aqueous) and extracted by ethyl acetate (200 mL). The organic layer was dried over magnesium sulfate and concentrated. The residue was purified by flash column chromatography (hexane: ethyl acetate 4:1) to give a white solid. Yield: 15%-40%.

### General procedure for the preparation of aryl (2-aryl-1H-imidazol-4-yl)methanones (12aa-ai, ba, ca, cb, da, db, ea, eb, fa, fb, ga, gb, ha, hb, ia, ib, ja, jb, pa; Figures 7 and 8).

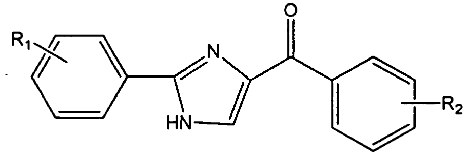

To a solution of aryl (2-aryl-1-(phenylsulfonyl)-1*H*-imidazol-4-yl)methanones (2.0 mmol) **11*aa-ai, ba, ca, cb, da, db, ea, eb, fa, fb, ga, gb, ha, hb, ia, ib, ja, jb, pa*** in THF (20.0 mL) was added 1.0M tetrabutyl ammonium fluoride (4.0 mmol) and stirred overnight. The reaction mixture was diluted by 50 mL of saturated NaHCO₃ solution (aqueous) and extracted by ethyl acetate (100 mL). The organic layer was dried over magnesium sulfate and concentrated. The residue was purified by flash column chromatography (hexane: ethyl acetate 3:1) or recrystallized from water and methanol to give a white solid. Yield: 80-95%.

### Preparation of (2-(4-hydroxyphenyl)-1H-imidazol-4-yl) (aryl)methanones (12ka, 11kb; Figure 8).

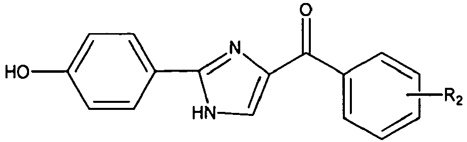

To a solution of (2-(4-(benzyloxy)phenyl)-1*H*-imidazol-4-yl)(aryl)methanone **12ja** or **12jb**, (1 mmol) in AcOH (20 mL) was added concentrated HCl (2 mL) and refluxed overnight. After removing the solvent, the residue was recrystallized from dichloromethane to give the titled compound as a yellow solid. Yield: 70-85%.

### Preparation of (2-aryl-1H-imidazol-4-yl) (3,4,5-trihydroxyphenyl)methanones 13ea, 13fa, 13ha (Figure 8).

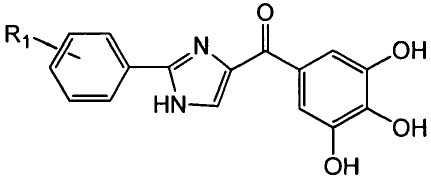

To a solution of aryl (2-aryl-1*H*-imidazol-4-yl)methanone **12ea**, **12fa or 12ha** (0.5 mmol) in CH₂Cl₂ (6.0 mL) was added 1.0 M of BBr₃ (2 mmol) in CH₂Cl₂ and stirred for 1 h at RT. Water was added to destroy excess BBr₃. The precipitated solid was filtered and recrystallized from MeOH to afford a yellow solid. Yield: 60-80%.

### Preparation of aryl (2-aryl-1H-imidazol-4-yl)methanone-HCl salt (12db-HCl).

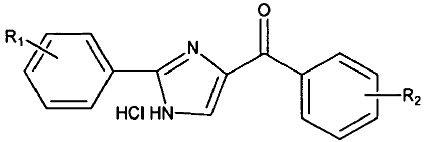

To a solution of **12db** (0.5 mmol) in methanol (20 mL) was added 2 M solution of hydrogen chloride (5 mmol) in ethyl ether and stirred overnight at RT. The reaction mixture was concentrated and the residue was washed by CH₂Cl₂ to yield the titled compound. Yield: 95%.

### Preparation of aryl (2-phenyl-1H-imidazol-1-yl)methanone (12aba, 12aaa; Figure 9).

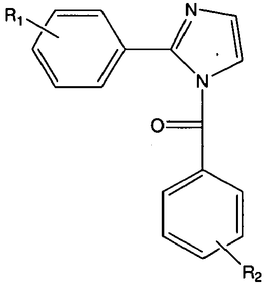

To a solution of 2-phenyl-1*H*-imidazole **9a** (10 mmol) in THF (20 mL) was added NaH (15 mmol) and substituted benzoyl chloride (12 mmol) at 0 °C. The reaction mixture was stirred overnight and diluted by saturated NaHCO₃ solution followed by extraction with ethyl acetate. The organic layer was dried over magnesium sulfate and concentrated. The residue was purified by flash column chromatography (chloroform) to give a white solid. Yield: 12-16%.

### Preparation of 1-substituted-(1-phenyl-1H-imidazol-1-yl)-aryl-methanone (12dc, 12fc, 12daa, 12 dab, 12 cba, 11gaa, 12la; Figures 10-11).

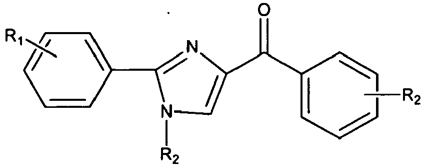

The synthesis of **12dc, 12fc** and **12daa, 12dab** and **12cba** is summarized in **Figure 10**. Compounds **12da, 12cb** and **12fa** were synthesized according to the synthesis decribed above and in **Figures 7** and **8**. Treatment of **12da** and **12fa** with aluminum chloride provided the *para*-demethylated **12dc, 12fc** with the 3,5-dimethoxy being intact. Compound **12daa** was prepared by benzylation of the N-1 position of **12da.** While methylation of the N-1 position of **12da** and **12cb** afforded compounds **12dab** and **12cba**, respectively.

### Synthesis of 12dc, 12fc, 12daa, 12dab, 12cba: Method D. (for 12dc and 12fc) [Figure 10]:

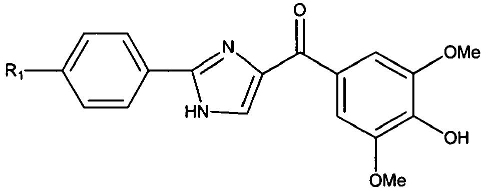

**R₁=CH₃ (12dc)**
**R₁=Cl (12fc)**

To a solution of **12da** and **12fa** (200 mg) in THF (20 mL) was added aluminum chloride (10 equiv). The reaction mixture was stirred overnight. Water was added followed by extraction with ethyl acetate. The organic layer was dried over magnesium sulfate and concentrated. The residue was subjected to flash column chromatography (hexane: ethyl acetate 1:1) to give a white-yellowish solid. Yield: 60%-80%.

### Synthesis of 12daa, 12dab, 12cba, Method E: [Figure 10]:

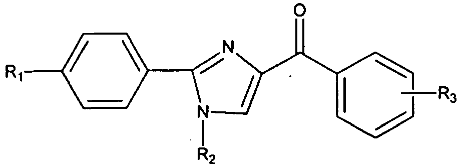

**R₁=Me; R₂=Bn; R₃=3,4,5-(OMe)₃ (12daa)**
**R₁=Me; R₂=CH₃; R₃=3,4,5-(OMe)₃(12dab)**
**R₁=OMe; R₂=CH₃; R₃=F (12cba)**

To a solution of **12da** and **12cb** (100 mg) in THF (10 mL) in an ice-bath was added sodium hydride (1.2 equiv) followed by the addition of methyl iodide (for **12dab, 12cba**) or benzyl bromide (for **12daa**) (2 equiv). The resulted reaction mixture was stirred for 5 h under reflux condition. After dilution by 50 mL of saturated NaHCO₃ solution (aqueous), the reaction mixture was extracted by ethyl acetate (100 mL). The organic layer was dried over magnesium sulfate and concentrated. The residue was purified by flash column chromatography (hexane: ethyl acetate 2:1) to give a white solid. Yield: 50%-98%.

### Synthesis of 11gaa and 12la (Figure 11):

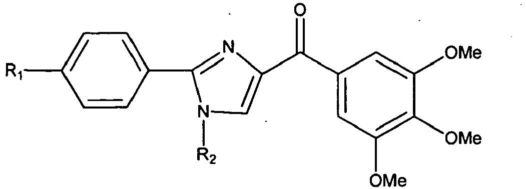

R₁=N(Me)₂; R₂=(4-OMe)PhSO₂ **(11gaa)**
R₁=Br; R₂=H **(12la)**

The substituted benzaldehyde compounds **8(l, g)** were converted to compounds **9(l, g)** in the presence of ammonium hydroxide and glyoxal to construct the imidazole scaffold. The imidazole rings of compounds **9(l, g)** were protected by an appropriate phenylsulfonyl group followed by coupling with 3,4,5-trimethoxybenzoyl chloride to achieve compound **11(la,gaa).** Treatment of **11la** with *tert*-butylammoniumfluoride to remove the protecting group afforded **12la.**

### Structural characterization of (1-Benzyl-2-(p-tolyl)-1H-imidazol-4-yl)(3,4,5-trimethoxyphenyl)methanone (12daa) (Figure 11).

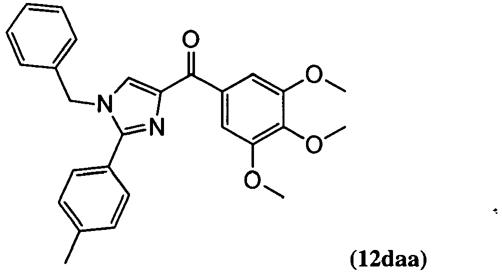

Yield: 92.8%; mp 135-137 °C. ¹H NMR (CDCl₃, 500 MHz) δ 7.81 (s, 1 H), 7.80 (d, *J* = 6.5 Hz, 2 H), 7.58 (d, *J* = 8.0 Hz, 2 H), 7.41-7.45 (m, 3 H), 7.31-7.33 (m, 2 H), 7.20 (d, *J* = 7.0 Hz, 2 H), 5.33 (s, 2 H), 3.99 (s, 3 H), 3.98 (s, 6 H), 2.47 (s, 3 H). MS (ESI) calcd for C₂₇H₂₆N₂O₄ 442.2, found 443.1 [M + Na]⁺. HPLC1: t_{R} 4.28 min, purity > 99%.

### Structural characterization of (2-(4-(dimethylamino)phenyl)-1-((4-methoxyphenyl)sulfonyl)-1H-imidazol-4-yl)(4-fluorophenyl)methanone (12gba).

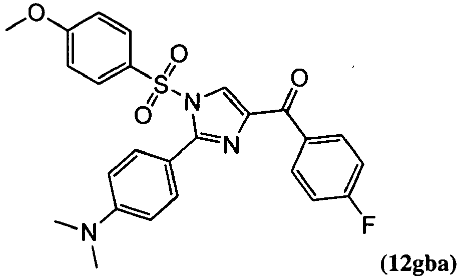

Yield: 34.1%; mp 147-149 °C. ¹H NMR (CDCl₃, 500 MHz) δ 8.07 (q, *J* = 8.5 Hz, 5.5 Hz, 2 H), 7.78 (d, *J* = 9.0 Hz, 2 H), 7.41 (d, *J* = 8.5 Hz, 2 H), 7.39 (s, 1 H), 7.23 (t, *J* = 8.5 Hz, 2 H), 6.91 (d, *J* = 9.0 Hz, 2 H), 6.68 (d, *J* = 9.0 Hz, 2 H), 3.89 (s, 3 H), 3.08 (s, 3 H). MS (ESI) calcd for C₂₅H₂₂FN₃O₄S 479.1, found 502.1 [M + Na]⁺. HPLC2: t_{R} 18.6 min, purity 96.9%.

### Synthesis of (2-(4-bromophenyl)-1H-imidazol-4-yl)(3,4,5-trimethoxyphenyl)methanone (12la) (Figure 11)

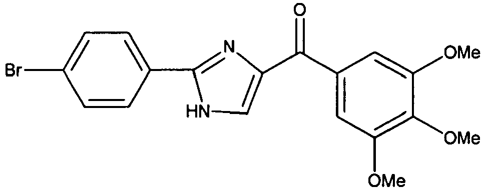

**Synthesis of 9l, 9g:** To a solution of appropriate benzaldehyde (**8l**, and **8g**, 100 mmol) in ethanol (400 mL) at 0 °C was added a solution of 40% oxalaldehyde (glyoxal) in water (1.1 equiv) and a solution of 29% ammonium hydroxide in water (10 equiv). After stirring for 2-3 days at RT, the reaction mixture was concentrated and the residue was subjected to flash column chromatography with dichloromethane as eluent to yield the titled compound as a yellow powder. Yield: 10%- 30%.

**Synthesis of 10la, 10gb:** To a solution of imidazoles **(9l, 9g)** (10 mmol) in anhydrous THF (200 mL) at 0 °C was added sodium hydride (60% dispersion in mineral oil, 1.2 equiv) and stirred for 20 min. 4-Methoxybenzenesulfonyl chloride (for **10gb**) or benzenesulfonyl chloride (for others)(1.2 equiv) was added and the reaction mixture was stirred overnight. After dilution by 200 mL of saturated NaHCO₃ solution (aqueous), the reaction mixture was extracted by ethyl acetate (600 mL). The organic layer was dried over magnesium sulfate and concentrated. The residue was purified by flash column chromatography (hexane: ethyl acetate 2:1) to give a pale solid. Yield: 40%-95%.

**Synthesis of 11la, 11gaa:** To a solution of 2-aryl-1-(phenylsulfonyl)-1*H-*imidazole **(101a, 10gb)** (5.0 mmol) in anhydrous THF (30 mL) at -78 °C was added 1.7 M *tert-*butyllithium in pentane (1.2 equiv) and stirred for 10 min. 3,4,5-Trimethoxybenzoyl chloride (1.2 equiv) was added at -78 °C and stirred overnight. The reaction mixture was diluted with 100 mL of saturated NaHCO₃ solution (aqueous) and extracted by ethyl acetate (300 mL). The organic layer was dried over magnesium sulfate and concentrated. The residue was purified by flash column chromatography (hexane: ethyl acetate 3:1) to give a white solid. Yield: 5%-45%.

**Synthesis of 12la:** To a solution of aryl (2-aryl-1-(phenylsulfonyl)-1*H*-imidazol-4-yl)methanone **(11la),** 2.0 mmol) in THF (25.0 mL) was added 1.0 M tetrabutyl ammonium fluoride (2 equiv) and stirred overnight. The reaction mixture was diluted by 60 mL of saturated NaHCO₃ solution (aqueous) and extracted by ethyl acetate (150 mL). The organic layer was dried over magnesium sulfate and concentrated. The residue was purified by flash column chromatography (hexane: ethyl acetate 4:1) or recrystallized from water and methanol to give a white solid. Yield: 80-98%.

### Synthesis of (4-Fluorophenyl)(2-(4-methoxyphenyl)-1H-imidazol-4-yl)methanone (12cb) (Figure 7).

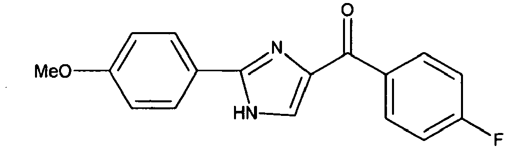

To a solution of (4-fluorophenyl)(2-(4-methoxyphenyl)-1-(phenylsulfonyl)-1*H-*imidazol-4-yl)methanone (**11cb**, 872 mg, 2.0 mmol) in THF (20.0 mL) was added 1.0 M tetrabutyl ammonium fluoride (4.0 mL, 4.0 mmol) and stirred overnight. The reaction mixture was diluted by 50 mL of saturated NaHCO₃ solution (aqueous) and extracted by ethyl acetate (100 mL). The organic layer was dried over magnesium sulfate and concentrated. The residue was recrystallized from water and methanol to give a white solid. Yield: 90%; mp 245 - 247 °C.

### Synthesis of (2-(p-Tolyl)-1H-imidazol-4-yl)(3,4,5-trimethoxyphenyl)methanone (12da) (Figure 8).

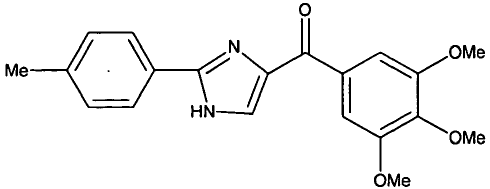

To a solution of (1-(phenylsulfonyl)-2-(p-tolyl)-1*H*-imidazol-4-yl)(3,4,5-trimethoxyphenyl)methanone (**11da,** 492 mg, 1.0 mmol) in THF (15.0 mL) was added 1.0 M tetrabutyl ammonium fluoride (2.0 mL, 2.0 mmol) and stirred overnight. The reaction mixture was diluted by 30 mL of saturated NaHCO₃ solution (aqueous) and extracted by ethyl acetate (80 mL). The organic layer was dried over magnesium sulfate and concentrated. The residue was recrystallized from water and methanol to give a white solid. Yield: 88.5%.

### Synthesis of (2-(4-Chlorophenyl)-1H-imidazol-4-yl)(3,4,5-trimethoxyphenyl)methanone (12fa) (Figures 8 and 14).

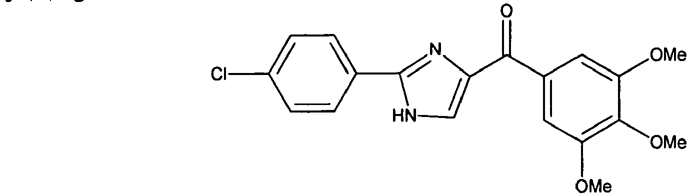

**2-(4-Chlorophenyl)-1*H*-imidazole (9f):** To a solution of 4-chlorobenzaldehyde (8f) (100 mmol) in ethanol (350 mL) at 0 °C was added a solution of 40% oxalaldehyde in water (12.8 mL, 110 mmol) and a solution of 29% ammonium hydroxide in water (1000 mmol, 140 mL). After stirring for 2-3 days at RT, the reaction mixture was concentrated and the residue was subjected to flash column chromatography with dichloromethane as eluent to yield the titled compound as a yellow powder. Yield: 19.8 %. ¹H NMR (500 MHz, DMSO-*d₆*) δ 13.60 (br, 1H), 7.94 (d, *J* = 8.5 Hz, 2H), 7.51 (d, *J* = 8.0 Hz, 2H), 7.27 (s, 1H), 7.03 (s, 1H). MS (ESI): calculated for C₉H₇ClN₂, 178.0, found 178.9 [M + H]⁺.

**2-(4-Chlorophenyl)-1-(phenylsulfonyl)-1*H*-imidazole (10f):** To a solution of 2-(4-chlorophenyl)-1*H*-imidazole (**9f**) (20 mmol) in anhydrous THF (200 mL) at 0 °C was added sodium hydride (60% dispersion in mineral oil, 1.2 g, 30 mmol) and stirred for 30 min. Benzenesulfonyl chloride (2.82 mL, 22 mmol) was added and the reaction mixture was stirred overnight. After dilution by 100 mL of saturated NaHCO₃ solution (aqueous), the reaction mixture was extracted by ethyl acetate (500 mL). The organic layer was dried over magnesium sulfate and concentrated. The residue was purified by flash column chromatography (hexane: ethyl acetate 2:1) to give a pale solid. Yield: 54.9%. ¹H NMR (500 MHz, CDCl₃) δ 7.65 (d, *J* = 2.0 Hz, 1H), 7.58 (t, *J* = 7.5 Hz, 1H), 7.43 (d, *J* = 8.5 Hz, 2H), 7.38 (t, *J* = 8.0 Hz, 2H), 7.34-7.36 (m, 4H), 7.12 (d, *J* = 1.5 Hz, 1H). MS (ESI): calculated for C₁₅H₁₁ClN₂O₂S, 318.0, found 341.0 [M + Na]⁺.

**(2-(4-Chlorophenyl)-1-(phenylsulfonyl)-1*H*-imidazol-4-yl)(3,4,5-trimethoxyphenyl)methanone (11fa):** To a solution of 2-(4-chlorophenyl)-1-(phenylsulfonyl)-1*H*-imidazole **(10f)** (6.0 mmol) in anhydrous THF (30 mL) at -78 °C was added 1.7 M *tert-*butyllithium in pentane (5.3 mL, 9.0 mmol) and stirred for 10 min. 3,4,5-Trimethoxybenzoyl chloride (7.2 mmol) was added at -78 °C and stirred for overnight. The reaction mixture was diluted with 100 mL of saturated NaHCO₃ solution (aqueous) and extracted by ethyl acetate (200 mL). The organic layer was dried over magnesium sulfate and concentrated. The residue was purified by flash column chromatography (hexane: ethyl acetate 4:1) to give a white solid. Yield: 36.8%; ¹H NMR (500 MHz, CDCl₃) δ 8.05 (d, *J* = 7.5 Hz, 2H), 7.77 (t, *J* = 7.5 Hz, 1H), 7.62 (t, *J* = 8.0 Hz, 2H), 7.48 (s, 1H), 7.44 (d, *J* = 9.0 Hz, 2H), 7.39 (d, *J* = 8.5 Hz, 2H), 7.37 (s, 2H). MS (ESI): calculated for C₂₅H₂₁ClN₂O₆S, 512.1, found 513.1 [M + H]⁺.

**(2-(4-Chlorophenyl)-1*H*-imidazol-4-yl)(3,4,5-trimethoxyphenyl)methanone (12fa):** To a solution of (2-(4-chlorophenyl)-1-(phenylsulfonyl)-1*H*-imidazol-4-yl)(3,4,5-trimethoxyphenyl)methanone **(11fa)** (2.0 mmol) in THF (20.0 mL) was added 1.0 M tetrabutyl ammonium fluoride (4.0 mmol) and stirred overnight. The reaction mixture was diluted by 50 mL of saturated NaHCO₃ solution (aqueous) and extracted by ethyl acetate (100 mL). The organic layer was dried over magnesium sulfate and concentrated. The residue was purified by flash column chromatography (hexane: ethyl acetate 3:1) or recrystallized from water and methanol to give a white solid. Yield: 80-95%. Yield: 36.9%; mp 193 - 195 °C. ¹H NMR (500 MHz, CDCl₃) δ 10.75 (br, 1H), 7.96 (d, *J* = 8.5 Hz, 2H), 7.83 (s, 1H), 7.47 (d, *J* = 9.0 Hz, 2H), 7.23 (s, 2H), 3.97 (s, 3H), 3.94 (s, 6H), 2.43 (s, 3H). MS (ESI): calculated for C₁₉H₁₇ClN₂O₄, 372.1, found 395.1 [M + Na]⁺, 370.9 [M - H]⁻. HPLC Gradient: Solvent A (water) and Solvent B (methanol): 0-15 min 40-100%B (linear gradient), 15-25 min 100%B: t_{R} 16.36 min, purity > 99%.

### Synthesis of (2-(4-Chlorophenyl)-1H-imidazol-4-yl)(4-fluorophenyl)methanone (12fb) (Figure 8).

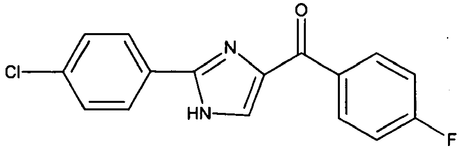

To a solution of (2-(4-chlorophenyl)-1-(phenylsulfonyl)-1*H*-imidazol-4-yl)(4-fluorophenyl)methanone (**11fb**, 440 mg, 1.0 mmol) in THF (12.0 mL) was added 1.0 M tetrabutyl ammonium fluoride (2.0 mL, 2.0 mmol) and stirred overnight. The reaction mixture was diluted by 20 mL of saturated NaHCO₃ solution (aqueous) and extracted by ethyl acetate (60 mL). The organic layer was dried over magnesium sulfate and concentrated. The residue was recrystallized from water and methanol to give a white solid. Yield: 83.7%.

**Physicochemical Characterization of Aryl-Benzoyl-Imidazole Compounds and Intermediates**

| **Compound** | **Physicochemical Cheracterization** |
|---|---|
| 2-phenyl-1*H*-imidazole (**9a**) | Yield: 36.8 %. ¹H NMR (500 MHz, DMSO-*d₆*) δ 12.52 (br, 1 H), 7.95 (d, *J* = 7.0 Hz, 2 H), 7.44 (t, *J* = 7.5 Hz, 2 H), 7.34 (t, *J* = 7.0 Hz, 1H), 7.25-7.27 m, 1 H), 7.04 - 7.07. m, 1 H). MS (ESI): calculated for C₉H₈N₂, 144.1, found 167.1 [M + Na]⁺. |
| 2-(4-fluorophenyl)-1*H*-imidazole (**9b**) | Yield: 56.5 %. ¹H NMR (300 MHz, DMSO-*d₆*) δ 12.46 (br, 1 H), 7.94-7.99 (m, 2 H), 7.24-7.30 (m, 2 H), 7.00- 7.03 (m, 2 H). MS (ESI): calculated for C₉H₇FN₂, 162.1, found 163 [M + H]⁺, 160.6 [M - H]⁻. |
| 2-(4-methoxyphenyl)-1*H*-imidazole (**9c**) | Yield: 22.2 %. ¹H NMR (500 MHz, CDCl₃) δ 7.80 (d, *J* = 10.0 Hz, 2 H), 7.15 (s, 2 H), 3.86 (s, 3 H). MS (ESI): calculated for C₁₀H₁₀N₂O, 174.1, found 175 [M + H]⁺, 172.8 [M - H]⁻. |
| 2-(*p*-tolyl)-1*H*-imidazole (**9d**) | Yield: 36.1 %. ¹H NMR (500 MHz, CDCl₃) δ 7.64 (d, *J* = 7.5 Hz, 2 H), 7.16 (d, *J* = 7.5 Hz, 2 H), 7.12 (s, 1 H), 7.02 (s, 1 H). MS (ESI): calculated for C₁₀H₁₀N₂, 158.1, found 159.0 [M + H]⁺, 156.8 [M - H]⁻. |
| 2-(3,4,5-trimethoxypheny1)-1*H*-imidazole (**9e**) | Yield: 26.0%. ¹H NMR (500 MHz, CDCl₃) δ 7.26 (s, 2 H), 7.08 (d, *J* = 1.5 Hz, 2 H), 3.86 (s, 3 H), 3.82 (s, 6 H). MS (ESI): calculated for C₁₂H₁₄N₂O₃, 234.1, found 234.9 [M + H]⁺. |
| 2-(4-chlorophenyl)-1*H*-imidazole (**9f**) | Yield: 19.8 %. ¹H NMR (500 MHz, DMSO-*d₆*) δ 13.60 (br, 1 H), 7.94 (d, *J* = 8.5 Hz, 2 H), 7.51 (d, *J* = 8.0 Hz, 2 H), 7.27 (s, 1 H), 7.03 (s, 1 H). MS (ESI): calculated for C₉H₇ClN₂, 178.0, found 178.9 [M + H]⁺. |
| 4-(1*H*-imidazol-2-yl)-*N*,*N*-dimethylaniline (**9g**) | Yield: 16.5 %. ¹H NMR (300 MHz, CDCl₃) δ 7.70 (dd, *J* = 7.0 Hz, 2.0 Hz, 2 H), 7.10 (s, 2 H), 6.75 (dd, *J* = 9.0 Hz, 2.0 Hz, 2 H), 3.02 (s, 6 H). MS (ESI): calculated for C₁₁H₁₃N₃, 187.1, found 187.9 [M + H]⁺, 185.8 [M - H]⁻. |
| 2-(3,4-dimethoxyphenyl)-1*H*-imidazole (**9h**) | Yield: 22.0 %. ¹H NMR (500 MHz, CDCl₃) δ 7.52 (d, *J* = 1.5 Hz, 1 H), 7.27-7.28 (m, 1 H), 7.14 (s, 2 H), 6.88 (d, *J* = 8.0 Hz, 1 H), 3.91 (s, 3 H), 3.87 (s, 3 H). MS (ESI): calculated for C₁₁H₁₂N₂O₂, 204.1, found 205.1 [M + H]⁺, 202.8 [M - H]⁻. |
| 2-(2-(trifluoromethyl)phenyl)-1*H*-imidazole (**9i**) | Yield: 25.5 %. ¹H NMR (500 MHz, DMSO-*d₆*) δ 12.31 (br, 1 H), 7.84 (d, *J* = 8.0 Hz, 1 H), 7.76 (t, *J* = 8.0 Hz, 1 H), 7.65 (t, *J* = 7.5 Hz, 1 H), 7.16 (br, 2 H). MS (ESI): calculated for C₁₀H₇F₃N₂, 212.1, found 212.9 [M + H]⁺, 210.7 [M - H]⁻. |
| 2-(4-(benzyloxy)phenyl)-1*H*-imidazole (**9j**) | Yield: 12.1 %. ¹H NMR (500 MHz, CDCl₃) δ 7.77 (d, *J* = 8.5 Hz, 2 H), 7.36-7.47 (m, 5 H), 7.10-7.18 (m, 2 H), 7.06 (d, *J* = 9.0 Hz, 2 H), 5.13 (s, 2 H). MS (ESI): calculated for C₁₆H₁₄N₂O, 250.1, found 251.1 [M + H]⁺, 248.8 [M - H]⁻. |
| 2-(4-Bromophenyl)-1*H*-imidazole (**9l**) | Yield: 19.5%. ¹H NMR (300 MHz, CDCl₃) δ 12.59 (s, 1 H), 7.87 (d, *J* = 8.1 Hz, 2 H), 7.64 (d, *J* = 8.1 Hz, 1 H), 7.27 (s, 1 H), 7.04 (s, 1 H). MS (ESI) calcd for C₉H₇BrN₂ 222.0, found 222.8 [M + H]⁺. |
| 2-(4-(Trifluoromethyl)phenyl)-1*H*-imidazole (**9p**) | Yield: 26.2 %; ¹H NMR (500 MHz, CDCl₃) δ 8.03 (d, *J* = 8.0 Hz, 2 H), 7.66 (d, *J* = 8.0 Hz, 2 H), 7.25 (s, 2 H). MS (ESI) calcd for C₁₀H₇F₃N₂ 212.1, found 213.1 [M + H]⁺ |
| 2-(4-nitrophenyl)-1*H*-imidazole (**9x**) | Yield: 53.7 %. ¹H NMR (500 MHz, DMSO-*d₆*) δ 12.97 (br, 1 H), 8.32 (d, *J* =9.0 Hz, 2 H), 8.17 (d, *J* = 9.0 Hz, 2 H), 7.42 (s, 1 H), 7.17 (s, 1H). MS (ESI): calculated for C₉H₇N₃O₂, 189.1, found 189.9 [M + H]⁺, 187.8 [M - H]⁻. |
| 2-phenyl-1-(phenylsulfonyl)-1*H*-imidazole (**10a**) | Yield: 50.3 %. ¹H NMR (500 MHz, CDCl₃) δ 7.64-7.67 (m, 1 H), 7.56 (t, *J* = 9.0 Hz, 1 H), 7.32-7.48 (m, 9 H), 7.12-7.16 (m, 1 H). MS (ESI): calculated for C₁₅H₁₂N₂O₂S, 284.1, found 307.1 [M + Na]⁺. |
| 2-(4-fluorophenyl)-1-(phenylsulfonyl)-1*H-*imidazole (**10b**) | Yield: 56.9 %. ¹H NMR (500 MHz, CDCl₃) δ 7.66 (d, *J* = 2.0 Hz, 1 H), 7.58 (t, *J* = 10.0 Hz, 1 H), 7.36-7.42 (m, 6 H), 7.12 (d, *J* = 2.0 Hz, 1 H), 7.06 (t, *J* = 10.0 Hz, 2 H). MS (ESI): calculated for C₁₅H₁₁FN₂O₂S, 302.1, found 300.8 [M - H]⁻. |
| 2-(4-methoxyphenyl)-1-(phenylsulfonyl)-1*H-*imidazole (**10c**) | Yield: 40.9 %. ¹H NMR (500 MHz, CDCl₃) δ 7.62 (d, *J* = 5.0 Hz, 1 H), 7.56 (tt, *J* = 15.0 Hz, 5.0 Hz, 1 H), 7.32-7.43 (m, 6 H), 7.10 (d, *J* = 5.0 Hz, 1 H), 6.88 (dt, *J* = 16.0 Hz, 6.0 Hz, 2 H), 3.87 (s; 3 H). MS (ESI): calculated for C₁₆H₁₄N₂O₃S, 314.1, found 337.1 [M + Na]⁺, 312.9 [M - H]⁻. |
| 1-(phenylsulfonyl)-2-(*p*-tolyl)-1*H*-imidazole (**10d**) | Yield: 46.6%. ¹H NMR (500 MHz, CDCl₃) δ 7.63 (d, *J* = 1.0 Hz, 1 H), 7.55 (t, *J* = 8.0 Hz, 1 H), 7.42 (d, *J* = 8.0 Hz, 2 H), 7.35 (t, *J* = 7.5 Hz, 2 H), 7.27-7.29 (m, 2 H), 7.16 (d, *J* = 7.5 Hz, 2 H), 7.10 (s, 1 H), 2.41 (s, 3 H). MS (ESI): calculated for C₁₆H₁₄N₂O₂S, 298.1, found 321.1 [M + Na]⁺. |
| 1-(phenylsulfonyl)-2-(3,4,5-trimethoxyphenyl)-1*H*-imidazole (**10e**) | Yield: 55.7%. ¹H NMR (500 MHz, CDCl₃) δ 7.68 (d, *J* = 1.5 Hz, 1 H), 7.55 (t, *J* = 7.0 Hz, 1 H), 7.42 (d, *J* = 7.5 Hz, 2 H), 7.35 (t, *J* = 8.5 Hz, 2 H), 7.11 (d, *J* = 1.5 Hz, 2 H), 6.60 (s, 1 H), 3.90 (s, 3 H), 3.79 (s, 6 H). MS (ESI): calculated for C₁₈H₁₈N₂O₅S, 374.1, found 397.1 [M + Na]⁺. |
| 2-(4-chlorophenyl)-1-(phenylsulfonyl)-1*H-*imidazole (**10f**) | Yield: 54.9%. ¹H NMR (500 MHz, CDCl₃) δ 7.65 (d, *J* = 2.0 Hz, 1 H), 7.58 (t, *J* = 7.5 Hz, 1 H), 7.43 (d, *J* = 8.5 Hz, 2 H), 7.38 (t, *J* = 8.0 Hz, 2 H), 7.34-7.36 (m, 4 H), 7.12 (d, *J* = 1.5 Hz, 1 H). MS (ESI): calculated for C₁₅H₁₁ClN₂O₂S, 318.0, found 341.0 [M + Na]⁺. |
| *N*,*N*-dimethyl-4-(1-(phenylsulfonyl)-1*H-*imidazol-2-yl) aniline (**10g**) | Yield: 48.3%. ¹H NMR (300 MHz, CDCl₃) δ 7.59 (d, *J* = 2.0 Hz, 1 H), 7.55 (t, *J* = 8.0 Hz, 1 H), 7.45 (d, *J* = 7.5 Hz, 2 H), 7.28-7.38 (m, 4 H), 7.07 (d, *J* = 2.0 Hz, 1 H), 6.68 (d, *J* = 8.5 Hz, 2 H), 3.04 (s, 3 H). MS (ESI): calculated for C₁₇H₁₇N₃O₂S, 327.10, found 350.0 [M + Na]⁺, 325.9 [M - H]⁻. |
| 4-(1-((4-Methoxyphenyl)sulfonyl)-1*H-*imidazol-2-yl)-*N*,*N*-dimethylaniline (**10gb**) | Yield: 61.5 %. ¹H NMR (500 MHz, CDCl₃) δ 7.58 (d, *J* = 1.5 Hz, 1 H), 7.36 (t, *J* = 8.43 Hz, 4 H), 7.03 - 7.09 (m, 1 H), 6.80 (d, *J* = 9.0 Hz, 2 H), 6.69 (d, *J* = 8.8 Hz, 2 H), 3.84 (s, 3 H), 3.05 (s, 6 H). MS (ESI): calculated for C₁₇H₁₇N₃O₂S, 327.1, found 358.2 [M + Na]⁺. |
| 2-(3,4-dimethoxyphenyl)-1-(phenylsulfonyl)-1*H*-imidazole (**10h**) | Yield: 60.3%. ¹H NMR (500 MHz, CDCl₃) δ 7.64 (d, *J* = 7.0 Hz, 1 H), 7.55 (t, *J* = 7.5 Hz, 1 H), 7.40 (dd, *J* = 8.5 Hz, 1.5 Hz, 2 H), 7.35 (t, *J* = 8.0 Hz, 2H), 7.09 (d, *J* = 2.0 Hz, 1 H), 7.02 (dd, *J* = 8.0 Hz, 2.0 Hz, 1 H), 6.89 (d, *J* = 1.5 Hz, 1 H), 6.86 (d, *J* = 8.0 Hz, 1 H), 3.95 (s, 3 H), 3.81 (s, 3 H). MS (ESI): calculated for C₁₇H₁₆N₂O₄S, 344.10, found 367.0 [M + Na]⁺. |
| 1-(phenylsulfonyl)-2-(2-(trifluoromethyl)phenyl)-1*H*-imidazole (**10i**) | Yield: 58.6%. ¹H NMR (500 MHz, CDCl₃) δ 7.64-7.67 (m, 2 H), 7.61-7.63 (m, 3 H), 7.40-7.46 (m, 5 H), 7.16 (d, *J* = 1.5 Hz, 1 H). MS (ESI): calculated for C₁₆H₁₁F₃N₂O₂S, 352.10, found 353.1 [M + H]⁺. |
| 2-(4-(benzyloxy)phenyl)-1-(phenylsulfonyl)-1*H*-imidazole (**10j**) | Yield: 62.0%; mp 102 - 104 °C. ¹H NMR (500 MHz, CDCl₃) δ 7.56 (d, *J* = 1.0 Hz, 1 H), 7.46 (t, *J* = 8.0 Hz, 1 H), 7.20-7.40 (m, 11 H), 7.03 (d, *J* = 1.0 Hz, 1H), 6.89 (t, *J* = 8.0 Hz, 2 H), 5.08 (s, 2 H). MS (ESI): calculated for C₂₂H₁₈N₂O₃S, 390.10, found 413.1 [M + Na]⁺. HPLC2: *t*_{R} 18.22 min, purity 95.9%. |
| 2-(4-Bromophenyl)-1-(phenylsulfonyl)-1*H-*imidazole **(10la)** | Yield: 61.2%. ¹H NMR (500 MHz, CDCl₃) δ 7.71 (d, *J* = 2.0 Hz, 1 H), 7.64 (t, *J* = 7.0 Hz, 1 H), 7.57 (d, *J* = 9.0 Hz, 2 H), 7.49 (d, *J* = 7.0 Hz, 2 H), 7.45 (t, *J* = 9.0 Hz, 2 H), 7.34 (d, *J* = 8.5 Hz, 2 H), 7.18 (d, *J* = 1.5 Hz, 1 H). MS (ESI) calcd for C₁₅H₁₁BrN₂O₂S 362.0, found 363.0 [M + H]⁺. |
| 1-(Phenylsulfonyl)-2-(4-(trifluoromethyl)phenyl)-1*H*-imidazole **(10p)** | Yield: 36.7 %; ¹H NMR (500 MHz, CDCl₃) δ 7.75 (d, *J* = 2.0 Hz, 1 H), 7.69 (d, *J* = 8.0 Hz, 2 H), 7.65 (t, *J* = 8.0 Hz, 1 H), 7.60 (d, *J* = 8.0 Hz, 2 H), 7.48 (d, *J* = 7.5 Hz, 2 H), 7.43 (t, *J* = 8.0 Hz, 2 H), 7.22 (d, *J* = 2.0 Hz, 1 H). MS (ESI) calcd for C₁₆H₁₁F₃N₂O₂S 352.1, found 553.1 [M + H]⁺ |
| 2-(4-nitrophenyl)-1-(phenylsulfonyl)-1*H-*imidazole (**10x**) | Yield: 50%; mp 145 - 147 °C. ¹H NMR (500 MHz, DMSO-*d₆*) δ 8.28 (d, *J* =8.5 Hz, 2 H), 8.03 (d, *J* = 1.5 Hz, 1 H), 7.78 (t, *J* = 7.5 Hz, 1 H), 7.64-7.68 (m, 4H), 7.60 (t, *J* = 8.0 Hz, 2 H), 7.30 (d, *J* = 1.5 Hz, 1 H). MS (ESI): calculated for C₁₅H₁₁N₃O₄S, 329.10, found 352.0 [M + Na]⁺, 327.9 [M - H]⁻. HPLC2: *t*_{R} 14.87 min, purity 98.8%. |
| (4-methoxyphenyl)(2-phenyl-1-(phenylsulfonyl)-1*H*-imidazol-4-yl)methanone (**11ab**) | Yield: 26.3%; mp 118 - 120 °C. ¹H NMR (500 MHz, DMSO-*d₆*) δ 8.37 (d, *J* = 1.0 Hz, 1 H), 8.15-8.18 (m, 2 H), 8.12 (d, *J* = 9.0 Hz, 2 H), 7.56-7.64 (m, 5 H), 7.46-7.50 (m, 3 H), 7.16 (d, *J* = 8.0 Hz, 2 H), 3.90 (s, 3 H). MS (ESI): calculated for C₂₃H₁₈N₂O₄S, 418.10, found 419.1 [M + H]⁺. HPLC2: *t*_{R} 17.72 min, purity 95.7%. |
| (3-methoxyphenyl)(2-phenyl-1-(phenylsulfonyl)-1*H*-imidazol-4-yl)methanone (**11ac**) | Yield: 31.2%; mp 136 - 138 °C. ¹H NMR (500 MHz, CDCl₃) δ 8.35 (s, 1 H), 7.86 (d, *J* = 8.0 Hz, 1 H),7.72 (s, 1 H), 7.60 (t, *J* = 7.5 Hz, 1 H), 7.51 (t, *J* = 7.5 Hz, 1 H), 7.35-7.42 (m, 9H), 7.14 (dd, *J* = 8.0 Hz, 2.0 Hz, 1 H), 3.88 (s, 3 H). MS (ESI): calculated for C₂₃H₁₈N₂O₄S, 418.10, found 419.1 [M + H]⁺. HPLC2: *t*_{R} 17.72 min, purity 95.7%. |
| (2-phenyl-1-(phenylsulfonyl)-1*H*-imidazol-4-yl)(*p*-tolyl)methanone (**11ah**) | 28.9%; mp 108 - 110 °C. ¹H NMR (500 MHz, CDCl₃) δ 8.00 (d, *J* = 7.5 Hz, 2 H), 7.98 (q, *J* = 8.0 Hz, 1.5 Hz, 2 H), 7.91 (d, *J* = 8.0 Hz, 1 H), 7.81 (s, 1 H), 7.44-7.48 (m, 3 H), 7.35-7.40 (m, 2 H), 7.30 (t, *J* = 8.0 Hz, 2 H), 7.20 (s, 2 H), 2.42 (s, 3 H). MS (ESI): calculated for C₂₃H₁₈N₂O₃S, 402.10, found 403.1 [M + H]⁺. HPLC2: *t*_{R} 16.06 min, purity 96.2%. |
| (4-fluorophenyl)(2-phenyl-1-(phenylsulfonyl)-1*H*-imidazol-4-yl)methanone (**11af**) | Yield: 25.4%; mp 114 - 116 °C. ¹H NMR (500 MHz, CDCl₃) δ 8.10 (q, *J* = 3.5 Hz, 5.5 Hz, 2 H), 7.88 (d, *J* = 7.5 Hz, 2 H), 7.67 (t, *J* = 7.5 Hz, 1 H), 7.48 - 7.54 (m, 3 H), 7.38 - 7.41 (m, 5 H), 7.24 (t, *J* = 8.5 Hz, 2 H). MS (ESI): calculated for C₂₂H₁₅FN₂O₃S, 406.10, found 429.1 [M + Na]⁺. HPLC2: *t*_{R} 15.43 min, purity 96.1%. |
| (3-fluorophenyl)(2-phenyl-1-(phenylsulfonyl)-1*H*-imidazo1-4-yl)methanone (**11ag**) | Yield: 18.3%; mp 102 - 104 °C. ¹H NMR (500 MHz, CDCl₃) δ 8.14 (d, *J* = 7.5 Hz, 1 H), 7.76 - 7.87 (m, 3 H), 7.74 (d, *J* = 9.0 Hz, 1 H), 7.37 - 7.57 (m, 10 H), 7.38 - 7.41 (m, 5 H), 7.24 (t, *J* = 8.5 Hz, 2 H). MS (ESI): calculated for C₂₂H₁₅FN₂O₃S, 406.10, found 429.1 [M + Na]⁺. HPLC2: *t*_{R} 15.75 min, purity 96.5%. |
| (4-fluorophenyl)(2-(4-methoxyphenyl)-1-(phenylsulfonyl)-1*H*-imidazol-4-yl)methanone (**11cb**) | Yield: 23.5%; mp 135 - 137 °C. ¹H NMR (500 MHz, CDCl₃) δ 8.00 (d, *J* = 5.5 Hz, 2 H), 7.74 - 7.76 (m, 2 H), 7.54-7.58 (m, 1 H), 7.40 (d, *J* = 7.0 Hz, 2 H), 7.28-7.30 (m, 3 H), 7.14 - 7.16 (m, 2 H), 6.80-6.82 (m, 2 H), 3.80 (s, 3 H). MS (ESI): calculated for C₂₃H₁₇FN₂O₄S, 436.10, found 459.0 [M + Na]⁺, 434.9 [M - H]⁻. HPLC2: *t*_{R} 16.53 min, Purity 96.1%. |
| (1-(phenylsulfonyl)-2-(*p*-tolyl)-1*H*-imidazol-4-yl)(3,4,5-trimethoxyphenyl)methanone (**11da**) | Yield: 33.8%; ¹H NMR (500 MHz, CDCl₃) δ 8.00 (d, *J* = 8.0 Hz, 2 H), 7.70 (t, *J* = 7.5 Hz, 1 H), 7.55 (t, *J* = 8.0 Hz, 2 H), 7.44 (s, 2 H), 7.34 (s, 2H), 7.31 (d, *J* = 8.0 Hz, 2 H), 7.21 (d, *J* = 8.0 Hz, 2 H), 4.00 (s, 3 H), 3.98 (s, 6 H). MS (ESI): calculated for C₂₆H₂₄N₂O₆S, 492.14, found 515.2 [M + Na]⁺. |
| (4-fluorophenyl)(1-(phenylsulfonyl)-2-(*p-*tolyl)-1*H*-imidazol-4-yl)methanone (**11db**) | Yield: 18.6%; mp 142 - 144 °C. ¹H NMR (500 MHz, CDCl₃) δ 8.07 (q, *J* = 8.5 Hz, 5.5 Hz, 2 H), 7.88 (d, *J* = 7.5 Hz, 2 H), 7.64 (t, *J* = 8.0 Hz, 1 H), 7.49 (d, *J* = 8.0 Hz, 2 H), 7.38 (s, 1H), 7.30 (d, *J* = 8.0 Hz, 2 H), 7.18 - 7.24 (m, 4 H), 2.43 (s, 3 H). MS (ESI): calculated for C₂₃H₁₇FN₂O₃S, 420.10, found 443.0 [M + Na]⁺, 418.9 [M - H]⁻. HPLC2: *t*_{R} 17.28 min, purity 97.3%. |
| (1-(phenylsulfonyl)-2-(3,4,5-trimethoxyphenyl)-1*H*-imidazol-4-yl)(3,4,5-trimethoxyphenyl)methanone (**11ea**) | Yield: 21.1%; mp 135 - 137 °C. ¹H NMR (500 MHz, CDCl₃) δ 7.91 (d, *J* =8.0 Hz, 2 H), 7.65 (t, *J* = 7.5 Hz, 1 H), 7.51 (t, *J* = 8.0 Hz, 2 H), 7.44 (s, 1 H), 7.34 (s, 2 H), 6.60 (s, 2 H), 3.98 (s, 3 H), 3.96 (s, 6 H), 3.91 (s, 3 H), 3.73 (s, 6 H). MS (ESI): calculated for C₂₈H₂₈N₂O₉S, 568.2, found 569.2 [M + H]⁺. HPLCl: *t*_{R} 17.86 min, purity 98.9%. |
| (4-fluorophenyl)(1-(phenylsulfonyl)-2-(3,4,5-trimethoxyphenyl)-1*H*-imidazol-4-yl)methanone (**11eb**) | Yield: 18.8%; mp 135 - 137 °C. ¹H NMR (500 MHz, CDCl₃) δ 8.11 (q, *J* =5.5 Hz, 3.0 Hz, 1 H), 8.00 -8.03 (m, 1 H), 7.82 (d, *J* = 7.5 Hz, 1 H), 7.78 (s, 1 H), 7.64 (t, *J* =7.0 Hz, 1 H), 7.48 (t, *J* =8.0 Hz, 1 H), 7.42 (s, 1 H), 7.21 - 7.26 (m, 4 H), 6.62 (s, 1 H), 3.98 (s, 3 H), 3.96 (s, 6 H), 3.93 (s, 3 H). MS (ESI): calculated for C₂₅H₂₁FN₂O₆S, 496.10, found 497.1 [M + H]⁺. HPLC2: *t*_{R} 15.26 min, purity 98%. |
| (2-(4-chlorophenyl)-1-(phenylsulfonyl)-1*H-*imidazol-4-yl)(4-fluorophenyl)methanone (**11fb**) | Yield: 36.8%; mp 153 - 155 °C. ¹H NMR (500 MHz, CDCl₃) δ 8.06 (q, *J* =5.5 Hz, 3.0Hz, 2 H), 7.89 (d, *J* =7.5 Hz, 2 H), 7.68 (t, *J* =8.0 Hz, 1 H), 7.52 (t, *J* = 8.0 Hz, 2 H), 7.34-7.38 (m, 5H), 7.23 (t, *J* =8.5 Hz, 2 H). MS (ESI): calculated for C₂₂H₁₄ClFN₂O₃S, 440.0, found 463.0 [M + Na]⁺. HPLC2: *t*_{R} 17.72 min, purity 97.38%. |
| (2-(4-(dimethylamino)phenyl)-1-(phenylsulfonyl)-1*H*-imidazol-4-yl)(3,4,5-trimethoxyphenyl)methanone (**11ga**) | Yield: 32.2%; mp 157 - 159 °C. ¹H NMR (500 MHz, CDCl₃) δ 7.89 (d, *J* =8.0 Hz, 2 H), 7.62 (t, *J* =7.5 Hz, 1 H), 7.48 (t, *J* =8.0 Hz, 2 H), 7.43 (s, 1 H), 7.32 (d, *J* =8.5 Hz, 2 H), 7.30 (s, 2H), 6.62 (d, *J* =9.0 Hz, 2 H), 3.97 (s, 3 H), 3.95 (s, 6 H), 3.05 (s, 6 H). MS (ESI): calculated for C₂₇H2₇N₃O₆S, 521.2, found 544.1 [M + Na]⁺, 519.8 [M - H]⁻. HPLC2: *t*_{R} 16.00 min, purity 97.9%. |
| (2-(4-(dimethylamino)phenyl)-1-(phenylsulfonyl)-1*H*-imidazol-4-yl)(4-fluorophenyl)methanone (**11gb**) | Yield: 38.5%; mp 125 - 127 °C. ¹H NMR (500 MHz, CDCl₃) δ 8.04 (q, *J* =5.5 Hz, 3.5Hz, 2 H), 7.80 (d, *J* =7.5 Hz, 2 H), 7.61 (t, *J* =8.0 Hz, 1 H), 7.45 (t, *J* =8.0 Hz, 2 H), 7.39 (s, 1 H), 7.35 (d, *J* =9.0 Hz, 2 H), 7.21 (t, *J* =8.5 Hz, 2 H), 6.62 (d, *J* =9.0 Hz, 2 H), 3.05 (s, 6 H). MS (ESI): calculated for C₂₄H₂₀FN₃O₃S, 449.10, found 472.1 [M + Na]⁺, 447.9 [M - H]⁻. HPLC2: *t*_{R} 16.85 min, purity 96.5%. |
| (2-(3,4-dimethoxyphenyl)-1-(phenylsulfonyl)-1*H*-imidazol-4-yl)(3,4,5-trimethoxyphenyl)methanone (**11ha**) | Yield: 28.6%; mp 136 - 138 °C. ¹H NMR (300 MHz, CDCl₃) δ 7.92 (dd, *J* =8.5 Hz, 1.5 Hz, 2 H), 7.66 (t, *J* =7.5 Hz, 2 H), 7.51 (t, *J* =7.5 Hz, 2 H), 7.43 (s, 1 H), 7.33 (s, 2 H), 7.02 (dd, *J* =8.0 Hz, 2.0 Hz, 1 H), 6.91 (d, *J* =2.0 Hz, 1 H), 6.86 (d, *J* =8.5 Hz, 1 H), 3.98 (s, 3 H), 3.96 (s, 9 H), 3.77 (s, 3 H). MS (ESI): calculated for C₂₇H₂₆N₂O₈S, 538.10, found 561.1 [M + Na]⁺, 536.8 [M - H]⁻. HPLC2: *t*_{R} 14.67 min, purity 98.2%. |
| (2-(3,4-dimethoxyphenyl)-1-(phenylsulfonyl)-1*H*-imidazol-4-yl)(4-fluorophenyl)methanone (**11hb**) | Yield: 31.9%; mp 144 - 145 °C. ¹H NMR (300 MHz, CDCl₃) δ 8.09 (q, *J* =5.5 Hz, 3.5 Hz, 2 H), 7.81 (d, *J* =8.0 Hz, 2 H), 7.62 (t, *J* =7.5 Hz, 2 H), 7.48 (t, *J* =7.5 Hz, 2 H), 7.40 (s, 1 H), 7.21-7.25 (m, 2 H), 7.04 (dd, *J* =8.0 Hz, 2.0 Hz, 1 H), 6.92 (d, *J* =2.0 Hz, 1 H), 6.86 (d, *J* =8.5 Hz, 1 H),3.96 (s, 3 H), 3.79 (s, 6 H). MS (ESI): calculated for C₂₄H₁₉FN₂O₅S, 466.10, found 489.1 [M + Na]⁺, 464.8 [M - H)⁻. HPLC2: *t*_{R} 15.52 min, purity 97.4%. |
| (1-(phenylsulfonyl)-2-(2-(trifluoromethyl)phenyl)-1*H*-imidazol-4-yl)(3,4,5-trimethoxyphenyl)methanone (**11ia**) | Yield: 25.0%; mp 155 - 157 °C. ¹H NMR (500 MHz, DMSO-*d₆*) δ 7.91 (d, *J* =8.0 Hz, 1 H), 7.84 (q, *J* =7.5 Hz, 5.0 Hz, 2 H), 7.77-7.80 (m, 2 H), 7.75 (s, 2 H), 7.66 (t, *J* =8.0 Hz, 2 H), 7.56 (d, *J* =7.5 Hz, 1 H), 7.18 (s, 2 H), 3.87 (s, 6 H), 3.81 (s, 3 H). MS (ESI): calculated for C₂₆H₂₁F₃N₂O₆S, 546.10, found 569.0 [M + Na]⁺. HPLC2: *t*_{R} 16.16 min, purity 98.9%. |
| (1-(phenylsulfonyl)-2-(2-(trifluoromethyl)phenyl)-1*H*-imidazol-4-yl)(4-fluorophenyl)methanone (**11ib**) | Yield: 25.0%; mp 151 - 153 °C. ¹H NMR (500 MHz, CDCl₃) δ 8.03 (q, *J* =5.5 Hz, 3.0 Hz, 2 H), 7.90 (d, *J* =8.0 Hz, 2 H), 7.80 (d, *J* =8.0 Hz, 1 H), 7.69 (q, *J* =7.0 Hz, 6.5 Hz, 2 H), 7.61 (t, *J* =8.0 Hz, 1 H), 7.52 (t, *J* =8.0 Hz, 2 H), 7.34 - 7.36 (m, 2 H), 7.23 (t, *J* =8.5 Hz, 2 H). MS (ESI): calculated for C₂₃H₁₄F₄N₂O₃S, 474.10, found 497.0 [M + Na]⁺. HPLC2: *t*_{R} 16.80 min, purity 98.2%. |
| (2-(4-(benzyloxy)phenyl)-1-(phenylsulfonyl)-1*H*-imidazol-4-yl)(4-fluorophenyl)methanone (**11jb**) | Yield: 22.3.0%; mp 149 - 151 °C. ¹H NMR (500 MHz, CDCl₃) δ 8.09 (q, *J* =5.5 Hz, 3.5 Hz, 2 H), 7.82 (d, *J* =7.5 Hz, 2 H), 7.63 (t, 7.5 Hz, 1 H), 7.36-7.50(m, 10 H), 7.25 (t, *J* =8.5 Hz, 2 H), 6.98 (d, *J* =8.0 Hz, 2 H), 5.17 (s, 2 H). MS (ESI): calculated for C₂₉H₂₁FN₂O₄S, 512.10, found 535.0 [M + Na]⁺. HPLC2: *t*_{R} 18.35 min, purity 95.1*%*. |
| (2-(4-bromophenyl)-1-(phenylsulfonyl)-1*H-*imidazol-4-yl)(3,4,5-trimethoxyphenyl)methanone (**11la**) | Yield: 32.6% ¹H NMR (500 MHz, CDCl₃) δ 8.06 (d, *J* = 8.0 Hz, 2 H), 7.88 (d, *J* = 8.5 Hz, 1 H), 7.77 (t, *J* = 7.0 Hz, 1 H), 7.54-7.63 (m, 4 H), 7.31-7.36 (m, 4 H), 4.04 (s, 3 H), 4.01 (s, 6 H). MS (ESI) calcd for C₂₅H₂₁BrN₂O₆S 556.0, found 557.0 [M + H]⁺. |
| (1-(phenylsulfonyl)-2-(4-(trifluoromethyl)phenyl)-1*H*-imidazol-4-yl)(3,4,5-trimethoxyphenyl)methanone (**11pa**) | Yield: 36.7 %; ¹H NMR (500 MHz, CDCl₃) δ 8.06 (d, *J* = 7.5 Hz, 2 H), 7.78 (t, *J* = 8.0 Hz, 1 H), 7.72 (d, *J* = 8.0 Hz, 2 H), 7.62 (d, *J* = 8.0 Hz, 2 H), 7.59 (d, *J* = 8.0 Hz, 2 H), 7.50 (s, 1 H), 7.37 (s, 2 H), 4.04 (s, 3 H), 4.02 (s, 6 H). MS (ESI) calcd for C₂₆H₂₁F₃N₂O₆S 546.1, found 547.1 [M+H]⁺. |
| (2-(4-(dimethylamino)phenyl)-1-((4-methoxyphenyl)sulfonyl)-1*H*-imidazol-4-yl)(3,4,5-trimethoxyphenyl)methanone (**11gaa**) | Yield: 34.1*%*; mp 147-149 °C. ¹H NMR (500 MHz, CDCl₃) δ 8.07 (q, *J* = 8.5 Hz, 5.5 Hz, 2 H), 7.78 (d, *J* = 9.0 Hz, 2 H), 7.41 (d, *J* = 8.5 Hz, 2 H), 7.39 (s, 1 H), 7.23 (t, *J* = 8.5 Hz, 2 H), 6.91 (d, *J* = 9.0 Hz, 2 H), 6.68 (d, *J* = 9.0 Hz, 2 H), 3.89 (s, 3 H), 3.08 (s, 3 H). MS (ESI) calcd for C₂₈H₂₉N₃O₇S 551.2, found 573.1 [M + Na]⁺. HPLC2: t_{R} 18.6 min, purity 96.9*%*. |
| (2-phenyl-1*H*-imidazol-4-yl)(3,4,5-trimethoxyphenyl)methanone (**12aa**) | Yield: 10.1 %; mp 227-229 °C. ¹H NMR (500 MHz, CDCl₃) δ 8.0-8.03 (m, 2 H), 7.83 (s, 1 H), 7.34-7.38 (m, 3 H), 7.21 (s, 2 H), 3.90 (s, 3 H), 3.84 (s, 6 H). MS (ESI): calculated for C₁₉H₁₈N₂O, 338.1, found 337.1 [M - H] HPLC2: *t*_{R}14.19 min, purity 96.3%. |
| (4-methoxyphenyl)(2-phenyl-1*H*-imidazol-4-yl)methanone (**12ab**) | Yield: 16.6%; mp 179 - 181 °C. ¹H NMR (500 MHz, CDCl₃) δ 11.1 (br, 1 H), 8.07-8.10 (m, 2 H), 8.04 (d, *J* = 8.5 Hz, 2 H), 7.84 (d, *J* = 1.0 Hz, 1 H), 7.49-7.51 (m, 3 H), 7.07 (d, *J* = 9.0 Hz, 2 H), 3.95 (s, 3 H). MS (ESI): calculated for C₁₇H₁₄N₂O₂, 278.10, found 279.0 [M + H]⁺. HPLC1: *t*_{R} 15.14 min, purity > 99*%*. |
| (3-methoxyphenyl)(2-phenyl-1*H*-imidazol-4-yl)methanone (**12ac**) | Yield: 22.5 %; mp 160 - 162 °C. ¹H NMR (500 MHz, CDCl₃) δ 11.2 (br, 1 H), 8.10-8.12 (m, 2 H), 7.87 (d, *J* = 1.0 Hz, 1 H), 7.61 (d, *J* = 7.5 Hz, 1 H), 7.48 - 7.52 (m, 5 H), 7.21 (dd, *J* = 2.5 Hz, 8.5Hz, 1 H), 3.91 (s, 3 H). MS (ESI): calculated for C₁₇H₁₄N₂O₂, 278.10, found 279.0 [M + H]⁺. HPLC2: *t*_{R} 15.07 min, purity > 99*%*. |
| (3,5-dimethoxyphenyl)(2-phenyl-1 *H-*imidazol-4-yl)methanone (**12ad**) | Yield: 26.2%; mp 168 - 170°C. ¹H NMR (500 MHz, CDCl₃) δ 8.04-8.06 (m, 2 H), 7.88 (s, 1 H), 7.50-7.52 (m, 3 H), 7.15 (d, *J* = 2.0 Hz, 2 H), 6.75 (t, *J* = 1.0 Hz, 1 H), 3.89 (s, 6 H). MS (ESI): calculated for C₁₈H₁₆N₂O₃, 308.10, found 331.1 [M + Na]⁺, 306.9 [M - H]⁻. HPLC2: *t*_{R} 15.59 min, purity > 99*%*. |
| (3,4-dimethoxyphenyl)(2-phenyl-1*H-*imidazol-4-yl)methanone (**12ae**) | Yield: 18.6%; mp 162 - 164 °C. ¹H NMR (500 MHz, CDCl₃) δ 10.9 (br, 1 H), 8.05 (dd, *J* = 1.5 Hz, 8.0 Hz, 2 H), 7.86 (d, *J* = 1.5 Hz, 1 H), 7.74 (dd, *J* = 2.0 Hz, 8.5 Hz, 1 H), 7.56 (d, *J* = 2.0 Hz, 1 H), 7.50-7.52 (m, 3 H), 7.04 (d, *J* = 8.5 Hz, 1 H), 4.03 (s, 3 H), 3.99 (s, 3 H). MS (ESI): calculated for C₁₈H₁₆N₂O₃, 308.10, found 331.1 [M + Na]⁺, 306.9 [M - H]⁻. HPLC2: *t*_{R} 13.54 min, purity > 99*%*. |
| (4-fluorophenyl)(2-phenyl-1*H*-imidazol-4-yl)methanone (**12af**) | Yield: 30.2*%*; mp 231 - 233 °C. ¹H NMR (500 MHz, CDCl₃) δ 10.6 (br, 1 H), 8.02-8.05 (m, 4 H), 7.81 (d, *J* = 1.0 Hz, 1 H), 7.51-7.54 (m, 3 H), 7.27 (t, *J* = 8.5 Hz, 2 H). MS (ESI): calculated for C₁₆H₁₁FN₂O, 266.10, found 267.0 [M +H]⁺, 264.8 [M - H]⁻. HPLC1: *t*_{R} 15.37 min, purity 98.9%. |
| (3-fluorophenyl)(2-phenyl-1*H*-imidazol-4-yl)methanone (**12ag**) | Yield: 23.4%; mp 212 - 214 °C. ¹H NMR (500 MHz, CDCl₃) δ 8.05 (dd, *J* = 1.5 Hz, 7.5 Hz, 2 H), 7.86 (s, 1 H), 7.84 (d, *J* = 7.0 Hz, 1 H), 7.74 (d, *J* = 8.5 Hz, 1 H), 7.52-7.58 (m, 4 H), 7.37 (dt, *J* =2.0 Hz, 6.0 Hz, 1 H). MS (ESI): calculated for C₁₆H₁₁FN₂O, 266.10, found 267.0 [M + H]⁺, 264.8 [M - H]⁻. HPLC1: *t*_{R} 15.29 min, purity > 99*%*. |
| (2-phenyl-1*H*-imidazol-4-yl)(*p-*tolyl)methanone (**12ah**) | Yield: 15.6%; mp 225 - 227°C. ¹H NMR (500 MHz, CDCl₃) δ 11.1 (br, 1H), 8.08 (d, *J* = 7.5 Hz, 2 H), 7.93 (d, *J* =9.0 Hz, 2 H), 7.84 (s, 1 H), 7.48-7.52 (m, 3 H), 7.38 (d, *J* = 10.0 Hz, 2 H), 2.50 (s, 3 H). MS (ESI): calculated for C₁₇H₁₄N₂O, 262.10, found 263.0 [M +H]⁺, 260.8 [M - H]⁻. HPLC2: *t*_{R} 15.86 min, purity 98.7%. |
| (2-phenyl-1*H*-imidazol-4-yl)(*m-*tolyl)methanone (**12ai**) | Yield: 20.5%; mp 168 - 169 °C. ¹H NMR (500 MHz, CDCl₃) δ 11.0 (br, 1 H), 8.09-8.11 (m, 2 H), 7.84 (d, *J* = 1.5 Hz, 1 H), 7.81-7.82 (m, 2 H), 7.47-7.52 (m, 5 H), 2.50 (s, 3 H). MS (ESI): calculated for C₁₇H₁₄N₂O, 262.10, found 285.0 [M +Na]⁺, 260.8 [M - H]⁻. HPLC2: *t*_{R} 15.89 min, purity > 99%. |
| (2-(4-fluorophenyl)-1*H*-imidazol-4-yl)(3,4,5-trimethoxyphenyl)methanone (**12ba**) | Yield: 12.2*%*. mp 176 - 178 °C. ¹H NMR (500 MHz, CDCl₃) δ 10.72 (br, 1 H), 8.02 (q, *J* = 5.0 Hz, 2 H), 7.84 (s, 1 H), 7.19 (t, *J* = 10.0 Hz, 2 H), 4.00 (s, 6 H), 3.97 (s, 3 H). MS (ESI): calculated for C₁₉H₁₇FN₂O₄, 356.10, found 379.1 [M + Na]+, 354.9 [M - H]⁻. HPLC1: *t*_{R} 17.23 min, purity > 99% |
| (2-(4-methoxyphenyl)-1*H*-imidazol-4-yl)(3,4,5-trimethoxyphenyl)methanone (**12ca**) | Yield: 10.2%; mp 220 - 222 °C. ¹H NMR (300 MHz, CDCl₃) δ 10.24 (br, 1H), 7.93 (d, *J =* 14.5 Hz, 2 H), 7.81 (s, 1H), 7.24 (s, 2 H), 7.03 (d, *J* = 14.5 Hz, 2 H), 3.97 (s, 3 H), 3.95 (s, 6 H), 3.90 (s, 3 H). MS (ESI): calculated for C₂₀H₂₀N₂O₅, 368.10, found 391.0 [M + Na]⁺, 367.0 [M - H]⁻. HPLC2: *t*_{R} 14.46 min, purity 98.4%. |
| (4-fluorophenyl)(2-(4-methoxyphenyl)-1*H-*imidazol-4-yl)methanone (**12cb**) | Yield: 15.2%; mp 245 - 247°C. ¹H NMR (500 MHz, CDCl₃) δ 10.20 (br, 1 H), 7.93-7.96 (m, 2 H), 7.85 (d, *J* = 5.0 Hz, 2 H), 7.68 (s, 1 H), 7.15-7.17 (m, 2 H), 6.95 (d, *J* = 6.0 Hz, 2 H), 3.82 (s, 3 H). MS (ESI): calculated for C₁₇H₁₃FN₂O₂, 296.10, found 319.1 [M + Na]⁺, 294.9 [M - H]⁻. HPLC2: *t*_{R} 15.40 min, purity 98.8%. |
| (2-(*p*-tolyl)-1*H*-imidazol-4-yl)(3,4,5-trimethoxyphenyl)methanone (**12da**) | Yield: 48.5%; mp 201 - 203 °C. ¹H NMR (500 MHz, CDCl₃) δ 10.40 (br, 1H), 7.88 (d, *J* = 8.0 Hz, 2 H), 7.82 (s, 1 H), 7.31 (d, *J* = 8.0 Hz, 2 H), 7.24 (s, 2 H), 3.96 (s, 3 H), 3.94 (s, 6 H), 2.43 (s, 3 H). MS (ESI): calculated for C₂₀H₂₀N₂O₄, 352.10, found 375.2 [M + Na]⁺. HPLC2: *t*_{R} 15.45 min, purity 97.4%. |
| (4-fluorophenyl)(2-(*p*-tolyl)-1*H*-imidazol-4-yl)methanone (**12db**) | Yield: 56.3%; mp 229 - 231 °C. ¹H NMR (500 MHz, CDCl₃) δ 10.50 (br, 1 H), 7.99-8.02 (m, 2 H), 7.88 (d, *J* = 8.0 Hz, 2 H), 7.60 (d, *J* = 1.0 Hz, 1 H), 7.30 (d, *J* = 8.0 Hz, 2 H), 7.23 (t, *J* = 9.0 Hz, 2 H), 2.43 (s, 3 H). MS (ESI): calculated for C₁₇H₁₃FN₂O, 280.10, found 281.0 [M + H]⁺, 278.9 [M - H]⁻. HPLC2: *t*_{R} 16.31 min, purity > 99*%*. |
| (4-hydroxy-3,5-dimethoxyphenyl)(2-(*p*-tolyl)-1*H*-imidazol-4-yl)methanone (**12dc**) | Yield: 56.8*%*; mp 220-222°C. ¹H NMR (500 MHz, CDCl₃) δ 8.02 (d, *J* = 8.0 Hz, 2H), 7.91(s, 1H), 7.39 (s, 2H), 7.28 (d, *J* = 7.5 Hz, 2H), 4.00 (s, 6H), 2.44 (s, 3H). MS (ESI) calcd for C₁₉H₁₈ FN₂O₄ 338.1, found 339.1 [M + H]⁺. HPLC1: t_{R} 3.91 min, purity > 99%. |
| (3,4,5-trimethoxyphenyl)(2-(3,4,5-trimethoxyphenyl)-1*H*-imidazol-4-yl)methanone (**12ea**) | Yield: 86.8%; mp 196 - 198 °C. ¹H NMR (500 MHz, DMSO-*d₆*) δ 13.3 (br, 0.47 H), 13.50 (br, 0.52 H), 8.19 (s, 0.49 H), 7.90 (s, 1 H), 7.83 (s, 0.5 H), 7.59 (s, 1 H), 7.40 (s, 1 H), 7.18 (s, 1 H), 3.89 (s, 6 H), 3.86 (s, 6 H), 3.77 (s, 3 H), 3.72 (s, 3 H). MS (ESI): calculated for C₂₂H₂₄N₂O₇, 428.2, found 451.1[M + Na]⁺, 426.9 [M - H]⁻. HPLC2: *t*_{R} 14.49 min, purity > 99*%*. |
| (4-fluorophenyl)(2-(3,4,5-trimethoxyphenyl)-1*H*-imidazol-4-yl)methanone (**12eb**) | Yield: 90.2*%*; mp 153 - 155 °C. ¹H NMR (500 MHz, CDCl₃) δ 10.42 (br, 1 H), 8.00 (q, *J* = 5.5 Hz, 3.0Hz, 2 H), 7.76 (s, 1 H), 7.23 (t, *J* = 8.5 Hz, 2 H), 7.19 (s, 2 H), 3.94 (s, 3 H), 3.92 (s, 3 H). MS (ESI): calculated for C₁₉H₁₇FN₂O₄, 356.1, found 379.0 [M + Na]⁺, 354.9 [M - H]⁻. HPLC2: *t*_{R} 15.31 min, purity > 99*%*. |
| (2-(4-chlorophenyl)-1*H*-imidazol-4-yl)(3,4,5-trimethoxyphenyl)methanone (**12fa**) | Yield: 36.9%; mp 193 - 195 °C. ¹H NMR (500 MHz, CDCl₃) δ 10.75 (br, 1 H), 7.96 (d, *J* = 8.5 Hz, 2 H), 7.83 (s, 1 H), 7.47 (d, *J* = 9.0 Hz, 2 H), 7.23 (s, 2 H), 3.97 (s, 3 H), 3.94 (s, 6 H), 2.43 (s, 3 H). MS (ESI): calculated for C₁₉H₁₇ClN₂O₄, 372.1, found 395.1 [M + Na]⁺, 370.9 [M - H]⁻. HPLC2: *t*_{R} 16.36 min, purity > 99*%*. |
| (2-(4-chlorophenyl)-1*H*-imidazol-4-yl)(4-fluorophenyl)methanone (**12fb**) | Yield: 83.7%; mp 232 - 234 °C. ¹H NMR (500 MHz, CDCl₃) δ 10.78 (br, 1 H), 8.00 (q, *J* = 5.5 Hz, 3.0Hz, 2 H), 7.96 (d, *J* = 9.0 Hz, 2 H), 7.78 (s, 1 H), 7.47 (d, *J* = 8.0 Hz, 2 H), 7.24 (t, *J* = 8.5 Hz, 2 H). MS (ESI): calculated for C₁₆H₁₀ClFN₂O, 300.1, found 323.0 [M + Na]⁺, 298.8 [M-H]⁻. HPLC2: *t*_{R} 17.08 min, purity > 99*%*. |
| (2-(4-chlorophenyl)-1*H*-imidazol-4-y1)(4-hydroxy-3,5-dimethoxyphenyl)methanone (**12fc**) | Yield: 80.2*%*; mp 216-218 °C. ¹H NMR (500 MHz, CD₃OD) δ 8.06 (d, *J* = 8.5 Hz, 2 H), 7.99 (s, 1 H), 7.61 (d, *J* = 8.0 Hz, 2 H), 7.52 (s, 2 H), 4.01 (s, 6 H). MS (ESI) calcd for C₁₈H₁₅ClN₂O₄ 358.1, found 359.1 [M + H]⁺. HPLC2: t_{R} 4.12 min, purity > 99*%*. |
| (2-(4-(dimethylamino)phenyl)-1*H*-imidazol-4-yl)(3,4,5-trimethoxyphenyl)methanone (**12ga**) | Yield: 91.2%; mp 195 - 197 °C. ¹H NMR (500 MHz, CDCl₃) δ 10.39 (br, 1 H), 7.87 (d, *J* = 8.5 Hz, 2 H), 7.80 (s, 1 H), 7.23 (s, 2 H), 6.75(d, *J =* 9.0 Hz, 2 H), 3.95 (s, 3 H), 3.94 (s, 6 H), 3.05 (s, 6 H). MS (ESI): calculated for C₂₁H₂₃N₃O₄, 381.2, found 404.2 [M + Na]⁺, 380.0 [M - H]⁻. HPLC2: *t*_{R} 15.20 min, purity 95.8%. |
| (2-(4-(dimethylamino)phenyl)-1*H*-imidazol-4-yl)(4-fluorophenyl)methanone (**12gb**) | Yield: 86.7%; mp 278 - 280 °C. ¹H NMR (500 MHz, CDCl₃) δ 10.21 (br, 1 H), 7.98 (q, *J* = 5.0 Hz, 3.5Hz, 2 H), 7.84 (d, *J* = 8.5 Hz, 2 H), 7.72 (s, 1 H), 7.20 (t, *J* = 8.5 Hz, 2 H), 6.76 (t, *J* = 9.0 Hz, 2 H), 3.06 (s, 6 H). MS (ESI): calculated for C₁₈H₁₆FN₃O, 309.1, found 332.1 [M + Nat, 307.9 [M-H]⁻. HPLC2: *t*_{R} 16.06 min, purity 95.6%. |
| (2-(3,4-dimethoxyphenyl)-1*H*-imidazol-4-yl)(3,4,5-trimethoxyphenyl)methanone (**12ha**) | Yield: 85.0 %; mp 100 - 102°C. ¹H NMR (500 MHz, CDCl₃) δ 10.19 (br, 1 H), 7.81 (s, 1 H), 7.58 (d, *J* = 1.5 Hz, 1 H), 7.48 (d, *J* = 8.0 Hz, 1 H), 7.25 (s, 2 H), 6.97 (d, *J* = 8.5 Hz, 1 H), 4.00 (s, 3 H), 3.96 (s, 6 H), 3.95 (s, 6 H). MS (ESI): calculated for C₂₁H₂₂N₂O₆, 398.2, found 399.1 [M + H]⁺, 397.0 [M - H]⁻. HPLC2: *t*_{R} 13.73 min, purity > 99*%*. |
| (2-(3,4-dimethoxyphenyl)-1*H*-imidazol-4-yl)(4-fluorophenyl)methanone (**12hb**) | Yield: 78.3%; mp 174 - 176 °C. ¹H NMR (500 MHz, CDCl₃) δ 8.02 (t, *J* = 9.0 Hz, 2 H), 7.75 (s, 1 H), 7.57 (s, 1 H), 7.48 (d, *J* = 8.5 Hz, 1 H), 7.23 (t, *J* = 8.5 Hz, 2 H), 6.95 (d, *J* = 8.5 Hz, 1 H), 3.99 (s, 3 H), 3.96 (s, 3 H). MS (ESI): calculated for C₁₈H₁₅FN₂O₃, 326.1, found 349.0 [M + Na]⁺ 324.9 [M - H]⁻. HPLC2: *t*_{R} 14.65 min, purity > 99*%*. |
| (2-(2-(trifluoromethyl)phenyl)-1*H*-imidazol-4-yl)(3,4,5-trimethoxyphenyl)methanone (**12ia**) | Yield: 83.8*%*; mp 75 - 77 °C. ¹H NMR (500 MHz, CDCl₃) δ 10.37 (br, 1 H), 8.00-8.02 (m, 1 H), 7.87 (s, 1 H), 7.82-7.85 (m, 1 H), 7.69-7.74 (m, 1 H), 7.62-7.66 (m, 1 H), 7.25 (s, 2 H), 3.99 (s, 3 H), 3.98 (s, 6 H). MS (ESI): calculated for C₂₀H₁₇F₃N₂O₄, 406.1, found 429.1 [M + Na]⁺, 405.0 [M-H]⁻. HPLC2: *t*_{R} 13.98 min, purity > 99*%*. |
| (4-fluorophenyl)(2-(2-(trifluoromethyl)phenyl)-1 *H*-imidazol-4-yl)methanone (**12ib**) | Yield: 91.1*%*; mp 152 - 154 °C. ¹H NMR (500 MHz, CDCl₃) δ 8.12-8.14 (m, 2 H), 7.97 (d, *J* = 7.5 Hz, 1 H), 7.82-7.85 (m, 2 H), 7.69 (t, *J* = 7.5 Hz, 1 H), 7.61 (t, *J* = 8.0 Hz, 1 H), 7.22 (t, *J* = 9.0 Hz, 2 H). MS (ESI): calculated for C₁₇H₁₀F₄N₂O, 334.1, found 357.1 [M + Na]⁺, 332.9 [M-H]⁻. HPLC2: *t*_{R} 15.10 min, purity > 99*%*. |
| (2-(4-(benzyloxy)phenyl)-1*H*-imidazol-4-yl)(3,4,5-trimethoxyphenyl)methanone (**12ja**) | Yield: 16.5%; mp 191 - 193 °C. ¹H NMR (500 MHz, CDCl₃) δ 10.22 (br, 1 H), 7.93 (d, *J* = 9.0 Hz, 2 H), 7.81 (s, 1 H), 7.37-7.47 (m, 5 H), 7.24 (s, 2 H), 7.11 (d, *J* = 8.5 Hz, 2 H), 5.16 (s, 2 H), 3.97 (s, 3 H), 3.95 (s, 6 H). MS (ESI): calculated for C₂₆H₂₄N₂O₅, 444.2, found 467.1 [M + Na]⁺, 442.9 [M - H]⁻. HPLC2: *t*_{R} 17.36 min, purity 95.5%. |
| (2-(4-(benzyloxy)phenyl)-1*H*-imidazol-4-yl)(4-fluorophenyl)methanone (**12jb**) | Yield: 84.7%; mp 212 - 214 °C. ¹H NMR (300 MHz, CDCl₃) δ 10.28 (br, 1 H), 799-8.04 (m, 2 H), 7.92-7.95 (m, 2 H), 7.76 (d, *J* = 1.5 Hz, 1 H), 7.38-7.48 (m, 5 H), 7.20-7.25 (m, 2 H), 7.09-7.12 (m, 2 H), 5.16 (s, 2 H). MS (ESI): calculated for C₂₃H₁₇FN₂O₂, 372.1, found 395.1 [M + Na]⁺. HPLC2: *t*_{R} 17.97 min, purity 97.8%. |
| (2-(4-hydroxyphenyl)-1*H*-imidazol-4-yl)(3,4,5-trimethoxyphenyl)methanone (**12ka**) | Yield: 72.3%. mp 191-193 °C. ¹H NMR (500 MHz, CD₃OD) δ 8.31 (s, 1 H), 7.90 (d, *J* = 8.5 Hz, 2 H), 7.31 (s, 2 H), 7.05 (s, 2 H), 3.95 (s, 6 H), 3.88 (s, 3 H). MS (ESI): calculated for C₁₉H₁₈N₂O₅, 354.1, found 355.1 [M + H]⁺, 352.9 [M - H]⁻. HPLC2: *t*_{R} 12.25 min, purity 98.7%. |
| (2-(4-(hydroxyphenyl)-1*H*-imidazol-4-yl)(4-fluorophenyl)methanone (**12kb**) | Yield: 89.0%; mp 276 - 278 °C. ¹H NMR (500 MHz, CDCl₃) δ 8.31 (s, 1 H), 8.13 (q, *J* = 5.5 Hz, 3.0 Hz, 2 H), 7.93 (d, *J* = 8.5 Hz, 2 H), 7.38 (t, *J* = 8.5 Hz, 2 H), 7.07 (d, *J* = 8.5 Hz, 2 H). MS (ESI): calculated for C₁₆H₁₁FN₂O₂, 282.1, found 283.0 [M + H]⁺, 280.9 [M - H]⁻. HPLC2: *t*_{R} 13.46 min, purity 97.65%. |
| (2-(4-bromophenyl)-1*H*-imidazol-4-yl)(3,4,5-trimethoxyphenyl)methanone (**121a**) | Yield: 25.6%; mp 190-192 °C. ¹H NMR (500 MHz, CDCl₃) δ 7.99 (d, *J* = 8.5 Hz, 2 H), 7.92 (s, 1 H), 7.70 (d, *J* = 8.5 Hz, 2 H), 7.32 (s, 2 H), 4.03 (s, 3 H), 4.00 (s, 6 H). MS (ESI) calcd for C₁₉H₁₇ BrN₂O₄ 416.0, found 417.0 [M + H]⁺. HPLC2: t_{R} 4.24 min, purity 98.8%. |
| (2-(4-(trifluoromethyl)phenyl)-1*H*-imidazol-4-yl)(3,4,5-trimethoxyphenyl)methanone (**12pa**) | Yield: 85.3%; mp 195 - 196 °C. ¹H NMR (500 MHz, CDCl₃) δ 8.22 (d, *J* = 8.5 Hz, 2 H), 7.96 (s, 1 H), 7.83 (d, *J* = 8.5 Hz, 2 H), 7.34 (s, 2 H), 4.04 (s, 3 H), 4.00 (s, 6 H). MS (ESI) calcd for C₂₀H₁₇F₃N₂O₄ 406.1; found 407.1 [M + H]⁺, HPLC2: t_{R} 18.00 min, purity >99%. |
| (2-phenyl-1*H*-imidazol-1-yl)(3,4,5-trimethoxyphenyl)methanone (**12aaa**) | Yield: 39.8%; mp 113 - 115 °C. ¹H NMR (500 MHz, CDCl₃) δ 7.53 (q, *J* = 5.0 Hz, 3.0 Hz, 2 H), 7.41 (d, *J* = 1.0 Hz, 1 H), 7.33-7.35 (m, 3 H), 7.23 (d, *J* = 1.0 Hz, 1 H), 7.03 (s, 2 H), 3.93 (s, 3 H), 3.85 (s, 6 H). MS (ESI): calculated for C₁₉H₁₈N₂O₄, 338.1, found 339.1 [M +H]⁺. HPLC2: *t*_{R} 13.8 min, purity 95.6%. |
| (4-methoxyphenyl)(2-pheny)-1*H*-imidazol-1-yl)methanone (**12aba**) | Yield: 56.3%; mp 68 - 70 °C. ¹H NMR (500 MHz, CDCl₃) δ 7.78 (d, *J* = 9.0 Hz, 2 H), 7.54-7.56 (m, 2 H), 7.32-7.34 (m, 4 H), 7.21 (d, *J* = 1.0 Hz, 1 H), 6.93 (d, *J* = 8.5 Hz, 2 H), 3.90 (s, 3 H). MS (ESI): calculated for C₁₇H₁₄N₂O₂, 278.1, found 301.0 [M +Na]⁺, 276.8 [M - H]⁻. HPLC2: *t*_{R} 14.72 min, purity 95.7%. |
| (4-fluorophenyl)(2-(*p*-tolyl)-1*H*-imidazol-4-yl)methanone HCl salt (**12db-HCl**) | Yield: 95%; mp 115 - 117°C. ¹H NMR (500 MHz, DMSO-*d₆*) δ 8.20-8.23 (m, 2 H), 8.18 (s, 1 H), 8.04 (d, *J* = 6.5 Hz, 2 H), 7.42 (t, *J* = 8.0 Hz, 2 H), 7.37 (d, *J* = 7.0 Hz, 2 H), 2.38 (s, 3 H). MS (ESI): calculated for C₁₇H₁₄FClN₂O, 316.1, found 281.0 [M - HCl + H]⁺. HPLC2: *t*_{R} 17.16 min, purity >99%. |
| (4-fluorophenyl)(2-(4-methoxyphenyl)-1-methyl-1*H*-imidazol-4-yl)methanone (**12cba**) | Yield: 90.2%; mp 148-150 °C. ¹H NMR (500 MHz, CDCl₃) δ 8.45 (q, *J* = 8.5 Hz, 5.5 Hz, 2 H), 7.79 (s, 1 H), 7.63 (d, *J* = 8.5 Hz, 2 H), 7.16 (t, *J* = 8.5 Hz, 2 H), 7.03 (d, *J* = 9.0 Hz, 2 H), 3.89 (s, 3 H), 3.82 (s, 3 H). MS (ESI) calcd for C₁₈H₁₅ FN₂O₂ 310.1, found 311.0 [M + H]⁺. HPLC2: t_{R} 4.01 min, purity 97.6%. |
| (1-benzyl-2-(*p*-tolyl)-1*H*-imidazol-4-yl)(3,4,5-trimethoxyphenyl)methanone (**12daa**) | Yield: 92.8%; mp 135-137 °C. ¹H NMR (500 MHz, CDCl₃) δ 7.81 (s, 1 H), 7.80(d, *J* = 6.5 Hz, 2 H), 7.58 (d, *J* = 8.0 Hz, 2 H), 7.41-7.45 (m, 3 H), 7.31-7.33 (m, 2 H), 7.20 (d, *J* = 7.0 Hz, 2 H), 5.33 (s, 2 H), 3.99 (s, 3 H), 3.98 (s, 6 H), 2.47 (s, 3 H). MS (ESI) calcd for C₂₇H₂₆N₂O₄ 442.2, found 443.1 [M + Na]⁺. HPLCl: t_{R} 4.28 min, purity > 99%. |
| (1-methyl-2-(*p*-tolyl)-1*H*-imidazol-4-yl)(3,4,5-trimethoxyphenyl)methanone (**12dab**) | Yield: 87.4%; mp 110-112 °C. ¹H NMR (500 MHz, CDCl₃) δ 7.87 (s, 2 H), 7.86 (d, *J* = 8.0 Hz, 1 H), 7.65 (d, *J* = 10 Hz, 2 H), 7.37 (d, *J =* 10 Hz, 2 H), 4.01 (s, 6 H), 4.00 (s, 3 H), 3.90 (s, 3 H). MS (ESI) calcd for C₂₁H₂₂N₂O₄ 366.2, found 367.2 [M + H]⁺. HPLCl: t_{R} 4.23 min, purity > 99*%*. |
| (2-(4-(dimethylamino)phenyl)-1-((4-methoxyphenyl)sulfonyl)-1H-imidazol-4-yl)(4-fluorophenyl)methanone (**12gba**) | Yield: 34.1*%*; mp 147-149 °C. ¹H NMR (CDCl₃. 500 MHz) δ 8.07 (q, *J* = 8.5 Hz, 5.5 Hz, 2 H), 7.78 (d, *J =* 9.0 Hz, 2 H), 7.41 (d, *J =* 8.5 Hz, 2 H), 7.39 (s, 1 H), 7.23 (t, *J* = 8.5 Hz, 2 H), 6.91 (d, *J* = 9.0 Hz, 2 H), 6.68 (d, *J* = 9.0 Hz, 2 H), 3.89 (s, 3 H), 3.08 (s, 3 H). MS (ESI) calcd for C₂₅H₂₂FN₃O₄S 479.1, found 502.1 [M + Na]⁺. HPLC2: t_{R} 18.6 min, purity 96.9%. |
| (3,4,5-trihydroxyphenyl)(2-(3,4,5-trihydroxyphenyl)-1*H*-imidazol-4-yl)methanone (**13ea**) | Yield: 66.1 *%*. mp 294 - 296 °C. ¹H NMR (500 MHz, CD₃OD) δ 8.07 (s, 1 H), 7.07 (s, 2 H), 7.02 (s, 2 H). MS (ESI): calculated for C₁₆H₁₂N₂O₇, 344.1, found 345.0[M + H]⁺, 342.9 [M - H]⁻. HPLC2: *t*_{R} 3.62 min, purity 97.9%. |
| (2-(4-chlorophenyl)-1*H*-imidazol-4-yl)(3,4,5-trihydroxyphenyl)methanone (**13fa**) | Yield: 79.3%; mp > 300 °C. ¹H NMR (500 MHz, CD₃OD) δ 8.02 (d, *J* = 8.5 Hz, 2 H), 7.77 (s, 1 H), 7.54 (d, *J* = 8.5 Hz, 2 H), 7.14 (s, 2 H). MS (ESI): calculated for C₁₆H₁₁ClN₂O₄, 330.0, found 331.1 [M + Na]⁺, 328.9 [M - H]⁻. HPLC2: *t*_{R} 11.9 min, purity 95.6%. |
| (2-(3,4-dihydroxyphenyl)-1*H*-imidazol-4-yl)(3,4,5-trihydroxyphenyl)methanone (**13ha**) | Yield: 62.2 %; mp > 300°C. ¹H NMR (500 MHz, CD₃OD) δ 8.11 (s, 1 H), 7.46 (d, *J* = 2.0 Hz, 1 H), 7.42 (dd, *J* = 8.5 Hz, 2.0 Hz, 1 H), 7.10 (s, 2 H), 7.02 (d, *J* = 8.5 Hz, 1 H). MS (ESI): calculated for C₁₆H₁₂N₂O₆, 328.1, found 329.0 [M + H]⁺, 326.9 [M - H]⁻. HPLC2: *t*_{R} 3.64 min, purity 97.9*%*. |
| 2-(4-nitrophenyl)-4,5-dihydro-1*H*-imidazole (**14x**) | Yield: 70.3 %. ¹H NMR (500 MHz, CDCl₃) δ 8.30 (d, *J* = 9.0 Hz, 2 H), 7.98 (d, *J* = 8.5 Hz, 2 H), 3.88-3.95 (m, 4 H). MS (ESI): calculated for C₉H₉N₃O₂, 191.10, found 191.9 [M + H]⁺, 189.7 [M - H]⁻. |
| 2-(4-fluorophenyl)4,5-dihydro-1*H*-imidazole (**14b**) | Yield: 60.2 %. ¹H NMR (500 MHz, CDCl₃) δ 7.80 (q, *J* = 7.0 Hz, 2 H), 7.11 (d, *J* = 10.0 Hz, 2 H), 3.82 (br, 4 H). MS (ESI): calculated for C₉H₉FN₂, 164.10, found 165 [M + H]⁺. |
| 2-(4-methoxyphenyl)-4,5-dihydro-1*H-*imidazole (**14c**) | Yield: 56.9 %. ¹H NMR (500 MHz, CDCl₃) δ 7.84 (d, *J* = 8.5 Hz, 2 H), 6.94 (d, *J* = 9.0 Hz, 2 H), 3.87 (s, 3 H), 3.85 (br, 4 H). MS (ESI): calculated for C₁₀H₁₂N₂O, 176.10, found 177.0 [M + H]⁺. |

### REFERENCE EXAMPLE 6

### SYNTHESIS OF SELECTED INDOLYL-BENZOYL-IMIDAZOLE COMPOUNDS

The synthesis of **15xaa** is outlined in **Figure 12****.** This route was originally designed for the synthesis of **12xa,** but the nonselectivity of the benzoylation at the indole-2 and imidazole-4 positions resulted in the formation of **15xaa,** which is a closely related but bulkier analog of **11xaa.** The indole-5-carboxaldehyde **8x** was protected by a phenylsulfonyl group on the indole NH to afford intermediate **8xa. 8xa** was reacted with glyoxal and ammonium hydroxide to generate the 2-aryl-imidazole **9xa.** Protection of the imidazole NH with phenylsulfonyl gave the intermediate **10xaa** which was coupled with 3,4,5-trimethoxybenzoyl chloride to produce **16xaa.** Removal of the protecting group from **16xaa** provided **15xaa.**

**Synthesis of 1-(Phenylsulfonyl)-1*H*-indole-5-carbaldehyde (8xa).** To a solution of indole-3-carboxaldehyde (100 mmol) in ethanol (500 mL) at room temperature was added potassium hydroxide (110 equiv), the mixture was stirred until total solubilization. The ethanol was completely removed in vacuum and acetone (250 mL) added followed by benzenesulfonyl chloride (110 equiv). The precipitate was filtered off and the filtrate was concentrated and recrystallized from methanol to give a white solid. Yield: 32.6% ¹H NMR (500 MHz, CDCl₃) δ 10.17 (s, 1 H), 8.25 - 8.39 (m, 2 H), 7.97 - 8.09 (m, 3 H), 7.69 (t, *J* = 7.33 Hz, 1 H), 7.59 (t, *J* = 7.5 Hz, 2 H), 7.39 -7.54 (m, 2 H). MS (ESI) calcd for C₁₅H₁₁NO₃S 285.1, found 286.0 [M+H]⁺.

**Synthesis of (5-(4-(3,4,5-Trimethoxybenzoyl)-1*H-*imidazol-2-yl)-1*H*-indol-2-yl)(3,4,5-trimethoxyphenyl)methanone (15xaa)**: To a solution of (1-(phenylsulfonyl)-2-(1-(phenylsulfonyl)-2-(3,4,5-trimethoxybenzoyl)-1*H*-indol-5-yl)-1*H*-imidazol-4-yl)(3,4,5-trimethoxyphenyl)methanone (**16xaa**) (1 mmol) in ethanol (20 mL) was added sodium hydroxide (10 equiv) and stirred overnight in darkness. The reaction mixture was diluted by 50 mL of water and extracted by ethyl acetate (250 mL). The organic layer was dried over magnesium sulfate and concentrated. The residue was purified by flash column chromatography (hexane: ethyl acetate 3:1) or recrystallized from water and methanol to give a white solid. Yield: 30-95%.

**5-(1*H*-Imidazol-2-yl)-1-(phenylsulfonyl)-1*H*-indole (9xa).** Yield: 12.0%. ¹H NMR (500 MHz, DMSO-*d₆*) δ 8.33 (d, *J* = 2.9 Hz, 2 H), 8.13 (d, *J* = 7.8 Hz, 2 H), 7.98 - 8.04 (m, 1 H), 7.62 - 7.67 (m, 1 H), 7.55 (d, *J* = 7.82 Hz, 2 H), 7.22 - 7.34 (m, 4 H). MS (ESI) calcd for C₁₇H₁₃N₃O₂S 323.1, found 324.0 [M + H]⁺.

**1-(Phenylsulfonyl)-5-(1-(phenylsulfonyl)-1*H*-imidazol-2-yl)-1*H*-indole (10xaa).** Yield: 23.6%. ¹H NMR (500 MHz, CDCl₃) δ 8.01 (d, *J* = 8.5 Hz, 1 H), 7.95 (d, *J* = 7.5 Hz, 2 H), 7.73 (d, *J* = 1.0 Hz, 1 H), 7.70 (d, *J* = 4.0 Hz, 1 H), 7.63-7.66 (m, 2 H), 7.52-7.56 (m, 3 H), 7.31-7.34 (m, 3 H), 7.22 (t, *J* = 8.5 Hz, 2 H), 7.17 (s, 1 H), 6.14 (d, *J* = 3.5 Hz, 1 H). MS (ESI) calcd for C₂₃H₁₇N₃O₄S₂ 463.1, found 464.0 [M+H]⁺.

**(1-(Phenylsulfonyl)-2-(1-(phenylsulfonyl)-2-(3,4,5-trimethoxybenzoyl)-1*H-*indol-5-yl)-1*H*-imidazol-4-yl)(3,4,5-trimethoxyphenyl)methanone (16xaa).** Yield: 15.9%. ¹H NMR (500 MHz, CDCl₃) δ 8.18 - 8.25 (m, 3 H), 8.04 (d, *J* = 8.1 Hz, 2H), 7.70 - 7.78 (m, 2 H), 7.61 - 7.69 (m, 3 H), 7.55 (t, *J* = 7.7 Hz, 3 H), 7.50 (s, 1 H), 7.38 (s, 2 H), 7.34 (s, 2 H), 6.94 (s, 1 H), 3.99 - 4.06 (m, 12 H), 3.94 - 3.99 (m, 6 H). MS (ESI) calcd for C₄₃H₃₇N₃O₁₂S₂ 851.2, found 852.1 [M + H]⁺.

**(5-(4-(3,4,5-Trimethoxybenzoyl)-1*H*-imidazol-2-yl)-1*H*-indol-2-yl)(3,4,5-trimethoxyphenyl)methanone (15xaa)**. Yield: 45.9%; mp 239-241 °C. ¹H NMR (500 MHz, CDCl₃) δ 10.45 (s, 1 H), 9.44 (s, 1 H), 8.41 (s, 1 H), 8.04 (d, *J* = 8.5 Hz, 1 H), 7.86 (s, 1 H), 7.61 (d, *J* = 8.5 Hz, 1H), 7.29 (s, 2 H), 7.26 (s, 2 H), 3.99 (s, 3 H), 3.95-3.97 (m, 15H). MS (ESI) calcd for C₃₁H₂₉N₃O₈ 571.2, found 572.2 [M + H]⁺. HPLC2: t_{R} 4.09 min, purity 96.3%.

### REFERENCE EXAMPLE 7

### SYNTHESIS OF (2-(1H-INDOL-3-YL)-1H-IMIDAZOL-4-YL)(3,4,5-TRIMETHOXYPHENYL)METHANONE (17ya) (FIGURE 13)

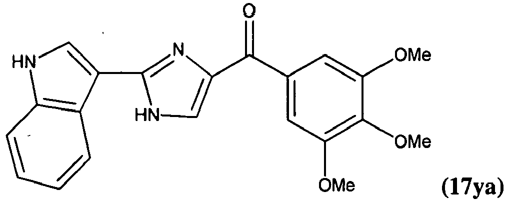

**Synthesis of 1-(phenylsulfonyl)-1*H*-indole-3-carboxaldehyde (8ya)**: To a solution of indole 3-carboxaldehyde (**8y**) (100 mmol) in ethanol (500 mL) at RT was added potassium hydroxide (1.1 equiv). The mixture was stirred until total solubilization. The ethanol was completely removed in vacuum and the residual was dissolved in acetone (250 mL) followed by adding benzenesulfonyl chloride (1.1 equiv, 110 mmol). The reaction mixture was stirred for half hour. The precipitate was filtered off and the filtrate was concentrated and recrystallized from methanol to give a white solid. Yield: 33%. ¹H NMR (500 MHz, CDCl₃) δ 10.17 (s, 1 H), 8.25-8.39 (m, 2 H), 7.97-8.09 (m, 3 H), 7.69 (t, *J* = 7.33 Hz, 1 H), 7.59 (t, *J* = 7.5 Hz, 2 H), 7.39-7.54 (m, 2 H). MS (ESI) calcd for C₁₅H₁₁NO₃S 285.1, found 286.0 [M + H]⁺.

**Synthesis of 3-(1*H*-imidazol-2-yl)-1-(phenylsulfonyl)-1*H*-indole (9ya)**: To a solution of 1-(phenylsulfonyl)-1*H*-indole-3-carboxaldehyde (**8ya**) (100 mmol) in ethanol (400 mL) at 0 °C was added a solution of 40% oxalaldehyde (glyoxal) in water (1.1 equiv, 110 mmol) and a solution of 29% ammonium hydroxide in water (10 equiv, 1000 mmol). After stirring for 2-3 days at RT, the reaction mixture was quenched by water and extracted by dichloromethane. The organic layer was removed by vacuum and the residue was subjected to flash column chromatography with hexane/ethyl acetate (4:1-2:1) as eluent to yield the titled compound as a yellow powder. Yield: 12%.¹H NMR (500 MHz, DMSO-*d₆*) δ 8.33 (d, *J* = 2.9 Hz, 2 H), 8.13 (d, *J* = 7.8 Hz, 2 H), 7.98 - 8.04 (m, 1 H), 7.62 - 7.67 (m, 1 H), 7.55 (d, *J* = 7.82 Hz, 2 H), 7.22 - 7.34 (m, 4 H). MS (ESI) calcd for C₁₇H₁₃N₃O₂S 323.1, found 324.0 [M + H]⁺.

**Synthesis of 1-(phenylsulfonyl)-3-(1-(phenylsulfonyl)-1*H*-imidazol-2-yl)-1*H-*indole (10ya)**: To a solution of 3-(1*H*-imidazol-2-yl)-1-(phenylsulfonyl)-1*H*-indole (**9ya**) (20 mmol) in anhydrous THF (300 mL) at 0 °C was added sodium hydride (60% dispersion in mineral oil, 1.2 equiv, 24 mmol) and stirred for 20 min. Benzenesulfonyl chloride (1.2 equiv, 24 mmol) was added and the reaction mixture was stirred overnight. After dilution by 200 mL of saturated NaHCO₃ solution (aqueous), the reaction mixture was extracted by ethyl acetate (600 mL). The organic layer was dried over magnesium sulfate and concentrated. The residue was purified by flash column chromatography (hexane: ethyl acetate 5:1) to give a white solid. Yield: 40%. ¹H NMR (CDCl₃, 300 MHz) δ 8.02-8.08 (m, 4 H), 7.72 (d, *J* = 1.5 Hz, 1 H), 7.35-7.60 (m, 8 H), 7.23 (d, *J* = 1.5 Hz, 1 H), 7.10-7.16 (m, 3 H). MS (ESI) calcd for C₂₃H₁₇N₃O₄S₂ 463.1, found 486.0 [M+Na]⁺.

**Synthesis of (1-(phenylsulfonyl)-2-(1-(phenylsulfonyl)-1*H*-indol-3-yl)-1*H-*imidazol-4-yl)(3,4,5-trimethoxyphenyl)methanone (17yaa)**: To a solution of 1-(phenylsulfonyl)-3-(1-(phenylsulfonyl)-1*H*-imidazol-2-yl)-1*H*-indole (**10ya**) (5.0 mmol) in anhydrous THF (100 mL) at -78 °C was added 1.7 M *tert*-butyllithium in pentane (1.2 equiv, 6.0 mmol) and stirred for 10 min. A solution of 3,4,5-trimethoxybenzoyl chloride (1.2 equiv, 6.0 mmol) in THF was added at -78 °C and stirred overnight. The reaction mixture was quenched with 100 mL of saturated NaHCO₃ solution (aqueous) and extracted by ethyl acetate (300 mL). The organic layer was dried over magnesium sulfate and concentrated. The residue was purified by flash column chromatography (hexane: ethyl acetate 3:1) to give a white solid. Yield: 30%. ¹H NMR (500 MHz, CDCl₃) δ 8.09 (d, *J* = 10 Hz, 1 H), 8.04 (d, *J* = 10 Hz, 2 H), 7.91 (s, 1 H), 7.76 (d, *J* = 5 Hz, 2 H), 7.65 (t, *J* = 10 Hz, 1 H), 7.55-7.58 (m, 5 H), 7.40 (s, 2 H), 7.33-7.36 (m, 3 H), 7.25 (t, *J* = 10 Hz, 1 H),4.05 (s, 3 H), 4.03 (s, 6 H). MS (ESI) calcd for C₃₃H₂₇N₃O₈ 657.0, found 680.1 [M+Na]⁺.

**Synthesis of (2-(1*H*-indol-3-yl)-1*H*-imidazol-4-yl)(3,4,5-trimethoxyphenyl)methanone (17ya):** To a solution of (1-(phenylsulfonyl)-2-(1-(phenylsulfonyl)-1*H*-indol-3-yl)-1*H*-imidazol-4-yl)(3,4,5-trimethoxyphenyl)methanone (**17yaa**) (1 mmol) in ethanol (40 mL) and water (4 mL) was added sodium hydroxide (10 equiv, 10 mmol) and stirred overnight under refluxing condition in darkness. The reaction mixture was diluted by 50 mL of water and extracted by ethyl acetate (200 mL). The organic layer was dried over magnesium sulfate and concentrated. The residue was purified by flash column chromatography (hexane: ethyl acetate 1:1) to give a yellow solid. Yield: 60%. ¹H NMR (500 MHz, CD₃OD) δ 8.31 (d, *J* = 6.5 Hz, 1 H), 7.99 (s, 1 H), 7.90 (s, 1 H), 7.48-7.52 (m, 3 H), 7.24-7.28 (m, 2 H), 4.00 (s, 6 H), 3.93 (s, 3 H). MS (ESI) calcd for C₂₁H₁₉N₃O₄ 377.1, found 400.1 [M + Na]⁺. Mp 208-210 °C.

### EXAMPLE 8

### SYNTHESIS OF (2-(1H-INDOL-5-YLAMINO)THIAZOL-4-YL)(3,4,5-TRIMETHOXYPHENYL)METHANONE (COMPOUND 55) (FIGURE 15).

A mixture of 5-nitro-1*H*-indole (11 g, 67.9 mmol) and Pd/C (5%; 1 g), dissolved in ethanol (50 mL), was hydrogenated for 3 h at 40 psi. The reaction mixture was filtered and the excess of ethanol was evaporated under reduced pressure. Solid product was recrystallized from hexane to obtain the pure compound 5-aminoindole (**55-1**). Yield: 92.5%. ¹H NMR (500 MHz, CDCl₃): δ 7.96 (br, 1 H), 7.20 (d, 1 H), 7.13 (s, 1 H), 6.95 (s, 1 H), 6.67 (dd, 1 H), 6.37 (s, 1 H), 3.50 (s, 2 H). MS (ESI) *m*/*z* 133.0 (M + H)⁺.

A solution of 5-aminoindole (8 g, 60.6 mmol) in acetone (150 mL) was reacted with benzoylisothiocyanate (9.88 g, 60. mmol) at RT for about 4 h. The resulting solid was filtered and treated with 2 N NaOH in THF (120 mL). The mixture was refluxed for about 6 h and allowed to warm to RT. The solvent was evaporated off under vacuum. The residue was diluted with water (20 mL) and neutralized to pH 7 with 1 N HCl. The resulting solid was filtered and dried under vacuum to afford 5-indolylthiourea (**55-2**). 5-Indolyl thiourea (0.01 mol) and ethyl bromopyruvate (0.011 mol) were dissolved in 3 mL ethanol and held at reflux for 2 h. The reaction was cooled, the crystalline ethyl 2-(1*H*-indol-5-ylamino)thiazole-4-carboxylate (**55-3**) was collected by filtration and washed with ethanol. Refluxing the mixture of ethyl esters with the NaOH-ethanol solution gave 2-(1*H*-indol-5-ylamino)thiazole-4-carboxylic acid (**55-4**) which was used directly in next steps. To a mixture of the crude acid (2.5 mmol), EDCI (2.9 mmol), HOBt (2.6 mmol) and NMM (5.3 mmol) in CH₂Cl₂ (30 mL) was added HNCH₃OCH₃HCl salt (2.6 mmol) and stirring continued at RT for overnight. The reaction mixture was diluted with CH₂Cl₂ (20 mL) and sequentially washed with water, satd. NaHCO₃, brine and dried over MgSO₄. The solvent was removed under reduced pressure to yield a crude product, which was purified by column chromatography to obtain pure compound 2-(1*H*-indol-5-ylamino)-*N-*methoxy-*N*-methylthiazole-4-carboxamide (**55-5**) (45.6% yield for overall 5 steps). At -78 °C, to a solution of 5-bromo-1,2,3-trimethoxybenzene (1.235 g, 5.0 mmol) in 30 mL THF was charged n-BuLi in hexane (2.5 N, 2.4 mL, 6 mmol) under Ar₂ protection and stirred for 10 min Weinreb amide (1 mmol) in 10 mL THF was added to lithium reagent and allowed to stir at RT for 2 h. The reaction mixture was quenched with satd. NH₄Cl, extracted with ethyl ether, dried with MgSO₄. The solvent was removed under reduced pressure to yield a crude product, which was purified by column chromatography to obtain pure compound **55** (51.7% yield). ¹H NMR (300 MHz, CDCl₃) δ 8.29 (br, 1 H), 7.68 (d, 1 H), 7.46 (s, 2 H), 7.39 (s, 1 H), 7.36 (s, 1 H),7.28 ∼ 7.26 (m, 1 H), 7.15∼7.12 (m, 1 H), 6.55 (m, 1 H), 3.93 (s, 3 H), 3.89 (s, 6 H). MS (ESI) *m*/*z* 432.1 (M + Na)⁺, 408.0 (M-H)⁻.

### REFERENCE EXAMPLE 9

### SYNTHESIS OF OUINOLINE- AND ISOOUINOLINE-ARYL COMPOUNDS (FIGURE 16).

A series of compounds were prepared by Suzuki coupling of 7-bromo-1-chloroisoquinoline with various arylboronic acids.

### Synthesis of 1-Chloro-7-(1H-indol-5-yl)-isoquinoline (6d) (Figure 16C):

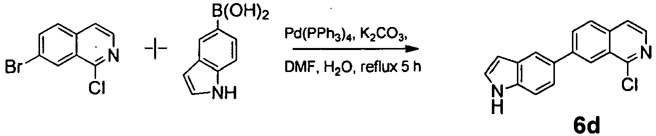

A mixture of 7-bromo-1-chloroisoquinoline (0.50 g, 2.1 mmol), 5-indoleboronic acid (0.40 g, 2.5 mmol), tetrakis(triphenylphosphene)palladium (0.035 g, 0.08 mmol), potassium carbonate (2.1 mL, 2 M, 4.1 mmol), *N,N*-dimethylformamide (11 mL) was stirred while purging the headspace with argon for 30 min. The mixture was then brought to reflux for 16 h before being allowed to cool to RT. The mixture was filtered through a bed of silica gel, diluted with water (50 mL), and extracted with ethyl acetate (50 mL). The organic layer was separated and washed with NaOH (2 x 20 mL, 10 % aq.), water (5 x 30 mL, until refractive changes were no longer seen at the organic-aqueous interface), and ammonium chloride (20 mL, sat.). The organic layer was then adsorbed onto silica gel and flash-chromatographed (ethyl acetate/hexanes) to afford 0.14 g (25 %) of a yellow solid. MS (ESI): calculated for C₁₇H₁₁ClN₂, 278.1, found 301.0 [M + Na]⁺. ¹H NMR (300 MHz, DMSO-*d₆*) δ 6.56 - 6.58 (m, 1 H), 7.44 (t, *J* = 2.77 Hz, 1 H), 7.57 - 7.59 (m, 2 H), 7.93(m, 1 H), 8.04 (s, 1 H), 8.13 - 8.20 (m, 1 H), 8.27 - 8.31 (m, 2 H), 8.43 (m, 1 H), 11.25 (brs, 1 H).

### 1,7-Bis-(1H-indol-5-yl)-isoquinoline (6b) (Figure 16E):

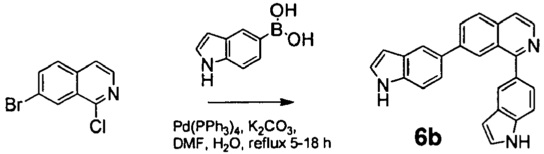

A mixture of 7-bromo-1-chloroisoquinoline (0.20 g, 2.1 mmol), 5-indoleboronic acid (0.80 g, 5.0 mmol), tetrakis(triphenylphosphene)palladium (0.19 g, 0.16 mmol), potassium carbonate (2.1 mL, 2 M, 4.1 mmol), *N*,*N*-dimethylformamide (11 mL) was stirred while purging the headspace with argon for 30 min. The mixture was then brought to reflux for 16 h before being allowed to cool to RT. The mixture was filtered through a bed of silica gel, diluted with water (50 mL), and extracted with ethyl acetate (50 mL). The organic layer was separated and washed with NaOH (2 x 20 mL, 10 % aq.), water (5 x 30 mL, until refractive changes were no longer seen at the organic-aqueous interface), and ammonium chloride (20 mL, sat.). The organic layer was then adsorbed onto silica gel and flash-chromatographed (ethyl acetate/hexanes) to afford 0.29 g (39 %) of a yellow solid. MS (ESI): calculated for C₂₅H₁₇N₃, 359.1, found 360.2 [M +H]⁺ 382.1 [M + Na]⁺, and 358.0 [M - H]⁻.¹H NMR (500 MHz, DMSO-*d₆*) δ 6.46 - 6.50 (m, 1 H) 6.52 - 6.59 (m, 1 H) 7.34 - 7.36 (m, 1 H) 7.36 - 7.41 (m, 2 H) 7.42 - 7.52 (m, 3 H) 7.58 (d, *J*=8.30 Hz, 1 H) 7.81 (dd, *J*=5.49, 5.00 Hz, 2 H) 7.92 (s, 1 H) 8.08 - 8.17 (m, 2 H) 8.33 (s, 1 H) 8.54 (d, *J*=5.61 Hz, 1 H) 11.18 (br. s., 1 H) 11.30(br. s., 1 H) ppm.

### 1-(4-Fluoro-phenyl)-7-(1H-indol-5-yl)-isoquinoline (6c) (Figure 16D):

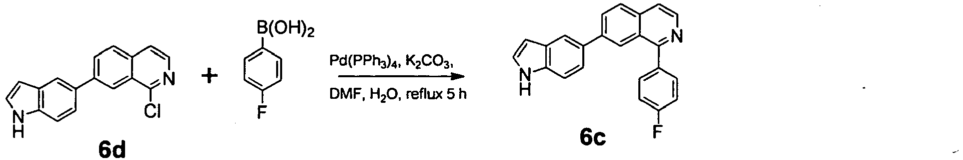

A mixture of **6d** (0.20 g, 0.72 mmol), 4-fluorophenylboronic acid (0.12 g, 0.86 mmol), tetrakis(triphenylphosphene)palladium (0.033 g, 0.03 mmol), potassium carbonate (0.72 mL, 2 M, 1.4 mmol), *N*,*N*-dimethylformamide (22 mL) was stirred while purging the headspace with argon for 30 min. The mixture was then brought to reflux for 16 h before being allowed to cool to RT. The mixture was filtered through a bed of silica gel, diluted with water (50 mL), and extracted with ethyl acetate (50 mL). The organic layer was separated and washed with NaOH (2 x 20 mL, 10 % aq.), water (5 x 30 mL, until refractive changes were no longer seen at the organic-aqueous interface), and ammonium chloride (20 mL, sat.). The organic layer was then adsorbed onto silica gel and flash-chromatographed (ethyl acetate/hexanes) to afford 0.038 g (16 %) of a yellow solid. MS (ESI): calculated for C₂₃H₁₅FN₂, 338.12, found 339.2 [M+H]⁺ and 361.2 [M + Na]⁺. ¹H NMR (300 MHz, DMSO-*d₆*) δ 6.47 - 6.55 (m, 1 H), 6.80 (d, *J* = 9.16 Hz, 2 H), 7.38 - 7.45 (m, 2 H), 7.47 - 7.62 (m, 3 H), 7.72 (d, *J* = 8.85 Hz, 2 H), 7.79 - 7.96 (m, 3 H), 11.18 (br. s., 1 H).

### 1,7-Bis-(4-fluoro-phenyl)-isoquinoline (40) (Figure 16A).

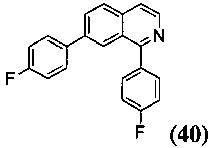

MS (ESI): calculated for C₂₁H₁₃F₂N, 317.10, found 318.1 [M + H]⁺, 340.1 [M + Na]⁺, and 315.9 [M - H]⁻. ¹H NMR (500 MHz, DMSO-*d₆*) δ 7.31 (br. s., 1 H) 7.31 - 7.37 (m, 2 H) 7.39 (br. s., 1 H) 7.41 (t, *J* = 8.54 Hz, 2 H) 7.72 - 7.77 (m, 2 H) 7.78 - 7.84 (m, 2 H) 7.89 (br. s., 1 H) 7.90 - 7.99 (m, 1 H) 8.09 - 8.19 (m, 3 H) 8.59 (br. s., 1 H) 8.60 - 8.65 (m, 1 H).

### Synthesis of 7-Bromo-1-(4-fluoro-benzenesulfonyl)-1,2,3,4-tetrahydroquinoline (43) and 1-(4-Fluoro-benzenesulfonyl)-7-(1H-indol-5-yl)-1,2,3,4-tetrahydroquinoline (41). (Figure 16B).

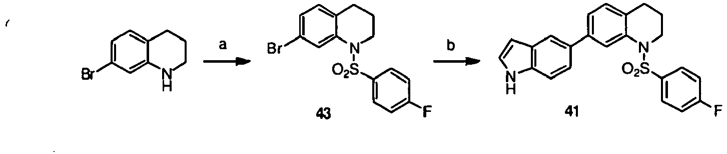

7-Bromo-1,2,3,4-tetrahydroquinoline (0.60 g, 2.8 mmol) was stirred with 4-fluorophenylsulphonyl chloride (1.65 g, 8.49 mmol) in pyridine (5 mL) at 80 °C for 3 h. The mixture was cooled, concentrated, and the residue was chromatagraphed (EtOAc/Hexanes on SiO₂) to give 845 mg of a brown solid (81 %) of compound **43**. C₁₅H₁₃BrFNO₂S 368.98, found 394.0 [M + Na]⁺ and 367.8 [M - H]⁻. ¹H NMR (500 MHz, CDCl₃) δ 1.58 - 1.67 (m, 2 H) 2.41 (t, *J* = 6.71 Hz, 2 H) 3.72 - 3.82 (m, 2 H) 6.89 (d, *J* = 8.30 Hz, 1 H) 7.08 - 7.17 (m, 2 H) 7.18 - 7.24 (m, 1 H) 7.59 - 7.68 (m, 2 H) 7.92 - 8.01 (m, 1 H).

**43** (0.46 g, 1.3 mmol), 5-indoleboronic acid (0.26 g, 1.6 mmol), tetrakis(triphenylphosphene)palladium (0.031 g, 0.03 mmol), potassium carbonate (1.35 mL, 2-M, 2.7 mmol), and *N*,*N*-dimethylformamide (135 mL) were stirred while purging the headspace with argon for 30 min. The mixture was then brought to reflux for 16 h before being allowed to cool to RT. The mixture was filtered through a bed of silica gel, diluted with water (50 mL), and extracted with ethyl acetate (50 mL). The organic layer was separated and washed with NaOH (2 × 20 mL, 10 % aq.), water (5 × 30 mL, until refractive changes were no longer seen at the organic-aqueous interface), and ammonium chloride (20 mL, sat.). The organic layer was then adsorbed onto silica gel and flash-chromatographed (ethyl acetate/hexanes) to afford 0.38 g (77 %) of a white crystalline solid of compound **41**. MS (ESI): calculated for C₂₃H₁₉FN₂O₂S, 406.12, found 404.9 [M - H]⁻ and 429.1 [M + Na]⁺. ¹H NMR (500 MHz, DMSO-*d₆*) δ 1.56 - 1.66 (m, 2 H) 2.48 (t, *J* = 6.59 Hz, 2 H) 3.76 - 3.86 (m, 2 H) 6.46 - 6.56 (m, 1 H) 7.14 (m, *J* = 7.81 Hz, 1 H) 7.33 - 7.37 (m, 1 H) 7.38 - 7.45 (m, 4 H) 7.49 (m, *J* = 8.54 Hz, 1 H) 7.66 - 7.74 (m, 2 H) 7.74 - 7.81 (m, 1 H) 7.85 - 7.94 (m, 1 H) 11.17 (br. s., 1 H).

### 7-Bromo-2-(4-fluoro-benzenesulfonyl)-1,2,3,4-tetrahydro-isoquinoline (42) (Figure 16B).

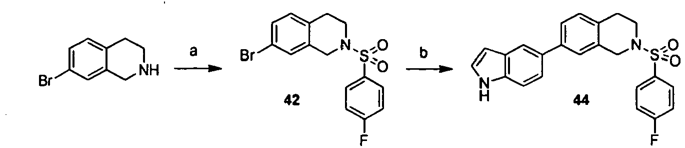

Yield 23 %. C₁₅H₁₃BrFNO₂S, 369.0, found 392.0 [M+Na]⁺ and 367.7 [M - H]⁻. ¹H NMR (500 MHz, DMSO-*d₆*) δ 2.75 - 2.82 (m, 2 H) 3.32 (t, *J* = 6.10 Hz, 2 H) 4.24 (s, 2 H) 7.07 (d, *J* = 8.30 Hz, 1 H) 7.29 - 7.37 (m, 1 H) 7.37 - 7.43 (m, 1 H) 7.47 (t, *J* = 8.79 Hz, 2 H) 7.87 . - 7.93 (m, 2 H).

### 2-(4-Fluoro-benzenesulfonyl)-7-(1H-indol-5-yl)-1,2,3,4-tetrahydroisoquinoline (44).

Yield 77 %. ¹H NMR (500 MHz, DMSO-*d₆*) δ 2.84 - 2.91 (m, 2 H) 3.35 (t, *J*=5.98 Hz, 2 H) 4.30 (s, 2 H) 6.44 - 6.48 (m, 1 H) 7.17 (d, *J* = 7.81 Hz, 1 H) 7.32 - 7.40 (m, 2 H) 7.41 - 7.51 (m, 3 H) 7.75 - 7.79 (m, 1 H) 7.89 - 7.96 (m, 1 H) 11.13 (br. s., 1 H).

### EXAMPLE 10

### WATER SOLUBILITY OF ARYL-BENZOYL-IMIDAZOLE (ABI) COMPOUNDS (Figure 17)

**Determination of water solubility.** To determine water solubility, 1 mg of each compound was suspended in 1 mL water and shaken for 48 h at room temperature (RT). The suspension was centrifuged at 10,000 rpm for 10 min and filtered on 0.22 µm filter. Concentrations of each compound were measured by LC-MS, consisting of an HP S1100 HPLC instrument (Agilent, Foster ceity, CA) and a Bruker ESQUIRE MS detector with electrospray/ion trap instrument in positive ion mode (Bruker, Fremont, CA). For HPLC, a reverse phase Nova-pak C18 column (150mm × 3.9 mm, Waters, Milford, MA) was used. The mobile phase was composed of 20:80 v/v water/acetonitrile. For MASS, the peak was extracted at 382 m/z (for imidazole compounds) and 399 m/z (for thiazole compounds) respectively. The concentration of each compound was calculated by MS peak area according to the following calibration equation: y=1.3295x + 114.24 (R²=1.00). To make the standard curve (**Figure 17**) from which the the equation was derived, 50, 100 µL of each 100 µg/mL, 10 µg/mL of ABI compound **12ga,** and its corresponding thiazole analog, as well as CA-4 (see **Figure 19** for structure) in acetonitrile, were injected into HPLC and monitored by mass spectroscopy. The amount (ng) in each injection was plotted again its relative mass peak area to generate the standard curve in **Figure 17**.

The HPLC retention times of ABI compound **12ga** (1.5 min) was compared to its corresponding thiazole analog (2.2 min) using 80/20 methanol/water mobile phase at 1 mL/min flow rate and a reversed phase column, indicating that the imidazole derivative was more hydrophilic than its corresponding thiazole analog. The calculated logP values for ABI compound **12ga** and the corresponding thiazole analog were approximately 2.9 and 4.4, respectively. The water solubility of compound **12ga** was 13 µg/mL, or about 200 times greater than its thiazole counterpart (72 ng/mL).

### EXAMPLE 11

### BIOLOGICAL EVALUATION OF COMPOUNDS OF THIS DISCLOSURE

### Example 11A: In Vitro Cell Growth Inhibitions.

**Cell Culture and Cytotoxicity Assay of Prostate Cancer and Melanoma.** All cell lines were obtained from ATCC (American Type Culture Collection, Manassas, VA, USA), while cell culture supplies were purchased from Cellgro Mediatech (Herndon, VA, USA). We examined the antiproliferative activity of our anti-tubulin compounds in four human prostate cancer cell lines (LNCaP, DU 145, PC-3, and PPC-1) and two human melanoma cell lines (A375 and WM-164). Human ovarian cell line OVCAR-8 and its resistant cell line that over-expresses P-gp (NCI/ADR-RES) were used as MDR models. Both ovarian cell lines were obtained from National Cancer Institutes (NCI). All cell lines were tested and authenticated by either ATCC or NCI. All prostate cancer and ovarian cancer cell lines were cultured in RPMI 1640, supplemented with 10% fetal bovine serum (FBS).

Melanoma cells were cultured in DMEM, supplemented with 5% FBS, 1% antibiotic/antimycotic mixture (Sigma-Aldrich, Inc., St. Louis, MO, USA) and bovine insulin (5 µg/mL; Sigma-Aldrich). The cytotoxic potential of the anti-tubulin compounds was evaluated using the sulforhodamine B (SRB) assay after 96 h of treatment.

All of the reported compounds were first evaluated for cytotoxicity in the mouse melanoma cell line B16-F1, human melanoma cell lines (A375 and WM-164) and prostate cancer cell lines (DU145, PC-3, LNCaP, PPC-1). Compound **1h** and ABT-751 (E7010, Abbott Laboratories/Eisai Co Ltd), which has entered phase II clinical studies in treating patients with different cancers, were included in the assays as examples of colchicine-site binding agents. IC₅₀ values for cell growth inhibition are shown in **Tables 1, 2** and 3. The only compounds which belong to the invention are the ones falling under the scope of formula (XI).

### Results:

**Table 1. SAR of B ring Optimizing Compounds**

| 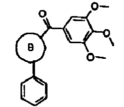 | B ring | IC₅₀ ± SEM (nM) | | | | | |
|---|---|---|---|---|---|---|---|
| | | B16-F1 | A375 | DU 145 | PC-3 | LNCaP | PPC-1 |
| **1a** | 1,3-phenyl | 500±200 | 87±15 | 178 | 81 | 234 | 85 |
| **1b** | 4,6-pyrimidine | >30000 | >30000 | 6900 | 8300 | 7000 | 3700 |
| **1c** | 2,6-pyridine | 39±12 | 30±14 | 33±3 | 32±2 | 27±2 | 25±1 |
| **1d** | 2,5-furan | 151±24 | 27±8 | 35 | 21 | 23 | 20 |
| **1e** | 2,5-thiazole | 12500±5200 | 13600±3800 | >10000 | >10000 | >10000 | >10000 |
| **1f** | 2,4-thiophene | 72±15 | 15±6 | 26 | 12 | 17 | 15 |
| **1g** | 1,4-piperidine | >30000 | >30000 | >20000 | >20000 | >20000 | >20000 |
| **1h** | 2,4-thiazole | 55±5 | 28±5 | 71±4 | 21±1 | 28±4 | 43±5 |
| **1i** | 3,5-isoxazole | >30000 | >30000 | >10000 | >10000 | >10000 | >10000 |
| **36a** | 2,4-oxazole | 600±200 | 300±100 | 292 | 294 | 310 | 324 |
| **35a** | 2,4-oxazoline | 6500±800 | 500±100 | 1200±100 | 1200±100 | 1200+100 | 1100±100 |

**Table 2. SAR of Carbonyl Linker Optimizing Compounds**

| 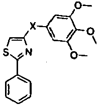 | | | | | | | | |
|---|---|---|---|---|---|---|---|---|
| | | IC₅₀ ± SEM (nM) | | | | | | |
| | X linker | | | | | | | |
| | | B16-F1 | A375 | WM-164 | DU 145 | PC-3 | LNCaP | PPC-1 |
| **1h** | C=O | 55±5 | 28±5 | 64±4 | 71±4 | 21±1 | 28±4 | 43±5 |
| **2a** | C=CMe₂ | 3800±1300 | 1900±800 | 3700±1200 | 2650 | 2470 | 1390 | 2040 |
| **2b** | CHOH | >30000 | >30000 | ND | >10000 | >10000 | >10000 | >10000 |
| **2 c-trans** | *syn*-C=C-CN | 5400±2100 | 4600±1500 | 4900±1300 | 2280 | 890 | 580 | 900 |
| **2c-cis** | *anti-*C=C-CN | 1200±300 | 1200±400 | 1000±200 | ∼10000 | ∼10000 | 1990 | ∼10000 |
| **2d-cis** | *syn-C*=N-NH₂ | 2000±800 | 900±300 | ND | 1210 | 1120 | 1800 | 872 |
| **2d-trans** | *Anti-C*=N-NH₂ | 1800±700 | 600±200 | ND | 1210 | 1040 | 1300 | 966 |
| **2 e-cis** | *syn*-*C*=N-OH | 300±100 | 200±100 | ND* | 102 | 120 | 189 | 160 |
| **2 e-trans** | *anti-C*=N-OH | 11400±2100 | 7800±1200 | ND | >10000 | >10000 | >10000 | >10000 |
| **2 f-cis** | *syn*-*C*=N-OMe | 3800±1600 | 2900±1200 | 3400±1800 | >10000 | >10000 | >10000 | >10000 |
| **2 f-trans** | *Anti-C*=N-OMe | >10000 | >10000 | >10000 | >10000 | >1 0000 | >10000 | >10000 |
| **2g** | CONH | >30000 | >30000 | ND | >10000 | >1 0000 | | >10000 |
| **2h** | NHCO | >30000 | >30000 | ND | >10000 | >10000 | >10000 | >10000 |
| **2i** | Bond (none) | >10000 | >10000 | >10000 | >10000 | >10000 | >10000 | >10000 |
| **2j** | C=N-CN | 60±21 | 28±12 | 27±13 | 42±2 | 27±1 | 23±2 | 20±1 |
| **3a** | *cis*-C=C | 11000±2800 | 46500±23300 | | >10000 | >10000 | >10000 | >10000 |
| **3b** | *trans-*C=C | 32800±13000 | >10000 | 30800±12000 | >10000 | >10000 | >10000 | >10000 |
| **4a** | S | 2400±900 | 11600±400 | 2000+1200 | >10000 | >10000 | 300±200 | 2300±100 |
| **4b** | SO₂ | >10000 | >10000 | >10000 | >10000 | >10000 | >10000 | >10000 |
| **4c** | SO | >10000 | >10000 | >10000 | >10000 | >10000 | >10000 | >10000 |
| **4d** | SO₂NH₂ | >10000 | > 10000 | >10000 | >10000 | > 10000 | >10000 | >10000 |

| | | | | | | | | |
|---|---|---|---|---|---|---|---|---|
| *ND = Not determined | | | | | | | | |

**Table 3. Antiproliferative Activity of Modified Compounds with Improved Aqueous Solubility**

| 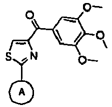 | A part | IC₅₀ ± SEM (nM) | | | | | |
|---|---|---|---|---|---|---|---|
| | | B16-F1 | A375 | DU 145 | PC-3 | LNCaP | PPC-1 |
| **58a** | 4-OTBDMSPh | 500±200 | 700±300 | 434±30 | 183±24 | 549 | 246±8 |
| **2l** | 4-OHPh | 110 | 100 | 116 | 87 | 103 | 76 |
| **62a** | 2-indolyl | 43±21 | 19±9 | 32 | 24 | 28 | 28 |
| **66a** | 5-indolyl | 25±13 | 8±1 | 13 | 7 | 10 | 8 |
| **68a** | 4-BocNHCH₂Ph | 2900±400 | 7900±500 | 4400 | 3100 | 2600 | 2700 |
| **2r** | 4-NH₂CH₂Ph | 38±11 | 41±13 | 25 | 80 | 13 | 34 |
| **2s** | 4-NHMeCH₂Ph | >10000 | >10000 | ∼10000 | >10000 | 114±80 | -1000 |
| **2u** | 4-NMe₂CH₂Ph | >10000 | >10000 | >10000 | >10000 | 1025±200 | >10000 |
| **5a** | PhNH | 65±12 | 45±8 | 70±4 | 57±3 | 51±1 | 54±1 |
| **5Hb** | 4-CH₃PhNH | ND* | ND | 35±1 | 38±2 | 35±1 | 36±1 |
| **5c** | 4-FPhNH | ND | ND | 63±1 | 43±1 | 41±1 | 37±1 |
| **1h** | Ph | 55±5 | 28±5 | 71±4 | 21±1 | 28±4 | 43±5 |
| **ABT-751** | | 2127±351 | 1111±108 | 839±719 | 786±89 | 658±117 | 701±307 |

| | | | | | | | |
|---|---|---|---|---|---|---|---|
| *ND = Not determined | | | | | | | |

**SAR of alternative "B" ring molecules.** The first series was targeted to alternatives to the thiazole "**B**" ring. Accordingly, a series of heterocyclic "B" rings were examined. As shown in **Table 1**, the successful replacements of the thiazole were pyridine **1c**, furan **1d** and thiophene **1f**. The IC₅₀s (12 nM ∼ 35 nM against prostate cancer cells) are close to the thiazole compound **1h**. Introducing phenyl (**1a**), oxazoline (**35a**), and oxazole (**36a**) maintained activity in the hundreds of nanomolar range. But introducing of pyrimidine (**1b**, IC₅₀: 3.7∼8.3 µM), a reversed 2,5-thiazole and 3,5-isoxazole (**1e** and **1i**, IC₅₀: > 10 µM) caused obvious losses of potency. Modification of "B" ring to the saturated ring of piperidine (**1g**) also totally abolished activity (IC₅₀ >20 µM).

**SAR of Alternative Linkers.** *In vitro* hepatic metabolic stability studies revealed that the carbonyl linker between "B" and "C" rings in SMART compounds caused short half lives (5-17 min) primarily due to carbonyl reduction. For the sake of blocking this ketone reduction to the inactive hydroxyl linker compound **2b**, the carbonyl linker in the second series of compounds was modified (**Table 2**). The carbonyl linker was replaced with double bonds (**2a, 3a** and **3b**), amides (**2g, 2h**), oximes (**2e-cis,trans and 2f-cis,trans**)**,** hydrazide (**2d-cis, 2d-trans**), acrylonitriles (**2c-trans, 2c-cis**), cyanoimine (**2j**), sulfonyl amide (**4d**), sulfur ether (**4a**), sulfonyl and sulfinyl compounds (**4b, 4c**). A direct link compound **2i** without any linker between "**B**" and "C" rings was also prepared. Among these linker modifications, only cyanoimine linkage (**2j**) showed promising potential (**20** ∼ 60 nM) compared with carbonyl compound **1h**, but an *in vitro* metabolism study showed that the half life of **2j** in human liver microsome was less than 5 min. This suggested that although the ketone reduction is blocked, it might introduce a new metabolic liability in compound **2j**. The isomer pairs of compounds containing double bonds, oximes and hydrazides were separated. Compound **3a** was designed to mimic the structure of CA-4, (**Figure 19**) which contain a *cis*-C=C between two aryl rings, unfortunately **3a** and other isomer pairs lost activity after replacing the C=O linker. One interesting phenomenon is *syn*-isomer of **2e-cis** (0.1 ~ 0.3 µM) showed 10 fold more activity than its *anti*-isomer **2e-trans** (>10 µM). The half life of 2e-cis in human liver microsome is extended to 35 min, while half lives of compounds **2d** can be prolonged to 55 min. But decreased activity (~1 µM) of **2d** also reduced their potency.

### Example 11B: Aqueous Solubility of compounds of the disclosure

The solubility of drugs was determined by Multiscreen Solubility Filter Plate (Millipore Corporate, Billerica, MA) coupled with LC-MS/MS. Briefly, 198 µL of phosphate buffered saline (PBS) buffer (pH 7.4) was loaded into 96-well plate, and 2 µL of 10 mM test compounds (in DMSO) was dispensed and mixed with gentle shaking (200-300 rpm) for 1.5 h at RT (N = 3). The plate was centrifuged at 800g for 5 min, and the filtrate was used to determine its concentration and solubility of test compound by LC-MS/MS as described below.

**Introducing polar and ionizable groups into the anti-tubulin agents.** One major limitation of the SMART agents is low aqueous solubility. Surfactants such as Tween 80, were used to study *in vivo* SMART behavior, accordingly favorable results were obtained. But these surfactants are biologically active and are responsible for many side effects. In addition, it was thought that low aqueous solubility of **1h** resulted in low oral bioavailability (3.3%, **Table 4**). In the third series of compounds, the aqueous solubility was successfully increased without impacting the potency by introducing polar groups like hydroxyl and indolyls. In addition, ionizable groups like amino and alkylamino groups were also introduced into "A" ring *para-*position. As shown in **Figure 5** and **Table 3**, introducing indolyl groups to the "A" ring especially 5-indolyl (**66a**, 7 ∼ 25 nM) increased the potency compared with the 4-OH compound **21** (76-116 nM). Aminomethyl -CH₂NH₂ at the "A" ring *para* position also maintained potency (**2r**, 13-80 nM), but *p*-NHMe (**2s**) or *p*-NMe₂ (**2u**) abrogated activity. As shown in **Figure 18**, analytical measurement to estimate aqueous solubility showed that indolyl compound **66a** increased solubility in PBS from 1.1 µg/mL (compound **1h**) to 3.8 µg/mL. Aminomethyl compound **2r** was converted to the HCl salt, which increased solubility over 35-fold (> 35µg/mL). Although compound **2r** showed satisfactory aqueous solubility, the pharmacokinetic studies showed this compound still had very poor bioavailability (F% = 0.2%). It was thought that compound **2r** was ionized in the stomach, and therefore not absorbed into the circulation system.

### Example 11C: Pharmacokinetic studies

**Pharmacokinetic Study.** Female Sprague-Dawley rats (n = 3 or 4; 254 ± 4 g) were purchased from Harlan Inc. (Indianapolis, IN). Rat thoracic jugular vein catheters were purchased from Braintree Scientific Inc. (Braintree, MA). On arrival at the animal facility, the animals were acclimated for 3 days in a temperature-controlled room (20-22 °C) with a 12 h light/dark cycle before any treatment. Compound **1h** was administered intravenously (i.v.) into the jugular vein catheters at a dose of 2.5 mg/kg (in DMSO/PEG300, 2/8), whereas **5Ha** and **5Hc** were dosed at 5 mg/kg (in DMSO/PEG300, 1/9). An equal volume of heparinized saline was injected to replace the removed blood, and blood samples (250 µL) were collected via the jugular vein catheters at 10, 20, 30 min, and 1, 2, 4, 8, 12, 24 h. Compounds **1h, 5Ha** and **5Hc** were given (p.o.) by oral gavage at 10 mg/kg (in Tween80/DMSO/H₂O, 2/1/7). All blood samples (250 µL) after oral administration were collected via the jugular vein catheters at 30, 60, 90 min, 120 min, 150 min, 180 min, 210 min, 240 min, and 8, 12, 24 h. Heparinized syringes and vials were prepared prior to blood collection. Plasma samples were prepared by centrifuging the blood samples at 8,000 g for 5 min. All plasma samples were stored immediately at -80 °C until analyzed.

Analytes were extracted from 100 µL of plasma with 200 µL of acetonitrile containing 200 nM the internal standard ((3,5-dimethoxyphenyl)(2-phenyl-1*H*-imidazol-4-yl)methanone). The samples were thoroughly mixed, centrifuged, and the organic extract was transferred to autosampler for LC-MS/MS analysis. Multiple reaction monitoring (MRM) mode, scanning *m*/*z* 356 → 188 (compound **1h**), *m*/*z* 371 → 203 (compound **5Ha**), *m*/*z* 389 → 221 (compound **5Hc**), and m/z 309 → 171 (the internal standard), was used to obtain the most sensitive signals. The pharmacokinetic parameters were determined using non-compartmental analysis (WinNonlin, Pharsight Corporation, Mountain View, CA)

### Results:

**Table 4. Pharmacokinetic Parameters for Compounds Tested in vivo.**

| | **1h** | | **2r** | | **5Ha** | | **5Hc** | |
|---|---|---|---|---|---|---|---|---|
| Route | IV | PO | IV | PO | IV | PO | IV | PO |
| N^{a} | 4 | 3 | 3 | 3 | 3 | 3 | 3 | 3 |
| Dose(mg/kg) | 2.5 | 10 | 2.5 | 4 | 5 | 10 | 5 | 10 |
| CL^{b}(mL/min/kg) | 7.7 ± 1.0 | - | 22 ± 13 | - | 17 ± 3 | - | 13 ± 2 | - |
| Vss^{c}(L/kg) | 4.9±1.9 | - | 0.33± 025 | - | 1.4 ± 0.2 | - | 1.4± 0.2 | - |
| AUC^{d}(min*mg/mL) | 279 ± 53 | 37 ± 20 | 139± 77 | 0.4 | 296 ± 46 | 65 ± 20 | 381 ± 65 | 160 ± 13 |
| Cₘₐₓ^{c}(ng/mL) | 3816±509 | 212 | 3.2 ± 1.6 | 3794±1580 | 4198±438 | 814±255 | 3349±686 | 1262 ± 362 |
| F^{f}(%) | 3.3 | | 0.2 | | 11 | | 21 | |

| | | | | | | | | |
|---|---|---|---|---|---|---|---|---|
| ^{a}Numbers of rats. ^{b}Systemic clearance. ^{c}Volume of distribution following intravenous dosing. ^{d} Area under the curve following intravenous dosing, integrated drug concentration with respect to time and integrated drug concentration with respect to time following oral dosing. ^{e}Maximum plasma concentration following intravenous dosing. ^{f}Percent oral bioavailability. | | | | | | | | |

**Modifying Substituted Methoxybenzoyl Aryl Thiazole (SMART) Molecules to Improve Oral Bioavailability.** Many established tubulin targeting anticancer drugs like taxanes and vinblastine require intravenous administration because of low oral bioavailability. Oral bioavailability is a complex parameter involving many chemical and physiological processes, such as solubility, permeability, and metabolic stability. The solubility of these tubulin inhibitors was improved by inserting an amino linker between the "A" and "B" rings as in **5a-d** (**Figure 6**), **Table 3** demonstrates that the NH bridged compounds (**5a-c**) had similar potency (35∼65 nM) as **1h** with increased solubility (15 and 19 µg/mL for **5a** and **5c**, respectively (**Figure 18**), but they are over 20 fold more active than ABT-751 (**Table 3** and **Figure 19** for the structure of ABT-751).

Rat pharmacokinetic studies were performed to study whether these new compounds exhibited improved bioavailability compared to compound **1h** (**Table 4**). The data clearly showed that **5Hc** (HCl salt of **5c**) exhibited more than 4.3-fold increased exposure (AUC) by the oral route as compared to **1h**, suggesting that improved aqueous solubility by the amino linker successfully improved oral bioavailability. In addition, the maximal concentration (Cmax) of **5Ha** and **5Hc** by oral administration was 814 and 1262 ng/mL, respectively. While Cmax of **1h** was only 212 ng/mL. Overall, the bioavailability of **5Ha** and **5Hc** was increased from 3.3% of **1h** to 11% and 21%, respectively (**Table 4**). Compound **5Hc** exhibited moderate clearance, moderate volume of distribution, and acceptable oral bioavailability. This data suggested that these new synthesized amino linked compounds have the potency and PK profile to be developed as a new class of orally bioavailable antitubulin agents.

### Example 11D: in vitro Tubulin Polymerization inhibition by compounds of the disclosure

***In Vitro* Tubulin Polymerization Assay.** Bovine brain tubulin (0.4 mg, >97% pure) (Cytoskeleton, Denver, CO) was mixed with 10 µM of the test compounds and incubated in 100 µl of general tubulin buffer (80 mM PIPES, 2.0 mM MgCl₂, 0.5 mM EGTA, and 1 mM GTP) at pH 6.9. The absorbance of wavelength at 340 nm was monitored every 1 min for 20 min by the SYNERGY 4 Microplate Reader (Bio-Tek Instruments, Winooski, VT). The spectrophotometer was set at 37 °C for tubulin polymerization.

### Results:

The inhibition of tubulin polymerization by selected potent compounds **1c**, **2j**, **66a**, and **5a** was investigated by all three design strategies (alternative B-rings, novel linkers, and solubilizing moieties) and compared with **1h**. Bovine brain tubulin (> 97% pure) was incubated with the individual compounds (10 µM) to test their effect on tubulin polymerization (**Figure 20a**). After 20 min incubation, tubulin polymerization was inhibited 47% by **1h**, as compared to vehicle. Both **1c** and **2j** inhibited 64% of polymerization at 20 min with different inhibition patterns. Compounds **5a** and **66a** provided greater inhibitions as 78% and 81%, respectively. These data suggest that these compounds exhibit strong antitubulin polymerization activity that corresponds well with their cytotoxicity.

The inhibition of tubulin polymerization by compound 5c by binding with colchicines binding site and compared with compound 1h is demonstrated in Figures 20b and 20c.

### Example 11E: Novel Anti-tubulin Compounds Overcome P-Glycoprotein Mediated Multidrug Resistance.

The P-glycoprotein (P-gp) system appears to be a primary physiological mechanism of multidrug resistance (MDR) which acts as an ATP-dependent drug efflux pump, actively removing a variety of structurally diverse cytotoxic compounds. Enhanced efflux of these compounds reduces their intracellular accumulation and so reduces their cytotoxicity. Therefore, novel compounds which are not susceptible to drug resistance could be of high therapeutic and economic value. In addition to P-gp, clinically used antitubulin agents have other resistance mechanisms such as changes in microtubule dynamics and mutations in β-tubulin which are known to limit sensitivity to the taxanes. The anti-tubulin compounds of the invention were tested against an ovarian cancer cell line OVCAR-8 (parent) and P-gp over-expressing NCI/ADR-RES cell line (**Tables 5A, 5B**).

### Results:

**Table 5A. Antiproliferative Activity of Selected Compounds against P-gp over-expressed MDR cell lines.**

| Compound | IC₅₀ (nM) | | Resistance factor |
|---|---|---|---|
| | OVCAR-8 | NCI/ADR-RES | |
| **1c** | 33±3 | 13±0.8 | 0.4 |
| **2j** | 34±2 | 14±1 | 0.4 |
| **66a** | 10±3 | 4±2 | 0.4 |
| **2r** | 26±2 | 11±2 | 0.4 |
| **5a** | 46 ±6 | 27 | 0.6 |
| **5b** | 28 | 21 | 0.8 |
| **5c** | 44±3 | 25±6 | 0.6 |
| **1h** | 35±2 | 13±1 | 0.4 |
| paclitaxel* | 4.7±0.1 | 6263±634 | 1333 |
| vinblastine | 3.9±0.1 | 582±57 | 149 |
| colchicine | 17±1 | 1113±79 | 65 |

Notably, the anti-tubulin compounds of the invention demonstrated equipotent antiproliferative effects against OVCAR-8 and NCI/ADR-RES cell lines, suggesting that they are not P-gp substrates and that they function in a P-gp-independent manner. This feature is distinct from that of paclitaxel, vinblastine, and colchicine in NCI/ADR-RES cells.

**Table 5B. Antiproliferative activity of selected phenyl-amino thiazole compounds**

| | R | IC₅₀ ± SEM (nM) | | | | | |
|---|---|---|---|---|---|---|---|
| | | B16-F1 | A375 | DU 145 | PC-3 | LNCaP | PPC-1 |
| **5a** | H | 65±12 | 45±8 | 70±4 | 57±3 | 51±1 | 54±1 |
| **5Hb** | 4-CH₃ | ND* | ND | 35±1 | 38±2 | 35±1 | 36±1 |
| **5c** | 4-F | ND | ND | 63±1 | 43±1 | 41±1 | 37±1 |
| **5d** | 4-Cl | ND | 25 ± 7 | 73±1 | 33±1 | 45±1 | 36±1 |
| **1h** | - | 55 ± 5 | 28 ± 5 | 71 ± 4 | 21 ± 1 | 28 ± 4 | 43 ± 5 |
| **ABT-751** | - | 2127±351 | 1111±108 | 839±719 | 786±89 | 658±117 | 701±307 |

The phenyl amino thiazole compounds **5a, 5Hb, 5c** and **5d** demonstrated potent activity in a number of prostate cancer cell lines. Unexpectedly, the phenyl amino imidazole compound **5e** demonstrated no activity (IC₅₀ > 1000 nM in LNCaP, PC-3, DU-145, and PPC-1) in these prostate cancer cell lines. The positive controls for this experiment were **55** and **17ya** which demonstrated IC₅₀ values between 7.5 nM and 24.1 nM in the same cell lines (**Table 5C**).

**Table 5C**

| **Table 5C.** | **IC₅₀ ± SEM (nM)** | | | | | |
|---|---|---|---|---|---|---|
| | **B16-F1** | **A375** | **DU 145** | **PC-3** | **LNCaP** | **PPC-1** |
| **5a** | 65±12 | 45±8 | 70±4 | 57±3 | 51±1 | 54±1 |
| **5Hb** | ND | ND | 35±1 | 38±2 | 35± 1 | 36±1 |
| **5c** | ND | ND | 63±1 | 43±1 | 41±1 | 37±1 |
| **5d** | ND | 25±7 | 73±1 | 33±1 | 45±1 | 36±1 |
| **1h** | 55±5 | 28±5 | 71±4 | 21±1 | 28±4 | 43±5 |
| **ABT-751** | 2127±351 | 1111±108 | 839±719 | 786±89 | 658±117 | 701±307 |
| **d** | ND | ND | >1000 | >1000 | >1000 | >1000 |
| **e** | ND | ND | >1000 | >1000 | >1000 | >1000 |
| **5e** | ND | ND | >1000 | >1000 | >1000 | >1000 |
| **55** | ND | ND | 24±6 | 12±1 | 13±1 | 15±1 |
| **17ya** | ND | ND | 11±1 | 5±2 | 8±2 | 8±1 |

A new series of tubulin polymerization inhibitors with acceptable oral bioavailability and equi-potent activity in multidrug resistant tumor cell lines has been discovered. Medicinal chemistry efforts starting from optimizing SMART reference compound **1h**. Chemical modifications of different substituted aryl in "B" ring and linkages between "B" and "C" rings were investigated based on biological evaluation against cancer cells *in vitro.* SAR studies revealed that optimal "B" rings include .pyridine (**1c**), thiophene (1**f**), and furan (1**d**) which maintain excellent *in vitro* potency. Replacing carbonyl linker with cyanoimine (**2j**) between "B" and "C" ring will increase the activity. Structure modifications to increase aqueous solubility and bioavailability were performed. Introducing an amino between "A" and "B" rings gave us the compounds of the invention **5a-c**, which showed similar *in vitro* antiproliferative potency against tested cancer cells as well as MDR(+) and MDR(-) cell lines, furthermore, the solubility and *in vivo* bioavailability were improved greatly over those of the **1h**. Therefore, these new anti-tubulin compounds represent a new family of compounds that may be very useful in the treatment of cancer.

### EXAMPLE 12

### ANTIPROLIFERATIVE ACTIVITY

The antiproliferative activity of analogs prepared by the methods of the invention are shown in Tables 6 and 6A.

**Table 6**

| IC₅₀ ±SEM (nM) | | | | | | | | | | | |
|---|---|---|---|---|---|---|---|---|---|---|---|
| ID | **LNCaP** | **PC-3** | **DU 145** | **PPC-1** | **A375** | **B16-F1** | **WM164** | **MES- SA** | **MES- SA/Dx5** | **OVCAR -8** | **NCI/AD R-RES** |
| Paclitaxel | 1.7 | 4.8 | 5.1 | 2.3 | 12 | 17 | | 2.7 | 6.6 | 4.7 | 6263 |
| Vinblastine | 1.1 | 2.1 | 1.8 | 1.1 | 1 | 4.7 | | 1.4 | 16 | 3.9 | 582 |
| Colchicine | 16 | 11 | 10 | 20 | 20 | 29 | | 8.4 | 22 | 17 | 1113 |
| **1k** | 101 | 101 | 140 | 84 | 100 | 245 | 220 | | | | |
| **2k** | 6 | 13 | 12 | 8 | 33 | 43 | | 11 | 19 | 34 | 12 |
| **2m** | 19 | 8.7 | 6.9 | 6.2 | 11 | 21 | | | | | |
| **2n** | 101 | 131 | 143 | 99 | 210 | 290 | | | | | |
| **20** | 65 | 73 | 121 | 73 | 38 | 42 | | | | | |
| **2p** | >10000 | 2385 | 1899 | 1079 | 2200 | 16560 | | | | | |
| **2q** | >10000 | >10000 | >10000 | >10000 | >20000 | >20000 | | | | | |
| **5c-HCl** | 53 | 53 | 70 | 43 | | | | | | | |
| **6d** | 703 | 908 | 1637 | 929 | | | | | | | |

| | | | | | | | | | | | |
|---|---|---|---|---|---|---|---|---|---|---|---|
| *ND: not determined | | | | | | | | | | | |

**Table 6A**

| IC₅₀ (nM) | | | | | | | | | | | | |
|---|---|---|---|---|---|---|---|---|---|---|---|---|
| **Structure** | **ID** | **LNCaP** | **PC-3** | **DU 145** | **PPC-1** | **A375** | **B16-F1** | **WM164** | **MES- SA** | **MES- SA/Dx5** | **OVCAR -8** | **NCI/AD R-RES** |
| 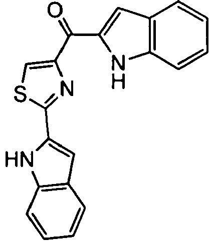 | **8** | 346 | 704 | 580 | 230 | 318 | 570 | 404 | | | | |
| 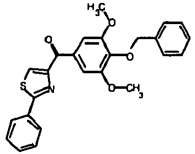 | **9** | -10000 | -10000 | ∼10000 | ∼10000 | | | | | | | |
| 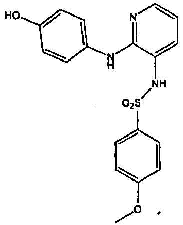 | **10** | 658 | 786 | 839 | 701 | 1111 | 2127 | 661 | | | | |

| **Structure** | **ID** | **LNCaP** | **PC-3** | **DU 145** | **PPC-1** | **A375** | **B16-F1** | **WM164** | **MES- SA** | **MES- SA/Dx5** | **OVCAR -8** | **NCI/AD R-RES** |
|---|---|---|---|---|---|---|---|---|---|---|---|---|
| 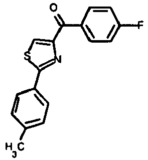 | **11** | >10000 | >10000 | -10000 | -10000 | 3470 | 4900 | 4700 | | | | |
| 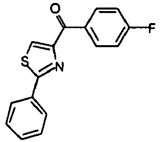 | **12** | >10000 | >10000 | >10000 | >10000 | >10000 | >10000 | | | | | |
| 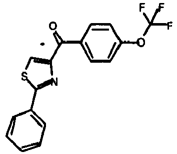 | **13** | >10000 | >10000 | >10000 | >10000 | >10000 | >10000 | >10000 | | | | |
| 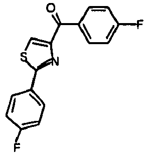 | **14** | >10000 | >10000 | >10000 | >10000 | >10000 | >10000 | | | | | |
| 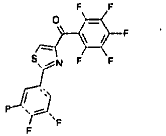 | **16** | >10000 | >10000 | >10000 | >10000 | 15200 | 6900 | | | | | |
| 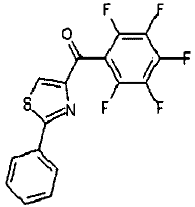 | **17** | 2100 | 1900 | 2600 | 1300 | 4300 | 9800 | | | | | |
| 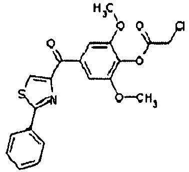 | **18** | ∼10000 | ∼10000 | ∼10000 | ∼10000 | | | | | | | |
| 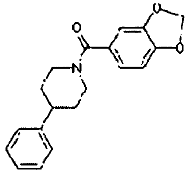 | **19** | >20000 | >20000 | >20000 | >20000 | >20000 | >20000 | | | | | |
| 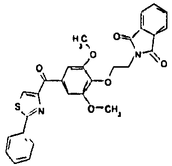 | **20** | 1452 | >10000 | 642 | 633 | 2300 | 3100 | 1300 | | | | |
| 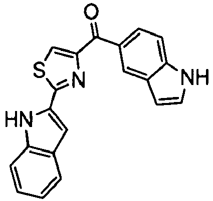 | **21** | 314 | 403 | 435 | 216 | 383 | 924 | 408 | | | | |
| 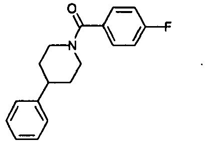 | **22** | >20000 | >20000 | >20000 | >20000 | >20000 | >20000 | | | | | |
| 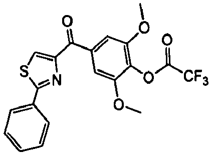 | **23** | ∼10000 | ∼10000 | ∼10000 | ∼10000 | | | | | | | |
| 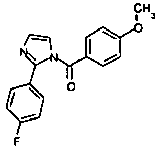 | **24** | >10000 | >10000 | >10000 | >10000 | >10000 | >10000 | >10000 | | | | |
| 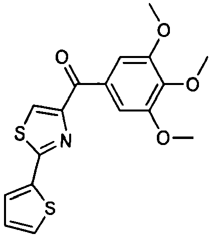 | **25** | 48 | 44 | 24 | 13 | 20 | 38 | | | | | |
| 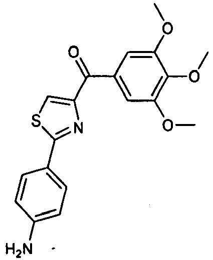 | **26** | 23 | 16 | 16 | 15 | 11 | 14 | | | | | |
| 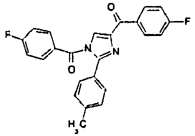 | **29** | 1788 | >10000 | >10000 | >10000 | >10000 | >10000 | | | | | |
| 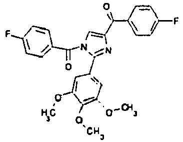 | **30** | >10000 | >10000 | >10000 | >10000 | >10000 | >10000 | | | | | |
| 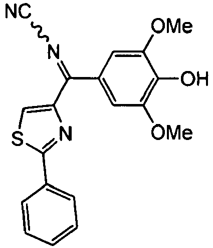 | **32** | 1664 | 2291 | 4601 | 1170 | 2700 | >10000 | 2600 | | | | |
| 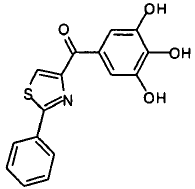 | **33** | >2000 | >2000 | >2000 | >2000 | 9800 | >20000 | | | | | |
| 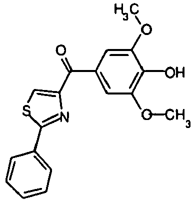 | **34** | >10000 | >10000 | >10000 | >10000 | >10000 | >10000 | >10000 | | | | |
| 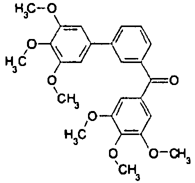 | **35** | 1500 | 40100 | 21900 | 15000 | | | | | | | |
| 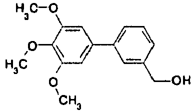 | **39** | 4300 | 32500 | 16800 | 21400 | | | | | | | |
| 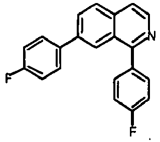 | **40** | 13400 | 19600 | 18400 | 6200 | | | | | | | |
| 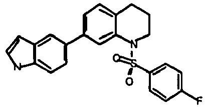 | **41** | 15750 | 18170 | 17040 | >20000 | | | | | | | |
| 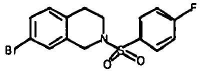 | **42** | 43590 | 23790 | 24880 | >20000 | | | | | | | |
| | **43** | 12690 | 14720 | 17210 | >20000 | | | | | | | |
| 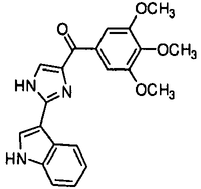 | **17ya** | 12 | 10 | 17 | 21 | 17.35 | 32.94 | 12.08 | | | | |
| 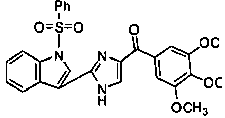 | **17yaa** | 233.7 | 148.3 | 592.1 | 208.9 | 481.2 | 538.7 | 467.6 | | | | |
| 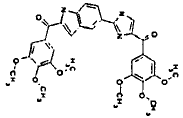 | **15xaa** | 1068 | 2628 | 5917 | 4575 | 1800 | 1390 | 1700 | | | | |
| 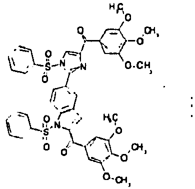 | **16xaa** | >10000 | >10000 | >10000 | >10000 | >10000 | >10000 | >10000 | | | | |

### EXAMPLE 13

### BIOLOGICAL EVALUATION OF ISOQUINOLINE DERIVATIVES

### Cell culture.

LNCaP, PC-3, DU-145, PPC-1, MES-SA, and MES-SA/DX5 were originally obtained from ATCC (Rockville, MD). All cells obtained from ATCC were immediately expanded and frozen down such that all cell lines could be restarted every 2-3 months from a frozen vial of the same batch of cells. For the *in vivo* xenograft studies, PC-3 was authenticated at Research Animal Diagnostic Laboratory (Columbia, MO) within four months before studies. Inter-species contamination was tested by PCR and the identity of the cell lines was verified by generating a genetic profile. MES-SA and MES-SA/DX5 were maintained in McCoy's 5A Medium containing 2 mM L-glutamine supplemented with 10% fetal bovine serum (FBS). All other cells were maintained in RPMI-1640 medium with 2 mM L-glutamine and 10% FBS.

### Growth Inhibition Assay.

The cytotoxic or antiproliferative activity of test compounds was investigated in several cell lines using the sulforhodamine B (SRB) assay. Cultured cells were plated into 96-well plates and incubated with medium containing different concentrations of the test compounds for 96 h. Cells were stained with SRB solution. The optical density was determined at 540 nm on a microplate reader (Dynex Technologies, Chantilly, VA). Plots of percent inhibition of cell growth versus drug concentration were constructed, and the concentration that inhibited cell growth by 50% relative to the untreated control (IC₅₀) was determined by nonlinear least squares regression using WinNonlin software (Pharsight Corporation, Cary, NC).

### Cell Cycle Analysis.

Cell cycle distribution was determined by propidium iodide (PI) staining. Treated cells were washed with PBS and fixed with 70% ice-cold ethanol overnight. Fixed cells were then stained with 20 µg/mL of PI in the presence of RNase A (300 µg/mL) at 37°C for 30 min. Cell cycle distribution was analyzed by fluorescence-activated cell sorting (FACS) analysis core services at the University of Tennessee Health Science Center, TN.

### In Vitro Metabolism Studies.

For both phase I, the incubation mixture, in 65 mM potassium phosphate buffer (pH 7.4), consisted of 1 mg/mL liver microsomal proteins, 3 mM NADPH, and 0.5 µM test compound. The concentration of methanol (used for dissolving the substrate) was 1 % (v/v). Total volume of the incubation was 200 µL and the reaction mixtures were incubated at 37 °C. To generate the stability curves for test compounds different incubations were stopped at 10, 20, 30, 60, and 90 minutes for analysis of compounds remaining. All reactions were stopped by the addition of 200 µL ice-cold acetonitrile. Subsequently, the samples were then centrifuged at 3000 g for 5 min and supernatant was analyzed by LC-MS/MS.

### Pharmacokinetic Studies in Mice.

Male ICR mice (5-6 weeks, 20-25 g) were used. For **6a**, **6b**, and **6c** a dose of 5mg/kg was administered *via* the i.v., i.p., and p.o. route. I.v. doses were administered via the tail vein. Oral doses were administered by gavage. At each time point, three to four mice were euthanized by isoflurane (Baxter Healthcare, Deerfield, IL) and blood samples (up to 600 µL each) were taken from the posterior vena cava. Plasma samples were stored at -20 °C prior to analysis. Plasma proteins were precipitated by the addition of acetonitrile (150 µL, containing the internal standard) to 100 µL of mouse plasma. Samples were vortexed and then centrifuged at 8000g for 10 min. The supernatant was transferred to a clean vial for injection into the mass spectrometer for analysis.

### In Vivo Antitumor Efficacy Study.

PC-3 cells (2.5×10⁶ cells/site) plus Matrigel (BD biosciences, San Jose, CA) were injected subcutaneously into flanks of male nu/nu mice. Tumor size was measured using calipers every 2-4 days and calculated as V = π / 6 × (length) × (width)². When tumors reached a volume of approximately 100-150 mm³, drug treatment was initiated. The control group was treated with vehicle (20% Captex200 in Tween80). During the treatment, tumor size and body weights were measured every 2-4 days.

### White Blood Cell Counting.

Whole blood was obtained from nude mice at the end of efficacy study. To count white blood cells (WBC) using a hemacytometer, 10 µL of whole blood sample was diluted with the 190 µL of 2% acetic acid. With proper light adjustment, the leukocytes appeared as dark dots on the hemacytometer. WBC in each sample was counted twice within one hours following dilution and average was calculated.

### Results

**Table 7. Anticancer efficacy of isoquinoline compounds in different cancer cell lines and MDR cell lines mediated by P-glycoprotein**

| | IC₅₀ (nM) | | | | |
|---|---|---|---|---|---|
| | **6a** | **6b** | **6c** | Vinblastine | Docetaxel |
| LNCaP | 80.6 ± 17.1 | 98.1 ± 17.9 | 38.3 ± 9.7 | 3.4 ± 0.9 | 4.7 ± 1.3 |
| PC-3 | 64.4 ± 12.2 | 71.8 ± 9.1 | 25.6 ± 8.3 | 1.4 ± 0.3 | 6.3 ± 0.4 |
| DU-145 | 91.7 ± 10.2 | 113.4 ± 21.4 | 46.6 ± 13.8 | 2.6 ± 1.0 | 5.2 ± 1.0 |
| PPC-1 | 60.6 ± 3.4 | 47.9 ± 10.0 | 27.7 ± 4.5 | 1.1 ± 0.4 | 2.7 ± 1.0 |
| P-gp | | | | | |
| MES-SA | 78.2 ± 1.8 | 129.8 ± 38.0 | 35.6 ± 2.8 | 2.3 ± 0.8 | 5.9 ± 1.1 |
| MES-SA/DX5 | 119.4 ±0.4 | 177.8 ± 32.8 | 59.2 ± 0.1 | 45.7 ± 5.3 | 76.4 ± 8.7 |
| Resistance factor | 1.5 | 1.4 | 1.7 | 20 | 13 |
| NOTE: P-gp is over-expressed in MES-SA/DX5. The resistance factor (RF) was calculated as the ratio of IC₅₀ values for the resistant cell subline to that of the parental cell line. All experiments were performed at least in three replicates. | | | | | |

**Table 8. Compound 6a, 6b, and 6c arrested PC-3 cells in G₂M phase.**

| | G₂M phase arrest EC₅₀ (nM) |
|---|---|
| **6a** | 53.4 |
| **6b** | 91.9 |
| **6c** | 23.3 |

**Table 9. Summary of half lives (phase I pathway) of 6a, 6b, and 6c in mouse, rat, hamster, rabbit, guinea pig, dog, monkey, and human liver microsomes.**

| | T ½ (min) | | |
|---|---|---|---|
| | **6a** | **6b** | **6c** |
| Mouse | 3.4 | 10 | 13. |
| Rat | 12 | 9 | 14 |
| Hamster | 6 | 11 | 20 |
| Rabbit | 17 | 16 | 16 |
| Guinea pig | 15 | 15 | 8 |
| Dog | 13 | 30 | 29 |
| Monkey | 16 | 13 | 9 |
| Human | 32 | 40 | 47 |

**Table 10. Summary of pharmacokinetic properties of compound 6a, 6b, and 6c in mice.**

| | | **6a** | **6b** | **6c** |
|---|---|---|---|---|
| | | 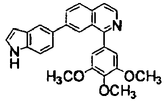 | 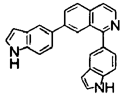 | 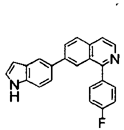 |
| MW | | 410.5 | 359.4 | 338.4 |
| IV CL (mL*min⁻¹kg⁻¹) | 5mg/kg | 51 | 14 | 30 |
| IV V_{d} (L*kg⁻¹) | 5mg/kg | 2.3 | 1.1 | 1.8 |
| IP Cₘₐₓ (ng/mL) | 5mg/kg | 678.4 | 1500 | 1100 |
| IP AUC (min*µg/mL) | 5mg/kg | 59 | 218 | 55 |
| IP Bioavailability | Fᵢₚ% | 60 | 60 | 33 |
| PO Cₘₐₓ (ng/mL) | 5mg/kg | 6.7 | 50 | 50 |
| AUC (min*µg/mL) | 5mg/kg | 5 | 7 | 4 |
| PO Bioavailability | Fₚₒ% | 5 | 2.1 | 2.7 |

Efficacy and tolerability of **6b** and **6c** was measured in xenograft models after i.p. injection (**Figure 34**). PC-3 xenografts were treated with vehicle (qd), **6b** (40 mg/kg, qd), or **6c** (40 mg/kg, qd) for 3 weeks. Dosing vehicles were composed of 20% Captex200 in Tween80. The tumor volumes (mm³) were plotted against time and are the means ± SD from eight animals. The tumor volumes and survival rates or body weights are shown in **Figure 34A**. The liver size (g) of each nude mouse was measured after 3 weeks treatment and is shown in **Figure 34B**. The number of white blood cells was counted in whole blood collected from animal after 3 weeks treatment and is shown in **Figure 34C**.

### EXAMPLE 14

### ANTIPROLIFERATIVE ACTIVITY OF SELECTED ABI COMPOUNDS

### Cell Culture Cytotoxicity Assay

### Materials and Methods

The antiproliferative activity of the ABI compounds in three melanoma cell lines (A375 and WM-164, human melanoma cell line; B16-F1, mouse melanoma cell line) and four human prostate cancer cell lines (LNCaP, DU 145, PC-3, and PPC-1) were studied. All these cell lines were purchased from ATCC (American Type Culture Collection, Manassas, VA) except the PPC-1 cell line. MDA-MB-435 and MDA-MB-435/LCCMDR1 cells were kindly provided by Dr. Robert Clarke at Georgetown University School of Medicine, Washington, DC. Melanoma cells were cultured in DMEM (Cellgro Mediatech, Inc., Herndon, VA) and prostate cancer cells were cultured in RPMI 1640 (Cellgro Mediatech, Inc., Herndon, VA) supplemented with 10% FBS (Cellgro Mediatech). Cultures were maintained at 37°C in a humidified atmosphere containing 5% CO₂. 1000 to 5000 cells were plated into each well of 96-well plates depending on growth rate and exposed to different concentrations of a test compound for 48 h (fast growing melanoma cells) or 96 h (slow growing prostate cancer cells) in three to five replicates. Cell numbers at the end of the drug treatment were measured by the sulforhodamine B (SRB) assay. Briefly, the cells were fixed with 10% trichloroacetic acid and stained with 0.4% SRB, and the absorbances at 540 nm were measured using a plate reader (DYNEX Technologies, Chantilly, VA). Percentages of cell survival versus drug concentrations were plotted, and the IC₅₀ (concentration that inhibited cell growth by 50% of untreated control) values were obtained by nonlinear regression analysis using GraphPad Prism (GraphPad Software, San Diego, CA).

### Results

The results of the *in vitro* antiproliferative activities of the compounds using three melanoma cell lines (one murine melanoma cell line, B16-F1, and two human metastatic melanoma cell lines, A375 and WM-164) and four human prostate cancer cell lines (LNCaP, PC-3, Du 145, and PPC-1) are summarized in **Tables 11-13**.

**Table 11. In vitro growth inhibitory effects of compounds without A ring substitutions.**

| ***Structure*** | **ID** | **R** | **IC₅₀ (nM)** | | | | | | |
|---|---|---|---|---|---|---|---|---|---|
| | | | **A375** | **B16-F1** | **WM164** | *LNCaP* | *PC-3* | *Du 145* | *PPC-1* |
| 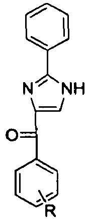 | **12aa** | 3,4,5-(OMe)₃ | 160 | 120 | 10 | 152 | 288 | 196 | 133 |
| | **12ab** | 4-OMe | >10000 | >10000 | >10000 | >10000 | >10000 | >10000 | >10000 |
| | **12ac** | 3-OMe | >10000 | >10000 | >10000 | >10000 | >10000 | >10000 | >10000 |
| | **12ad** | 3,5-(OMe)₂ | 2800 | 5400 | 2100 | 3611 | 3274 | 2590 | 2129 |
| | **12ae** | 3,4-(OMe)₂ | >10000 | >10000 | >10000 | >10000 | >10000 | >10000 | >10000 |
| | **12af** | 4-F | 580 | 930 | 630 | 613 | 2197 | 846 | 575 |
| | **12ag** | 3-F | >10000 | >10000 | >10000 | >10000 | >10000 | >10000 | >10000 |
| | **12ah** | 4-Me | >10000 | >10000 | >10000 | >10000 | >10000 | >10000 | >10000 |
| | **12ai** | 3-Me | >10000 | >10000 | >10000 | >10000 | >10000 | >10000 | >10000 |
| 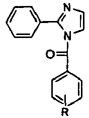 | **12aba** | 4-OMe | >10000 | >10000 | >10000 | >10000 | >10000 | >10000 | >10000 |
| | **12aaa** | 3,4,5-(OMe)₃ | >10000 | >10000 | >10000 | >10000 | >10000 | >10000 | >10000 |
| 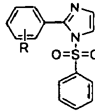 | **10a** | H | >10000 | >10000 | >10000 | >10000 | >10000 | >10000 | >10000 |
| | **10x** | 4-NO₂ | >10000 | >10000 | >10000 | >10000 | >10000 | >10000 | >10000 |
| | **10j** | 4-OBn | >10000 | >10000 | >10000 | >10000 | >10000 | >10000 | >10000 |

From Table 11, compounds **12aa-12ai** showed moderate activity with IC₅₀ values in the µM range (average of all seven cell lines). The most potent compound of this series was **12aa** with an average IC₅₀ value of 160 nM. The removal of one of the methoxy groups from the 3,4,5-trimethoxy on the C ring (**12ad, 12ae**) led to a significant loss of activity (IC₅₀ >10 µM for **12ae** and an average IC₅₀ of 3.1 µM for **12ad**). Compound with 4-fluoro on the C ring (**12af**) also showed relatively good activity (IC₅₀ = 0.91 µM), a finding that has an important implication, because replacing the trimethoxy moiety with a 4-fluoro group may provide good activity and improved metabolic stability. The position of the fluorine on the C ring was critical for activity because a shift from 4-fluoro to 3-fluoro resulted in a total loss of activity (IC₅₀ >10 µM for **12ag** compared with 0.91 µM for **12af**). This result suggested that a potential hydrogen bond donor is present close to the 4-position of this ring.

As clearly indicated in Table 11, the positions of the A and C rings were critical. A simple shift of the C-ring moiety from position 4 to position 1 in the imidazole ring (B ring) resulted in total loss of activity (IC₅₀ >10 µM for **12aba, 12aaa, 10a, 10x, 10j**).

**Table 12. In vitro growth inhibitory effects of compounds with substitutions on A ring.**

| | **ID** | **R¹** | **R²** | *IC₅₀ ± SEM (nM)* | | | | | | | | |
|---|---|---|---|---|---|---|---|---|---|---|---|---|
| | | | | **A375** | **B16-F1** | *WM164* | *LNCaP* | *PC-3* | *Du 145* | *PPC-1* | *OVCAR-8* | *NCI*/*ADR- RES* |
| 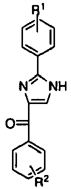 | **12ba** | 4-F | 3,4,5-(OMe)3 | 205±19 | 320±41 | 73±8 | 98±2 | 169±12 | 132±24 | 81±1 | | |
| | **12ca** | 4-OMe | 3,4,5-(OMe)3 | 30±5 | 108±12 | **31±4** | **31±1** | **45±1** | **48±0.5** | **34±0.3** | | |
| | **12cb** | 4-OMe | 4-F | 31±5 | 63±7 | 28±3 | 28±2 | 31±2 | 41±38 | **29±1** | | |
| | **12da** | 4-Me | 3,4,5-(OMe)3 | 9±2 | 46±5 | 8±2 | 12±1 | 9±0.4 | 15±0.5 | 11±0.1 | | |
| | **12db** | 4-Me | 4-F | 143±13 | 222±10 | 156±19 | 45±2 | 56±3 | 78±5 | 34±1 | | |
| | **12db-HCl** | | | 108±11 | 297±23 | 112±9 | WD | ND | ND | ND | | |
| | **12dc** | 4-Me | 3,5-(OMe)₂-4-OH | 105 | 387 | 123 | 134 | 127 | 174 | 110 | | |
| | **12ea** | 3,4,5-(OMe)₃ | 3,4,5-(OMe)3 | 4800 | >10000 | >10000 | >10000 | >10000 | >10000 | > 10000 | | |
| | **12eb** | 3,4,5-(OMe)₃ | 4-F | >10000 | >10000 | >10000 | >10000 | >10000 | >10000 | >10000 | | |
| | **12fa** | 4-Cl | 3,4,5-(OMe)3 | 43±5 | 168±14 | 26±3 | 24±1 | 35±1 | 36±0.4 | 26±0.2 | 47 | 19 |
| | **12fb** | 4-Cl | 4-F | 52±4 | 73±6 | 74±9 | 49±2 | 81±2 | 65±1 | 32±1 | | |
| | **13fa** | 4-Cl | 3,4,5-(OH)₃ | 3900 | 1810 | 2100 | 10000 | 10000 | 10000 | >10000 | | |
| | **12ga** | 4-N(Me)₂ | 3,4,5-(OMe)3 | 82±9 | 361±29 | 80±11 | 58±2 | 92±4 | 95±1 | 67±0.7 | | |
| | **12gb** | 4-N(Me)₂ | 4-F | 56±7 | 129±11 | 62±8 | 57±6 | 81±3 | 72±0.4 | 45±0.3 | | |
| | **12ha** | 3,4-(OMe)₂ | 3,4,5-(OMe)₃ | 113±14 | 1400±200 | 191±18 | 121±10 | 203±7 | 168±15 | 117±1 | | |
| | **12hb** | 3,4-(OMe)₂ | 4-F | 10000 | 4210 | 1400 | 2533 | 10000 | 10000 | 2172±48 | | |
| | **12ia** | 2-CF₃ | 3,4,5-(OMe)₃ | >10000 | >10000 | >10000 | >10000 | > 10000 | >10000 | >10000 | | |
| | **12ib** | 2-CF₃ | 4-F | >10000 | >10000 | >10000 | >10000 | >10000 | >10000 | >10000 | | |
| | **13ea** | 3,4,5-(OH)₃ | 3,4,5-(OPH)₃ | >10000 | >10000 | >10000 | >10000 | >10000 | >10000 | >10000 | | |
| | **12ja** | 4-OBn | 3,4,5-(OMe)₃ | 5200 | 10000 | 5500 | 2786 | 10000 | 10000 | 2844 | | |
| | **12jb** | 4-OBn | 4-F | 93±8 | 117±16 | 90±12 | 44±7 | 79±0.4 | 60±3 | 43±0.2 | | |
| | **12ka** | 4-OH | 3,4,5-(OMe)₃ | 1600 | 2400 | 1800 | ND | ND | ND | ND | | |
| | **12kb** | 4-OH | 4-F | 10000 | >10000 | >10000 | 10000 | >10000 | >10000 | >10000 | | |
| | **12kc** | 4-OH | 3-OH, 4,5-(OMe)₂ | 10000 | 5600 | 6400 | | | | | | |
| | **121a** | 4-Br | 3,4,5-(OMe)₃ | 32 | 74 | 36 | 34 | 36 | 49 | 33 | | |
| | **12pa** | 4-CF3 | 3,4,5-(OMe)₃ | 163.1 | 468.7 | 175 | 134 | 127 | 174 | 110 | | |
| | **13ha** | 3,4-(OH)₂ | 3,4,5-(OH)₃ | >10000 | >10000 | >10000 | ND | ND | ND | ND | | |
| | | | | | | | | | | | | |
| | | | | | | | | | | | | |
| | | | | | | | | | | | | |
| | **Colchicine** | | | 20±3 | 29±5 | ND | 16±4 | 11±1 | 10±2 | 20±1 | | |
| | | | | | | | | | | | | |

| | | | | | | | | | | | | |
|---|---|---|---|---|---|---|---|---|---|---|---|---|
| ND- not determined | | | | | | | | | | | | |

From **Table 12** compounds with 3,4,5-trimethoxy and 4-fluoro substitutions on the C ring showed good activity with different substitutions on the A ring. These compounds demonstrated excellent antiproliferative activity with IC₅₀ values as low as 8.0 nM on WM164 cell line (**12da**). In general, compounds incorporating a single substituent on the *para*-position of the A ring were more potent as can be seen from the activities of **12ca, 12cb, 12da, 12db, 12fa, 12fb, 12ga,** and **12gb** (IC₅₀ = 7.9-110 nM). **12db-**HCl salt (IC₅₀ = 172 nM) showed slightly diminished activity compared with the corresponding free base **12db** (IC₅₀ = 109 nM). Compound **12fb** (IC₅₀ = 63.7 nM), with a single halogen substituent in the para-position of the A and C rings, demonstrated potent and was devoid of a methoxy moiety. Compounds with 3,4,5-trimethoxy substituents on the A ring lost activity completely (IC₅₀ > 10 µM for **12ea, 12eb**), suggesting very different binding environments near the A ring and C ring. Removal of the 5-methoxy substituent from the A-ring improved activity significantly (IC₅₀ = 330 nM and >10 µM for **12ha, 12ea** respectively). Demethylation of the 3,4,5-trimethoxy decreased activity sharply from 43 nM (**12fa**) to 3.89 µM (**13fa**)**.** Similar results were observed for **13ea, 12ka, 12kb,** and **13ha** due to the demethylation of subsituents on either the A or C ring. Electron-donating groups (4-methoxy, 4-dimethylamino, 4-methyl) and electron-withdrawing groups (4-chloro, 2-trifluoromethyl) on the A ring did not show substantial differences in activity. The introduction of a trifluoromethyl group at the *ortho* position of the A ring caused complete loss of activity (IC₅₀ >10 µM for **12ia, 12ib**)**.** The presence of a benzyloxy group at the para position of A ring (IC₅₀ = 75 nM for **12jb**) resulted in a 440-fold increase in activity when compared with the *para*-hydroxy compound **12kb** (IC₅₀=33 µM). It is worthwhile to note that compound **12jb**, with the 4-fluoro in the C ring, has better activity than does its counterpart **12ja**, which has a 3,4,5-trimethoxy group in the C ring (IC₅₀ is 75 nM for **12jb**, and 7.3 µM for **12ja**).

**Table 13. In vitro growth inhibitory effects of compounds with protection on B ring.**

| **Structure** | **ID** | **R¹** | **R²** | **R³** | **IC₅₀ ± SEM (nM)** | | | | | | |
|---|---|---|---|---|---|---|---|---|---|---|---|
| | | | | | **A375** | **B16-F1** | **WM164** | ***LNCaP*** | ***PC-3*** | ***Du 145*** | ***PPC-1*** |
| | **11ab** | H | 4-OMe | SO₂Ph | >10000 | >10000 | >10000 | >10000 | >10000 | > 10000 | >10000 |
| | **11ac** | H | 3-OMe | SO₂Ph | >10000 | >10000 | >10000 | >10000 | >10000 | >10000 | >10000 |
| | **11ah** | H | 4-Me | SO₂Ph | >10000 | >10000 | >10000 | >10000 | >10000 | >10000 | >10000 |
| | **11af** | H | 4-F | SO₂Ph | 630±72 | 946±86 | 596±61 | 573 | 2233 | 846 | 575 |
| | **11ag** | H | 3-F | SO₂Ph | >10000 | >10000 | >10000 | >10000 | >10000 | >10000 | >10000 |
| | **11cb** | 4-OMe | 4-F | SO₂Ph | 36±5 | 71±8 | 43±6 | 31±2 | 33±2 | 52±3 | 32±0.7 |
| 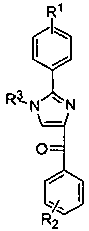 | **11db** | 4-Me | 4-F | SO₂Ph | 113±14 | 287±31 | 107±14 | 55±3 | 80±1 | 80±1 | 57±1 |
| | **11ea** | 3,4,5-(OMe)₃ | 3,4,5-(OMe)₃ | SO₂Ph | >10000 | >10000 | >10000 | >10000 | >10000 | >10000 | >10000 |
| | **11eb** | 3,4,5-(OMe)₃ | 4-F | SO₂Ph | 3840 | >10000 | >10000 | >10000 | >10000 | >10000 | >10000 |
| | **11fb** | 4-Cl | 4-F | SO₂Ph | 88±9 | 107±12 | 70±6 | 48±1 | 76±2 | 64±1 | 54±1 |
| | **11ga** | 4-N(Me)₂ | 3,4,5-(OMe)₃ | SO₂Ph | 162±13 | 1200±90 | 308±32 | 62±2 | 93±6 | 99±2 | 72±0.4 |
| | **11gb** | 4-N(Me)₂ | 4-F | SO₂Ph | 55±7 | 242±26 | 56±4 | 56±6 | 83±3 | 74±0.5 | 48±0.3 |
| | **11ha** | 3,4-(OMe)₂ | 3,4,5-(OMe)₃ | SO₂Ph | 192±15 | 970±68 | 139±15 | 114±6 | 197±9 | 144±29 | 117±2 |
| | **11hb** | 3,4-(OMe)₂ | 4-F | SO₂Ph | 960±59 | 2000±400 | 1400±30 | 1915±77 | 10000 | 3312 | 1441±49 |
| | **11ia** | 2-CF₃ | 3,4,5-(OMe)₃ | SO₂Ph | >10000 | >10000 | >10000 | >10000 | >10000 | >10000 | >10000 |
| | **11ib** | 2-CF₃ | 4-F | SO₂Ph | >10000 | >10000 | >10000 | >10000 | >10000 | >10000 | >10000 |
| | **11jb** | 4-OBn | 4-F | SO₂Ph | 64±7 | 110±15 | 48±5 | 35±1 | 75±0.5 | 58±1 | 38±0.2 |
| | **12dab** | 4-Me | 3,4,5-(OMe)₃ | Me | 32 | 134 | 40 | 32 | 46 | 36 | 28 |
| | **12cba** | 4-OMe | 4-F | Me | >10000 | >10000 | >10000 | >10000 | >10000 | >10000 | >10000 |
| | **12daa** | 4-Me | 3,4,5-(OMe)₃ | CH₂Ph | | | | 683.2 | 465.8 | 1501 | 777.9 |
| | **12gba** | 4-N(Me)₂ | 4-F | SO₂PhOMe | ∼100 | ∼100 | ∼100 | 73.2 | 44.14 | 129.4 | 63.4 |

From **Table 13**, compounds with a phenylsulfonyl protection group attached to the nitrogen of the imidazole ring (**11cb, 11db, 11fb, 11ga, 11gb, 11ha, 11jb**) were also very active with IC₅₀ in the nM range (**Table 13**). Generally the activities of these compounds are comparable to their corresponding unprotected counterparts as exemplified by comparing the activities of **11cb** (43 nM), **11db** (111 nM), **11fb** (72 nM), **11ga** (285 nM), **11gb** (87 nM), **11ha** (268 nM), and **11jb** (61 nM) with their corresponding unprotected counterparts **12cb** (36 nM), **12db** (109 nM), **12fb** (64 nM), **12ga** (131 nM), **12gb** (72 nM), **12ha** (330 nM), and **12jb** (75 nM). Other compounds (**11ab-11ag, 11ea, 11eb, 11hb, 11ia,** and **11ib,** 1-50 µM) were generally much less active, also in line with their counterparts (**12ab-12ag, 12ea, 12eb, 12hb, 12ia,** and **12ib**, 1-50 µM).

### EXAMPLE 15

### ACTIVITY OF ARYL-BENZOYL-IMIDAZOLE (ABI) COMPOUNDS IN DRUG-RESISTANT MELANOMA CELLS

P-glycoprotein (Pgp)-mediated drug efflux represents a major mechanism for cancer cells to prevent the build up of effective anticancer intracellular drug concentrations. The activity of the ABI compounds were compared against multidrug-resistant (MDR) melanoma cells (MDA-MB-435/LCCMDR1) and their parental nonresistant cancer cells (MDA-MB-435). Although MDA-MB-435 was originally designated as a breast cancer cell line, it has been shown definitively to originate from the M14 melanoma cell line. Compounds **12da, 12fb, 12cb, 11cb**, and **11fb** together with other tubulin-targeting agents including colchicine, paclitaxel, and vinblastine were tested on both the MDR melanoma cell line and its parental melanoma cell line (**Table 14A**). Paclitaxel and vinblastine are clinically used anticancer drugs known to target cell tubulin. Although colchicine is not an FDA-approved drug for cancer treatment, its prodrug, ZD6126, is in clinical trial for solid tumors. Bortezomib is the first therapeutic proteasome inhibitor and was approved in 2003 by the FDA for use in multiple myeloma. ABT-751 is known to target the tubulin colchicine binding site. It is a promising drug candidate in clinical trial for children with relapsed or refractory neuroblastoma. Compounds **12da, 12fb, 12cb, 11**cb, **11fb** had much better resistance indices (3.0 for **12da**, 0.9 for **12fb**, 1.3 for **12cb**, 0.8 for **11eb**, 0.7 for **11fb**) than colchicine (65.8), paclitaxel (69.3), and vinblastine (27.5). Although colchicine, paclitaxel, and vinblastine showed excellent activity in nonresistant melanoma cell lines (0.5-10 nM), these compounds were significantly less potent in the MDR melanoma cell line (277-658 nM). In contrast, **12cb**, **11cb**, **11fb** had essentially equivalent potency on both MDR (15 nM, 38 nM, 30 nM, 30 nM, 35 nM for **12da**, **12fb, 12cb, 11cb** and **11fb** respectively) and nonresistant melanoma cell lines (5 nM, 41 nM, 24 nM, 38 nM, 50 nM for **12da**, **12fb, 12cb, 11cb** and **11fb** respectively). Compound **12da** was more active than paclitaxel and colchicine on A375 and WM-164 cells.

**Table 14A. In vitro growth inhibitory effects of the ABI compounds in comparison to other anticancer drugs on multidrug-resistant melanoma cell line (MDR cell) and the matching sensitive parent cell line (Normal Melanoma cell).**

| **Compound ID** | **IC₅₀ ± SEM (nM) (*n*=*3*)** | | | | | | |
|---|---|---|---|---|---|---|---|
| | **A375** | **B16-F1** | **WM- 164** | **Tubulin binding (µM)** | **MDA- MB- 435** | **MDA-MB- 435/LCC6MDR1** | **Resistance index*** |
| **12da** | 9±2 | 46±5 | 8±2 | 0.2+0.1 | 5±1 | 15±2 | 3.0 |
| **12fb** | 52±4 | 73±6 | 74±9 | 3.9±2.1 | 41±2 | 38±2 | 0.9 |
| **12cb** | 31±5 | 63±7 | 28±3 | 3.4±1.5 | 24±2 | 30±4 | 1.3 |
| **11cb** | 36±5 | 71±8 | 43±6 | ND | 38±3 | 30±2 | 0.8 |
| **11fb** | 88±9 | 107±12 | 74±8 | ND | 50±6 | 35±3 | 0.7 |
| Paclitaxel | 12±3 | 17±2 | 18±3 | N/A | 4±1 | 277±41 | 69.3 |
| Vinblastine | 1.1±0.2 | 4.7±0.7 | 0.6±0.1 | ND | 0.4±0.1 | 11±1 | 27.5 |
| Colchicine | 20±3 | 29±5 | 10±2 | 1.8+0.5 | 10±1 | 658±50 | 65.8 |
| Bortezomib | 8±1 | 24±2 | 8±1 | ND | ND | ND | ND |
| ABT-751 | 1111±108 | 2127±351 | 661±56 | ND | ND | ND | ND |

| | | | | | | | |
|---|---|---|---|---|---|---|---|
| *Resistance indexes were calculated by dividing IC₅₀ values on multidrug-resistant cell line MDA-MB-435/LCC6MDR1 by IC₅₀ values on the matching sensitive parental cell line MDA-MB-435. Abbreviations: N/A, value not available; ND, not determined. | | | | | | | |

**Table 14B. Anticancer efficacy and colchicine site binding affinity of ABIs in different cancer and MDR cell lines with different resistance mechanisms. ABIs showed excellent potency against all tested melanoma cell lines including highly metastatic and multidrug resistant cell lines. High binding affinity of ABIs to the colchicine binding site in tubulin confirmed their target inside cells.**

| | IC_{5O} ± SEM (nmol/L) (*n*=*3*) | | | | | | | |
|---|---|---|---|---|---|---|---|---|
| | **12cb** | **12da** | **12fb** | Paclitaxel | Vinblastine | Colchicine | ABT-751 | SN-38 |
| A375 | 31±5 | 9±2 | 52±4 | 12±3 | 1±0.1 | 20±3 | 685±108 | ND |
| A375MA2 | 44±5 | 8±1 | 55±4 | 8±1 | 1±0.2 | 18±2 | 265±36 | ND |
| B16-F1 | 63±7 | 46±5 | 73±6 | 17±2 | 5±1 | 29±5 | 2127±351 | ND |
| WM-164 | 28±3 | 8±2 | 74±9 | 18±3 | 0.6±0.1 | 10±2 | 661±56 | ND |
| **MDR1** | | | | | | | | |
| MDA-MB-435* | 24±2 | 5±1 | 41±2 | 4±1 | 0.4±0.1 | 10±1 | 417±23 | ND |
| MDA-MB-435/LCC6MDR1 | 30±4 (**1**) | 11±2 (**2**) | 38±2 (**1**) | 277±4 (**69**) | 11±1 (**28**) | 658±50 (**66**) | 577±31 (**1**) | ND |
| OVCAR-8* | 25±2 | 11±1 | 45±2 | 10±0.2 | 2±0.1 | 12±1 | 785±17 | 2±0.2 |
| NCI/ADR-RES | 13±1 (**0.5**) | 5±0.1 (**0.5**) | 20±6 (**0.4**) | 5109±170 (**511**) | 570±84 (**285**) | 737±51 (**61**) | 864±42 (**1**) | 10±1 (**5**) |
| **MRP** | | | | | | | | |
| HEK293 - pcDNA3.1 * | 12±2 | 9±1 | 54±0.3 | 9±0.3 | 5±0.1 | 3±0.4 | 645±153 | 3±0.4 |
| HEK293-MRP1 | 16±2 (**1**) | 8±1 (**0.9**) | 33±7 (**0.6**) | 30±3 (**3**) | 24±1 (**5**) | 5±0.1 (**2**) | 717±28 (**1**) | 9±0.04 (**3**) |
| HEK293-MRP2 | 14±4 (**1**) | 8±0.3 (**0.9**) | 39±12 (**0.7**) | 37±2 (**4**) | 28±2 (**6**) | 3±0.3 (**1**) | 747±7 (**1**) | 7±0.1 (**2**) |
| **BCRP** | | | | | | | | |
| HEK293-482R2 | 17±1 (**1**) | 8±1 (**0.9**) | 23±3 **(0.4)** | 50±1 (**6**) | 25±1 (**5**) | 5±0.1 (**2**) | 653±72 (**1**) | 123±28 (**41**) |
| Tubulin binding (µM)⁺ | 311 | 0.2±0.1 | 4±1 | N/A | ND | 2±1 | 3.1⁺⁺ | ND |

| | | | | | | | | |
|---|---|---|---|---|---|---|---|---|
| Notes: *: parental cell line to drug resistant cell subline; MDR1 were overexpressed in MDA-MB-435/LCC6MDR1 and NCI/ADR-RES; MRP1, MRP2 and BCRP were overexpressed in HEK293-MRP1, HEK293-MRP2, and HEK293-482R2. The resistance indexes (numbers in the parenthesis) were calculated by dividing IC₅₀ values on the resistant cell subline by that of the matching parental cell line. ⁺: IC₅₀ for tubulin binding was calculated from [³H]colchicine competition-binding scintillation proximity assay. ⁺⁺: binding affinity reported in the literature for ABT-751. Abbreviations: N/A, not applicable since they bind to tubulin at different sites. | | | | | | | | |

The results of **Table 14A** showed that cell line MDA-MB-435/LCCMDR1 was very resistant to colchicine, paclitaxel, and vinblastine. But the ABIs of this invention showed equal potency to the drug-resistant cell line and the sensitive parent cell line. This result strongly suggests that ABIs are not substrates for P-gp. Thus, they overcame the multidrug resistance found in MDA-MB-435/LCCMDR1 cells. The dose response curves are shown in **Figure 21** for **12fb, 12da,** and **12cb. Table 14B** explores further the resistance mechanisms for paclitaxel, SN-38, vinblastine, and colchicine as compared to the ABIs **12cb, 12da,** and **12fb.** MRP and BCRP conferred moderate resistance to pacleitaxel (resistance indexes of 4 and 6, respectively), vinblastine (resistance indexes of 6 and 5, respectively), and BCRP conferred significant resistance to SN-38 (resistance index of 41). However, none of the ABIs were susceptible to MRP- or BCRP-mediated resistance (resistance indexes ranged from 0.4 to 1.0). ABT-751, like the ABIs, was not susceptible to MDR1, MRP, or BCRP.

### EXAMPLE 16

### IN VITRO MICROTUBULE POLYMERIZATION ASSAY

### Materials and Methods

Bovine brain tubulin (0.4 mg) (Cytoskeleton, Denver, CO) was mixed with 10 µM of the test compound and incubated in 110 µl of general tubulin buffer (80 mM PIPES, 2.0 mM MgCl₂, 0.5 mM EGTA, and 1 mM GTP) at pH 6.9. The absorbance at 340 nm was monitored every 1 min for 15 min by the SYNERGY 4 Microplate Reader (Bio-Tek Instruments, Winooski, VT). The spectrophotometer was set at 37 °C for tubulin polymerization.

### Results

The inhibition of tublin polymerization by Aryl-Benzoyl-Imidazole (ABI) compounds was examined. Bovine brain tubulin (>97% pure) was incubated with three potent ABI compounds, **12cb, 12da,** and **12db** at a concentration of 10 µM, to determine the effect of these ABI compounds on tubulin polymerization (**Figure 22**). Tubulin polymerization was completely inhibited by compound **12da**, while ∼ 80% inhibition was observed during incubation with compounds **12cb** and **12db**.

This microtubule destabilization effect was similar to that of colchicine and vinblastine but was opposite to that of paclitaxel. The results not only confirmed that ABIs can directly interact with tubulin but also suggested that they may share the same binding site with colchicine (or vinblastine).

### EXAMPLE 17

### MELANOMA INHIBITION IN VITRO

### Materials and Methods

B16-F1 melanoma cells were plated at a colony-forming density (2000 cells per well on six-well plates) on top of 0.8% base agar. Cells were grown in 0.4% agar together with DMEM medium supplemented with fetal bovine serum and an antibiotic-antimycotic solution at 37 °C in an atmosphere of 95% air and 5% CO₂. Cells were treated with compounds **12da, 12cb** and **12fb** at different concentrations (20, 100, and 500 nM). Compounds were added to the media from 1 mM DMSO stock solutions, and a corresponding dilution of DMSO was used as control. Cells were grown for 14 days. Plates were photographed, and the number of colonies was measured by Artek 880 Automated Colony Counter (Artek Systems Corporation, Farmingdale, NY).

### Results

Four representative photos are shown in **Figure 23**. After 14 days of incubation, about 130 detectable colonies (diameter larger than 100 µm) were formed in controls (no treatment).

Compounds **12cb** and **12da** effectively inhibited B16-F1 melanoma colony formation even at the lowest tested concentration, 20 nM (p<0.05 compared with control). **12fb** showed effective inhibition at 100 nM. All three tested compounds showed complete inhibition of colony formation at 0.5 µM, further proving ABIs' antimelanoma efficacy.

### EXAMPLE 18

### IN VIVO ANTI-TUMOR ACTIVITY

### Materials and Methods

**Animals:** Female C57/BL mice, age 4-6 weeks, were purchased from Harlan Laboratories (Harlan Laboratories Inc., Indianapolis, IN). The animal housing met the Association for Assessment and Accreditation and Laboratory Animal Care specifications. All of the procedures were conducted in accordance with guidelines of our Institutional Animal Care and Use Committee.

***In vivo* evaluation of efficacy.** Mouse melanoma B16-F1 cells were prepared in FBS-free DMEM medium (Cellgro Mediatech) at a concentration of 5 x 10⁶ viable cells/mL. The cell suspension (100 µL) was injected subcutaneously in the right dorsal flank of each mouse. When tumor size reached about 100-150 mm³, about 7 days after cell inoculation, all mice bearing tumors were divided into control and treatment groups based on tumor size (n = 5 per group). Each group had similar average tumor size. Mice in control groups (negative control) were injected intraperitoneally with 50 µL vehicle solution only or DTIC at 60 mg/kg (positive control) once daily. Tumor volume was measured every 2 days with a traceable electronic digital caliper (Fisher Scientific, Inc., Pittsburgh, PA) and calculated using the formula a × b² ×0.5, where a and b represented the larger and smaller diameters, respectively. Tumor volume was expressed in cubic millimeters. Data were expressed as mean ± SE for each group and plotted as a function of time. Percentage tumor reduction at the conclusion of the experiment (14 days after starting treatment) was calculated with the formula 100-100 × [(T - T₀)/(C - C₀)], where T represents mean tumor volume of a treated group on a specific day, T₀ represents mean tumor volume of the same group on the first day of treatment, C represents mean tumor volume of a control on a specific day, and C₀ represents mean tumor volume of the same group on the first day of treatment. Animal activity and average body weight of each group were monitored during the entire experiment period to assess compound toxicity. At the end of treatment, all mice were euthanized by CO₂ followed by cervical dislocation, and tumors were harvested for further studies.

### Results

To evaluate efficacy of ABI analogs in *vivo,* we tested the antitumor activity of compound **12cb** on mice melanoma B16-F1 xenograft. against DTIC, the gold standard in malignant melanoma treatment, was used as a positive control (**Figure 24A**). Twenty female C57/BL mice were divided into four groups: a vehicle control group, a DTIC (60 mg/kg) treatment group, a **12cb** (10 mg/kg) treatment group, and a **12cb** (30 mg/kg) treatment group. Each mouse was injected with 0.5 million B16-F1 melanoma cells subcutaneously. Seven days after tumor inoculation, treatment started with each compound injected intraperitoneally daily (**Figure 24**). Tumor volume was significantly (p<0.05) reduced 47%, 51%, and 73% for **12cb** (10 mg/kg), DTIC (60 mg/kg), and **12cb** (30 mg/kg), respectively, after 14 days of treatment. No significant weight loss was observed in any of the treatment groups during the experiment.

Two dose levels of **12fb**, 15 and 45 mg/kg, were chosen. DTIC at 60 mg/kg was used as a positive control. B16-F1 melanoma allograft model on C57BL/6 mice was first chosen for study. After 13 days of treatment (**Figure 24B**), compound **12fb** inhibited melanoma tumor growth (TGI value) by 32% at 15 mg/kg and 82% at 45 mg/kg. Student's t test p value of **12fb** at 45 mg/kg compared with control was less than 0.001, indicating a significant difference. The t test p value of **12fb** at 15 mg/kg compared with control was 0.08, suggesting that this dose was not effective. Comparing **12fb** at 45 mg/kg with DTIC at 60 mg/kg, which had a TGI of 51%, the t test p value was about 0.001, suggesting that **12fb** had substantially better activity than did DTIC. For the control and **12fb** 15 mg/kg treatment groups, average body weight increased slightly throughout the experiment period.

To further confirm ABIs' *in vivo* activity, A375 human melanoma xenograft model on SHO mice was used, and **12fb** at 25 mg/kg was tested. DTIC at 60 mg/kg was used as a positive control again. After 31 days of treatment (**Figure 24C**), **12fb** inhibited melanoma tumor growth (TGI value) by 69%, whereas DTIC inhibited growth by 52%. The *t* test p value of **12fb** treatment versus control was less than 0.001, suggesting that **12fb** significantly inhibited melanoma tumor growth at 25 mg/kg. The *t* test p value of **12fb** treatment versus DTIC was less than 0.05, suggesting again that **12fb** had better activity than did DTIC. Average body weight of all groups increased slightly throughout the experiment period. Physical activities for the mice also looked normal, suggesting that 25 mg/kg was a well tolerated dose for SHO mice.

### EXAMPLE 19

### BINDING TO COLCHICINE

### Materials and Methods

Each test compound was prepared at 20 × concentration in G-PEM buffer (Cytoskeleton Inc., Denver, CO) followed by pipetting 10 µL of test compound into the 96-well plates. Ten microliters of tritiated labeled colchicine (Perkin-Elmer, Waltham, MA) was added to each testing well. Subsequently, 180 µL bead/tubulin (GE Healthcare Bio-Sciences Corp., Piscataway, NJ) suspension was added into each well. The plate was incubated for 45 min at 37 °C before it was read by a Topcount NXT plate reader (Perkin-Elmer, Waltham, MA). Nonradiolabeled "cold" colchicine was included as a positive control and paclitaxel as a negative control because paclitaxel binds to a different site in tubulin and does not compete for the colchicine site binding. Data were processed using GraphPad Prism software.

### Cell cycle analysis

Flow cytometry analysis was performed to study cell cycle phase distribution. A375 cells were cultured in 10-cm tissue culture dishes until the confluence was about 80%, and then cells were treated with 0, 10, 50, 200, and 1000 nM of colchicine, **12da, 12fb** and **12cb**, for 24 h in growth media. Cellular DNA was stained with PBS containing 50 µg/mL propidium iodide and 100 µg/mL RNase A. The cell cycle was determined using a BD LSR-II cytometer (BD Biosciences, San Jose, CA) with 10,000 cells scored. Data were analyzed and graphs were prepared using the Modfit 2.0 program (Verity Software House, Topsham, ME).

### Results

Three ligand binding sites in tubulin α/β-heterodimer have been reported: paclitaxel binding site, vinblastine binding site, and colchicine binding site. The binding affinity of compound **12cb** using ³H-labeled colchicine and a competitive binding scintillation proximity assay (SPA) was measured. The results confirmed the strong binding of **12cb** with a binding affinity of 3.4±1.5 µM (**Figure 25A**). Colchicine bound tubulin with an IC₅₀ value of 1.8±0.5 µM under these conditions. These results clearly indicated that ABI compounds effectively inhibit tubulin polymerization.

The binding graph (**Figure 25A**) clearly shows that ABIs can competitively bind to the tubulin colchicine binding site. As the concentration of the three tested compounds increased from 0.03 µM to 100 µM, increased tritiated colchicine was competitively stripped away from tubulin and emitted lower SPA counts. The negative control, paclitaxel, gave only a flat line, because theoretically it should not bind to the colchicine binding site on tubulin. Second, ABIs have relatively high binding affinity to the tubulin colchicine binding site. GraphPad Prism calculated IC₅₀ values for binding showed that **12da** has the highest binding affinity. The binding affinity was positively correlated to *in vitro* antimelanoma activity; the higher the binding affinity, the higher the antimelanoma activity.

ABIs demonstrated that they arrest cells by cell cycle analysis in the G2/M phase as indication that they target tubulin. Compounds **12da, 12fb** and **12cb** were tested together with colchicine as a positive control on A375 cells (**Figure 25B**). Four different concentrations - 10, 50, 200, and 1000 nM - of each compound were chosen to show the dose effect (**Figure 25C** **and** **25D**). For controls (no treatment) without interference, about 16% of A375 cells were distributed in the G2/M phase. For the colchicine treatment group, as concentration increased from 10 nM to 50 nM, the percentage of cells distributed in the G2/M phase increased from 14% to 85%. ABIs had similar results for A375 cells, in arresting them in the G2/M phase in a dose-dependent manner. The potency of the different concentrations in arresting cells in the G2/M phase positively correlated with *in vitro* activity.

### EXAMPLE 20

### IN VITRO AND IN VIVO PHARMACOLOGY OF COMPOUNDS 17ya, 12fa. AND 55 Materials and Methods

**Cell culture and cytotoxicity assay of prostate cancer.** All prostate cancer cell lines (LNCaP, PC-3, and DU145, PPC-1) were obtained from ATCC (American Type Culture Collection, Manassas, VA, USA). Human PC-3_TxR, was resistant to paclitaxel and used a MDR model compared with PC-3. Cell culture supplies were purchased from Cellgro Mediatech (Herndon, VA, USA). All cell lines were used to test the antiproliferative activity of compounds **17ya**, **12fa**, and **55** by sulforhodamine B (SRB) assay. All cancer cell lines were maintained in RPMI 1640 media with 2 mM glutamine and 10% fetal bovine serum (FBS).

***In vitro* microtubule polymerization assay.** Porcine brain tubulin (0.4 mg) (Cytoskeleton, Denver, CO) was mixed with 1 and 5 µM of the test compound or vehicle (DMSO) and incubated in 100 µL of buffer (80 mM PIPES, 2.0 mM MgCl₂, 0.5 mM EGTA, pH 6.9 and 1 mM GTP). The absorbance at 340 nm wavelength was monitored every min for 15 min (SYNERGY 4 Microplate Reader, Bio-Tek Instruments, Winooski, VT). The spectrophotometer was maintained at 37 °C for tubulin polymerization.

**Metabolic incubations.** Metabolic stability studies were conducted by incubating 0.5 µM of test compounds in a total reaction volume of 1 mL containing 1 mg/mL microsomal protein in reaction buffer [0.2 M of phosphate buffer solution (pH 7.4), 1.3 mM NADP⁺, 3.3 mM glucose-6-phosphate, and 0.4 U/mL glucose-6-phosphate dehydrogenase] at 37 °C in a shaking water bath. The NADPH regenerating system (solution A and B) was obtained from BD Biosciences (Bedford, MA). For glucuronidation studies, 2 mM UDP-glucuronic acid (Sigma, St. Louis, MO) cofactor in deionized water was incubated with 8 mM MgCl₂, 25 µg of alamethicin (Sigma, St. Louis, MO) in deionized water, and NADPH regenerating solutions (BD Biosciences, Bedford, MA) as described previously. The total DMSO concentration in the reaction solution was approximately 0.5% (v/v). Aliquots (100 µL) from the reaction mixtures used to determine metabolic stability were sampled at 5, 10, 20, 30, 60, and 90 min. Acetonitrile (150 µL) containing 200 nM of the internal standard was added to quench the reaction and to precipitate the proteins. Samples were then centrifuged at 4,000g for 30 min at RT, and the supernatant was analyzed directly by LC-MS/MS.

**Analytical method**. Sample solution (10 µL) was injected into an Agilent series HPLC system (Agilent 1100 Series Agilent 1100 Chemstation, Agilent Technology Co, Ltd). All analytes were separated on a narrow-bore C18 column (Alltech Alltima HP, 2.1×100 mm, 3 µm, Fisher, Fair Lawn, NJ). Two gradient modes were used. For metabolic stability studies, gradient mode was used to achieve the separation of analytes using mixtures of mobile phase A [ACN/H₂O (5%/95%, v/v) containing 0.1% formic acid] and mobile phase B [ACN/H₂O (95%/5%, v/v) containing 0.1% formic acid] at a flow rate of 300 µL/min. Mobile phase A was used at 10% from 0 to 1 min followed by a linearly programmed gradient to 100% of mobile phase B within 4 min, 100% of mobile phase B was maintained for 0.5 min before a quick ramp to 10% mobile phase A. Mobile phase A was continued for another 10 min towards the end of analysis.

A triple-quadruple mass spectrometer, API Qtrap 4000™ (Applied Biosystems/MDS SCIEX, Concord, Ontario, Canada), operating with a TurboIonSpray source was used. The spraying needle voltage was set at 5 kV for positive mode. Curtain gas was set at 10; Gas 1 and gas 2 were set 50. Collision-Assisted-Dissociation (CAD) gas at medium and the source heater probe temperature at 500°C. Multiple reaction monitoring (MRM) mode, scanning *m*/*z* 378 → 210 (**17ya**), *m*/*z* 373 → 205 (**12fa**), *m*/*z* 410 → 242 (**55**) and m/z 309 → 171 (internal standard), was used to obtain the most sensitive signals. Data acquisition and quantitative processing were accomplished using Analyst™ software, Ver. 1.4.1 (Applied Biosystems).

**Aqueous solubility.** The solubility of drugs was determined by Multiscreen Solubility Filter Plate (Millipore Corporate, Billerica, MA) coupled with LC-MS/MS. Briefly, 198 µL of phosphate buffered saline (PBS) buffer (pH 7.4) was loaded into 96-well plate, and 2 µL of 10 mM test compounds (in DMSO) was dispensed and mixed with gentle shaking (200-300 rpm) for 1.5 hours at RT (N = 3). The plate was centrifuged at 800g for 10 min, and the filtrate was used to determine its concentration and solubility of test compound by LC-MS/MS as described previously.

**Pharmacokinetic study.** Male ICR mice (n = 3 per group) 6 to 8 weeks of age were purchased from Harlan Inc., and used to examine the pharmacokinetics (PK) of **17ya, 12fa**, and **55**. All compounds (10 mg/kg) were dissolved in DMSO/ PEG300 (1/9) and administered by a single intravenously (i.v.) injection (50 µL) into the tail vein. Blood samples were collected at 5, 15, and 30 min, 1, 1.5, 2, 3, 4, 8, 12, and 24 h after i.v. administration. Mice were given (p.o.) by oral gavage at 20 mg/kg (in Tween80/DMSO/H₂O, 2/2/6) of each test compound to evaluate their oral bioavailability. Blood samples were collected at 0.5, 1, 1.5, 2, 3, 4, 8, 12, and 24 h after p.o. administration.

Female Sprague-Dawley rats (n = 3; 254 ± 4 g) were purchased from Harlan Inc. (Indianapolis, IN). Rat thoracic jugular vein catheters were purchased from Braintree Scientific Inc. (Braintree, MA). On arrival at the animal facility, the animals were acclimated for 3 days in a temperature-controlled room (20-22 °C) with a 12 h light/dark cycle before any treatment. Compounds **17ya, 12fa,** and **55** were administered i.v. into the thoracic jugular vein at a dose of 5 mg/kg (in DMSO/PEG300, 1/9). An equal volume of heparinized saline was injected to replace the removed blood, and blood samples (250 µL) were collected via the jugular vein catheter at 10, 20, 30 min, and 1, 2, 4, 8, 12, 24 h. Rats were given (p.o.) by oral gavage at 10 mg/kg (in Tween80/DMSO/H₂O, 2/2/6) of each test compound to evaluate their oral bioavailability. All blood samples (250 µL) after oral administration were collected via the jugular vein catheter at 30, 60, 90 min, 120 min, 150 min, 180 min, 210 min, 240 min, and 8, 12, 24 h. Heparinized syringes and vials were prepared prior to blood collection. Plasma samples were prepared by centrifuging the blood samples at 8,000g for 5 min. All plasma samples were stored immediately at -80 °C until analyzed.

Analytes were extracted from 100 µL of plasma with 200 µL of acetonitrile containing 200 nM the internal standard. The samples were thoroughly mixed, centrifuged, and the organic extract was transferred to autosampler for LC-MS/MS analysis.

**PC-3_TxR xenograft studies.** PC-3_TxR cells (10×0⁷ per mL) were prepared in RPMI1640 growth media containing 10% FBS, and mixed with Matrigel (BD Biosciences, San Jose, CA) at 1:1 ratio. Tumors were established by injecting 100 µL of the mixture (5×10⁶ cells per animal) subcutaneously (s.c.) into the flank of 6-8-week-old male athymic nude mice. Length and width of tumors were measured and the tumor volume (mm³) was calculated by the formula, π/6 × L × W², where length (L) and width (W) were determined in mm. When the tumor volumes reached 300 mm³, the animals bearing PC-3_TxR tumors were treated with vehicle [Tween80/DMSO/H₂O (2/2/6)], or **17ya** (10 mg/kg) orally. The dosing schedule was 3 times a week for four weeks.

### Results

**17a and 55 exhibit broad cytotoxicity in cells, including multidrug-resistant cells.** The ability of **17ya** and **55** to inhibit the growth of cancer cell lines was evaluated using SRB assay (**Table 15**). Both compounds inhibited the growth of several human cancer cell lines, including five prostate and one glioma cancer cell lines, with IC₅₀ values in the low nanomolar range. **17ya** exhibited 1.7-4.3 fold higher potency than **55** in these cell lines. Paclitaxel-resistant PC-3 (PC-3/TxR) cell line that over-expresses P-glycoprotein (P-gp), was used to study the effect of drug resistance on **17ya** and **55** and to compare against its parent, PC-3 cell line. The IC₅₀ values of docetaxel were 1.2 ± 0.1 nM and 17.7 ± 0.7 nM in PC-3 and PC-3/TxR cells, respectively. **17ya** and **55** were both equipotent against parent PC-3 and PC-3/TxR, whereas paclitaxel and docetaxel exhibited relative resistance of 85- and 15-fold, respectively. These data indicate that both **17ya** and **55** circumvent P-gp-mediated drug resistance.

**Table 15. Cytotoxicity data of 17ya and 55.**

| **Cell line** | **Type** | **Cytotoxicity [IC₅₀ values, mean ± SD nM]** | | |
|---|---|---|---|---|
| | | **17ya** | **55** | **Paclitaxel** |
| | | 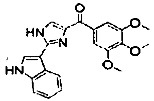 | 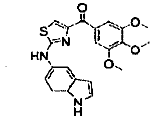 | 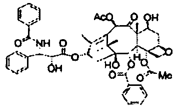 |
| PC-3 | Prostate | 5.2 ± 0.2 | 16 ± 1.5 | 0.6 ± 0.05 |
| PC-3/TxR | Prostate | 2.1 ± 0.1 (**0.4**) | 6.7 ± 0.5 (**0.4**) | 51 ± 2.3 (**85**) |
| LNCaP | Prostate | 12 ± 0.1 | 27 ± 0.6 | 1.7 ± 0.2 |
| Du-145 | Prostate | 17 ± 0.2 | 38 ± 0.6 | 5.1 ± 0.1 |
| PPC-1 | Prostate | 21 ± 0.1 | 36 ± 0.4 | 2.3 ± 0.8 |
| U87MG | Glioma | 10 ± 1.6 | 22 ± 3.0 | NR |

| | | | | |
|---|---|---|---|---|
| IC₅₀ values (mean ± SD) were determined after 96 h treatment (N = 3). Paclitaxel was used as a positive control. Data in parentheses indicated resistance factor when compared IC₅₀ values in PC-3 and PC-3/TxR. NR, Not Reported. | | | | |

**17ya and 55 bind to colchicine-binding site on tubulin, inhibit tubulin polymerization, and induce cell apoptosis (****Figure 26****).** A competitive mass binding assay was developed to study the interaction of small molecule inhibitors with tubulin. In this study, varying concentrations of **17ya** or **55** were used to compete with colchicine-tubulin binding. Both compounds competed effectively with colchicine for tubulin binding (**Figure 26A**); however, their competitive binding curves deviated substantially from zero at higher concentrations when compared to podophylltoxin, a known potent colchicine-site binding ligand. This suggests that both **17ya** and **55** exhibited less affinity than podophylltoxin or they partially bind to the colchicine-binding site. Vinblastine, the negative control, did not inhibit the colchicine-tubulin binding, successfully demonstrating the specificity of this competitive mass binding assay

Porcine brain tubulin (>97% pure) was incubated with **17ya** or **55** (5 µM) to test their effect on tubulin polymerization (**Figure 26B**). **17ya** and **55** inhibited tubulin polymerization by 47% and 40% at 15 min, respectively. Colchicine at 5 µM was used as a positive control and inhibited tubulin polymerization by 32%. These data suggest that both **17ya** and **55** have slightly greater inhibition of tubulin polymerization than colchicine. Therefore, the molecular mechanism of these compounds is binding to the colchicine-binding site, inhibiting tubulin polymerization, and inducing cytotoxicity.

PC-3 and PC-3/TxR cells were exposed to 0.8 to 600 nmol/L of **17ya, 55,** or docetaxel for 24 h. The levels of DNA-histone complexes were used to represent cell apoptosis. Both **17ya** and **55** were equally potent to induce cell apoptosis in PC-3 (**Figure 26C**) and PC-3/TxR (**Figure 26D**) in 24 h. Though, docetaxel was highly potent to induce apoptosis of PC-3 cells, it was weaker in PC-3/TxR cells due to over-expression of P-gp.

**17ya and 55 exhibited favorable drug-like properties**. Drug-like properties, such as metabolic stability, permeability, aqueous solubility, and drug-drug interactions, were examined for **17ya** and **55** (**Table 16A**). **17ya** exhibited greater metabolic stability, and aqueous solubility than **55**. Both chemicals exhibited more than adequate permeability values, suggesting their potential to be orally used. In addition, both **17ya** and **55** showed high IC₅₀ values in micromolar range on CYP enzyme inhibition assays, indicating that both compounds may avoid drug-drug interactions through main CYP liver enzymes. Overall, both compounds exhibited favorable drug-like properties.

**Table 16A. Drug-like properties of compound 17a and 55. Metabolic stability, permeability, solubility, and potential drug-drug interactions were evaluated. Each value represents the mean from duplicate studies.**

| **Measurment** | **Units** | **17ya** | **55** | **positive controls (mean)** |
|---|---|---|---|---|
| **Metabolic stability** | | | | |
| half-life in human liver microsomes | min | > 60 | 28 | Verapamil (12) |

| **Permeability** | | | | |
|---|---|---|---|---|
| P_{app(A→B)} in CaCO-2 assay | 10⁻⁶ cm/s | 36 | 99 | Propranolol (19) |
| **Aqueous solubility** | µg/mL | > 75 | 19 | 1h (1.1) |

| **Drug-drug interactions** | | | | |
|---|---|---|---|---|
| IC₅₀ value in Cyp3A4 (substrate: Testosterone) | µM | 20 | 5.5 | Ketoconazole (0.02) |
| IC₅₀ value in Cyp2D6 (substrate: Dextromethorphan) | µM | > 50 | 34 | Quinindine (0.1) |
| IC₅₀ value in Cyp2C19 (substrate: (S)-mephenytoin) | µM | 6.6 | 5.3 | Ticlopidine (0.37) |
| IC₅₀ value in Cyp2C9 (substrate: Diclofenac) | µM | 17 | 4.9 | Sulfaphenazole (0.5) |
| IC₅₀ value in Cyp1A2 (substrate: Phenacetin) | µM | 9.2 | 8.1 | Furafytline (2.2) |

**Table 16B. Summary of drug-like and pharmacokinetic properties of 17ya, 12fa, 55, and 1h.**

| | | **17ya** | **12fa** | **55** | **1h** |
|---|---|---|---|---|---|
| | | 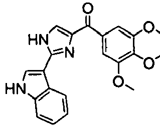 | 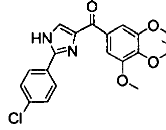 | 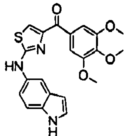 | 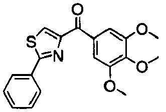 |
| Molecular weight | | 377 | 372 | 409 | 355 |
| IC₅₀ in PC3 (nM) | nM | 10 | 35 | 28 | 21 |
| Half-life in HLM (Phase I) | min | -80 | 44 | 30 | 17 |
| Half-life in HLM (Phase I+II) | min | -90 | NA | 43 | 17 |
| Solubility | µg/mL | >75 | 12 | 19 | 1 |
| RatPK_IV5mgk_Cl | mL/min/kg | | 16 | | 7.7 (2.5mpk) |
| RatPK_IV5mgk_V | L/kg | | 1.9 | | 4.9 (2.5mpk) |
| RatPK_PO10mgk_Cmax | ng/mL | | 1109 | | 212 |
| RatPK_PO1 0mgk_AUC | min*µg/mL | | 218 | | 37 |
| RatPK_Bioavailability | %F | | 35 | | 3.3 |
| MousePK_IV10mgk_Cl | mL/min/kg | | 61 | | 130 |
| MousePK_IV10mgk_V | L/kg | | 4 | | 4.9 |
| MousePK_PO20mgk_Cmax | ng/mL | | 2592 | | NA |
| MousePK_PO20mgk_AUC | min*µg /mL | | 201 | | NA |
| MousePK_Bioavailability | %F | | 62 | | NA |

As shown in Table 16B, **17ya** had a half-life of 80 min by phase I reaction, suggesting that **17ya** was stable in phase **I** metabolic processes. The half-life (90 min) in the presence of UDP-glucuronic acid was similar to that observed in its absence. These data suggested that **17ya** is stable in human liver microsomes, and it was hoped that low clearance and long half-life will be obtained in human. On the other hand, **55** exhibited 30 and 43 min as half lives when it was in the presence and absence of UDP-glucuronic acid, respectively. Compound **12fa** shows the half-life with 44 in phase **I**. These data suggested that all three compounds showed acceptable stability in human liver microsomes, and **17ya** is more stable than **12fa** and **55**. When investigating their metabolism, it was found that **12fa** and **55** exhibited higher levels of ketone-reduction (data not shown), suggesting that **12fa** and **55** are more labile than **17ya**.

### Compound 17ya exhibited great aqueous solubility, 12fa and 55 showed acceptable solubility.

Compound **17ya** contained an imidazole ring, and this ring improved aqueous solubility, resulting in > 75 µg/mL aqueous solubility (**Table 16A**). Compounds **12fa** and **55** exhibited less aqueous solubility, and exhibited 12 and 19 µg/mL, respectively. Overall, **17ya** demonstrated a great aqueous solubility, and **12fa** and **55** showed acceptable aqueous solubility, and much improved over **1h**. The greater solubility of **12fa** translated into much improved oral bioavailability compared to **1h** (35% vs. 3.3% in rat). Similarly for **17ya** and **55,** aqueous solubility correlated with much improved oral bioavailability as discussed *infra* (Table 17).

**Pharmacokinetic studies of 17ya and 55 in mice**, **rats and dogs**. The pharmacokinetic parameters of **17ya** and **55** given in a single (i.v. or p.o.) dose in ICR mice, Sprague-Dawley rats, and beagle dogs are summarized in **Table 17. 17ya** exhibited low clearance in mice and rats, suggesting that **17ya** exhibited metabolic stability, and minimal first-pass metabolism in these species. In addition, **17ya** had moderate volume of distribution in mice and rats, indicating that it may properly distribute into tissues, including tumors. Unlike in mice and rats, surprisingly, the total clearance of **17ya** in dogs was high. Two abundant metabolites in dog plasma, a hydroxylated metabolite and an unknown metabolite with +34 m/z of the parent (data not shown), were consistent with those found in dog liver microsomes. In summary, higher clearance and lower oral exposure was obtained for **17ya** compared to **55** in dogs, but not in mice and rats. In addition, **17ya** exhibited abundant metabolites only in dog liver microsomes, but not in mouse, rat or human liver microsomes (data not shown). **17ya** showed acceptable 21%, 36%, and 50% oral bioavailability in rats, mice, and dogs, respectively. Meanwhile, **55** had low clearance in rats, and moderate clearance in mice and dogs. Similar to **17ya, 55** exhibited moderate volume of distribution in these species. **55** had constant oral bioavailability rates among three species (24%-36%). These properties indicate that both **17ya** and **55** are potential orally available tubulin inhibitors.

**Table 17. Pharmacokinetic studies of compounds 17ya and 55 in mice, rats, and dogs.**

| | **17ya** | | **55** | |
|---|---|---|---|---|
| | **IV** | **PO** | **IV** | **PO** |
| **Mouse PK (N=3)** | | | | |
| Dose, mg/kg | 10 | 20 | 10 | 20 |
| Clearance, mL/min/kg | 19 | NR | 40 | NR |
| Vss, L/kg | 2.9 | NR | 1.3 | NR |
| t_{1/2}, min | 101 | 339 | 46 | 126 |
| AUC, min*µg/mL | 540 | 384 | 249 | 171 |
| Cₘₐₓ, ng/mL | 4800 | 1560 | 7739 | 1253 |
| F, % | | 36% | | 34% |
| | | | | |
| **Rat PK (N=3)** | | | | |
| Dose, mg/kg | 5 | 10 | 5 | 10 |
| Clearance, mL/min/kg | 9.5 ± 2.3 | NR | 10 ± 1.4 | NR |
| Vss, L/kg | 1.8 ± 0.2 | NR | 1.0 ± 0.1 | NR |
| t_{1/2}, min | 139 ± 24 | 206 ± 12 | 73 ± 5.0 | 350 ± 214 |
| AUC, min*µg/mL | 553 ± 143 | 233 ± 134 | 509 ± 73 | 246 ± 163 |
| Cₘₐₓ, ng/mL | 3672 ± 519 | 999 ± 445 | 4609 ± 55 | 757 ± 520 |
| F, % | | 21% | | 24% |
| | | | | |
| **Dog PK (N=4)** | | | | |
| Dose, mg/kg | 2 | 5 | 2 | 5 |
| Clearance, mL/min/kg | 109 ± 29 | NR | 15 ± 3.2 | NR |
| Vss, L/kg | 94 ± 95 | NR | 0.9 ± 0.2 | NR |
| t_{1/2}, min | 2757 ± 1573 | 1695 ± 439 | 82 ± 15 | 191 ± 9.0 |
| AUC, min*µg/mL | 18.5 ± 4.7 | 23.1 ± 11.3 | 141 ± 30 | 128 ± 154 |
| Cₘₐₓ, ng/mL | 400 ± 118 | 210 ± 133 | 2552 ± 576 | 862 ± 1010 |
| F, % | | 50% | | 36% |

**17ya and 55 inhibit paclitaxel resistant prostate (PC-3/TxR) xenografts growth.** PC-3 (Figure 27A) and paclitaxel-resistant prostate cancer (PC-3/TxR) (Figure 27B) cells were inoculated in nude mice and the tumor volumes were allowed to reach about 150-300 mm³. Docetaxel (10 or 20 mg/kg), which is in clinic for prostate cancer, was used to evaluate its effectiveness in models of P-gp-mediated drug resistance *in vivo.* PC-3/TxR tumor was found to be fast-growing and the volume reached 1500-2500 mm³ at the termination of the study. Though 10 and 20 mg/kg intravenously administered docetaxel exhibited a dose response in both models (**Figures 27A** and **27B**), the tumor growth inhibition (TGI) effect decreased from 84% TGI in PC-3 tumors to 14% TGI in PC-3/TxR tumors when intravenously dosed at 10 mg/kg (**Table 18**). In addition, at the higher dose (20 mg/kg), docetaxel elicited partial regression (>100% TGI) of PC-3 tumors, but barely 56% TGI in PC-3/TxR tumors. The effectiveness of docetaxel in PC-3/TxR tumors was dramatically decreased when compared to that in PC-3 tumors, suggesting that the efficacy was impaired by P-gp-mediated drug resistance, and these results are in very good agreement with our *in vitro* cytotoxicity or apoptosis data. In contrast to the lack of efficacy of docetaxel in PC-3/TxR tumors, orally administered **17ya** (6.7 mg/kg) demonstrated more than 100% TGI without an effect on their body weights (**Figure 27B** and **Table 18**). In addition, 2 out of 4 nude mice bearing PC-3/TxR tumors were tumor free on day 19 (data not shown).

The PC-3/TxR xenograft model was further utilized to evaluate efficacies of **17ya** (in other dosing schedules) and **55**. The maximal tolerated dose (body weight loss > 20%) of **17ya** was found to be 10 mg/kg, when orally dosed once daily for four days; or at 3.3 mg/kg twice a day (b.i.d.) for five days (data not shown). As shown in **Figure 27C**, 3.3 mg/kg of **17ya** was dosed b.i.d. for first consecutive four days in the first week, and the schedule was then changed to once daily between weeks 2 and 4. The result shows that partial regression was obtained during day 4-19, and the TGI was 97%; and one of the seven mice was tumor free on day 26. Higher dose (10 mg/kg) with lower dosing frequency (q2d) of **17ya** (**Figure 27D**) elicited partial regression during days 13 to 29. These data suggest that regimens with optimized doses and dosing schedules will facilitate **17ya** to successfully inhibit PC-3/TxR tumors. **55**, was orally administered to nude mice with 10 or 30 mg/kg b.i.d., and five times a week between weeks 1 and 4. As shown in **Fig 27C**, the inhibition profiles exhibit a dose-response in PC-3/TxR tumor. The TGI value was 59% for the treatment group with a lower dose (10 mg/kg). Moreover, the higher dose (30 mg/kg) started to show partial regression (>100% TGI) from day 19 to the termination of the study (day 26). Some mice in the vehicle group lost body weight at the endpoint, in part, due to cancer cachexia. On the contrary, mice treated with **17ya** (3.3 mg/kg) or **55** (30 mg/kg) were gaining weight (**Table 18**), suggesting that these optimized doses of **17ya** or **55** may be well-tolerated and were preventive of cancer cachexia.

**Table 18. Antitumor activity of compounds 17ya and 55 versus concomitantly evaluated docetaxel in vivo.**

| | **Dosing Schedule** | **End point** | **Number End/Start** | **Body weight (g)** | | **Tumor size (mm³)** | | **TGI (%)** |
|---|---|---|---|---|---|---|---|---|
| | | | | **Start** | **End** | **Start** | **End** | |
| **PC-3 xenograft** | | | | | | | | |
| Vehicle_IV | day 1and 9 | day 19 | 6/6 | 30 ± 2 | 32 ± 4 | 271 ± 83 | 875 ± 292 | - |
| Docetaxel_IV_10mpk | day 1and 9 | day 19 | 5/5 | 29 ± 2 | 24 ± 2 | 247 ± 49 | 341 ± 101 | 84 |
| Docetaxel_IV_20mpk | day land 9 | day 19 | 5/5 | 28 ± 3 | 24 ± 3 | 243 ± 68 | 172 ± 62 | > 100 |
| | | | | | | | | |

| **PC-3/TxR xenograft** | | | | | | | | |
|---|---|---|---|---|---|---|---|---|
| Vehicle_IV | day 1and 9 | day 19 | 5/5 | 33 ± 1 | 26 ± 5 | 171 ± 57 | 2061 ± 858 | - |
| Docetaxel_IV_10mpk | day land 9 | day 19 | 4/4 | 31 ± 2 | 25 ± 2 | 143 ± 20 | 1774 ± 183 | 14 |
| Dooetaxel_IV_20mpk | day land 9 | day 19 | 4/4 | 30 ± 1 | 25 ± 4 | 170 ± 86 | 999 ± 905 | 56 |
| 17ya_PO_6.7mpk | qd × 5/w | day 19 | 4/4 | 33 ± 3 | 34 ± 3 | 172 ± 69 | 126 ± 100 | > 100 |
| | | | | | | | | |
| Vehicle_PO | b.i.d × 5/w | day 26 | 6/7 | 30 ± 2 | 25 ± 2 | 156 ± 30 | 2591 ± 1423 | - |
| 55_PO_10mpk | b.i.d × 5/w | day 26 | 7/7 | 29 ± 2 | 26 ± 3 | 143 ± 44 | 1152 ± 433 | 59 |
| 55_PO_30mpk | b.i.d × 5/w | day 26 | 7/7 | 29 ± 3 | 30 ± 2 | 134 ± 34 | 101 ± 19 | > 100 |
| 17ya_PO_3.3mpk^{e} | qd × 5/w | day 26 | 7/7 | 29 ± 2 | 30 ± 2 | 139 ± 44 | 214 ± 172 | 97 |
| | | | | | | | | |
| Vehicle_PO | q2d × 3/w | day 29 | 5/5 | 24 ± 2 | 21 ± 1 | 299 ± 40 | 1521 ± 580 | - |
| 17ya_PO_10mpk | q2d × 3/w | day 29 | 5/5 | 24 ± 2 | 28 ± 2 | 294 ± 156 | 237 ± 103 | > 100 |

| | | | | | | | | |
|---|---|---|---|---|---|---|---|---|
| Dosing schedule: qd × 5/w = one administration given on five consecutive days per week; b.i.d. × 5/w = two administrations given on five consecutive days per week; or q2d × 3/w = every other day administration or three times a week. ^{a} Dose schedule was two administrations given on four consecutive days of the first week, and dose schedule was changed (because of toxicity) to one administration given on five consecutive days per week for the second to fourth week. | | | | | | | | |

**Brain penetration of 17ya and 55 in nude mice**. Whole brain concentrations in nude mice at 1 h and 4 h after oral administration of 20 mg/kg **17ya** or **55** were determined (**Table 19**). The ratios of brain to plasma concentrations were determined and compared to docetaxel in the nude mice. **55** exhibited greater brain penetration than **17ya** and docetaxel. **17ya** only exhibited slightly greater brain/plasma concentration ratios than docetaxel at both 1 and 4 h. The brain concentrations of **55** reached 14 to 19% of plasma concentrations at 1 h and 4 h, respectively, showing a 3.2-fold higher brain/plasma ratio at both 1 h and 4 h compared to docetaxel. These data suggest that **55** exhibited potentially favorable properties to treat glioma, since it has greater brain penetration and high potency (22 nM, **Table 15**) in glioma cells.

**Table 19. Brain-Blood Barrier (BBB) studies of compounds 17ya and 55. Brain and plasma concentrations were determined in nude mice at 1 and 4 h after administration of docetaxel (IP, 10 mpk), 17ya (PO, 20 mpk), and 55 (PO, 20 mpk). Each value represents the mean ± SD from 3 nude mice.**

| | **Docetaxel** | | **17ya** | | **55** | |
|---|---|---|---|---|---|---|
| **Measurment** | **1hr** | **4hr** | **1hr** | **4hr** | **1hr** | **4hr** |
| Brain (ng/mL) | 33 ± 14 | 20 ± 9 | 124 ± 108 | 49 ± 32 | 180 ± 44 | 73 ± 18 |
| Plasma (ng/mL) | 768 ± 92 | 345 ± 94 | 2058 ± 1252 | 570 ± 438 | 1669 ± 867 | 380 ± 32 |
| Brain/plasma (%) | 4.4 ± 2.0 | 6.0 ± 2.9 | 5.4 ± 1.9 | 8.9 ± 1.7 | 14 ± 7.9 | 19 ± 3.1 |

### EXAMPLE 21

### PHARMACOKINETICS OF COMPOUNDS

**Table 20.**

| Compound ID | Half life in Human liver microsome (min) | Half life in Mouse liver microsome (min) | Half life in Rat liver microsome (min) | Half life in Dog liver microsome (min) | Half life in Monkey liver microsome (min) |
|---|---|---|---|---|---|
| **1h** | 17 | <5 | 31 | 19 | <5 |
| **2e-cis** | 35 | | | | |
| **2i** | 32 | | | | |
| **2k** | 10 | 9 | 32 | 16 | <5 |
| **2l** | 20 | 11 | 49 | 30 | 8 |
| **6a** | 32 | 3.43 | 12 | 13 | 16 |
| **6b** | 40 | 10 | 9 | 30 | 13 |
| **6c** | 47 | 13 | 14 | 29 | 9 |
| **7d** | 24 | 37 | 42 | 29 | 15 |
| **12da** | 23 | 8 | 28 | 17 | |
| **12fa** | 56 | 23 | 46 | 26 | |
| **12fb** | 37 | | | | |
| **12dab** | 21 | <5 | 12 | 46 | |

### EXAMPLE 22

### BIOLOGICAL ACTIVITY OF 4-SUBSTITUTED METHOXYBENZOYL-ARYL THIAZOLE (SMART) COMPOUNDS 1h, 2k, and 2l: ACTIVE MICROTUBULE INHIBITORS

### Materials and Methods

***In vitro* microtubule polymerization assay.** Bovine brain tubulin (0.4 mg) (Cytoskeleton, Denver, CO) was mixed with 10 µM of the test compound or vehicle (DMSO) and incubated in 100 µl of buffer (80 mM PIPES, 2.0 mM MgCl₂, 0.5 mM EGTA, pH 6.9 and 1 mM GTP). The absorbance at 340 nm wavelength was monitored every min for 15 min (SYNERGY 4 Microplate Reader, Bio-Tek Instruments, Winooski, VT). The spectrophotometer was maintained at 37 °C for tubulin polymerization.

**MS competition binding assay.** Colchicine, vinblastine, and paclitaxel (1.2 µM for each) were incubated with tubulin (1.2 mg/mL) in the incubation buffer (80 mM PIPES, 2.0 mM MgCl₂, 0.5 mM EGTA, pH 6.9) at 37 °C for 1 hr. **1h** (0.5-125 µM) was examined to individually compete with colchicine-, vinblastine-, and paclitaxel-tubulin binding. The free-form ligands were separated from tubulin or microtubule using an ultrafiltration method (microconcentrator) (Microcon, Bedford, MA) with a molecular cutoff size of 30k Da. Colchicine, vinblastine and paclitaxel were determined by LCMS/ MS method. The ability of **1h** to inhibit the binding of ligands was expressed as a percentage of control binding in the absence of any competitor. Each reaction was run in triplicate.

**Cell culture and cytotoxicity assay of prostate and melanoma cancer.** All prostate and melanoma cell lines were obtained from ATCC (American Type Culture Collection, Manassas, VA, USA), while cell culture supplies were purchased from Cellgro Mediatech(Herndon, VA, USA). The antiproliferative activity of the compounds was examined in four human prostate cancer cell lines (LNCaP, DU 145, PC-3, and PPC-1) and two human melanoma cell lines (A375 and WM-164). Human ovarian cell line OVCAR-8 and its resistant cell line that over-expresses P-gp, NCI/ADR-RES, were used as MDR models. Both ovarian cell lines were obtained from National Cancer Institutes (NCI). All prostate cancer cell lines were cultured with 10% fetal bovine serum (FBS).

**Cell cycle analysis.** Flow cytometry was performed to study the effects of the compounds on cell cycle distribution. PC-3 and A375 cells were treated in growth media with the indicated concentrations of compounds **1h**, **2k**, **2**l for 24 h. Cellular DNA was stained with 100 µg/mL propidium iodide and 100 µg/mL RNase A in PBS and flow cytometry was performed to determine the cell cycle distribution of the cells.

**Apoptosis detection by ELISA.** Quantification of the enrichment of mono- and oligonucleosomes in the cytoplasm was used to determine the ability of the compounds to induce apoptosis (cell death detection ELISA PLUS, Roche, Germany) following the manufacturer's instructions.

**Pharmacokinetic study.** Male ICR mice (n = 3 or 4 per group) 6 to 8 weeks of age were purchased from Harlan Inc., and used to examine the pharmacokinetics (PK) of the compounds. **1h**, **2k**, **2l** (15 mg/kg) were dissolved in PEG300/DMSO (1/4) and administered by a single i.v. injection into the tail vein. Blood samples were collected at 2, 5, 15, and 30 min, 1, 2, 4, 8, 16, and 24 hr after administration. Male Sprague-Dawley rats (n = 4; 254_{.} ± 4 g) were purchased from Harlan Inc. (Indianapolis, IN). **1h**, **2k**, were administered intravenously into the jugular venous catheters at 2.5 mg/kg (in DMSO/PEG300, 1/4). Blood samples (250 µL) were collected at 10, 20, 30 min, and 1, 2, 4, 8, 12, 24, 48 h. A protein precipitation method was used for sample preparation. An aliquot (200 µL) of acetonitrile (ACN) was added to 100 µL of plasma and then was thoroughly vortexed for 15 s. After centrifugation, the supernatant was analyzed by liquid chromatography tandem mass spectrometry (LC-MS/MS). The PK parameters were determined using Non compartment analysis (WinNonlin, Pharsight Corporation, Mountain View, CA).

**PC-3 and A375 tumor xenograft studies.** PC-3 and A375 cells (5×10⁷ per mL) were prepared in phenol red-free growth media containing 10% FBS, and mixed with Matrigel (BD Biosciences, San Jose, CA) at 1:1 ratio. Tumors were established by injecting 100 µL of the mixture (2.5×10⁶ cells per animal) subcutaneously (s.c.) into the flank of 6-8- week-old male athymic nude mice. Length and width of tumors were measured and the tumor volume (mm³) was calculated by the formula, π/6 ×L ×W², where length (L) and width (W) were determined in mm. When the tumor volumes reached 150 mm³, the animals bearing PC-3 tumors were treated with vehicle [Captex200/Tween80 (1/4)], **1h** (5 and 15 mg/kg), **2k** (5 and 15 mg/kg) and **21** (50 mg/kg) intraperitorally for 21 days. Vinblastine (0.5 mg/kg) was used as the positive control and dosed q2d with vehicle [DMSO/PEG300 (1/9)]. On the other hand, A375 tumor bearing mice were treated for 34 days with vehicle [Captex200/Tween80 (1/4)], **1h** (20 mg/kg) or **2k** (15 mg/kg). Doses were selected based on acute toxicity studies of **1h** and **2k** in ICR mice (n = 2/group) showing that doses up to 30 mg/kg and 15 mg/kg, respectively, did not cause greater than 10% loss of body weight after 4 consecutive days of intraperitoneal dosing.

***In vivo* antitumor activity [tumor growth inhibition** (% **T/C), tumor growth delay (T-C value), and tumor cell kill (total log cell kill)].** Evidence of drug effect is described by the following parameters: % T/C = [Δ tumor volume of treated group] / [Δ tumor volume of control group] × 100%. The T-C values (tumor growth delay) were based on the median time (in days), required for the treatment (T) and the control group (C) tumors, to reach a predetermined size (600 mm³ in this study). These values were then used for the quantitation of the tumor cell kill following the equation: log cell kill = (T-C) / (3.32 xTd). Td is the tumor volume-doubling time in days. In this study, we defined the doubling time required for the tumor to increase from 300 to 600 mm³.

**Rotarod test.** ICR mice received training three times a day for two days to enable them to stay on the rotating rod for > 120 seconds at 12 rpm. Mice were then randomized by the length of time that they could stay on the rotating rod and divided into 7-8 mice per group. **1h** at a dose of 5 or 15 mg/kg in Captex200/Tween80 (1/4) was administered by intraperitoneal injection. Vinblastine at a dose of 0.5 mg/kg/day was used as a positive control under the same conditions. The rotarod test was performed twice a week. Treatment was stopped on day 31, and post observation was examined on weeks 1, 2, and 4 after termination of the treatment. The rod speed was increased from 59 rpm to 40 rpm over a period of 5 min. Performance was measured as the length of time that a mouse could stay on the rotating rod.

***In vivo* drug resistance studies.** At the end of the PC-3 xenograft studies, solid tumors from control and **1h** treated (15 mg/kg) groups were removed and digested with 0.1% collagenase (Type I) and 50 mg/mL DNAse (Worthington Biochemical Corp., Freehold, NJ). Dispersed cells were plated in RPMI medium + 10% FBS and incubated at 37°C and 5% CO₂ for 24 hr to allow attachment. The antiproliferative effects of **1h** were compared to determine whether tumor cells remaining in PC-3 xenografts retained sensitivity to drug. The PC-3 cells obtained from ATCC were used as *in vitro* control. Statistical analyses were performed using simple t-Test.

### Results

Based on structure-activity relationship studies, three compounds (**Figure 28A**) were selected for biological characterization. While **1h** and **2k** are highly potent molecules with low nanomolar cytotoxic properties, **2l**, which was rationally designed as a potential metabolite with improved solubility, had the least potent antiproliferative effects (**Table 21**).

**Table 21. In vitro efficacy of compounds on prostate, melanoma and drug resistant cell lines (n = 3, mean ±SE). Paciltaxel, vinblastine, and colchicine were used as positive controls as previously reported.**

| | | IC**₅₀ ± SEM (nM)** | | | | | |
|---|---|---|---|---|---|---|---|
| **Cell line** | **Cell type** | **SMART-H** | **SMART-F** | **SMART-OH** | **Paclitaxel** | **Vinblastine** | **Colchicine** |
| LNCaP | Prostate | 28±4^{a} | 6±1^{a} | 103±9 | 1.7±0.2 | 1.1±0.1 | 16±4 |
| PC-3 | Prostate | 21±1^{a} | 13±1^{a} | 87±5 | 4.8±0.3 | 2.1±0.2 | 11±1 |
| Dn-145 | Prostate | 71±4^{a} | 12±1^{a} | 116± 14 | 5.1±0.1 | 1.8±1.1 | 10±2 |
| PPC-1 | Prostate | 43±5^{a} | 8±1^{a} | 76±2 | 2.3±0.8 | 1.1±0.4 | 20±1 |
| B16-F1 | Melanoma | 55±5^{a} | 43±21^{a} | 113±6 | 17±2 | 4.7±0.7 | 29±5 |
| A375 | Melanoma | 28±5^{a} | 33±14^{a} | 93±11 | 12±3 | 1.1±0.2 | 20±3 |
| OVCAR-8 | Ovarian | 35±2 | 34±3 | 110±8 | 4.7±0.1 | 3.9±0.1 | 17±1 |
| NCI/ADR-RES | Ovarian | 13 ± 1 | 12±1 | 45±5 | 6263±634 | 582±57 | 1113±79 |
| Resistance Factor | | 0.4 | 0.4 | 0.4 | 1333 | 149 | 65 |

| | | | | | | | |
|---|---|---|---|---|---|---|---|
| SMART-H in Table 21 is **1h**; SMART-F in Table 21 is **2k**; and SMART-OH in Table 21 is **2l**. | | | | | | | |

### SMARTs inhibit microtubule polymerization by binding to the colchicine binding site on tubulin.

Bovine brain tubulin (>97% pure) was incubated with the individual compounds (10 µM) to test their effect on tubulin polymerization (**Figure 28B**). While **1h** and **2k** inhibited tubulin polymerization by 90%, **2l** inhibited the polymerization by only 55%. Previous studies demonstrated a concentration-dependent inhibition of tubulin polymerization by **1h**. In addition, under the same experimental conditions, the IC₅₀ for **1h** (4.23 µM) is similar to that of colchicine (4.91 µM). These data suggest that the compounds exhibit strong antitubulin polymerization activity that corresponds well with their cytotoxicity (**Table 21).** The ability of the compounds to compete for known binding sites on tubulin was determined using a novel MS competitive binding assay, which was developed in our laboratory. Three tubulin ligands, corresponding to the three binding sites on tubulin, colchicine, vinblastine, and paclitaxel were used for these competitive binding studies. It was found that, over a concentration range of 0.1-125 µM, **1h** specifically competed with colchicine binding to tubulin, but it did not compete with either vinblastine or paclitaxel binding to tubulin (**Figure 28C**).

### SMART compounds inhibit the growth of multidrug-resistant cancer cell lines.

The ability of the compounds to inhibit the growth of cancer cell lines was evaluated using the SRB assay. As shown in **Table 21,** the compounds inhibited the growth of several human cancer cell lines, including four prostate cancer cell lines, and two melanoma cell lines, with IC₅₀ values in the low nanomolar range. Out of the three compounds, **2l** was the least potent (IC₅₀ 76∼116 nM). **2k** exhibited the best antiproliferative effect with IC₅₀ values between 6 and 43 nM in prostate cancer and melanoma cell lines. In addition, the effect of the compounds in the OVCAR-8 and NCI/ADR-RES cell lines was also evaluated (**Table 21**). The compounds were equally potent against MDR cell (NCI-ADR-RES) and the parent cell line (OVCAR-8). Paclitaxel, vinblastine, and colchicine exhibited relative resistance values of 1333, 149, and 65 times, respectively (**Table 21**). These data indicate that the compounds circumvent P-gp-mediated drug resistance.

**SMART compounds arrest PC-3 (prostate) and A375 (melanoma) cells in G2/M phase of cell cycle and induce cell apoptosis**. PC-3 and A375 cells were exposed to 10, 50, 200, and 1000 nM of the compounds for 24 h. Treatment with the SMART compounds resulted in concentration-dependent accumulation of both PC-3 and A375 cells in the G2/M phase with concomitant decreases in the percentage of cells in G0/G1 phase (**Figures 29A and 29B**). The proportion of cells in G2/M phase significantly increased when treated with 50 to 200 nM of **1h**, **2k**, **2l**. Apoptosis was then examined by measuring the level of cytoplasmic DNA-histone complexes in PC-3 and A375 cells after 24 h treatment. Increasing concentration of the SMART compounds increased the level of cytoplasmic DNA-histone complexes in PC-3 and A375 cells (**Figure 29C**). The effect was more pronounced in A375 cells than PC-3 cells, but apoptosis was evident in both cell types. **1h** and **2k** induced moderate apoptosis at a concentration of 50 nM, while **2l** induced apoptosis only at concentrations greater than or equal to 200 nM.

***In vivo* PK profile of SMART compounds.** A single dose bolus of each compound (15 mg/kg) was administered by tail vein injection to ICR mice to characterize their pharmacokinetics (**Figure 30A**). **1h** and **2k** exhibited similar PK properties, but **2l** exhibited slightly greater AUC than **1h** and **2k** indicative of a lower clearance for **2l (Table 22). 2l** also had 2-3 times higher Vₛₛ than that of **1h** and **2k**. The clearance values for all three compounds were equal to or higher than 90 mL/min/kg, the hepatic blood flow rate in mice, suggesting that in addition to hepatic removal, other degradation routes may be involved in the elimination of the compounds. The pharmacokinetics of **1h** and **2k** (2.5 mg/kg) were also examined in rats (**Figure 30B**). Interestingly, low clearance values and hepatic extraction rates were obtained by both compounds, suggesting that these compounds exhibit species differences in clearance. In rats, **1h** exhibited favorable pharmacokinetic properties, which are low clearance (6 mL/min/kg), moderate volume of distribution (7.6 L/kg), long half-life (24 hr), and high exposure (AUC, 5.8 hr*µg/mL) (**Table 22**) when administered iv.

**Table 22. Pharmacokinetic parameters of SMART compounds. SMARTs were administrated 15 mg/kg and 2.5 mg/kg i.v. in mice and rats, respectively.**

| ***In vivo*, pharmacokinetic parameters of SMART compounds** | | | | | |
|---|---|---|---|---|---|
| **Species** | **Parameter** | **Unit** | **SMART-H** | **SMART-F** | **SMART-OH** |
| **Mice** | **AUC** | **lu· * µg/mL** | **1.9** | **2.2** | **2.6** |
| | **t_{1/2}** | **min** | **140** | **141** | **740** |
| | **Vₛₛ** | **L/kg** | **4.9** | **6.6** | **16.5** |
| | **CL** | **mL/min/kg** | **130** | **1 12** | **90** |
| **Rats** | **AUC** | **lu· * (µg/mL** | **5.8** | **1.6** | **NA** |
| | **t_{1/2}** | **min** | **1431** | **2410** | **NA** |
| | **Vₛₛ** | **L/kg** | **7.6** | **34** | **NA** |
| | **CL** | **mL/min/kg** | **6** | **11** | **NA** |

| | | | | | |
|---|---|---|---|---|---|
| **NA, not available** SMART-H in Table 22 is **1h**; SMART-F in Table 22 is **2k;** and SMART-OH in Table 22 is **21.** | | | | | |

**SMART compounds inhibit prostate and melanoma xenografts growth without neurotoxicity.** Prostate cancer PC-3 and melanoma A375 tumors in mice were allowed to reach a volume of 150 mm³ and then tumor-bearing mice were treated with the SMART compounds. As shown in **Figure 31A****,** tumor volumes in the control group increased to 680 mm³ over the 21 day duration of the study. Tumor volumes in the **1h** treated group increased to 370 mm³ (5 mg/kg treatment) and 176 mm³ (15 mg/kg treatment) by day 21, indicating strong antitumor activity for this compound. Tumors in the **2k**-treated animals increased to 269 mm³ (5 mg/kg treatment) and 292 mm³ (15 mg/kg treatment), while animals in the **2l** (50 mg/kg) treated group had tumors of 331 mm³ at day 21. This reduction in tumor volume reversed upon withdrawal of SMART compounds (data not shown). **Table 23** summarized the *in vivo* efficacy (%T/C, T-C values, and log cell kill) of SMART compounds.

**Table 23. In vivo efficacy of SMART compounds (administered i.p.) on prostate (PC-3), melanoma (A375). %T/C, T-C value, and log cell kill are summarized. The doubling time of melanoma xenograft was 4.6 d. Vinblastine was used as the positive control. % T/C ≤ 42% is considered to be moderately active by National Cancer Institute criteria. NA, not available.**

| **Compound** | **Dosage (mg/kg)** | **Xenograft model** | **% T/C** | **Median time to reach 600 mm³.** | **T-C (days)** | **Total log cell kill** |
|---|---|---|---|---|---|---|
| Vehicle | NA | Prostate | 100 | 19 days | NA | NA |
| Vinblastine | 0.5 | Prostate | 29 | N.A | NA | NA |
| SMART-H | 5 | Prostate | 29 | NA | NA | NA |
| SMART-H | 15 | Prostate | 4 | NA | NA | NA |
| SMART-F | 5 | Prostate | 21 | NA | NA | NA |
| SMART-F | 15 | Prostate | 24 | NA | NA | NA |
| SMART-OH | 50 | Prostate | 34 | NA | NA | NA |
| Vehicle | NA | Melanoma | 100 | 18 days | NA | NA |
| SMART-H | 20 | Melanoma | 30 | 28 days | 10 | 0.7 |
| SMART-F | 15 | Melanoma | 28 | 29 days | 11 | 0. 7 |

| | | | | | | |
|---|---|---|---|---|---|---|
| SMART-H in Table 23 is **1h**; SMART-F in Table 23 is **2k;** and SMART-OH in Table 23 is **2l**. | | | | | | |

**1h** tumor elicited %T/C = 29% and 4% at 5 and 15 mg/kg treatment (all doses were intraperitoneal (i.p.)), respectively, whereas, **2k** elicited % T/C of 21% and 24% at 5 and 15 mg/kg treatment, respectively. The high dose of **2l** (50 mg/kg) exhibited the %T/C of 34%. Vinblastine, the positive control, showed %T/C of 29% at day 22 in PC-3 xenografts (**Figure 31B**). Body weight measurements, to monitor toxicity, indicated that only 1 of 8 mice treated with **1h** (15 mg/kg), and 2 out of 7 mice treated with **2k** (15 mg/kg) lost more than 15% body weight. In addition to the antitumor effects of the compounds on PC-3 prostate tumors, **1h** (20 mg/kg) and **2k** (15 mg/kg) demonstrated a significant reduction of A375 tumors. As shown in **Figure 31C****,** the tumor volumes of control group increased to 2183 mm³, whereas the 14 volumes in **1h** and **2k** treatment groups increased to 775 mm³ and 722 mm³, respectively. **1h** and **2k** treatment evoked %T/C of 28% and 29%, respectively. Rotarod tests were performed to examine the *in vivo* neurotoxic effects of **1h**. Based on the result of *in vivo* efficacy experiments, 5 or 15 mg/kg [i.p. administration, Captex200/Tween80 (1/4)] of **1h** was chosen to study the effect on motor coordination. A 0.5 mg/kg treatment with vinblastine was used as the positive control under the same conditions. As shown in **Figure 31D****,** vinblastine gradually reduced the time (in seconds) that the mice could stay on the rotating rod, and attained significance by days 27 and 31 (*p* < 0.05) compared to the vehicle group. However, no significant difference was observed in the **1h** treatment groups, suggesting that **1h** did not cause neurotoxicity in ICR mice at doses that are associated with antitumor effects.

**1h did not develop drug-resistance in PC-3 tumor bearing mice.** We excised the PC-3 tumors from nude mice after 21 days of treatment with vehicle (n = 3) or 15 mg/kg **1h** (n = 3). Solid tumors were digested and dispersed into cells as described in the methods section. PC-3 cell line from ATCC (American Type Culture Collection, Manassas, VA, USA) was used as a control. IC₅₀ values were 29.1 ± 1.1, 29.1 ± 0.8, and 30.4 ± 0.5 nM in PC-3 cells from ATCC, and dissociated cells from vehicle and **1h** treated tumors, respectively. These data demonstrate that **1h** did not induce drug-resistance in PC-3 tumors after 21 days of continuous **1h** treatment.

### EXAMPLE 23

### MOLECULAR MODELING

### Methods

All molecular modeling studies were performed with Schrödinger Molecular Modeling Suite 2008 (Schrödinger LLC, New York, NY), running on a Dell Linux workstation. Because the size of ABI compounds are much closer to that of ABT-751, rather than DAMA-colchichine, we selected tubulin complex with ABT-751 (PDB code: 3KHC) as our modeling system. ABIs were built and prepared using the Ligprep module, and they were docked into the ABT-751 site using the Glide module in Schrödinger Suite. The best docking complexes were subject to restricted molecular dynamics to release any strains using Macromodel module with OPLS-2005 forcefield. The ligand and its surrounding residues within 15 Ǻ were allowed to move freely, while residues outside the 15 Ǻ radius were kept rigid.

### Results

Molecular modeling for binding ABI compounds in tubulin was studied. Several crystal structures of the ligand-tubulin complex are available in the PDB databank, with the most recent one from Dorleans *et al*. In general, the colchicine binding pocket tolerates a variety of molecular structures, which may indicate substantial conformation changes upon ligand binding. In fact, Dorleans *et al*. solved the crystal structures of both the empty tubulin dimer and the ligand-tubulin complex. They found that, without the presence of ligand, loop 7 (T7, residues 244-251, **Figure 32**) in the beta-monomer folds in to occupy the binding pocket, while it flips out upon ligand binding. The associated helix 7 (H7, residues 224-243) and helix 8 (H8, residues 252-260) were displaced upon ligand binding. It is conceivable that the extent to which T7 is displaced depends on the size of individual ligand. This flexibility presents a significant challenge to understand the precise binding modes for individual ligands without solving actual crystal structures. Nevertheless, careful analysis of the possible binding modes could provide some insights into the binding of different ligands.

The binding modes of **12cb** and **llcb** (stick model) are shown in **Figure 32A** and **32B****.** For comparison, the crystal structure complexes of ABT-751 and DAMA-colchicine (wire models) along with ABI-**12cb**/tubulin complex in **Figure 32A** is displayed. For clarity, only the related secondary structures forming the binding pocket in P-tubulin are shown in **Figure 32A****.** The overall structures of **12cb**, ABT-751 and DAMA-colchicine overlapped very well in the binding pocket. Several potential hydrogen bonding interactions between compound **12cb** and tubulin were identified. The carbonyl group in **12cb** was in sufficient proximity to form two hydrogen bond interactions with the backbone NH of Leu-252 in H8 and the sidechain of Asp-251 in T7 of the tubulin β-monomer. The *para*-fluorine substituent in the C-ring was close to the sidechain of Cys241 in T7 and Tyr202 in S6, possibly forming one or two hydrogen bonds. The imidazole proton is very close and likely to form a hydrogen bond to Thr179 in T5 loop (residues 173-182) of the tubulin α-monomer (**Figure 32A**). Together with the hydrophobic interactions provided by the aromatic rings, the likely formation of these hydrogen bonds would contribute to the high binding affinity to the tubulin dimer, resulting in high antiproliferative potency.

The binding mode of **llcb** will be conceivably less defined since two of the three aromatic rings may occupy the binding pocket in the β-monomer while the third ring may extend toward the interface of the α/β-monomers, similar to how the sidechain of DAMA-colchicine binds. Our modeling indicates that the protecting group likely extends to the tubulin dimer interface, while the A, C rings of **llcb** occupy similar binding pocket and orientation as **12cb** (**Figure 32B**). This may explain the similar activity between the two compounds, even though **llcb** has an extra ring system. From the molecular modeling studies presented in **Figures 32A** and **32B****,** the hydrogen bond donor is likely to be the thiol group in Cys-241 in loop 7 of the β-subuint in α/β-tubulin dimer.

The binding mode of ABI **12fb** was modeled (not shown) and compared to DAMA-colchicine (see Figure 19 for structure of colchicine) in the α/β-tubulin heterodimer. The overall structure of **12fb** and DAMA-cochicine overlapped very well. The *p*-fluoro phenyl moiety overlaps with the trimethoxylpheny moiety which is interacting with the T7 loop in the β-subunit. Similarly, the *p*-chloro phenyl moiety occupies the other side of the pocket where the seven-member ring of the DAMA-cochicine is, with the chlorine atom occupying the pocket where the methoxy moiety interacts.

### EXAMPLE 24

### MICROTOBULE IMAGING

### Materials and Methods

Cellomics Cytoskeleton rearrangement kit (Thermo Scientific, Rockford, IL) was used to get a visually appreciable proof of ABIs interacting with tubulin inside the cells. WM-164 melanoma cells were treated with each compound for 18 h in duplicate using a collagen-coated 96-well plate (Becton Dickinson Labware, Bedford, MA). Then cells were fixed with 4% paraformaldehyde (Thermo Scientific, Rockford, IL) and permeabilized using permeabilization buffer supply from the kit. Primary antibody for tubulin and fluorescence-labeled secondary antibody were subsequently added to the cells. Cell nuclei were stained by DAPI. Whole Cell Stain Green was also applied to all cells. All images were acquired with an Olympus IX71 inverted fluorescence microscope (Olympus Corp., Tokyo, Japan) with overlays from separate images of tubulin (red), nuclei (blue), and whole cells (green). For comparison, paclitaxel, colchicine and ABT-751, along with ABIs are included.

### Results

Visual proof of ABIs interacting with tubulin inside the cells was examined. The mictotubule arrangement in human melanoma WM-164 cells upon treatment with different compounds is presented in **Figure 33**. The microtubule images clearly showed that all five tested compounds resulted in cytoskeleton rearrangement. There was a significant difference between paclitaxel and the other four compounds (colchicine, ABT-751, **12cb**, and **12da**). Treatment with paclitaxel resulted in a condensation of microtubules orderly lying around the nuclei compared with controls, consistent with its mechanisms of action for stabilizing microtubules. On the contrary, treatment with colchicine, ABT-751, **12cb,** and **12da** had similar effects on microtubules and resulted in some degree of microtubule fragmentation, consistent with their common mechanism of action for destabilizing microtubules. These results also confirmed that ABIs shared the same cellular target with colchicine and induced the same cellular effect.

### EXAMPLE 25

### VASCULAR DISRUPTING ACTIVITY OF COMPOUNDS 17ya AND 55

### Method

**Cells.** HUVECs (Human Umbilical Vein Endothelial Cells) were cultured and grown in EGM-2 BulletKit (Lonza, Cat No. CC-3162), which contains growth supplements including hydrocortisone, human fibroblast growth factor-basic with heparin (hFGF-B), vascular endothelial growth factor (VEGF), R3-insulin-like growth factor 1 (IGF-1), ascorbic acid, heparin, fetal bovine serum, human epidermal growth factor (hEGF), and GA-1000 (gentamicin and amphotericin B) in Endothelial Cell Basal Medium-2. Cells between the third and fifth passages were used for experiments. PC-3 human prostate cancer cells and T47D human breast cancer cells were cultured in RPMI-1640 medium with 5% fetal bovine serum.

**Cell growth inhibition studies.** Cytotoxic or antiproliferative activity of test compounds was investigated in several cell lines using the sulforhodamine B (SRB) assay. Cultured cells were plated into 96-well plates and incubated in medium containing different concentrations of the test compounds for 24 h or 48 h. Cells were stained with sulphorhodamine B (SRB) solution. The optical density was determined at 540 nm on a microplate reader (Dynex Technologies, Chantilly, VA). Plots of percent inhibition of cell growth versus drug concentration were constructed, and the concentration that inhibited cell growth by 50% relative to the vehicle control (IC₅₀) was determined by nonlinear least squares regression using WinNonlin software (Pharsight Corporation, Cary, NC).

**Capillary formation and disruption assays.** Capillary formation assays were performed in 96-well plates by plating 12,000 cells/well of HUVECs on a Matrigel layer (BD Biosciences). In order to evaluate the anti-capillary action, capillaries were allowed to form over a 16 h period before the addition of test compound or vehicle-control. In addition, capillary formation inhibitory effect of test compound was investigated by treating HUVEC cells with test compounds before capillary formation. Images were acquired immediately following compound addition, 5, 10, 15, and 25 h after exposure to test compound. Capillary formation was quantified by counting the number of tubes and nodes having at least three edges.

**Endothelial monolayer permeability assay.** The permeability of an endothelial cell monolayer was assessed in the transwell system. HUVECs were plated at 2 × 10⁶ cells per insert of 24 well plate in EGM-2 medium and incubated for 72 h to reach 100% confluency. Test compounds were diluted in EGM-2 medium and added to the upper chamber of the apparatus. Following 1, 2, and 4 h of incubation, the compounds were removed and 75 µg/mL FITC-conjugated dextran (MW 40,000) was added for 5 minutes. Fluorescent measurements of the lower chamber were taken after excitation at 485 nm and emission was measured at 520 nm using a BioTek Synergy 4 Microplate Reader.

### Result

**17ya and 55 exhibited high antiproliferative activity against endothelial cells. 17ya** and **55** were evaluated for cytotoxic activity against growth factor-supplemented endothelial cells and growth factor-deprived endothelial cell cultures. Combretastatin A-4 (CA4) and doxorubicin were used as positive and negative control, respectively. Compound **17ya** exhibited higher potency than compound **55** against actively proliferating endothelial cells **(Table 24** and **Figure 35**). Both **17ya** and **55** exhibited selectivity for endothelial cells showing lower IC₅₀ values compared to one of the prostate cancer cells. CA4, **17ya** and **55** were 8, 5 and 3 times more active against endothelial cells than against cancer cells, respectively, while doxorubicin was not specific to endothelial cells **(Table 24** and **Figure 35****).** However no selectivity was observed between quiescent and active endothelial cells with these compounds (data not shown).

**Table 24. Endothelial cell growth inhibition of 17ya and 55. N=3**

| | CA4 | Doxorubicin | **17ya** | **55** |
|---|---|---|---|---|
| | 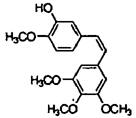 | 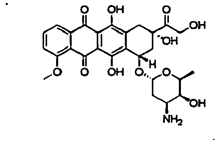 | 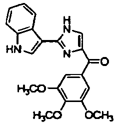 | 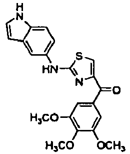 |
| PC3 | 3.2 | 397.0 | 7.8 | 23.3 |
| T47D | 6.0 | 352.8 | 18.0 | 37.4 |
| HUVEC | 1.2 | 273.6 | 2.8 | 9.7 |
| Selectivity ratio*, cancer cells/ HUVEC | 7.6 | 1.4 | 4.6 | 3.1 |

| | | | | |
|---|---|---|---|---|
| * To obtain the selectivity ratio between cancer cells and HUVEC cells, the mean IC₅₀ (nM) values of test compounds in PC3 and T47D cells were used. | | | | |

**17ya disrupts the formation of endothelial capillaries but does not disrupt preformed capillaries.** The activity of **17ya** was investigated on endothelial cells engaged in capillary tube formation *in vitro.* Endothelial cells were placed on a Matrigel matrix and the formation and construction of capillary tubes in the presence or absence of compounds were observed (CA4, doxorubicin, and **17ya**).

To avoid confusion between early stage of tube formation and disruption of tube construction, HUVEC cells on matrix in the presence of drug treatment were incubated for 15 h. Then disruption of capillary was determined by counting the number of tubes and nodes in each treatment group. On the other hand, to evaluate the effect of test compound in preformed capillaries, HUVEC cells on matrix were allowed to form capillary tube for 16 h and the capillaries were treated with test compounds.

As a result, the number of tubes and nodes was gradually decreased over time due to deficiency or consumption of nutrient by HUVEC cells (**Figure 36**). This trend was observed in every drug treatment group (**Figure 36**). In order to examine the difference between untreated and pretreated capillaries 15 h incubation groups were compared (Figure 36).

**17ya and 55 increased the permeability of endothelial cell monolayers.** Antitubulin agents could modify the integrity of endothelial cell layers lining blood vessels by targeting cytoskeleton of the endothelial cells. Thus, the vascular disruption effect of antitubulin agent is known to increase the permeability of blood vessel and thus could lead to protein leakage and high blood viscosity. This could result in reduction of blood flow, causing subsequent tumor death from hypoxia and nutrient deprivation.

The effect of **17ya** and **55** was evaluated on vascular permeability using *in vitro* study using transwell system with confluent HUVEC monolayers. The change in permeability by test compound was measured by the leakage of dextran (MW 40,000) after 1,2, and 4 h of drug treatment. CA4 was used as a positive control. CA4, **17ya,** and **55** resulted in increased permeability and the effect was more pronounced at 1 h incubation (data was not shown). **17ya** showed a potency similar to CA4 (**Figure 38**). Doxorubicin did not induce any change in the permeability of endothelial cell monolayer (**Figure 38**).

## Claims

1. A compound represented by the structure of formula **XI**: 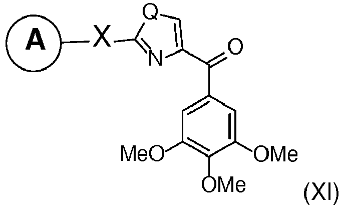 wherein
**X** is NH;
**Q** is S; and
**A** is a substituted or unsubstituted phenyl or indolyl ring;
wherein said A ring is optionally substituted by 1-5 substituents which are independently O-alkyl, O-haloalkyl, F, Cl, Br, I, haloalkyl, CF₃, CN, -CH₂CN, NH2, hydroxyl, -(CH₂)ᵢNHCH₃, -(CH₂)ᵢNH₂, -(CH₂)ᵢN(CH₃)₂, -OC(O)CF₃, C₁-C₅ linear or branched alkyl, haloalkyl, alkylamino, aminoalkyl, -OCH2Ph, -NHCO-alkyl, COOH, - C(O)Ph, C(O)O-alkyl, C(O)H, -C(O)NH₂ or NO₂; and
i is an integer between 0-5.

2. The compound of claim 1, wherein said compound is represented by the structure of formula **VIII:** 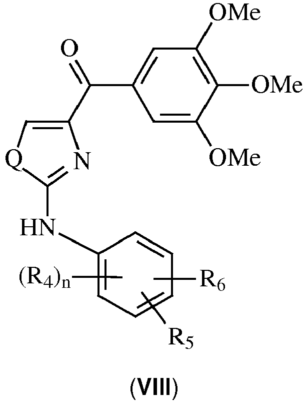 wherein
**R**₄, **R**₅ and **R**₆ are independently hydrogen, O-alkyl, O-haloalkyl, F, Cl, Br, I, haloalkyl, CF₃, CN, -CH₂CN, NH2, hydroxyl, -(CH₂)ᵢNHCH₃, -(CH₂)ᵢNH₂, - (CH₂)ᵢN(CH₃)₂, -OC(O)CF₃, C₁-C₅ linear or branched alkyl, haloalkyl, alkylamino, aminoalkyl, -OCH2Ph, -NHCO-alkyl, COOH, -C(O)Ph, C(O)O-alkyl, C(O)H, -C(O)NH₂ or NO₂;
Q is S;
**i** is an integer between 0-5; and
**n** is an integer between 1-3.

3. The compound of claim 1, wherein said compound is represented by the structure of formula **XI(c)**: 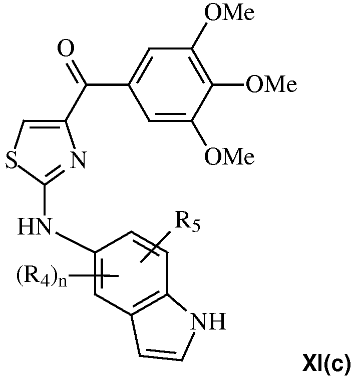 wherein
**R**₄ and **R**₅ are independently hydrogen, O-alkyl, O-haloalkyl, F, Cl, Br, I, haloalkyl, CF₃, CN, -CH₂CN, NH₂, hydroxyl, -(CH₂)ᵢNHCH₃, -(CH₂)ᵢNH₂, -(CH₂)ᵢN(CH₃)₂, - OC(O)CF₃, C₁-C₅ linear or branched alkyl, haloalkyl, alkylamino, aminoalkyl, - OCH₂Ph, -NHCO-alkyl, COOH, -C(O)Ph, C(O)O-alkyl, C(O)H, -C(O)NH₂ or NO₂;
**i** is an integer from 0-5; and
**n** is an integer between 1-4.

4. The compound of claim 3 wherein said compound is compound **55**, represented by the structure:

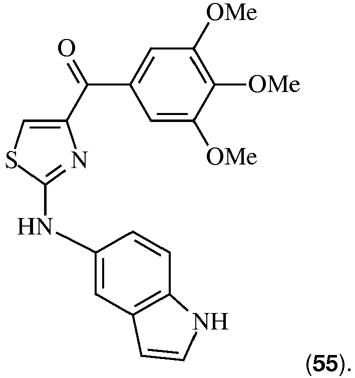

5. The compound of claim 2, wherein said compound is (2-(phenylamino)thiazol-4-yl)(3,4,5-trimethoxyphenyl)methanone (**5a**), (2-(p-tolylamino)thiazol-4-yl)(3,4,5-trimethoxyphenyl)methanone (**5b**), (2-(*p*-fluorophenylamino)thiazol-4-yl)(3,4,5-trimethoxyphenyl)methanone (**5c**), or (2-(4-chlorophenylamino)thiazol-4-yl)(3,4,5-trimethoxyphenyl)methanone (**5d**).

6. The compound according to any of claims 1-5, or its pharmaceutically acceptable salt, pharmaceutical product, tautomer, hydrate, *N*-oxide, or combinations thereof.

7. A pharmaceutical composition comprising a compound according to claim 6 and a pharmaceutically acceptable carrier.

8. A compound according to any of claims 1-6 for use in treating, suppressing, reducing the severity, reducing the risk, or inhibiting cancer in a subject, wherein said cancer is selected from the group consisting of prostate cancer, breast cancer, ovarian cancer, skin cancer, melanoma cancer, metastatic melanoma cancer, lung cancer, colon cancer, leukemia, renal cancer, CNS cancer, and combinations thereof.

9. A compound according to any of claims 1-6 for use in treating a drug resistant tumor or tumors in a subject, wherein said tumor is selected from the group consisting of melanoma cancer tumor, metastatic melanoma cancer tumor, prostate cancer tumor and ovarian cancer tumor, and combinations thereof.

10. The compound for use according to claim 8 or claim 9 wherein said compound is administered in combination with another cancer therapy.

11. A compound according to any of claims 1-6 for use in destroying a cancerous cell *in vivo.*

12. An *ex vivo* method of destroying a cancerous cell comprising providing a compound according to any of claims 1-6 and contacting the cancerous cell with the compound under conditions effective to kill the cancer cell.

## Patentansprüche

1. Verbindung, die durch die Strukturformel **XI** repräsentiert wird: 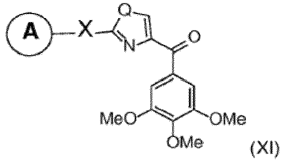 wobei
**X** NH ist;
**Q** S ist; und
**A** ein substituierter oder nicht substituierter Phenyl- oder Indolylring ist;
wobei der A-Ring wahlweise mit 1-5 Substituenten substituiert ist, bei denen es sich unabhängig voneinander um O-Alkyl, O-Haloalkyl, F, Cl, Br, I, Haloalkyl, CF₃, CN, -CH₂CN, NH₂, Hydroxyl, - (CH₂)ᵢNHCH₃, -(CH₂)ᵢNH₂, -(CH₂)ᵢN(CH₃)₂, -OC(O)CF₃, C₁-C₅ lineares oder verzweigtes Alkyl, Haloalkyl, Alkylamino, Aminoalkyl, -OCH₂Ph, -NHCO-Alkyl, COOH, -C(O)Ph, C(O)O-Alkyl, C(O)H, -C(O)NH₂ oder NO₂ handelt; und
**i** eine ganze Zahl zwischen 0 und 5 ist.

2. Verbindung nach Anspruch 1, wobei die Verbindung durch die Strukturformel **VIII** repräsentiert wird: 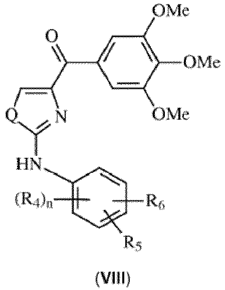 wobei
**R**₄, **R**₅ und **R**₆ unabhängig voneinander für Wasserstoff, O-Alkyl, O-Haloalkyl, F, Cl, Br, I, Haloalkyl, CF₃, CN, -CH₂CN, NH₂, Hydroxyl, -(CH₂)ᵢNHCH₃, -(CH₂)ᵢNH₂, -(CH₂)ᵢN(CH₃)₂, - OC(O)CF₃, C₁-C₅ lineares oder verzweigtes Alkyl, Haloalkyl, Alkylamino, Aminoalkyl, -OCH₂Ph, - NHCO-Alkyl, COOH, -C(O)Ph, C(O)O-Alkyl, C(O)H, -C(O)NH₂ oder NO₂ stehen;
**Q** S ist;
**i** eine ganze Zahl zwischen 0 und 5 ist; und
n eine ganze Zahl zwischen 1 und 3 ist.

3. Verbindung nach Anspruch 1, wobei die Verbindung durch die Strukturformel **XI**(**c**) repräsentiert wird: 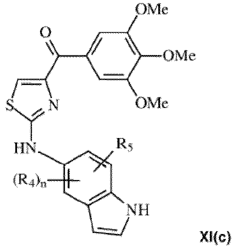 wobei
**R**₄ und **R**₅ unabhängig voneinander für Wasserstoff, O-Alkyl, O-Haloalkyl, F, Cl, Br, I, Haloalkyl, CF₃, CN, -CH₂CN, NH₂, Hydroxyl, -(CH₂)ᵢNHCH₃, -(CH₂)ᵢNH₂, -(CH₂)ᵢN(CH₃)₂, -OC(O)CF₃, C₁-C₅ lineares oder verzweigtes Alkyl, Haloalkyl, Alkylamino, Aminoalkyl, -OCH₂Ph, -NHCO-Alkyl, COOH, -C(O)Ph, C(O)O-Alkyl, C(O)H, -C(O)NH₂ oder NO₂ stehen;
**i** eine ganze Zahl zwischen 0 und 5 ist; und
**n** eine ganze Zahl zwischen 1 und 4 ist.

4. Verbindung nach Anspruch 3, wobei es sich um die Verbindung **55** handelt, die durch die folgende Strukturformel repräsentiert wird:

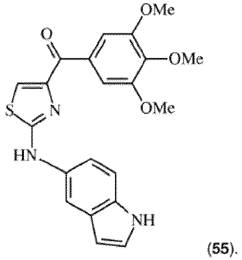

5. Verbindung nach Anspruch 2, wobei die Verbindung (2-(Phenylamino)thiazol-4-yl)(3,4,5-trimethoxyphenyl)methanon (**5a**), (2-(ρ-Tolylamino)thiazol-4-yl)(3,4,5-trimethoxyphenyl)methanon (**5b**), (2-(ρ-Fluorphenylamino)thiazol-4-yl)(3,4,5-trimethoxyphenyl)methanon (**5c**) oder (2-(4-Chlorphenylamino)thiazol-4-yl)(3,4,5-trimethoxyphenyl)methanon (**5d**) ist.

6. Verbindung nach einem der Ansprüche 1-5 oder ein pharmazeutisch akzeptables Salz, pharmazeutischen Produkt, Tautomer, Hydrat, *N-Oxid* oder Kombinationen davon.

7. Pharmazeutische Komposition umfassend eine Verbindung nach Anspruch 6 und einen pharmazeutisch akzeptablen Träger.

8. Verbindung nach einem der Ansprüche 1-6 zur Verwendung beim Behandeln, Unterdrücken, Mindern der Schwere, Reduzieren des Risikos oder Hemmens einer Krebserkrankung bei einem Subjekt, wobei die Krebserkrankung von der Gruppe bestehend aus Prostatakrebs, Brustkrebs, Eierstockkrebs, Hautkrebs, malignes Melanom, metastasiertes malignes Melanom, Lungenkrebs, Darmkrebs, Leukämie, Nierenkrebs, Krebserkrankung des zentralen Nervensystems und Kombinationen davon ausgewählt ist.

9. Verbindung nach einem der Ansprüche 1-6 zur Verwendung beim Behandeln eines arzneimittelresistenten Tumors bei einem Subjekt, wobei der Tumor von der Gruppe bestehend aus malignem Melanom-Tumor, metastasiertem malignem Melanom-Tumor, Prostata-Krebstumor und Eierstock-Krebstumor oder Kombinationen davon ausgewählt ist.

10. Verbindung zur Verwendung nach Anspruch 8 der Anspruch 9, wobei die Verbindung in Kombination mit einer weiteren Krebstherapie verabreicht wird.

11. Verbindung nach einem der Ansprüche 1-6 zur Verwendung beim Zerstören einer Krebszelle *in vivo.*

12. Verfahren zum Zerstören einer Krebszelle *ex vivo* Folgendes umfassend: Bereitstellen einer Verbindung nach einem der Ansprüche 1-6 und Kontaktieren der Krebszelle mit der Verbindung unter Bedingungen, die ein effektives Abtöten der Krebszelle verursachen.

## Revendications

1. Composé représenté par la structure de formule XI : 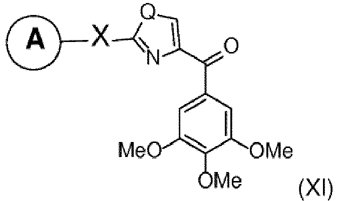 dans lequel
**X** est NH;
**Q** est S; et
**A** est un cycle phényle ou indolyle substitué ou non substitué ;
dans lequel ledit cycle A est éventuellement substitué par 1 à 5 substituants qui sont indépendamment O-alkyle, O-halogénoalkyle, F, Cl, Br, I, halogénoalkyle, CF3., CN, -CH2CN, NH2, hydroxyle, -(CH2)iNHCH3, -(CH2)iNH2, -(CH2)iN(CH3), -OC(O)CF3, alkyle linéaire ou ramifié en C1-C5, halogénoalkyle, alkylamino, aminoalkyle, -OCH2Ph, -NHCO-alkyle, COOH, - C(O)Ph, C(O)O-alkyle, C(O)H, -C(O)NH2 ou NO ; et
i est un entier compris entre 0 et 5.

2. Composé selon la revendication 1, dans lequel ledit composé est représenté par la structure de formule **VIII** : 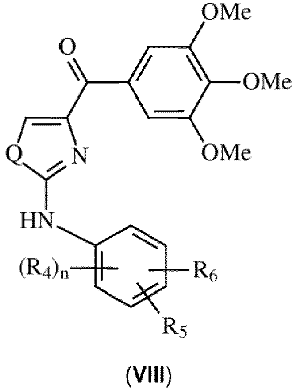 dans lequel
R4, R5 et R6 sont indépendamment hydrogène, O-alkyle, O-halogénoalkyle, F, Cl, Br, I, halogénoalkyle, CF3, CN, CH2CN, NH2, hydroxyle, -(CH2)iNHCH3, -(CH2)iNH2, - (CH2)iN(CH3), -OC(O)CF3, alkyle, halogénoalkyle, alkylamino, aminoalkyle linéaire ou ramifié en C1-C5, -OCH2Ph, -NHCO-alkyle, COOH, -C(O)Ph, C(O)O-alkyle, C(O)H, -C(O)NH ou NO2 ;
Q est S;
**i** est un entier compris entre 0 et 5 ; et
**n** est un entier compris entre 1-3 et

3. Composé selon la revendication 1, dans lequel ledit composé est représenté par la structure de formule **XI(c)** : 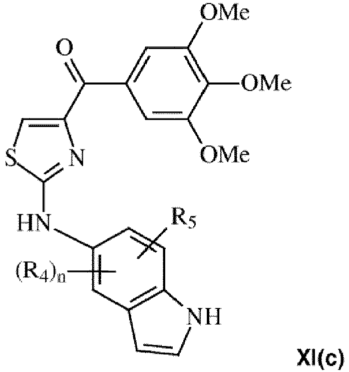 dans lequel
R4 et R5 sont indépendamment hydrogène, O-alkyle, O-halogénoalkyle, F, Cl, Br, I, groupe halogénalkyle, CF3, CN, CH2CN, NH2, hydroxyle, -(CH2)iNHCH3, -(CH2)iNH2, -(CH2)iN(CH3) OC(O)CF3, alkyle, halogénalkyle, alkylamino, aminoalkyle, linéaire ou ramifié en C1-C5, - OCHPh, -NHCO-alkyle, COOH, -C(O)Ph, C(O)O-alkyle, C(O)H, -C(O)NH2 ou NO2 ;
**i** est un entier de 0 à 5 ; et
**n** est un entier compris entre 1 et 4.

4. Composé selon la revendication 3, dans lequel ledit composé est le composé 55, représenté par la structure :

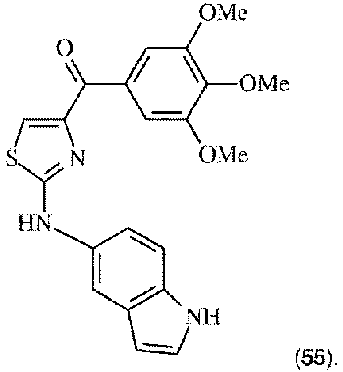

5. Composé selon la revendication 2, dans lequel ledit composé est (2-(phénylamino) thiazol-4-yl) (3,4,5-triméthoxyphényl) méthanone (**5a**), (2-(p-tolylamino) thiazol-4-yl). (3,4,5-triméthoxyphényl) méthanone (**5b**), (2-(p-fluorophénylamino) thiazol-4-yl) (3,4,5-triméthoxyphényl) méthanone (**5c**) ou (2-(4-chlorophénylamino)) thiazol-4-yl) (3,4,5-triméthoxyphényl) méthanone (**5d**).

6. Composé selon l'une quelconque des revendications 1 à 5, ou son sel, produit pharmaceutique, tautomère, hydrate, N-oxyde ou leurs combinaisons acceptables d'un point de vue pharmaceutique.

7. Composition pharmaceutique comprenant un composé selon la revendication 6 et un support acceptable d'un point de vue pharmaceutique.

8. Composé selon l'une quelconque des revendications 1 à 6, destiné à être utilisé dans le traitement, la suppression, la réduction de la gravité, la réduction du risque ou l'inhibition d'un cancer chez un sujet, ledit cancer étant choisi dans le groupe comprenant le cancer de la prostate, le cancer du sein et le cancer de l'ovaire, le cancer de la peau, le mélanome, le mélanome métastatique, le cancer du poumon, le cancer du colon, la leucémie, le cancer du rein, le cancer du SNC et leurs combinaisons.

9. Composé selon l'une quelconque des revendications 1 à 6, destiné à être utilisé dans le traitement d'une tumeur ou de plusieurs tumeurs résistantes aux médicaments chez un sujet, ladite tumeur étant choisie dans le groupe comprenant une tumeur cancéreuse de mélanome, une tumeur cancéreuse de mélanome métastatique, une tumeur cancéreuse de la prostate et une tumeur cancéreuse de l'ovaire, et leurs combinaisons.

10. Composé à utiliser selon la revendication 8 ou la revendication 9, dans lequel ledit composé est administré en combinaison avec un autre traitement du cancer.

11. Composé selon l'une quelconque des revendications 1 à 6, destiné à être utilisé pour détruire une cellule cancéreuse *in vivo.*

12. Procédé *ex vivo* de destruction d'une cellule cancéreuse comprenant la fourniture d'un composé selon l'une quelconque des revendications 1 à 6 et la mise en contact de la cellule cancéreuse avec le composé dans des conditions efficaces pour tuer la cellule cancéreuse.
